(19) **Europäisches Patentamt / European Patent Office / Office européen des brevets**

(11) **EP 2 375 255 A1**

(12) **EUROPEAN PATENT APPLICATION**

(43) Date of publication:
**12.10.2011 Bulletin 2011/41**

(51) Int Cl.:
***G01N 33/574*** (2006.01)   ***G01N 33/68*** (2006.01)

(21) Application number: **11172448.0**

(22) Date of filing: **05.06.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **06.06.2006 GB 0611116**
**07.06.2006 US 811681 P**

(62) Document number(s) of the earlier application(s) in accordance with Art. 76 EPC:
**07729912.1 / 2 074 426**

(71) Applicant: **Oxford Biotherapeutics Ltd.**
**Abingdon**
**Oxfordshire OX14 4RY (GB)**

(72) Inventor: **Rohlff, Christian**
**Abingdon, Oxfordshire OX14 4RY (GB)**

(74) Representative: **Teuten, Andrew John**
**Sagittarius IP**
**Taylor House**
**39 High Street**
**Marlow, Bucks SL7 1AF (GB)**

Remarks:
This application was filed on 01-07-2011 as a divisional application to the application mentioned under INID code 62.

(54) **Proteins**

(57) The present invention provides methods and compositions for screening, diagnosis and prognosis of colorectal cancer, for monitoring the effectiveness of colorectal cancer treatment, and for drug development.

**EP 2 375 255 A1**

**EP 2 375 255 A1**

**Description**

INTRODUCTION

The present invention relates to the identification of marker proteins not previously reported for human colorectal cancer which have utility as diagnostic and prognostic markers for colorectal cancer and colorectal cancer metastases. These proteins may also form biological targets against which therapeutic antibodies (or other affinity reagents such as Affibodies, Nanobodies or Unibodies) or other pharmaceutical agents can be made.

BACKGROUND OF THE INVENTION

**Colorectal Cancer**

Colorectal cancer (CRC) is one of the leading causes of cancer-related morbidity and mortality, responsible for an estimated half a million deaths per year, mostly in Western, well developed countries. In these territories, CRC is the third most common malignancy (estimated number of new cases per annum in USA and EU is approximately 350,000 per year). Estimated healthcare costs related to treatment for colorectal cancer in the United States are more than $8 billion.

**Colorectal Cancer diagnosis:**

Today, the fecal occult blood test and colonoscopy, a highly invasive procedure, are the most frequently used screening and diagnostic methods for colorectal cancer. Other diagnostic tools include Flexible Sigmoidoscopy (allowing the observation of only about half of the colon) and Double Contrast Barium Enema (DCBE, to obtain X-ray images).

**Colorectal Cancer staging:**

CRC has four distinct stages: patients with stage I disease have a five-year survival rate of >90%, while those with metastatic stage IV disease have a <5% survival rate according to the US National Institutes of Health (NIH).

**Colorectal Cancer treatment:**

Once CRC has been diagnosed, the correct treatment needs to be selected. Surgery is usually the main treatment for colorectal cancer, although radiation and chemotherapy will often be given before surgery. Possible side effects of surgery include bleeding from the surgery, blood clots in the legs, and damage to nearby organs during the operation.
Currently, 60 percent of colorectal cancer patients receive chemotherapy to treat their disease; however, this form of treatment only benefits a few percent of the population, while carrying with it high risks of toxicity, thus demonstrating a need to better define the patient selection criteria.
Colorectal cancer has a 30 to 40 percent recurrence rate within an average of 18 months after primary diagnosis. As with all cancers, the earlier it is detected the more likely it can be cured, especially as pathologists have recognised that the majority of CRC tumours develop in a series of well-defined stages from benign adenomas.

**Colon Cancer Survival by Stage**

| Stage | Survival Rate |
| --- | --- |
| I | 93% |
| IIA | 85% |
| IIB | 72% |
| IIIA | 83% |
| IIIB | 64% |
| IIIC | 44% |
| IV | 8% |

**Therapeutic Challenges**

The major challenges in colorectal cancer treatment are to improve early detection rates, to find new non-invasive markers that can be used to follow disease progression and identify relapse, and to find improved and less toxic therapies, especially for more advanced disease where 5 year survival is still very poor. There is a great need to identify targets which are more specific to the cancer cells e.g. ones which are expressed on the surface of the tumour cells so that they can be attacked by promising new approaches like immunotherapeutics and targeted toxins.

SUMMARY OF THE INVENTION

The present invention provides methods and compositions for screening, diagnosis, prognosis and therapy of colorectal cancer, for colorectal cancer patients' stratification, for monitoring the effectiveness of colorectal cancer treatment, and for drug development for treatment of colorectal cancer.

We have used mass spectrometry to identify peptides generated by gel electrophoresis and tryptic digest of membrane proteins extracted from colorectal tissue samples. Peptide sequences were compared to existing protein and cDNA databases and the corresponding gene sequences identified. For these membrane proteins, soluble forms exist, e.g. in serum, some of which are reported herein, and others which are known in the art. Many of these have not been previously reported to originate from colorectal cell membranes and represent a new set of proteins of potential diagnostic and/or therapeutic value.

Thus, a first aspect of the invention provides methods for diagnosis of colorectal cancer that comprises analysing a sample of serum e.g. by two-dimensional electrophoresis to detect at least one Colorectal Cancer Marker Protein (CRCMP), e.g., one or more of the CRCMPs disclosed herein or any combination thereof. These methods are also suitable for screening, prognosis, monitoring the results of therapy, drug development and discovery of new targets for drug treatment.

In particular there is provided a method of diagnosing colorectal cancer in a subject, differentiating causes of colorectal cancer in a subject, guiding therapy in a subject suffering from colorectal cancer, assessing the risk of relapse in a subject suffering from colorectal cancer, or assigning a prognostic risk of one or more future clinical outcomes to a subject suffering from colorectal cancer, the method comprising:

(a) performing assays configured to detect a soluble polypeptide derived from a protein selected from the list consisting of proteins defined by SEQ ID Nos 1-18 as a marker in one or more samples obtained from said subject; and
(b) correlating the results of said assay(s) to the presence or absence of colorectal cancer in the subject, to a therapeutic regimen to be used in the subject, to a risk of relapse in the subject, or to the prognostic risk of one or more clinical outcomes for the subject suffering from colorectal cancer.

Suitably such a method involves determining that when the level of said detected marker is higher in the subject than a control level, said determination indicates the presence of colorectal cancer in the subject, indicates a greater risk of relapse in the subject, or indicates a worse prognosis for the subject. Suitably if the level of said detected marker reduced in response to therapy, this indicates that the subject is responding to therapy. In particular such a method is a method for diagnosing colorectal cancer in a subject.

Diagnosing cancer embraces diagnosing primary cancer and relapse.

Colorectal cancer includes metastatic colorectal cancer.

Suitably the method may comprise performing one or more additional assays configured to detect one or more additional markers in addition to the soluble polypeptide derived from a protein selected from the list consisting of proteins defined by SEQ ID Nos 1-18 and wherein said correlating step comprises correlating the results of said assay(s) and the results of said additional assay(s) to the presence or absence of colorectal cancer in the subject, to a risk of relapse in the subject, or to the prognostic risk of one or more clinical outcomes for the subject suffering from colorectal cancer.

Suitably in methods according to the invention the subject is a human.

There is also provided a method for identifying the presence or absence of colorectal cancer cells in a biological sample obtained from a human subject, which comprises the step of identifying the presence or absence of one or more soluble polypeptides derived from a protein selected from the list consisting of proteins defined by SEQ ID Nos 1-18.

The presence of a soluble polypeptide may typically be determined qualitatively or quantitatively (eg quantitatively) for example by a method involving imaging technology (eg use of a labeled affinity reagent such as an antibody or an Affibody) as described herein.

There is also provided a method of detecting, diagnosing colorectal cancer in a subject, differentiating causes of colorectal cancer in a subject, guiding therapy in a subject suffering from colorectal cancer, assessing the risk of relapse in a subject suffering from colorectal cancer, or assigning a prognostic risk of one or more future clinical outcomes to a subject suffering from colorectal cancer, the method comprising:

(a) bringing into contact with a sample to be tested from said subject one or more antibodies (or other affinity reagents such as Affibodies, Nanobodies or Unibodies) capable of specific binding to a soluble polypeptides derived from a protein selected from the list consisting of proteins defined by SEQ ID Nos 1-18; and

(b) thereby detecting the presence of one or more soluble polypeptides derived from a protein selected from the list consisting of proteins defined by SEQ ID Nos 1-18 in the sample.

In such a method the presence of one or more said soluble polypeptides may indicate the presence of colorectal cancer in the patient.

There is also provided a method for identifying the presence of colorectal cancer in a subject which comprises the step of carrying out a whole body scan of said subject to determine the localisation of colorectal cancer cells, particularly metastatic colorectal cancer cells, in order to determine presence or amount of one or more soluble polypeptides derived from a protein selected from the list consisting of proteins defined by SEQ ID Nos 1-18, wherein the presence or amount of one or more of said soluble polypeptides indicates the presence of colorectal cancer in the subject.

There is also provided a method for identifying the presence of colorectal cancer in a subject which comprises determining the localisation of colorectal cancer cells by reference to a whole body scan of said subject, which scan indicates the presence or amount of one or more soluble polypeptides derived from a protein selected from the list consisting of proteins defined by SEQ ID Nos 1-18, wherein the presence or amount of one or more of said soluble polypeptides indicates the presence of colorectal cancer in the subject.

There is also provided a method of detecting, diagnosing colorectal cancer in a subject, differentiating causes of colorectal cancer in a subject, guiding therapy in a subject suffering from colorectal cancer, assessing the risk of relapse in a subject suffering from colorectal cancer, or assigning a prognostic risk of one or more future clinical outcomes to a subject suffering from colorectal cancer, the method comprising:

(a) bringing into contact with a sample to be tested one or more soluble polypeptides derived from a protein selected from the list consisting of proteins defined by SEQ ID Nos 1-18, or one or more antigenic or immunogenic fragments thereof; and

(b) detecting the presence of antibodies (or other affinity reagents such as Affibodies,

Nanobodies or Unibodies) in the subject capable of specific binding to one or more of said polypeptides, or antigenic or immunogenic fragments thereof.

A second aspect of the invention provides methods of treating colorectal cancer, comprising administering to a patient a therapeutically effective amount of a compound that modulates (e.g., upregulates or downregulates) or complements the expression or the biological activity (or both) of a CRCMP in patients having colorectal cancer, in order to (a) prevent the onset or development of colorectal cancer; (b) prevent the progression of colorectal cancer; or (c) ameliorate the symptoms of colorectal cancer.

A third aspect of the invention provides methods of screening for compounds that modulate (e.g., upregulate or downregulate) the expression or biological activity of a CRCMP.

A fourth aspect of the invention provides monoclonal and polyclonal antibodies or other affinity reagents such as Affibodies, Nanobodies or Unibodies capable of immunospecific binding to a CRCMP, e.g., a CRCMP disclosed herein.

Thus, in a fifth aspect, the present invention provides a method for screening for and/or diagnosis of colorectal cancer in a human subject, which method comprises the step of identifying the presence or absence of one or more of the CRCMPs as defined in Tables 1 and 2 herein, in a biological sample obtained from said human subject.

In a sixth aspect, the present invention provides a method for monitoring and/or assessing colorectal cancer treatment in a human subject, which comprises the step of identifying the presence or absence of one or more of the CRCMPs as defined in Tables 1 or 2 herein, in a biological sample obtained from said human subject.

In a seventh aspect, the present invention provides a method for identifying the presence or absence of metastatic colorectal cancer cells in a biological sample obtained from a human subj ect, which comprises the step of identifying the presence or absence of one or more of the CRCMPs as defined in Tables 1 or 2 herein.

In an eighth aspect, the present invention provides a method for monitoring and/or assessing colorectal cancer treatment in a human subject, which comprises the step of determining whether one or more of the CRCMPs as defined in Tables 1 or 2 herein is increased/decreased in a biological sample obtained from a patient.

The biological sample used can be from any source such as a serum sample or a tissue sample, e.g. colorectal tissue. For instance, when looking for evidence of metastatic colorectal cancer, one would look at major sites of colorectal cancer metastasis, e.g. the liver, the peritoneal cavity, the pelvis, the retroperitoneum and the lungs.

Preferably, the methods of the present invention are not based on looking for the presence or absence of all of the CRCMPs defined in Tables 1 and 2, but rather on "clusters" or groups thereof.

Other aspects of the present invention are set out below and in the claims herein.

BRIEF DESCRIPTION OF THE FIGURES

Figures 1-5, 7 and 9 show Box plot data for CRCMP#19, CRCMP#6, CRCMP#22, CRCMP#10 and CRCMP#9 as described in Examples 3 and 4.
Figures 6 and 8 show ROC curve data for CRCMP#19 and CRCMP#9 respectively as described in Example 4.
Figures 10(a) -10(r) show the sequences of the CRCMPs and mass match and tandem peptide fragments etc which are discussed in the Examples.

DETAILED DESCRIPTION OF THE INVENTION

The invention described in detail below provides methods and compositions for clinical screening, diagnosis and prognosis of colorectal cancer in a mammalian subject, for identifying patients most likely to respond to a particular therapeutic treatment, for monitoring the results of colorectal cancer therapy, for drug screening and drug development. The invention also encompasses the administration of therapeutic compositions to a mammalian subject to treat or prevent colorectal cancer. The mammalian subject may be a non-human mammal, but is preferably human, more preferably a human adult, i.e. a human subject at least 21 (more preferably at least 35, at least 50, at least 60, at least 70, or at least 80) years old. For clarity of disclosure, and not by way of limitation, the invention will be described with respect to the analysis of colon tissue and serum samples. However, as one skilled in the art will appreciate, the assays and techniques described below can be applied to other types of patient samples, including another body fluid (*e.g.* urine or saliva), a tissue sample from a patient at risk of having colorectal cancer (*e.g.* a biopsy such as a colon tissue biopsy) or homogenate thereof. The methods and compositions of the present invention are specially suited for screening, diagnosis and prognosis of a living subject, but may also be used for postmortem diagnosis in a subject, for example, to identify family members at risk of developing the same disease.
As used herein, colon tissue refers to the colon itself, as well as the tissue adjacent to and/or within the strata underlying the colon.

Colorectal Cancer Marker Proteins (CRCMPs)

In one aspect of the invention, two-dimensional electrophoresis is used to analyze serum samples from a subject, preferably a living subject, in order to measure the expression of one or more Colorectal Cancer Marker Proteins (CRCMPs) for screening or diagnosis of colorectal cancer, to determine the prognosis of a colorectal cancer patient, or to monitor the effectiveness of colorectal cancer therapy.
As used herein, the term "Colorectal Cancer Marker Protein" (CRCMP) refers to a soluble polypeptide derived from a protein believed to be associated with colorectal cancer. 18 such proteins are recited in Tables 1 and 2 by reference to their accession numbers. Soluble polypeptides derived therefrom have been detected by 1 or 2D electrophoresis of colorectal cancer tissue sample as shown in Table 1 and 2. Table 2 recited those proteins that have been detected as features on a gel by 2D gel analysis and Table 1 recites those proteins that have been detected as features on a gel by 1D gel analysis.
In particular, some of the features in Tables 1 and 2 have entries in the SwissProt database (available online at http://www.expasy.org), which is an annotated database for proteins. For these entries, the SwissProt database contains information on the structure of the proteins, when known, and this includes a definition of the sequence making up soluble parts of the proteins. In addition, methods suitable for predicting soluble forms of membrane proteins include, but are not limited to, primary structure analysis to identify membrane spanning helices and extracellular domains, which is provided by a number of bioinformatics tools, such as the Dense Alignment Surface method, the HMMTOP method, the TMpred method, the TopPred method, the TMHMM method, the TMAP method, the SOSUI method, the PredictProtein method, all of which are available online through the Topology Prediction section of the expasy webserver (http://www.expasy.org).
The CRCMPs disclosed herein have been identified as soluble forms of membrane proteins, cell surface proteins, secreted proteins or GPI anchored proteins extracted from colorectal tissue samples through the methods and apparatus of the technologies described herein (generally 1D and 2D gel electrophoresis and tryptic digest of membrane proteins extracted from colorectal tissue samples). Peptide sequences were compared to the SWISS-PROT and trEMBL databases (held by the Swiss Institute of Bioinformatics (SIB) and the European Bioinformatics Institue (EBI) which are available at http://www.expasy.com/) and the GenBank database (held by the National Institute of Health (NIH) which is available at http://www.ncbi.nlm.nih.gov/GenBank/) and corresponding genes identified. Each protein in Table 1 and Table 2 is identified by a Swiss Prot, TrEMBL or a Genbank Accession Number and each sequence is incorporated herein by reference. The apparent molecular weight and the amino acid sequences of tryptic digest peptides of these CRCMPs (Table 2) and CRCMP features (Table 1) identified by tandem mass spectrometry and database searching as described in the Examples, *infra,* are also listed in these Tables.

Table 3 provides further characterisation of the CRCMPs based on sample source, predictions and prior knowledge.

The proteins of the invention are useful as are fragments e.g. antigenic or immunogenic fragments thereof and derivatives thereof. Antigenic or immunogenic fragments will typically be of length 12 amino acids or more e.g. 20 amino acids or more e.g. 50 or 100 amino acids or more. Fragments may be 95% or more of the length of the full protein e.g. 90% or more e.g. 75% or 50% or 25% or 10% or more of the length of the full protein.

Antigenic or immunogenic fragments will be capable of eliciting a relevant immune response in a patient. DNA encoding the proteins of the invention are also useful as are fragments thereof e.g. DNA encoding fragments of the proteins of the invention such as immunogenic fragments thereof. Fragments of nucleic acid (e.g. DNA) encoding the proteins of the invention may be 95% or more of the length of the full coding region e.g. 90% or more e.g. 75% or 50% or 25% or 10% or more of the length of the full coding region. Fragments of nucleic acid (e.g. DNA) may be 36 nucleotides or more e.g. 60 nucleotides or more e.g. 150 or 300 nucleotides or more in length.

Derivatives of the proteins of the invention include variants on the sequences in which one or more (e.g. 1-20 such as 15 amino acids, or up to 20% such as up to 10% or 5% or 1% by number of amino acids based on the total length of the protein) deletions, insertions or substitutions have been made. Substitutions may typically be conservative substitutions. Derivatives will typically have essentially the same biological function as the protein from which they are derived. Derivatives will typically be comparably antigenic or immunogenic to the protein from which they are derived.

In one embodiment the soluble polypeptide markers of use according to the invention comprises one or more (e.g. one) amino acid sequences recited in column 4 of Table 1 (i.e. the column of tryptic digest peptides). In another embodiment the soluble polypeptides of use according to the invention comprises one or more (e.g. one) amino acid sequences recited in column 4 of Table 2 (i.e. the column of tryptic digest peptides).

Soluble peptides may typically be at least 5 amino acids in length e.g. at least 6 amino acids in length e.g. at least 10 or at least 12 or at least 15 e.g. at least 20 amino acids in length.

Suitably the marker polypeptide is derived from a protein in an isoform characterized by a pI and MW as listed in columns 2 and 3 of Table 2. An isoform is still considered to be characterized by a pI and MW as listed in columns 2 and 3 of Table 2 if the pI and MW values as determined experimentally fall within a spread of 10 %, suitably 5% either side of the stated value.

Suitably the marker polypeptide will be immunologically detectable.

Certain marker polypeptides disclosed herein are novel and are claimed as an aspect of the invention.

In one embodiment, suitably assays intended to detect the marker polypeptides are configured to detect two or more said markers. Suitably the two or more said markers are derived from at least two different proteins.

In another embodiment, suitably assays intended to detect the marker polypeptides are configured to detect three or more said markers. Suitably the three or more said markers are derived from at least three different proteins.

In another embodiment, suitably assays intended to detect the marker polypeptides are configured to detect four or more said markers. Suitably the four or more said markers are derived from at least four different proteins.

In another embodiment, suitably assays intended to detect the marker polypeptides are configured to detect five or more said markers. Suitably the five or more said markers are derived from at least five different proteins.

**Table 1: Features detected by 1D gel for the CRCMPs of the invention (see Example 1)**

| CRCMP # | MW (kDa) Range | Predicted MW (Da) | Amino Acid Sequences of Tryptic Digest Peptides [SEQ ID No] | Acc. number |
|---|---|---|---|---|
| 1 | 91 - 126 | 92219 | AENPEPLVFGVK [37], DAYVFYAVAK [62], DEENTANSFLNYR [64], DEYGKPLSYPLEIHVK [65], DINDNRPTFLQSK [68], DNVESAQASEVKPLR [70], EGLLYYNR [81], GDTRGWLK [104], HTEFEER [118], IDHVTGEIFSVAPLDR [119], TGAISLTR [202], VSEDVALGTK [220], WNDPGAQYSLVDK [227] | Q12864 |
| 2 | 47-54 | 35632 | EAYEEPPEQLR [76], EGLIQWDK [80], EGSPTPQYSWK [82], EREEEDDYR [86], EREEEDDYRQEEQR [87], LLLTHTER [146], NYIHGELYK [165], SVTLPCTYHTSTSSR [195], VTVDAISVETPQDVLR [222], YNILNQEQPLAQPASGQPVSLK [240] | Q99795 |

(continued)

| CRCMP # | MW (kDa) Range | Predicted MW (Da) | Amino Acid Sequences of Tryptic Digest Peptides [SEQ ID No] | Acc. number |
|---|---|---|---|---|
| 5 | 69 - 153 | 87327 | DRNHRPK [72], FGQIVNTLDK [92], IPIRWTAPEAIQYR [127], MIRNPNSLK [158], QLGLTEPR [170], TVAGYGRYSGK [209], VSDFGLSR [219], WTAPEAIQYR [229], YLADMNYVHR [237] | P29323 |
| 6 | 75 - 78 | 91938 | FTTPGFPDSPYPAHAR [101], GDADSVLSLTFR [103], HPGFEATFFQLPR [117], IFQAGVVSWGDGCAQR [122], SFVVTSVVAFPTDSK [189], VVMLPPR [223] | Q9Y5Y6 |
| 7 | 108 | 90138 | TEDVEPQSVPLLAR [200], YPPLPVDK [241] | P18433 |
| 8 | 88 - 104 | 112927 | ALLSDER [41], FLRPGHDPVR [95], GGASELQEDESFTLR [107], QPGLVMERALLSDER [171], REDVSGIASCVFTK [177], SAEDSFTGFVR [183], TLYFADTYLK [206], VFEMVEALQEHPR [213], VPPAERR [217], VSVAHFGSR [221], YGQGFYLISPSEFER [234] | Q6P1 M3 |
| 9 | 19 | 19171 | IMFVDPSLTVR [126], NLSPDGQYVPR [161] | Q8TD06 |
| 10 | 130 | 116727 | CSVPEGPFPGHLVDVR [60] | Q9UN66 |
| 12 | 42-43 | 34932 | APEFSMQGLK [44], DTEITCSER [73], EKPYDSK [83], EMGEMHR [85], GESLFHSK [106], KKRMAK [133], LAAKCLVMK [141], TQNDVDIADVAYYFEK [207], TQNDVDIADVAYYFEKDVK [208], YEKAEIK [233] | P16422 |
| 14 | 65 - 93 | 86705 | EGHQSEGLR [79], LQEDGLSVWFQR [151], QETDYVLNNGFNPR [167], RKNLDLAAPTAEEAQR [178], RPELEEIFHQYSGEDR [179] | ENST00 000322765 |
| 17 | 62 - 72 | 55711 | LNLWISR [147], QVVEAAQAPIQER [174], RSESVKSR [182] | 000515 |
| 18 | 79 - 96 | 82683 | AVINSAGYK [53], CPTQFPLILWHPYAR [59], EELCKSIQR [78], FEEVLSK [90], FQELIFEDFAR [98], HEIEGTGLPQAQLLWR [114], HNLYR [116], KHNLYR [132], KYDYDSSSVR [137], KYDYDSSSVRK [138], KYDYDSSSVRKR [139], LGEYMEK [145], MESLRLDGLQQR [156], MGWMGEK [157], TDMDQIITSK [199], VEGPAFTDAIR [211], VQQVQPAMQAVIR [218], YDYDSSSVR [230], YDYDSSSVRK [231], YDYDSSSVRKR [232] | Q96TA1 |
| 19 | 19 | 19979 | GWGDQLIWTQTYEEALYK [112], HLSPDGQYVPR [115], IMFVDPSLTVR [126], LPQTLSR [149], LYAYEPADTALLLDNMK [153] | 095994 |

(continued)

| CRCMP # | MW (kDa) Range | Predicted MW (Da) | Amino Acid Sequences of Tryptic Digest Peptides [SEQ ID No] | Acc. number |
|---|---|---|---|---|
| 20 | 118 -128 | 154374 | ATFAFSPEEQQAQR [51], AYPQYYR [55], FDTHEYRNESRR [89], FRPHQDANPEKPR [99], GHSPAFLQPQNGNSR [109], GYTIHWNGPAPR [113], IEEYEPVHSLEELQR [120], MDNYLLR [155], MPAMLTGLCQGCGTR [159], NSWQLTPR [164], SDEGESMPTFGKK [184] | Q9UHN6 |
| 22 | 71-126 | 83283 | AAGSRDVSLAK [34], ADAAPDEK [35], AFVNCDENSR [40], ANLTNFPENGTFVVNIAQLSQDDSGR [42], AQYEGR [47], ASVDSGSSEEQGGSSR [50], DGSFSVVITGLR [66], DQADGSR [71], DVSLAKADAAPDEK [74], EEFVATTESTTETK [77], FSSYEK [100], GGCITLISSEGYVSSK [108], GSVTFHCALGPEVANVAK [111], IIEGEPNLK [123], ILLNPQDK [124], KYWCR [140], LSLLEEPGNGTFTVILNQLTSR [152], QGHFYGETAAVYVAVEER [168], QGHFYGETAAVYVAVEERK [169], QSSGENCDVVVNTLGK [172], QSSGENCDVVVNTLGKR [173], RAPAFEGR [175], TDISMSDFENSR [198], VLDSGFR [214], VLDSGFREIENK [215], VPCHFPCK [216], VYTVDLGR [224], YKCGLGINSR [236], YLCGAHSDGQLQEGSPIQAWQLFVNEESTIPR [238] | P01833 |
| 23 | 37 | 35964 | DYEILFK [75], FFNVLTTNTDGK [91], IEFISTMEGYK [121], NLDGISHAPNAVK [160], SINGILFPGGSVDLR [190], YLESAGAR [239], YPVYGVQWHPEKAPYEWK [243] | Q92820 |
| 25 | 33-42 | 35463 | AEVDLQGIK [38], ASSPQGFDVDR [48], ASSPQGFDVDRDAKK [49], ATFQAYQILIGK [52], AYLTLVR [54], CAQDCEDYFAER [56], DIEEAIEEETSGDLQK [67], DLYDAGEGR [69], FQEKYQK [96], FQEKYQKSLSDMVR [97], GAGTDEETLIR [102], GDTSGNLKK [105], GMGTNEAAIIEILSGR [110], LFDRSLESDVK [144], ILVSLLQANR [125], SDTSGDFR [186], SELSGNFEK [187], SLESDVK [193], SLSDMVR [194], TALALLDRPSEYAAR [197], WGTDELAFNEVLAK [225], WGTDELAFNEVLAKR [226] | P27216 |
| 26 | | 29379 | SDPVTLNVR [185], TLVLLSATK [205] | Q14002 |

**Table 2: CRCMPs detected by 2D gel (see Example 2)**

| CRCMP # | MW (Da) | pI | Amino Acid Sequences of Tryptic Digest Peptides [SEQ ID No] | Acc. number |
|---|---|---|---|---|
| 7 | 39716 | 5,07 | TEDVEPQSVPLLAR [200] | P18433 |
| 7 | 40419 | 7,88 | KFCIQQVGDMTNR [130], QAGSHSNSFR [166] | P18433 |
| 7 | 73852 | 6,31 | QAGSHSNSFR [166] | P18433 |

(continued)

| CRCMP # | MW (Da) | pI | Amino Acid Sequences of Tryptic Digest Peptides [SEQ ID No] | Acc. number |
|---|---|---|---|---|
| 9 | 11481 | 7,96 | LYTYEPR [154], NLSPDGQYVPR [161], RPPQTLSR [180] | Q8TD06 |
| 9 | 12984 | 8,51 | IMFVDPSLTVR [126], LYTYEPR [154], NLSPDGQYVPR [161], RPPQTLSR [180] | Q8TD06 |
| 9 | 13055 | 8,46 | IMFVDPSLTVR [126], LYTYEPR [154], NLSPDGQYVPR [161], RPPQTLSR [180] | Q8TD06 |
| 9 | 13391 | 8,48 | FIMLNLMHETTDK [94], IMFVDPSLTVR [126], LYTYEPR [154], NLSPDGQYVPR [161], RPPQTLSR [180], VFAQNEEIQEMAQNK [212] | Q8TD06 |
| 9 | 14158 | 9,96 | IMFVDPSLTVR [126] | Q8TD06 |
| 10 | 56273 | 5,09 | AEYNVTITVTDLGTPR [39] | Q9UN66 |
| 17 | NULL | NULL | DEDEDIQSILR [63], ELEIPPR [84], KELEIPPR [129], LNLWISR [147], LPDNTVK [148], LPSVEEAEVPKPLPPASK [150], NLSSTTDDEAPR [162], QVVEAAQAPIQER [174], RATASEQPLAQEPPASGGSPATTK [176], SLAPGMALGSGR [192], TLEDEEEQER [203] | 000515 |
| 18 | NULL | NULL | AQIHMR [46], EVTDMNLNVINEGGIDK [88], FQELIFEDFAR [98], IVFSGNLFQHQEDSK [128], VQQVQPAMQAVIR [218] | Q96TA1 |
| 18 | NULL | NULL | FQELIFEDFAR [98], IVFSGNLFQHQEDSK [128], VQQVQPAMQAVIR [218] | Q96TA1 |
| 19 | 12993 | 9,02 | HLSPDGQYVPR [115], IMFVDPSLTVR [126] | 095994 |
| 19 | 13055 | 8,46 | IMFVDPSLTVR [126], KVFAENK [136] | 095994 |
| 19 | 13391 | 8,48 | IMFVDPSLTVR [126] | 095994 |
| 19 | 14158 | 9,96 | HLSPDGQYVPR [115], IMFVDPSLTVR [126], KVFAENK [136], LPQTLSR [149], LYAYEPADTALLLDNMK [153] | 095994 |
| 20 | 76700 | 5,76 | FIGVEAGGTLELHGAR [93], TLNSSGLPFGSYTFEK [204] | Q9UHN6 |
| 22 | 58949 | 4,65 | DGSFSVVITGLR [66] | P01833 |
| 22 | 59920 | 4,74 | AFVNCDENSR [40], APAFEGR [43], AQYEGR [47], DGSFSVVITGLR [66], FSSYEK [100], IIEGEPNLK [123], ILLNPQDK [124], QGHFYGETAAVYVAVEER [168], RAPAFEGR [175], YWCLWEGAQNGR [244] | P01833 |
| 22 | 64282 | 5,05 | APAFEGR [43], DGSFSVVITGLR [66], IIEGEPNLK [123], RAPAFEGR [175], VYTVDLGR [224] | P01833 |
| 22 | 72124 | 5,15 | APAFEGR [43], DGSFSVVITGLR [66], IIEGEPNLK [123], QGHFYGETAAVYVAVEER [168], RAPAFEGR [175], TENAQKR [201], VLDSGFR [214], VYTVDLGR [224] | P01833 |
| 22 | 72683 | 5,03 | AFVNCDENSR [40], ANLTNFPENGTFVVNIAQLSQDDSGR [42], APAFEGR [43], AQYEGR [47], CGLGINSR [57], CPLLVDSEGWVK [58], DGSFSVVITGLR [66], FSSYEK [100], IIEGEPNLK [123], ILLNPQDK [124], QGHFYGETAAVYVAVEER [168], QSSGENCDVVVNTLGK [172], RAPAFEGR [175], VLDSGFR [214], VYTVDLGR [224], YWCLWEGAQNGR [244] | P01833 |

(continued)

| CRCMP # | MW (Da) | pl | Amino Acid Sequences of Tryptic Digest Peptides [SEQ ID No] | Acc. number |
|---|---|---|---|---|
| 22 | 73988 | 4,96 | AFVNCDENSR [40], APAFEGR [43], AQYEGR [47], CGLGINSR [57], DGSFSVVITGLR [66], FSSYEK [100], IIEGEPNLK [123], ILLNPQDK [124], QGHFYGETAAVYVAVEER [168], QSSGENCDVVVNTLGK [172], RAPAFEGR [175], TVTINCPFK [210], VLDSGFR [214], VYTVDLGR [224], YLCGAHSDGQLQEGSPIQAWQLFVNEESTIPR [238], YWCLWEGAQNGR [244] | P01833 |
| 22 | 76022 | 5,63 | AFVNCDENSR [40], APAFEGR [43], AQYEGR [47], CGLGINSR [57], DGSFSVVITGLR [66], FSSYEK [100], IIEGEPNLK [123], QGHFYGETAAVYVAVEER [168], QSSGENCDVVVNTLGK [172], RAPAFEGR [175], TENAQKR [201], VLDSGFR [214], VPCHFPCK [216], VYTVDLGR [224], YWCLWEGAQNGR [244] | P01833 |
| 22 | 76452 | 5,02 | AFVNCDENSR [40], APAFEGR [43], AQYEGR [47], CGLGINSR [57], CPLLVDSEGWVK [58], DAGFYWCLTNGDTLWR [61], DGSFSVVITGLR [66], FSSYEK [100], IIEGEPNLK [123], ILLNPQDK [124], KYWCR [140], LDIQGTGQLLFSVVINQLR [142], QGHFYGETAAVYVAVEER [168], QSSGENCDVVVNTLGK [172], RAPAFEGR [175], VLDSGFR [214], VYTVDLGR [224], YLCGAHSDGQLQEGSPIQAWQLFVNEESTIPR [238], YWCLWEGAQNGR [244] | P01833 |
| 22 | 76788 | 5,09 | AFVNCDENSR [40], APAFEGR [43], AQYEGR [47], CGLGINSR [57], DGSFSVVITGLR [66], FSSYEK [100], IIEGEPNLK [123], ILLNPQDK [124], KYWCR [140], LDIQGTGQLLFSVVINQLR [142], LSLLEEPGNGTFTVILNQLTSR [152], QGHFYGETAAVYVAVEER [168], QSSGENCDVVVNTLGK [172], RAPAFEGR [175], VLDSGFR [214], VYTVDLGR [224], YLCGAHSDGQLQEGSPIQAWQLFVNEESTIPR [238] | P01833 |
| 22 | 76811 | 5,20 | AFVNCDENSR [40], APAFEGR [43], AQYEGR [47], CGLGINSR [57], DGSFSVVITGLR [66], FSSYEK [100], IIEGEPNLK [123], ILLNPQDK [124], KYWCR [140], LDIQGTGQLLFSVVINQLR [142], QGHFYGETAAVYVAVEER [168], RAPAFEGR [175], VYTVDLGR [224], YLCGAHSDGQLQEGSPIQAWQLFVNEESTIPR [238], YWCLWEGAQNGR [244] | P01833 |
| 22 | 76905 | 4,84 | AFVNCDENSR [40], APAFEGR [43], AQYEGR [47], CGLGINSR [57], DGSFSVVITGLR [66], FSSYEK [100], IIEGEPNLK [123], ILLNPQDK [124], QGHFYGETAAVYVAVEER [168], RAPAFEGR [175], VLDSGFR [214], VYTVDLGR [224], YLCGAHSDGQLQEGSPIQAWQLFVNEESTIPR [238], YWCLWEGAQNGR [244] | P01833 |
| 22 | 77049 | 5,03 | AFVNCDENSR [40], APAFEGR [43], CGLGINSR [57], DGSFSVVITGLR [66], FSSYEK [100], IIEGEPNLK [123], QGHFYGETAAVYVAVEER [168], RAPAFEGR [175], VYTVDLGR [224] | P01833 |
| 22 | 77219 | 5,09 | AFVNCDENSR [40], APAFEGR [43], CGLGINSR [57], DGSFSVVITGLR [66], IIEGEPNLK [123], QGHFYGETAAVYVAVEER [168], RAPAFEGR [175], VYTVDLGR [224] | P01833 |

(continued)

| CRCMP # | MW (Da) | pI | Amino Acid Sequences of Tryptic Digest Peptides [SEQ ID No] | Acc. number |
|---------|---------|------|---|---|
| 22 | 77291 | 5,63 | AFVNCDENSR [40], ANLTNFPENGTFVVNIAQLSQDDSGR [42], APAFEGR [43], AQYEGR [47], CGLGINSR [57], DGSFSVVITGLR [66], FSSYEK [100], IIEGEPNLK [123], LFAEEK [143], QGHFYGETAAVYVAVEER [168], QSSGENCDVVVNTLGK [172], RAPAFEGR [175], VLDSGFR [214], VYTVDLGR [224], YWCLWEGAQNGR [244] | P01833 |
| 22 | 77900 | 4,80 | AFVNCDENSR [40], ANLTNFPENGTFVVNIAQLSQDDSGR [42], APAFEGR [43], AQYEGR [47], CGLGINSR [57], DGSFSVVITGLR [66], FSSYEK [100], IIEGEPNLK [123], ILLNPQDK [124], KYWCR [140], QGHFYGETAAVYVAVEER [168], QSSGENCDVVVNTLGK [172], RAPAFEGR [175], VLDSGFR [214], VYTVDLGR [224], YLCGAHSDGQLQEGSPIQAWQLFVNEESTIPR [238], YWCLWEGAQNGR [244] | P01833 |
| 22 | 77980 | 5,00 | ADEGWYWCGVK [36], AFVNCDENSR [40], APAFEGR [43], AQYEGR [47], CGLGINSR [57], CPLLVDSEGWVK [58], DGSFSVVITGLR [66], FSSYEK [100], GSVTFHCALGPEVANVAK [111], IIEGEPNLK [123], ILLNPQDK [124], KNADLQVLKPEPELVYEDLR [134], KYWCR [140], QGHFYGETAAVYVAVEER [168], RAPAFEGR [175], TVTINCPFK [210], VLDSGFR [214], VYTVDLGR [224], YWCLWEGAQNGR [244] | P01833 |
| 22 | 79500 | 4,91 | ADEGWYWCGVK [36], AFVNCDENSR [40], APAFEGR [43], AQYEGR [47], CGLGINSR [57], CPLLVDSEGWVK [58], DGSFSVVITGLR [66], FSSYEK [100], GGCITLISSEGYVSSK [108], GSVTFHCALGPEVANVAK [111], IIEGEPNLK [123], ILLNPQDK [124], KNADLQVLKPEPELVYEDLR [134], KYWCR [140], QGHFYGETAAVYVAVEER [168], QSSGENCDVVVNTLGK [172], RAPAFEGR [175], TENAQKR [201], VLDSGFR [214], VYTVDLGR [224], YLCGAHSDGQLQEGSPIQAWQLFVNEESTIPR [238], YWCLWEGAQNGR [244] | P01833 |
| 22 | 79705 | 5,05 | AFVNCDENSR [40], APAFEGR [43], AQYEGR [47], CGLGINSR [57], DGSFSVVITGLR [66], QGHFYGETAAVYVAVEER [168], RAPAFEGR [175], VLDSGFR [214], VYTVDLGR [224], YLCGAHSDGQLQEGSPIQAWQLFVNEESTIPR [238] | P01833 |
| 22 | 80272 | 5,97 | AFVNCDENSR [40], APAFEGR [43], AQYEGR [47], CGLGINSR [57], DGSFSVVITGLR [66], FSSYEK [100], IIEGEPNLK [123], ILLNPQDK [124], LDIQGTGQLLFSVVINQLR [142], QGHFYGETAAVYVAVEER [168], RAPAFEGR [175], VLDSGFR [214], VYTVDLGR [224], YLCGAHSDGQLQEGSPIQAWQLFVNEESTIPR [238], YWCLWEGAQNGR [244] | P01833 |
| 22 | 80654 | 5,02 | ANLTNFPENGTFVVNIAQLSQDDSGR [42], DGSFSVVITGLR [66], QGHFYGETAAVYVAVEER [168], RAPAFEGR [175], VLDSGFR [214], VYTVDLGR [224] | P01833 |

(continued)

| CRCMP # | MW (Da) | pl | Amino Acid Sequences of Tryptic Digest Peptides [SEQ ID No] | Acc. number |
|---|---|---|---|---|
| 22 | 80735 | 5,78 | AFVNCDENSR [40], APAFEGR [43], AQYEGR [47], CGLGINSR [57], DGSFSVVITGLR [66], FSSYEK [100], IIEGEPNLK [123], ILLNPQDK [124], QGHFYGETAAVYVAVEER [168], RAPAFEGR [175], VLDSGFR [214], VYTVDLGR [224], YLCGAHSDGQLQEGSPIQAWQLFVNEESTIPR [238], YWCLWEGAQNGR [244] | P01833 |
| 22 | 83246 | 5,15 | AFVNCDENSR [40], ANLTNFPENGTFVVNIAQLSQDDSGR [42], APAFEGR [43], AQYEGR [47], CGLGINSR [57], DGSFSVVITGLR [66], FSSYEK [100], IIEGEPNLK [123], ILLNPQDK [124], QGHFYGETAAVYVAVEER [168], RAPAFEGR [175], VLDSGFR [214], VYTVDLGR [224] | P01833 |
| 22 | 83366 | 4,72 | AFVNCDENSR [40], ANLTNFPENGTFVVNIAQLSQDDSGR [42], APAFEGR [43], AQYEGR [47], CGLGINSR [57], DGSFSVVITGLR [66], FSSYEK [100], IIEGEPNLK [123], ILLNPQDK [124], KYWCR [140], QGHFYGETAAVYVAVEER [168], QSSGENCDVVVNTLGK [172], RAPAFEGR [175], VLDSGFR [214], VYTVDLGR [224], YLCGAHSDGQLQEGSPIQAWQLFVNEESTIPR [238], YWCLWEGAQNGR [244] | P01833 |
| 22 | 83750 | 4,96 | APAFEGR [43], AQYEGR [47], DGSFSVVITGLR [66], FSSYEK [100], IIEGEPNLK [123], QGHFYGETAAVYVAVEER [168], RAPAFEGR [175], VLDSGFR [214], VYTVDLGR [224] | P01833 |
| 22 | 83905 | 5,07 | APAFEGR [43], AQYEGR [47], DGSFSVVITGLR [66], QGHFYGETAAVYVAVEER [168], RAPAFEGR [175], VLDSGFR [214], VYTVDLGR [224] | P01833 |
| 22 | 84555 | 5,07 | DGSFSVVITGLR [66], QGHFYGETAAVYVAVEER [168], RAPAFEGR [175], VLDSGFR [214] | P01833 |
| 22 | 84742 | 4,90 | AFVNCDENSR [40], APAFEGR [43], AQYEGR [47], DGSFSVVITGLR [66], IIEGEPNLK [123], LDIQGTGQLLFSVVINQLR [142], QGHFYGETAAVYVAVEER [168], RAPAFEGR [175], VLDSGFR [214], VYTVDLGR [224] | P01833 |
| 22 | 86180 | 4,86 | AFVNCDENSR [40], APAFEGR [43], AQYEGR [47], CGLGINSR [57], DGSFSVVITGLR [66], FSSYEK [100], IIEGEPNLK [123], QGHFYGETAAVYVAVEER [168], RAPAFEGR [175], VLDSGFR [214], VYTVDLGR [224] | P01833 |
| 22 | 90403 | 4,78 | AFVNCDENSR [40], APAFEGR [43], AQYEGR [47], DGSFSVVITGLR [66], FSSYEK [100], IIEGEPNLK [123], QGHFYGETAAVYVAVEER [168], RAPAFEGR [175], VLDSGFR [214], VYTVDLGR [224] | P01833 |
| 22 | 91105 | 4,74 | DGSFSVVITGLR [66], LDIQGTGQLLFSVVINQLR [142], QGHFYGETAAVYVAVEER [168], RAPAFEGR [175], VLDSGFR [214], VYTVDLGR [224] | P01833 |
| 22 | 92925 | 4,74 | APAFEGR [43], DGSFSVVITGLR [66], QGHFYGETAAVYVAVEER [168], RAPAFEGR [175], VLDSGFR [214], VYTVDLGR [224] | P01833 |

(continued)

| CRCMP # | MW (Da) | pl | Amino Acid Sequences of Tryptic Digest Peptides [SEQ ID No] | Acc. number |
|---|---|---|---|---|
| 23 | 32654 | 5,31 | APYEWK [45], DYEILFK [75], FFNVLTTNTDGK [91], IEFISTMEGYK [121], KNNHHFK [135], NLDGISHAPNAVK [160], SINGILFPGGSVDLR [190], TAFYLAEFFVNEAR [196], WSLSVK [228], YLESAGAR [239], YPVYGVQWHPEK [242], YYIAASYVK [245] | Q92820 |
| 23 | 32772 | 5,46 | APYEWK [45], NLDGISHAPNAVK [160], TAFYLAEFFVNEAR [196], YLESAGAR [239], YPVYGVQWHPEK [242] | Q92820 |
| 23 | 33240 | 5,56 | IEFISTMEGYK [121], SINGILFPGGSVDLR [190], TAFYLAEFFVNEAR [196] | Q92820 |
| 23 | 33503 | 5,50 | APYEWK [45], DYEILFK [75], FFNVLTTNTDGK [91], IEFISTMEGYK [121], KFFNVLTTNTDGK [131], KNNHHFK [135], NLDGISHAPNAVK [160], RSDYAK [181], SESEEEK [188], SINGILFPGGSVDLR [190], TAFYLAEFFVNEAR [196], WSLSVK [228], YLESAGAR [239], YPVYGVQWHPEK [242], YYIAASYVK [245] | Q92820 |
| 23 | 34247 | 5,32 | APYEWK [45], DYEILFK [75], FFNVLTTNTDGK [91], IEFISTMEGYK [121], KFFNVLTTNTDGK [131], KNNHHFK [135], NLDGISHAPNAVK [160], NNHHFK [163], RSDYAK [181], SESEEEK [188], SINGILFPGGSVDLR [190], TAFYLAEFFVNEAR [196], WSLSVK [228], YLESAGAR [239], YPVYGVQWHPEK [242], YYIAASYVK [245] | Q92820 |
| 23 | 34827 | 5,20 | APYEWK [45], DYEILFK [75], FFNVLTTNTDGK [91], IEFISTMEGYK [121], KNNHHFK [135], NLDGISHAPNAVK [160], SINGILFPGGSVDLR [190], YLESAGAR [239], YPVYGVQWHPEK [242], YYIAASYVK [245] | Q92820 |
| 23 | 34996 | 5,01 | APYEWK [45], DYEILFK [75], FFNVLTTNTDGK [91], IEFISTMEGYK [121], KNNHHFK [135], NLDGISHAPNAVK [160], NNHHFK [163], SINGILFPGGSVDLR [190], TAFYLAEFFVNEAR [196], YLESAGAR [239], YPVYGVQWHPEK [242], YYIAASYVK [245] | Q92820 |
| 23 | 35025 | 5,42 | APYEWK [45], DYEILFK [75], IEFISTMEGYK [121], NLDGISHAPNAVK [160], SINGILFPGGSVDLR [190], SINGILFPGGSVDLRR [191], TAFYLAEFFVNEAR [196], YLESAGAR [239], YPVYGVQWHPEK [242] | Q92820 |

**Table 3: CRCMP Categories**

| CRCMP # | Trans Membrane Type | Known Truncated Isoforms | GPI Anchored Cell Surface | Secreted Isoform |
|---|---|---|---|---|
| 1 | I | | | |
| 2 | I | | | |
| 5 | I | | | |
| 6 | II | | | |
| 7 | I | yes | | |
| 8 | unknown | yes | | |
| 9 | | | | yes |
| 10 | I | yes | | |

(continued)

| CRCMP # | Trans Membrane Type | Known Truncated Isoforms | GPI Anchored Cell Surface | Secreted Isoform |
|---------|---------------------|--------------------------|----------------------------|------------------|
| 12 | I | | | |
| 14 | | | Probable | |
| 17 | | yes | | yes |
| 18 | unknown | yes | | |
| 19 | | yes | | yes |
| 20 | unknown | yes | | |
| 22 | I | yes | | yes |
| 23 | | yes | | yes |
| 25 | unknown | | | |
| 26 | | yes | yes | |

Membrane proteins come in numerous types with a few different suggested classifications. One of the most commonly used to date is the classification method suggested by JS Singer: Type I proteins have a single TM stretch of hydrophobic residues, with the portion of the polypeptide on the NH2-terminal side of the TM domain exposed on the exterior side of the membrane and the COOH-terminal portion exposed on the cytoplasmic side. The proteins are subdivided into types Ia (cleavable signal sequences) and Ib (without cleavable signal sequence). Most eukaryotic mebrane proteins with single spanning regions are of Type Ia. Type II membrane proteins are similar to the type I class in that they span the membrane only once, but they have their amino terminus on the cytoplasmic side of the cell and the carboxy terminus on the exterior. Type III membrane proteins have multiple transmembrane domains in a single polypeptide chain. They are also sub divided into a and b: Type IIIa molecules have cleavable signal sequences while type IIIb have their amino termini exposed on the exterior surface of the membrane, but do not have a cleavable signal sequences. Type IIIa proteins include the M and L peptides of the photoreaction center. Type IIIb proteins include e.g. cytochrome P450, and leader peptidase of E. coli. Type IV proteins have multiple homologous domains which make up an assembly that spans the membrane multiple times. The domains may reside on a single polypeptide chain or be on more than one indivdual chain. This nomenclature is used in Table 3.

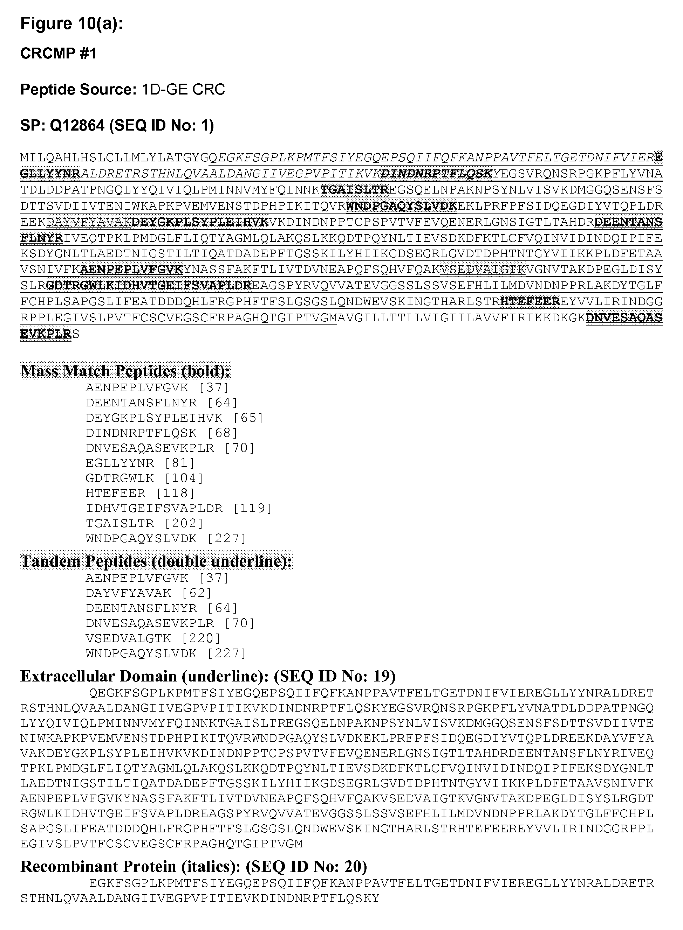

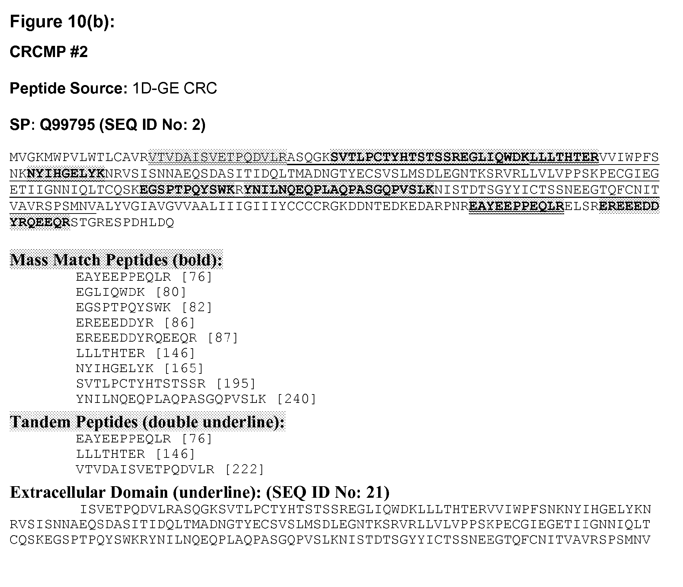

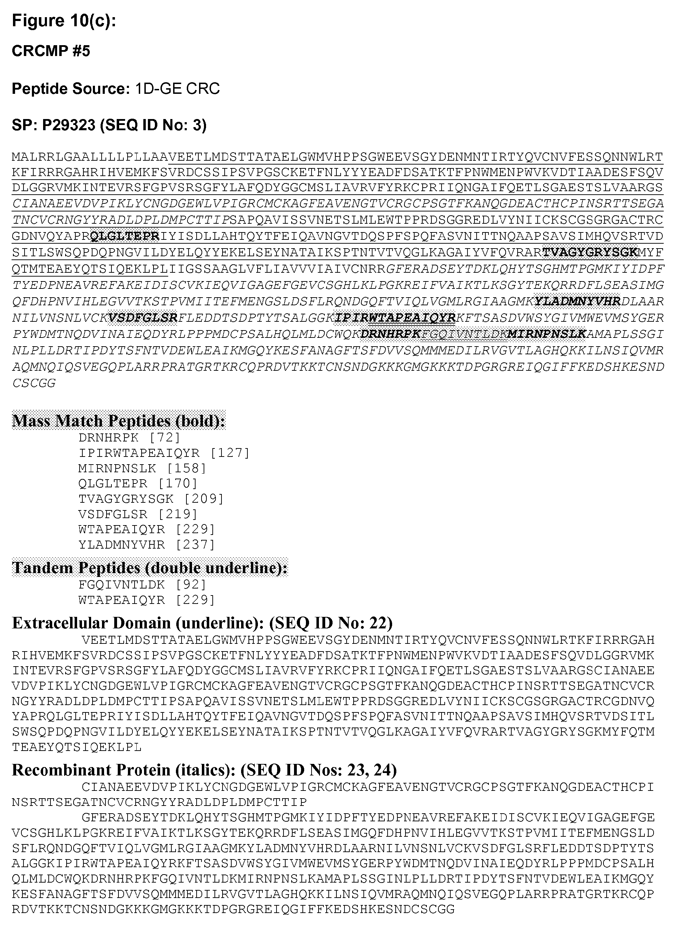

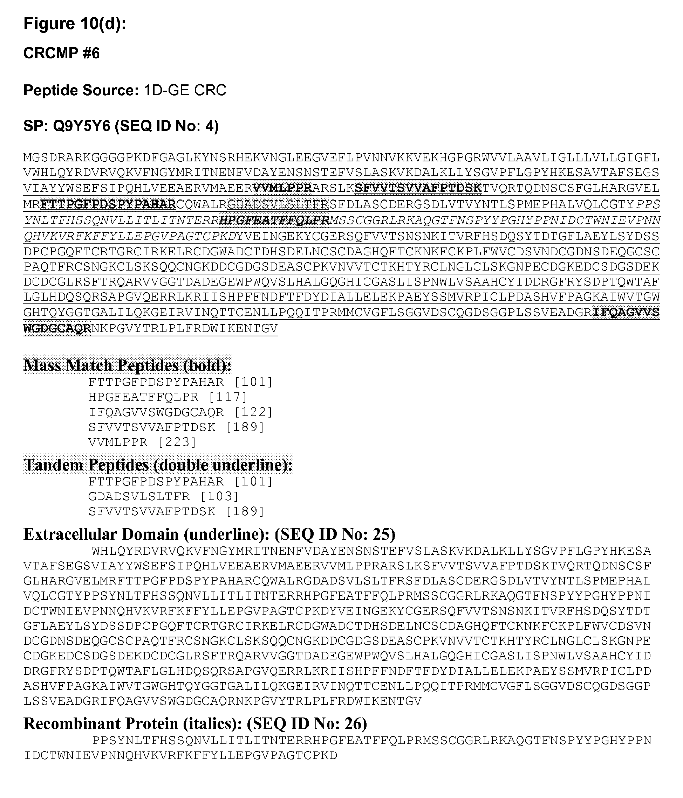

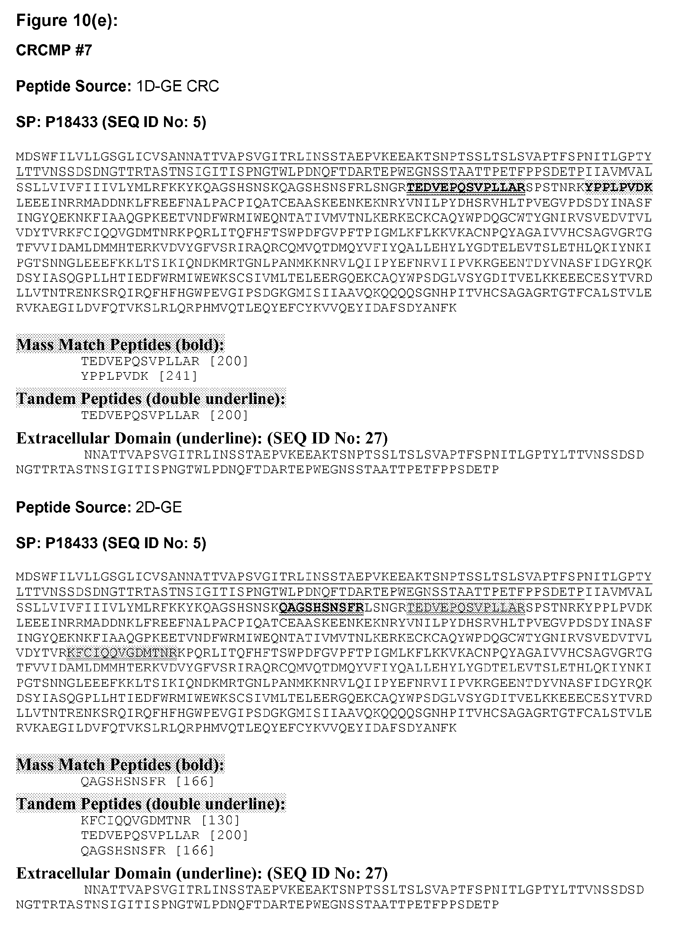

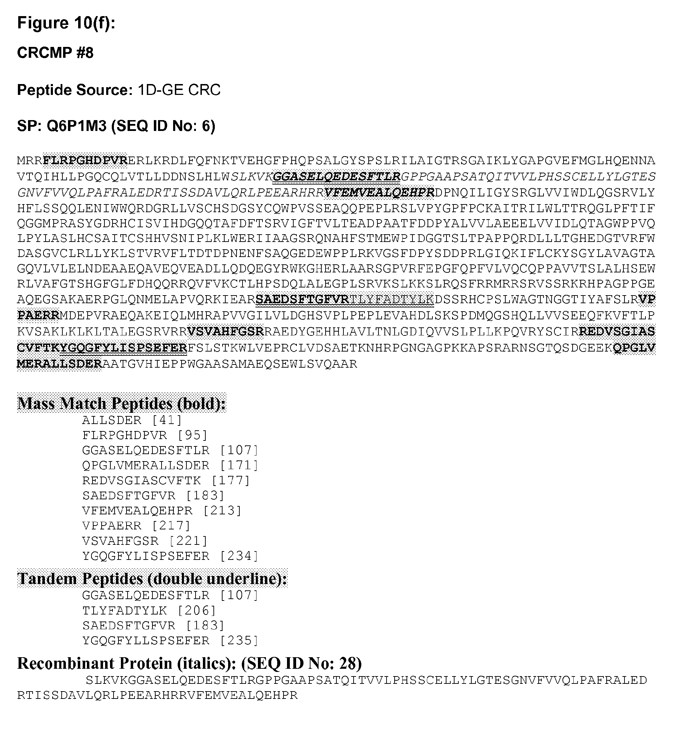

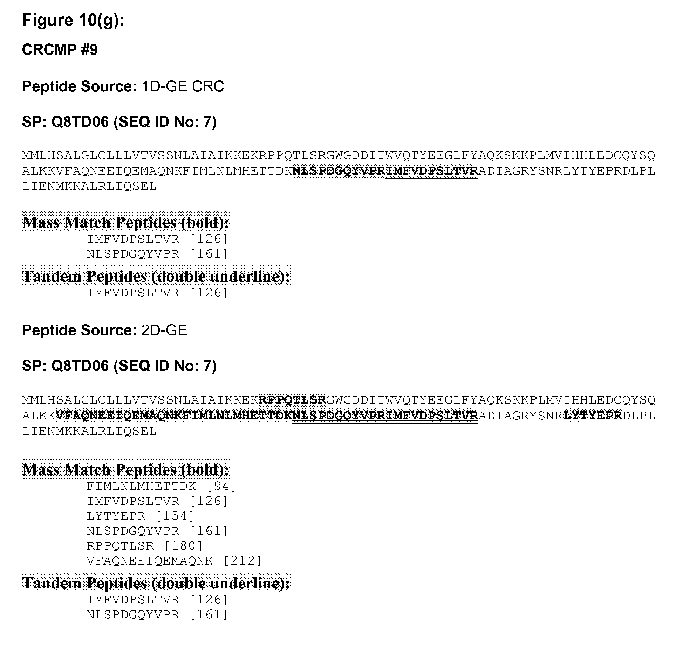

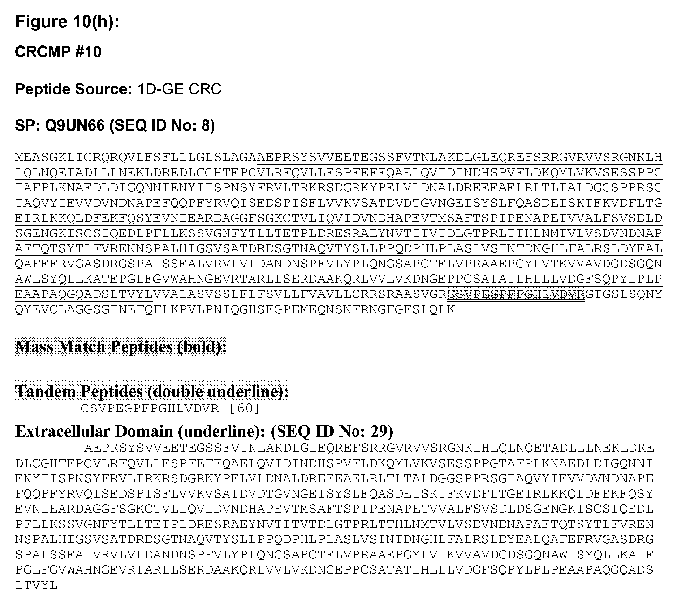

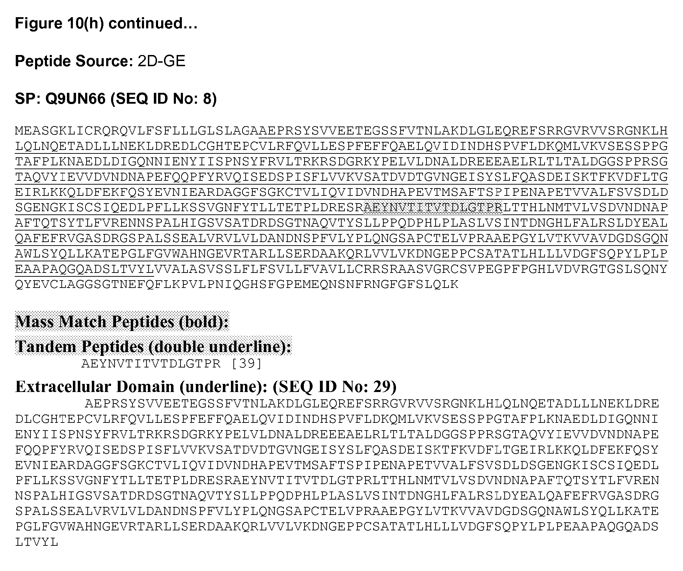

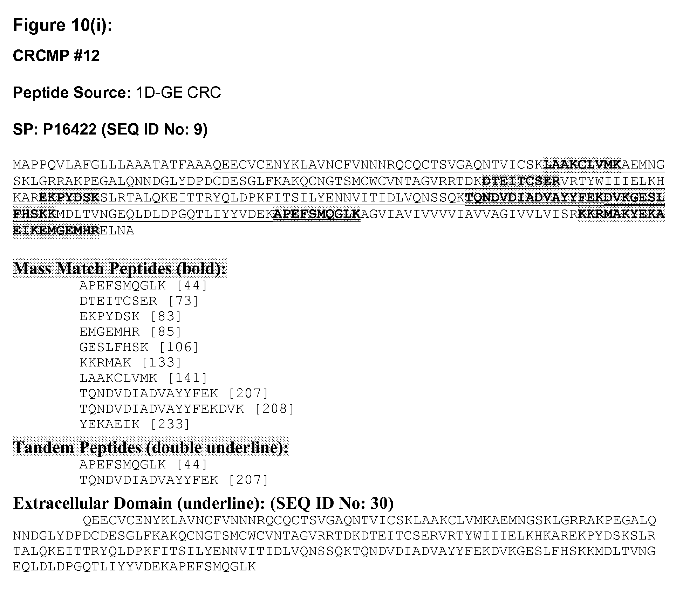

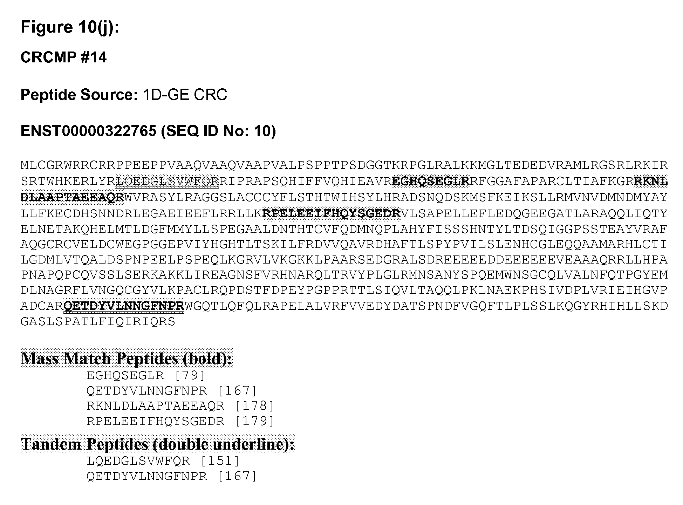

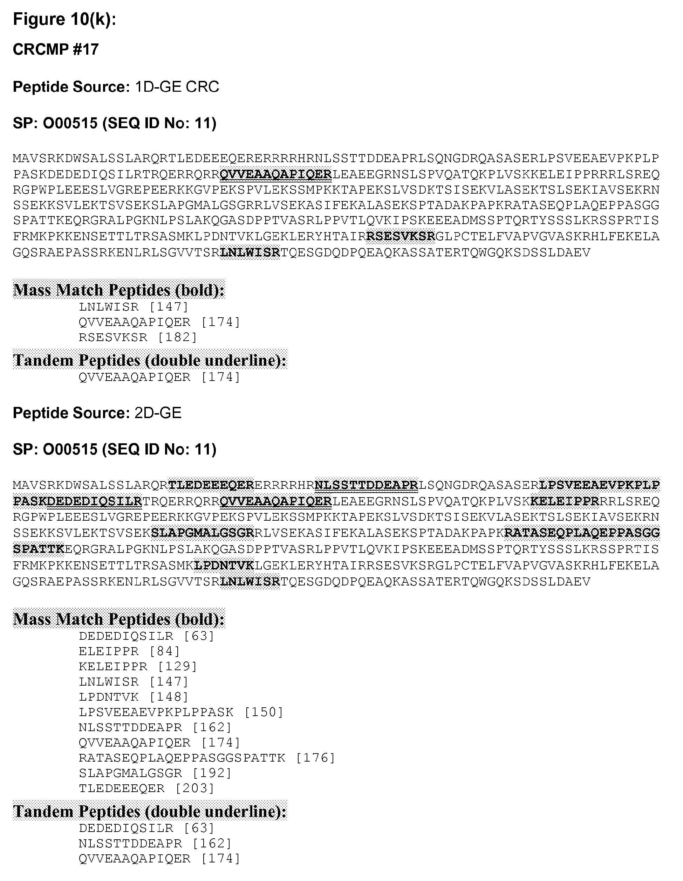

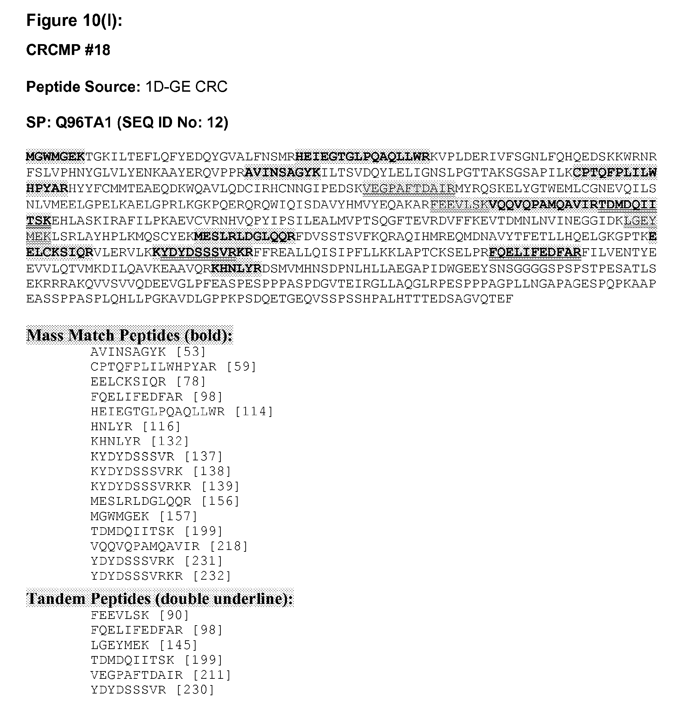

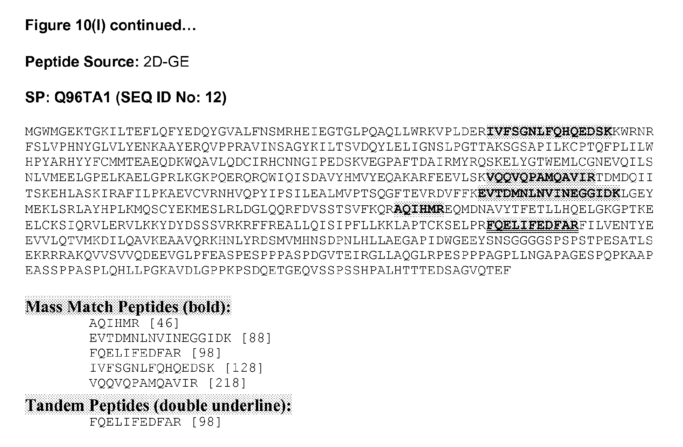

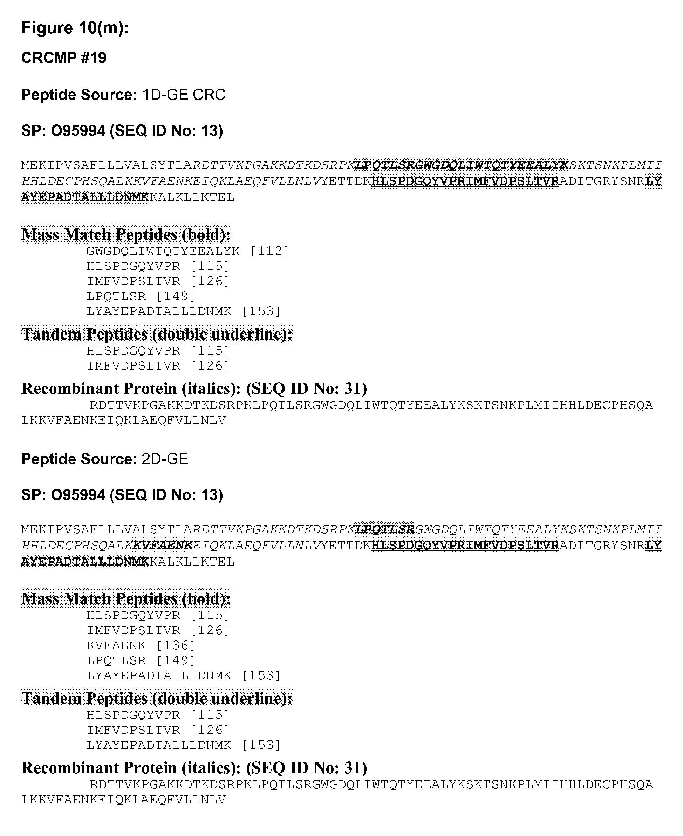

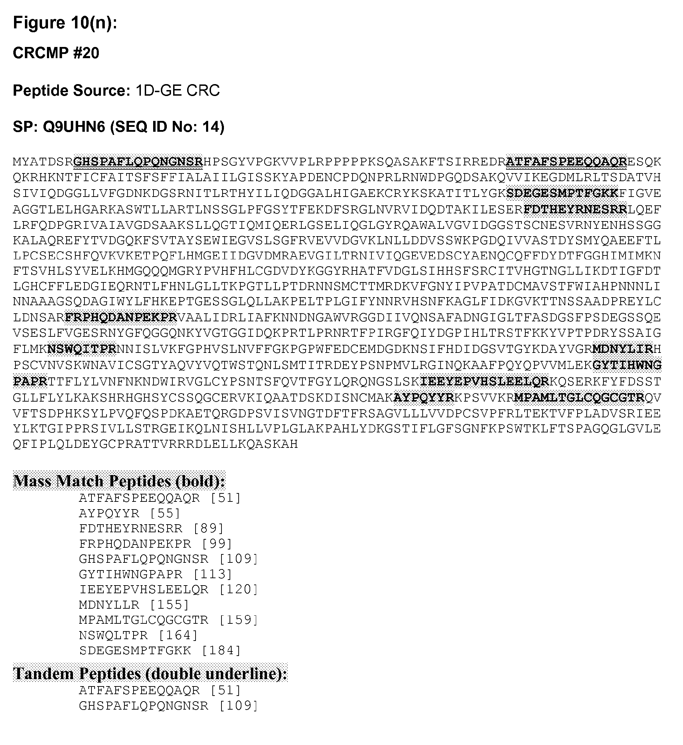

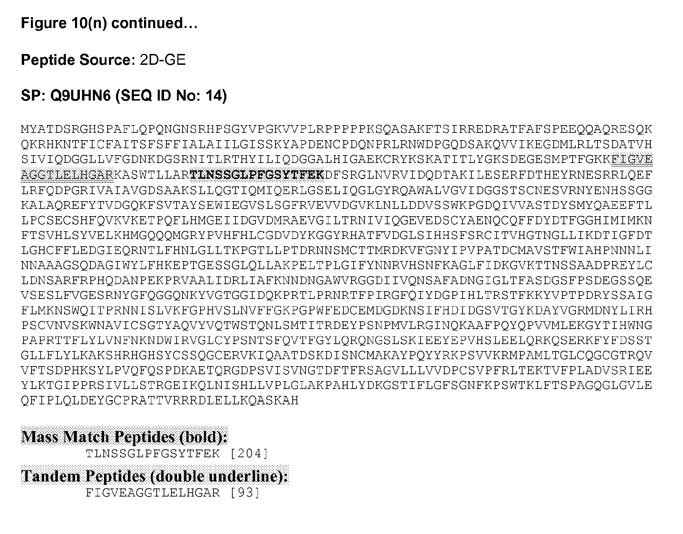

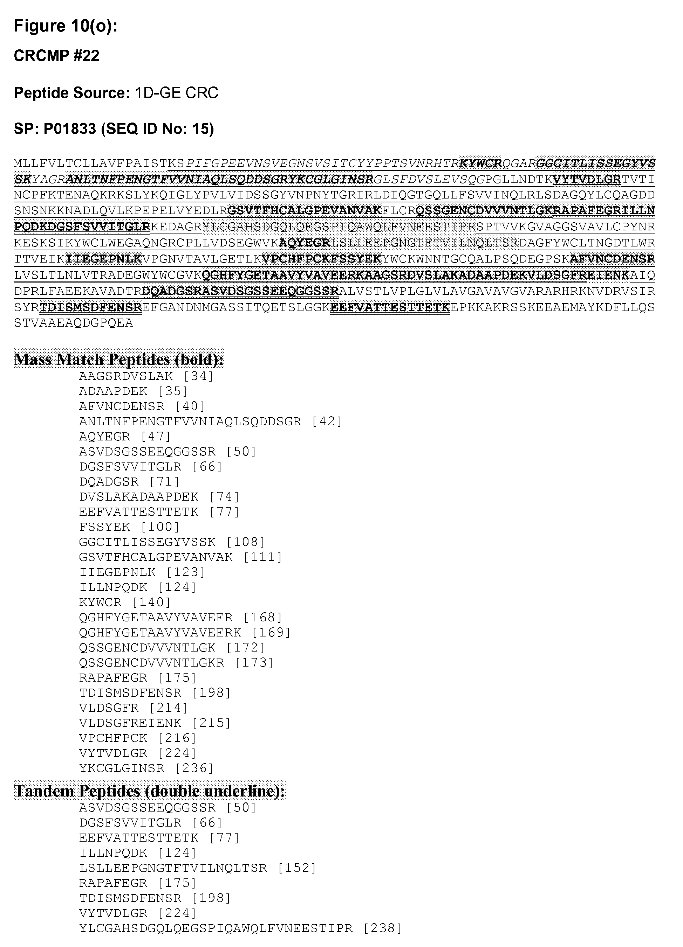

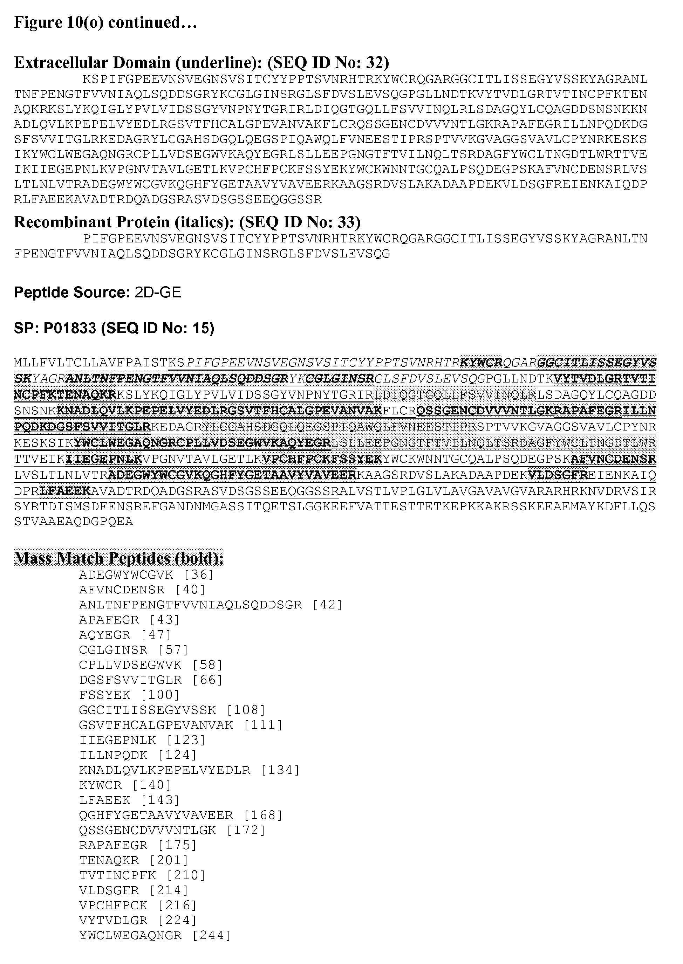

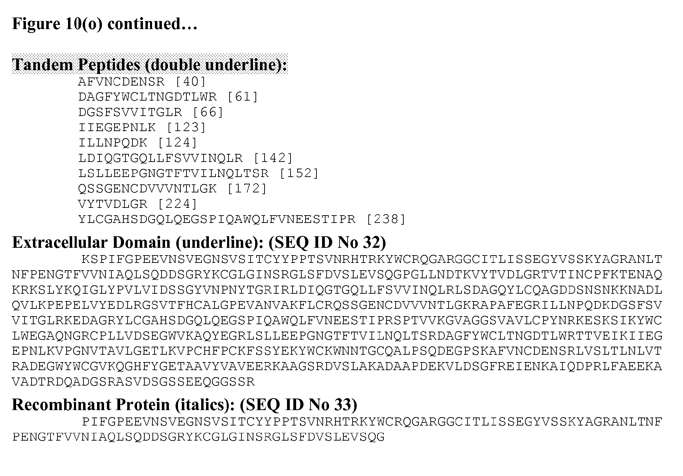

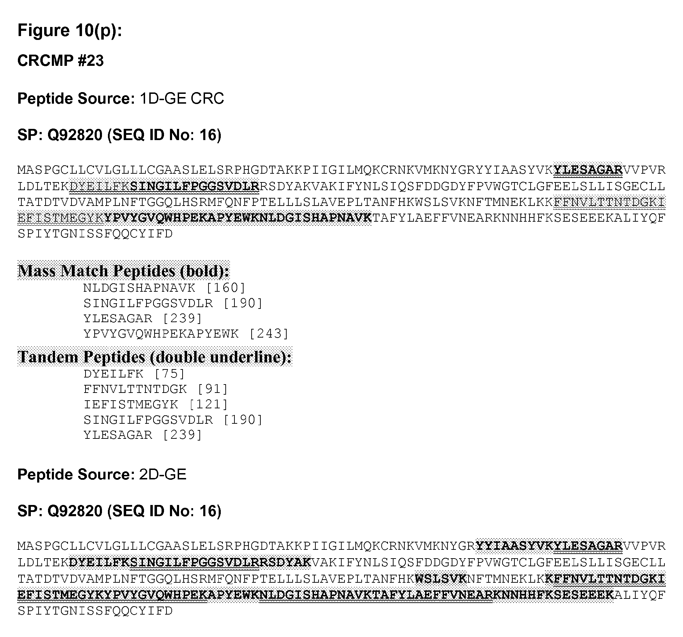

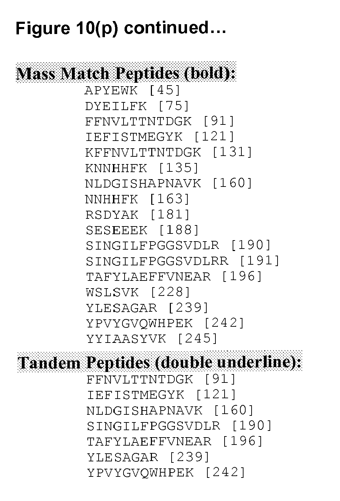

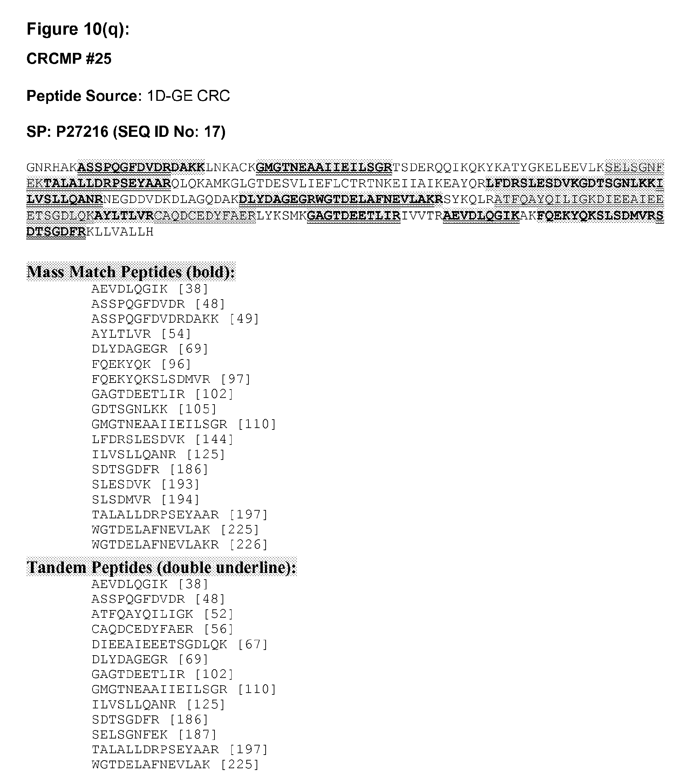

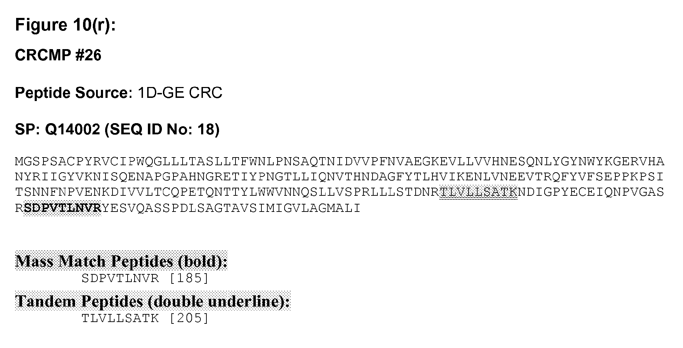

The sequences of the 18 proteins referred to in Table 1 and 2 are recited in Figures 10(a) to (r). The portions of the sequence which correspond to the Mass Match Peptides are shown in bold. The portions of the sequence which correspond to the Tandem Peptides are shown in double underline. The portion(s) of the sequences which correspond to an extracellular part of the whole protein are shown in underline (SEQ ID Nos 19, 21, 22, 25, 27, 29, 30 and 32). Preferred soluble peptides/CRCMPs according to the invention have sequences which overlap with or are preferably within an extracellular part of the whole protein.

Portions of the sequence which correspond to commercially available recombinant proteins are shown in italics (SEQ ID Nos 20, 23, 24, 26, 28, 31 and 33). These may, for example, be readily employed to raise antibodies for use according to the invention, especially when they overlap with or are preferably within the extracellular part of the whole protein. Other non-commercially available portions of the whole protein or, other soluble polypeptides according to the invention, may be prepared using conventional methods known to a skilled person e.g. expression of protein in a host cell containing a suitable vector (bacterial or mammalian system) or by stepwise peptide synthesis.

For any given CRCMP, the detected level obtained upon analyzing serum from subjects having colorectal cancer relative to the detected level obtained upon analyzing serum from subjects free from colorectal cancer will depend upon the particular analytical protocol and detection technique that is used, provided that such CRCMP is differentially expressed between normal and disease tissue. Accordingly, the present invention contemplates that each laboratory will establish a reference range for each CRCMP in subjects free from colorectal cancer according to the analytical protocol and detection technique in use, as is conventional in the diagnostic art. Preferably, at least one control positive serum sample from a subject known to have colorectal cancer or at least one control negative serum sample from a subject known to be free from colorectal cancer (and more preferably both positive and negative control samples) are included in each batch of test samples analysed.

In an assay the objective may be to detect the presence of a marker polypeptide. Alternatively it may be to determine the level of a marker polypeptide. Assay design may provide for an appropriate threshold of detection such that detection of a marker polypeptide can be correlated with detection of a specified level of that polypeptide.

In one embodiment, the level of expression of a protein is determined relative to a background value, which is defined as the level of signal obtained from a proximal region of the image that (a) is equivalent in area to the particular feature in question; and (b) contains no discernable protein feature.

CRCMPs can be used for detection, prognosis, diagnosis, or monitoring of colorectal cancer or for drug development. In one embodiment of the invention, serum from a subject (*e.g.*, a subject suspected of having colorectal cancer) is analysed by 2D electrophoresis for detection of one or more of the CRCMPs as defined in Tables 1 and 2. A decreased or increased abundance of said one or more CRCMPs in the serum from the subject relative to serum from a subject or subjects free from colorectal cancer (*e.g.*, a control sample) or a previously determined reference range indicates the presence or absence of colorectal cancer. More details are provided below in the section entitled Assay Measurement Strategies.

In a preferred embodiment, serum from a subject is analysed for quantitative detection of clusters of CRCMPs as defined in Tables 1 and 2.

As will be evident to one of skill in the art, a given CRCMP can be described according to the data provided for that CRCMP in Table 1 and in Table 2. The CRCMP is a protein comprising a peptide sequence described for that CRCMP (preferably comprising a plurality of, more preferably all of, the peptide sequences described for that CRCMP).

In one embodiment, serum from a subject is analysed for quantitative detection of one or more of the CRCMPs as defined in Tables 1 and 2, wherein a change in abundance of the CRCMP or CRCMPs in the serum from the subject relative to serum from a subject or subjects free from colorectal cancer (*e.g.*, a control sample or a previously determined reference range) indicates the presence of colorectal cancer.

In a preferred embodiment, serum from a subject is analysed for quantitative detection of a cluster of CRCMPs as defined in Tables 1 and 2.

For each CRCMP the present invention additionally provides: (a) a preparation comprising the isolated CRCMP; (b) a preparation comprising one or more fragments of the CRCMP; and (c) antibodies or other affinity reagents such as Affibodies, Nanobodies or Unibodies that bind to said CRCMP, to said fragments, or both to said CRCMP and to said fragments. As used herein, a CRCMP is "isolated" when it is present in a preparation that is substantially free of contaminating proteins, *i.e.,* a preparation in which less than 10% (preferably less than 5%, more preferably less than 1%) of the total protein present is contaminating protein(s). A contaminating protein is a protein having a significantly different amino acid sequence from that of the isolated CRCMP, as determined by mass spectral analysis. As used herein, a "significantly different" sequence is one that permits the contaminating protein to be resolved from the CRCMP by mass spectral analysis, performed according to the Reference Protocol.

The CRCMPs of the invention can be assayed by any method known to those skilled in the art, including but not limited to, the technology described herein in the examples, kinase assays, enzyme assays, binding assays and other functional assays, immunoassays, and western blotting. In one embodiment, the CRCMPs are separated on a 1-D gel by virtue of their MWs and visualized by staining the gel. In one embodiment, the CRCMPs are stained with a fluorescent dye and imaged with a fluorescence scanner. Sypro Red (Molecular Probes, Inc., Eugene, Oregon) is a suitable dye for this purpose. A preferred fluorescent dye is disclosed in U.S. Application No. 09/412,168, filed on October 5, 1999, which is incorporated herein by reference in its entirety.

Alternatively, CRCMPs can be detected in an immunoassay. In one embodiment, an immunoassay is performed by contacting a sample from a subject to be tested with an anti-CRCMP antibody (or other affinity reagent such as an Affibody, Nanobody or Unibody) under conditions such that immunospecific binding can occur if the CRCMP is present, and detecting or measuring the amount of any immunospecific binding by the affinity reagent. Anti-CRCMP affinity reagents can be produced by the methods and techniques taught herein.

CRCMPs may be detected by virtue of the detection of a fragment thereof e.g. an immunogenic or antigenic fragment thereof. Fragments may have a length of at least 10, more typically at least 20 amino acids e.g. at least 50 or 100 amino acids e.g. at least 200 or 500 amino acids e.g at least 800 or 1000 amino acids.

In one embodiment, binding of antibody (or other affinity reagent such as an Affibody, Nanobody or Unibody) in tissue sections can be used to detect aberrant CRCMP localization or an aberrant level of one or more CRCMPs. In a specific embodiment, an antibody (or other affinity reagent such as an Affibody, Nanobody or Unibody) to a CRCMP can be used to assay a patient tissue (*e.g.*, a serum sample) for the level of the CRCMP where an aberrant level of CRCMP is indicative of colorectal cancer. As used herein, an "aberrant level" means a level that is increased or decreased compared with the level in a subject free from colorectal cancer or a reference level.

Any suitable immunoassay can be used, including, without limitation, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

For example, a CRCMP can be detected in a fluid sample (e.g., blood, urine, or saliva) by means of a two-step sandwich assay. In the first step, a capture reagent (e.g., an anti-CRCMP antibody or other affinity reagent such as an Affibody, Nanobody or Unibody) is used to capture the CRCMP. The capture reagent can optionally be immobilized on a solid phase. In the second step, a directly or indirectly labeled detection reagent is used to detect the captured CRCMP. In one embodiment, the detection reagent is a lectin. Any lectin can be used for this purpose that preferentially binds to the CRCMP rather than to other isoforms that have the same core protein as the CRCMP or to other proteins that share the antigenic determinant recognized by the affinity reagent. In a preferred embodiment, the chosen lectin binds to the CRCMP with at least 2-fold greater affinity, more preferably at least 5-fold greater affinity, still more preferably at least 10-fold greater affinity, than to said other isoforms that have the same core protein as the CRCMP or to said other proteins that share the antigenic determinant recognized by the affinity reagent. Based on the present description, a lectin that is suitable for detecting a given CRCMP can readily be identified by methods well known in the art, for instance upon testing one or more lectins enumerated in Table I on pages 158-159 of Sumar et al., Lectins as Indicators of Disease-Associated Glycoforms, In: Gabius H-J & Gabius S (eds.), 1993, Lectins and Glycobiology, at pp. 158-174 (which is incorporated herein by reference in its entirety). In an alternative embodiment, the detection reagent is an antibody (or other affinity reagent such as an Affibody, Nanobody or Unibody), e.g., an antibody that immunospecifically detects other post-translational modifications, such as an antibody that immunospecifically binds to phosphorylated amino acids. Examples of such antibodies include those that bind to phosphotyrosine (BD Transduction Laboratories, catalog nos.: P11230-050/P11230-150; P11120; P38820; P39020), those that bind to phosphoserine (Zymed Laboratories Inc., South San Francisco, CA, catalog no. 61-8100) and those that bind to phosphothreonine (Zymed Laboratories Inc., South San Francisco, CA, catalogue nos. 71-8200, 13-9200).

If desired, a gene encoding a CRCMP, a related gene, or related nucleic acid sequences or subsequences, including complementary sequences, can also be used in hybridization assays. A nucleotide encoding a CRCMP, or subsequences thereof comprising at least 8 nucleotides, preferably at least 12 nucleotides, and most preferably at least 15 nucleotides can be used as a hybridization probe. Hybridization assays can be used for detection, prognosis, diagnosis, or monitoring of conditions, disorders, or disease states, associated with aberrant expression of genes encoding CRCMPs, or for differential diagnosis of subjects with signs or symptoms suggestive of colorectal cancer. In particular, such a hybridization assay can be carried out by a method comprising contacting a subject's sample containing nucleic acid with a nucleic acid probe capable of hybridizing to a DNA or RNA that encodes a CRCMP, under conditions such that hybridization can occur, and detecting or measuring any resulting hybridization. Nucleotides can be used for therapy of subjects having colorectal cancer, as described below.

The invention also provides kits e.g. diagnostic kits comprising one or more reagents for use in the detection and/or determination of one or more soluble polypeptide markers according to the invention. Suitably such kits comprise an anti-CRCMP antibody (or other affinity reagent such as an Affibody, Nanobody or Unibody) i.e. an affinity reagent capable of immunospecific binding to a soluble polypeptide marker according to the invention or for example a plurality of distinct such affinity reagents. Conveniently labeled affinity reagents may be employed to determine the presence of one or more of said soluble polypeptide markers. For example a kit may contain one or more containers with one or more affinity reagents against one or more said soluble polypeptide markers. Conveniently, such a kit may further comprise a labeled binding partner to the or each affinity reagent and/or a solid phase (such as a reagent strip) upon which the or each affinity reagent is immobilized. In addition, such a kit may optionally comprise one or more of the following: (1) instructions for using the anti-CRCMP affinity reagent for diagnosis, prognosis, therapeutic monitoring or any combination of these applications; (2) a labeled binding partner to the affinity reagent; (3) a solid phase (such as a reagent strip) upon which the anti-CRCMP affinity reagent is immobilized; and (4) a label or insert indicating regulatory approval for diagnostic, prognostic or therapeutic use or any combination thereof. If no labeled binding partner to the affinity reagent is provided, the anti-CRCMP affinity reagent itself can be labeled with a detectable marker, *e.g.*, a chemiluminescent, enzymatic, fluorescent, or radioactive moiety.

Antibodies (or other affinity reagents such as Affibodies, Nanobodies or Unibodies) and kits may be used for diagnosing colorectal cancer in a subject, differentiating causes of colorectal cancer in a subject, guiding therapy in a subject suffering from colorectal cancer, assessing the risk of relapse in a subject suffering from colorectal cancer, or assigning a prognostic risk of one or more future clinical outcomes to a subject suffering from colorectal cancer.

Kits may also be of use in the detection, diagnosis of colorectal cancer in a subject, for differentiating causes of colorectal cancer in a subject, for guiding therapy in a subject suffering from colorectal cancer, for assessing the risk of relapse in a subject suffering from colorectal cancer, or for assigning a prognostic risk of one or more future clinical outcomes to a subject suffering from colorectal cancer, which kit comprises one or more soluble polypeptides derived from a protein selected from the list consisting of proteins defined by SEQ ID Nos 1-18, and/or one or more antigenic or immunogenic fragments thereof.

The invention also provides a kit comprising a nucleic acid probe capable of hybridizing to RNA encoding a CRCMP. In a specific embodiment, a kit comprises in one or more containers a pair of primers (*e.g.*, each in the size range of 6-30 nucleotides, more preferably 10-30 nucleotides and still more preferably 10-20 nucleotides) that under appropriate reaction conditions can prime amplification of at least a portion of a nucleic acid encoding a CRCMP, such as by polymerase chain reaction (see, *e.g.,* Innis et al., 1990, PCR Protocols, Academic Press, Inc., San Diego, CA), ligase chain reaction (see EP 320,308) use of Qβ replicase, cyclic probe reaction, or other methods known in the art.

Kits are also provided which allow for the detection of a plurality of CRCMPs or a plurality of nucleic acids each encoding a CRCMP. A kit can optionally further comprise a predetermined amount of an isolated CRCMP protein or a nucleic acid encoding a CRCMP, e.g., for use as a standard or control.

Use in Clinical Studies

The diagnostic methods and compositions of the present invention can assist in monitoring a clinical study, *e.g.* to evaluate drugs for therapy of colorectal cancer. In one embodiment, candidate molecules are tested for their ability to restore CRCMP levels in a subject having colorectal cancer to levels found in subjects free from colorectal cancer or, in a treated subject (*e.g.* after treatment with taxol or doxorubacin), to preserve CRCMP levels at or near non-colorectal cancer values. The levels of one or more CRCMPs can be assayed.

In another embodiment, the methods and compositions of the present invention are used to screen candidates for a clinical study to identify individuals having colorectal cancer; such individuals can then be excluded from the study or can be placed in a separate cohort for treatment or analysis. If desired, the candidates can concurrently be screened to identify individuals with colorectal cancer; procedures for these screens are well known in the art.

Production of Proteins of the Invention and Corresponding Nucleic Acids

A DNA of the present invention can be obtained by isolation as a cDNA fragment from cDNA libraries using as starter materials commercial mRNAs and determining and identifying the nucleotide sequences thereof. That is, specifically, clones are randomly isolated from cDNA libraries, which are prepared according to Ohara et al's method (DNA Research Vol.4, 53-59 (1997)). Next, through hybridization, duplicated clones (which appear repeatedly) are removed and then in vitro transcription and translation are carried out. Nucleotide sequences of both termini of clones, for which products of 50 kDa or more are confirmed, are determined.

Furthermore, databases of known genes are searched for homology using the thus obtained terminal nucleotide sequences as queries. The entire nucleotide sequence of a clone revealed to be novel as a result is determined. In addition to the above screening method, the 5' and 3' terminal sequences of cDNA are related to a human genome sequence. Then an unknown long-chain gene is confirmed in a region between the sequences, and the full-length of the cDNA is analyzed. In this way, an unknown gene that is unable to be obtained by a conventional cloning method that depends on known genes can be systematically cloned.

Moreover, all of the regions of a human-derived gene containing a DNA of the present invention can also be prepared using a PCR method such as RACE while paying sufficient attention to prevent artificial errors from taking place in short fragments or obtained sequences. As described above, clones having DNA of the present invention can be obtained.

In another means for cloning DNA of the present invention, a synthetic DNA primer having an appropriate nucleotide sequence of a portion of a polypeptide of the present invention is produced, followed by amplification by the PCR method using an appropriate library. Alternatively, selection can be carried out by hybridization of a DNA of the present invention with a DNA that has been incorporated into an appropriate vector and labeled with a DNA fragment or a synthetic DNA encoding some or all of the regions of a polypeptide of the present invention. Hybridization can be carried out by, for example, the method described in Current Protocols in Molecular Biology (edited by Frederick M. Ausubel et al., 1987). DNA of the present invention may be any DNA, as long as they contain nucleotide sequences encoding the polypeptides of the present invention as described above. Such a DNA may be a cDNA identified and isolated from cDNA libraries or the like that are derived from colorectal tissue. Such a DNA may also be a synthetic DNA or the like. Vectors for use in library construction may be any of bacteriophages, plasmids, cosmids, phargemids, or the like. Furthermore, by the use of a total RNA fraction or a mRNA fraction prepared from the above cells and/or tissues, amplification can be carried out by a direct reverse transcription coupled polymerase chain reaction (hereinafter abbreviated as "RT-PCR method").

DNA encoding the above polypeptides consisting of amino acid sequences that are substantially identical to the amino acid sequences of the CRCMPs or DNA encoding the above polypeptides consisting of amino acid sequences derived from the amino acid sequences of the CRCMPs by deletion, substitution, or addition of one or more amino acids composing a portion of the amino acid sequence can be easily produced by an appropriate combination of, for example, a site-directed mutagenesis method, a gene homologous recombination method, a primer elongation method, and the PCR method known by persons skilled in the art. In addition, at this time, a possible method for causing a polypeptide to have substantially equivalent biological activity is substitution of homologous amino acids (e.g. polar and nonpolar amino acids, hydrophobic and hydrophilic amino acids, positively-charged and negatively charged amino acids, and aromatic amino acids) among amino acids composing the polypeptide. Furthermore, to maintain substantially equivalent biological activity, amino acids within functional domains contained in the polypeptide of the present invention are preferably conserved.

Furthermore, examples of DNA of the present invention include DNA comprising nucleotide sequences that encode the amino acid sequences of the CRCMPs and DNA hybridizing under stringent conditions to the DNA and encoding polypeptides (proteins) having biological activity (function) equivalent to the function of the polypeptides consisting of the amino acid sequences of the CRCMPs. Under such conditions, an example of such DNA capable of hybridizing to DNA comprising the nucleotide sequences that encode the amino acid sequences of the CRCMPs is DNA comprising a nucleotide sequence that has a degree of overall mean homology with the entire nucleotide sequence of the DNA, such as approximately 80% or more, preferably approximately 90% or more, and more preferably approximately 95% or more. Hybridization can be carried out according to a method known in the art such as a method described in Current Protocols in Molecular Biology (edited by Frederick M. Ausubel et al., 1987) or a method according thereto. Here, "stringent conditions" are, for example, conditions of approximately "1*SSC, 0.1% SDS, and 37°C, more stringent conditions of approximately "0.5*SSC, 0.1% SDS, and 42°C, or even more stringent conditions of approximately "0.2*SSC, 0.1% SDS, and 65°C. With more stringent hybridization conditions, the isolation of a DNA having high homology with a probe sequence can be expected. The above combinations of SSC, SDS, and temperature conditions are given for illustrative purposes. Stringency similar to the above can be achieved by persons skilled in the art using an appropriate combination of the above factors or other factors (for example, probe concentration, probe length, and reaction time for hybridization) for determination of hybridization stringency.

A cloned DNA of the present invention can be directly used or used, if desired, after digestion with a restriction enzyme or addition of a linker, depending on purposes. The DNA may have ATG as a translation initiation codon at the 5' terminal side and have TAA, TGA, or TAG as a translation termination codon at the 3' terminal side. These translation initiation and translation termination codons can also be added using an appropriate synthetic DNA adapter.

Where they are provided for use with the methods of the invention the CRCMPs are preferably provided in isolated form. More preferably the CRCMP polypeptides have been purified to at least to some extent. The CRCMP polypeptides may be provided in substantially pure form, that is to say free, to a substantial extent, from other proteins. The CRCMP polypeptides can also be produced using recombinant methods, synthetically produced or produced by a combination of these methods. The CRCMPs can be easily prepared by any method known by persons skilled in the art, which involves producing an expression vector containing a DNA of the present invention or a gene containing a DNA of the present invention, culturing a transformant transformed using the expression vector, generating and accumulating a polypeptide of the present invention or a recombinant protein containing the polypeptide, and then collecting the resultant.

Recombinant CRCMP polypeptides may be prepared by processes well known in the art from genetically engineered host cells comprising expression systems. Accordingly, the present invention also relates to expression systems which comprise CRCMP polypeptides or nucleic acids, to host cells which are genetically engineered with such expression systems and to the production of CRCMP polypeptides by recombinant techniques. For recombinant CRCMP polypeptide production, host cells can be genetically engineered to incorporate expression systems or portions thereof for nucleic acids. Such incorporation can be performed using methods well known in the art, such as, calcium phosphate transfection, DEAD-dextran mediated transfection, transvection, microinjection, cationic lipid-mediated transfection, electroporation, transduction, scrape loading, ballistic introduction or infection (see e.g. Davis et al., Basic Methods in Molecular Biology, 1986 and Sambrook et al. , Molecular Cloning: A Laboratory Manual, 2nd Ed., Cold Spring Harbour laboratory Press, Cold Spring Harbour, NY, 1989).

As host cells, for example, bacteria of the genus Escherichia, Streptococci, Staphylococci, Streptomyces, bacteria of the genus Bacillus, yeast, Aspergillus cells, insect cells, insects, and animal cells are used. Specific examples of bacteria of the genus Escherichia, which are used herein, include Escherichia coli K12 and DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), and HB101 (Journal of Molecular Biology, Vol. 41, 459 (1969)). As bacteria of the genus Bacillus, for example, Bacillus subtilis MI114 (Gene, Vol. 24, 255 (1983)) and 207-21 (Journal of Biochemistry, Vol. 95, 87 (1984)) are used. As yeast, for example, Saccaromyces cerevisiae AH22, AH22R-, NA87-11A, DKD-5D, and 20B-12, Schizosaccharomyces pombe NCYC1913 and NCYC2036, and Pichia pastoris are used. As insect cells, for example, Drosophila S2 and Spodoptera Sf9 cells are used. As animal cells, for example, COS-7 and Vero monkey cells, CHO Chinese hamster cells (hereinafter abbreviated as CHO cells), dhfr-gene-deficient CHO cells, mouse L cells, mouse AtT-20 cells, mouse myeloma cells, rat GH3 cells, human FL cells, COS, HeLa, C127,3T3, HEK 293, BHK and Bowes melanoma cells are used.

Cell-free translation systems can also be employed to produce recombinant polypeptides (e.g. rabbit reticulocyte lysate, wheat germ lysate, SP6/T7 in vitro T&T and RTS 100 E. Coli HY transcription and translation kits from Roche Diagnostics Ltd., Lewes, UK and the TNT Quick coupled Transcription/Translation System from Promega UK, Southampton, UK).

The expression vector can be produced according to a method known in the art. For example, the vector can be produced by (1) excising a DNA fragment containing a DNA of the present invention or a gene containing a DNA of the present invention and (2) ligating the DNA fragment downstream of the promoter in an appropriate expression vector. A wide variety of expression systems can be used, such as and without limitation, chromosomal, episomal and virus-derived systems, e.g. plasmids derived from Escherichia coli (e.g. pBR322, pBR325, pUC18, and pUC118), plasmids derived from Bacillus subtilis (e.g. pUB110, pTP5, and pC194), from bacteriophage, from transposons, from yeast episomes (e.g. pSH19 and pSH15), from insertion elements, from yeast chromosomal elements, from viruses such as baculoviruses, papova viruses such as SV40, vaccinia viruses, adenoviruses, fowl pox viruses, pseudorabies viruses and retroviruses, and vectors derived from combinations thereof, such as those derived from plasmid and bacteriophage (such as [lambda] phage) genetic elements, such as cosmids and phagemids. The expression systems may contain control regions that regulate as well as engender expression. Promoters to be used in the present invention may be any promoters as long as they are appropriate for hosts to be used for gene expression. For example, when a host is Escherichia coli, a trp promoter, a lac promoter, a recA promoter, a pL promoter, an lpp promoter, and the like are preferred. When a host is Bacillus subtilis, an SPO1 promoter, an SPO2 promoter, a penP promoter, and the like are preferred. When a host is yeast, a PHO5 promoter, a PGK promoter, a GAP promoter, an ADH promoter, and the like are preferred. When an animal cell is used as a host, examples of promoters for use in this case include an SRa promoter, an SV40 promoter, an LTR promoter, a CMV promoter, and an HSV-TK promoter. Generally, any system or vector that is able to maintain, propagate or express a nucleic acid to produce a polypeptide in a host may be used.

The appropriate nucleic acid sequence may be inserted into an expression system by any variety of well known and routine techniques, such as those set forth in Sambrook et al., supra. Appropriate secretion signals may be incorporated into the CRCMP polypeptide to allow secretion of the translated protein into the lumen of the endoplasmic reticulum, the periplasmic space or the extracellular environment. These signals may be endogenous to the CRCMP polypeptide or they may be heterologous signals. Transformation of the host cells can be carried out according to methods known in the art. For example, the following documents can be referred to: Proc. Natl. Acad. Sci. U.S.A., Vol. 69, 2110 (1972); Gene, Vol. 17, 107 (1982); Molecular & General Genetics, Vol. 168, 111 (1979); Methods in Enzymology, Vol. 194, 182-187 (1991); Proc. Natl. Acad. Sci. U.S.A.), Vol. 75, 1929 (1978); Cell Technology, separate volume 8, New Cell Technology, Experimental Protocol. 263-267 (1995) (issued by Shujunsha); and Virology, Vol. 52, 456 (1973). The thus obtained transformant transformed with an expression vector containing a DNA of the present invention or a gene containing a DNA of the present invention can be cultured according to a method known in the art. For example, when hosts are bacteria of the genus Escherichia, the bacteria are generally cultured at approximately 15°C to 43°C for approximately 3 to 24 hours. If necessary, aeration or agitation can also be added. When hosts are bacteria of the genus Bacillus, the bacteria are generally cultured at approximately 30°C to 40°C for approximately 6 to 24 hours. If necessary, aeration or agitation can also be added. When transformants whose hosts are yeast are cultured, culture is generally carried out at approximately 20°C to 35°C for approximately 24 to 72 hours using media with pH adjusted to be approximately 5 to 8. If necessary, aeration or agitation can also be added. When transformants whose hosts are animal cells are cultured, the cells are generally cultured at approximately 30°C to 40°C for approximately 15 to 60 hours using media with the pH adjusted to be approximately 6 to 8. If necessary, aeration or agitation can also be added.

If a CRCMP polypeptide is to be expressed for use in cell-based screening assays, it is preferred that the polypeptide be produced at the cell surface. In this event, the cells may be harvested prior to use in the screening assay. If the CRCMP polypeptide is secreted into the medium, the medium can be recovered in order to isolate said polypeptide. If produced intracellularly, the cells must first be lysed before the CRCMP polypeptide is recovered.

CRCMP polypeptides can be recovered and purified from recombinant cell cultures or from other biological sources by well known methods including, ammonium sulphate or ethanol precipitation, acid extraction, anion or cation exchange chromatography, phosphocellulose chromatography, affinity chromatography, hydrophobic interaction chromatography, hydroxylapatite chromatography, molecular sieving chromatography, centrifugation methods, electrophoresis methods and lectin chromatography. In one embodiment, a combination of these methods is used. In another embodiment, high performance liquid chromatography is used. In a further embodiment, an antibody (or other affinity reagent such as an Affibody, Nanobody or Unibody) which specifically binds to a CRCMP polypeptide can be used to deplete a sample comprising a CRCMP polypeptide of said polypeptide or to purify said polypeptide.

To separate and purify a polypeptide or a protein of the present invention from the culture products, for example, after culture, microbial bodies or cells are collected by a known method, they are suspended in an appropriate buffer, the microbial bodies or the cells are disrupted by, for example, ultrasonic waves, lysozymes, and/or freeze-thawing, the resultant is then subjected to centrifugation or filtration, and then a crude extract of the protein can be obtained. The buffer may also contain a protein denaturation agent such as urea or guanidine hydrochloride or a surfactant such as Triton X-100(TM). When the protein is secreted in a culture solution, microbial bodies or cells and a supernatant are separated by a known method after the completion of culture and then the supernatant is collected. The protein contained in the thus obtained culture supernatant or the extract can be purified by an appropriate combination of known separation and purification methods. The thus obtained polypeptides (proteins) of the present invention can be converted into salts by a known method or a method according thereto. Conversely, when the polypeptides (proteins) of the present invention are obtained in the form of salts, they can be converted into free proteins or peptides or other salts by a known method or a method according thereto. Moreover, an appropriate protein modification enzyme such as trypsin or chymotrypsin is caused to act on a protein produced by a recombinant before or after purification, so that modification can be arbitrarily added or a polypeptide can be partially removed. The presence of polypeptides (proteins) of the present invention or salts thereof can be measured by various binding assays, enzyme immunoassays using specific antibodies, and the like.

Techniques well known in the art may be used for refolding to regenerate native or active conformations of the CRCMP polypeptides when the polypeptides have been denatured during isolation and or purification. In the context of the present invention, CRCMP polypeptides can be obtained from a biological sample from any source, such as and without limitation, a blood sample or tissue sample, e.g. a colorectal tissue sample.

CRCMP polypeptides may be in the form of "mature proteins" or may be part of larger proteins such as fusion proteins. It is often advantageous to include an additional amino acid sequence which contains secretory or leader sequences, a pre-, pro- or prepro- protein sequence, or a sequence which aids in purification such as an affinity tag, for example, but without limitation, multiple histidine residues, a FLAG tag, HA tag or myc tag.

An additional sequence that may provide stability during recombinant production may also be used. Such sequences may be optionally removed as required by incorporating a cleavable sequence as an additional sequence or part thereof. Thus, a CRCMP polypeptide may be fused to other moieties including other polypeptides or proteins (for example, glutathione S-transferase and protein A). Such a fusion protein can be cleaved using an appropriate protease, and then separated into each protein. Such additional sequences and affinity tags are well known in the art. In addition to the above, features known in the art, such as an enhancer, a splicing signal, a polyA addition signal, a selection marker, and an SV40 replication origin can be added to an expression vector, if desired.

Diagnosis of Colorectal Cancer

In accordance with the present invention, test samples of serum, plasma or urine obtained from a subject suspected of having or known to have colorectal cancer can be used for diagnosis or monitoring. In one embodiment, a change in the abundance of one or more CRCMPs in a test sample relative to a control sample (from a subject or subjects free from colorectal cancer) or a previously determined reference range indicates the presence of colorectal cancer; CRCMPs suitable for this purpose are defined in Tables 1 and 2, as described in detail above. In another embodiment, the relative abundance of one or more CRCMPs in a test sample compared to a control sample or a previously determined reference range indicates a subtype of colorectal cancer (*e.g.*, familial or sporadic colorectal cancer). In yet another embodiment, the relative abundance of one or more CRCMPs in a test sample relative to a control sample or a previously determined reference range indicates the degree or severity of colorectal cancer (e.g., the likelihood for metastasis). In any of the aforesaid methods, detection of one or more CRCMPs as defined in Tables 1 and 2 herein may optionally be combined with detection of one or more additional biomarkers for colorectal cancer. Any suitable method in the art can be employed to measure the level of CRCMPs, including but not limited to the technology described herein in the examples, kinase assays, immunoassays to detect and/or visualize the CRCMPs (*e.g.*, Western blot, immunoprecipitation followed by sodium dodecyl sulfate polyacrylamide gel electrophoresis, immunocytochemistry, etc.). In cases where a CRCMP has a known function, an assay for that function may be used to measure CRCMP expression. In a further embodiment, a change in the abundance of mRNA encoding one or more CRCMPs as defined in Tables 1 and 2 in a test sample relative to a control sample or a previously determined reference range indicates the presence of colorectal cancer. Any suitable hybridization assay can be used to detect CRCMP expression by detecting and/or visualizing mRNA encoding the CRCMP (*e.g.*, Northern assays, dot blots, *in situ* hybridization, etc.).

In another embodiment of the invention, labeled antibodies (or other affinity reagents such as Affibodies, Nanobodies or Unibodies), derivatives and analogs thereof, which specifically bind to a CRCMP can be used for diagnostic purposes to detect, diagnose, or monitor colorectal cancer. Preferably, colorectal cancer is detected in an animal, more preferably in a mammal and most preferably in a human.

Assay Measurement Strategies

Preferred assays are "configured to detect" a particular marker. That an assay is "configured to detect" a marker means that an assay can generate a detectable signal indicative of the presence or amount of a physiologically relevant concentration of a particular marker of interest. Such an assay may, but need not, specifically detect a particular marker (*i.e.*, detect a marker but not some or all related markers). Because an antibody epitope is on the order of 8 amino acids, an immunoassay will detect other polypeptides (*e.g.*, related markers) so long as the other polypeptides contain the epitope(s) necessary to bind to the antibody used in the assay. Such other polypeptides are referred to as being "immunologically detectable" in the assay, and would include various isoforms (*e.g.*, splice variants). In the case of a sandwich immunoassay, related markers must contain at least the two epitopes bound by the antibody used in the assay in order to be detected. Taking $BNP_{79-108}$ as an example, an assay configured to detect this marker may also detect

BNP$_{77-108}$ or BNP$_{1-108}$, as such molecules may also contain the epitope(s) present on BNP$_{79-108}$ to which the assay antibody binds. However, such assays may also be configured to be "sensitive" to loss of a particular epitope, *e.g.*, at the amino and/or carboxyl terminus of a particular polypeptide of interest as described in US2005/0148024, which is hereby incorporated by reference in its entirety. As described therein, an antibody may be selected that would bind to the amino terminus of BNP$_{79-108}$ such that it does not bind to BNP$_{77-108}$. Similar assays that bind BNP$_{3-108}$ and that are "sensitive" to loss of a particular epitope, *e.g.*, at the amino and/or carboxyl terminus are also described therein.

Numerous methods and devices are well known to the skilled artisan for the detection and analysis of the markers of the instant invention. With regard to polypeptides or proteins in patient test samples, immunoassay devices and methods are often used. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, each of which is hereby incorporated by reference in its entirety, including all tables, figures and claims. These devices and methods can utilize labeled molecules in various sandwich, competitive, or non-competitive assay formats, to generate a signal that is related to the presence or amount of an analyte of interest. Additionally, certain methods and devices, such as biosensors and optical immunoassays, may be employed to determine the presence or amount of analytes without the need for a labeled molecule. *See, e.g.,* U.S. Patents 5,631,171; and 5,955,377, each of which is hereby incorporated by reference in its entirety, including all tables, figures and claims. One skilled in the art also recognizes that robotic instrumentation including but not limited to Beckman Access, Abbott AxSym, Roche ElecSys, Dade Behring Stratus systems are among the immunoassay analyzers that are capable of performing the immunoassays taught herein.

Preferably the markers are analyzed using an immunoassay, and most preferably sandwich immunoassay, although other methods are well known to those skilled in the art (for example, the measurement of marker RNA levels). The presence or amount of a marker is generally determined using antibodies (or other affinity reagents such as Affibodies, Nanobodies or Unibodies) specific for each marker and detecting specific binding. Any suitable immunoassay may be utilized, for example, enzyme-linked immunoassays (ELISA), radioimmunoassays (RIAs), competitive binding assays, and the like. Specific immunological binding of the affinity reagent to the marker can be detected directly or indirectly. Direct labels include fluorescent or luminescent tags, metals, dyes, radionuclides, and the like, attached to the affinity reagent. Indirect labels include various enzymes well known in the art, such as alkaline phosphatase, horseradish peroxidase and the like.

The use of immobilized antibodies (or other affinity reagents such as Affibodies, Nanobodies or Unibodies) specific for the markers is also contemplated by the present invention. The affinity reagents could be immobilized onto a variety of solid supports, such as magnetic or chromatographic matrix particles, the surface of an assay place (such as microtiter wells), pieces of a solid substrate material or membrane (such as plastic, nylon, paper), and the like. An assay strip could be prepared by coating the affinity reagent or a plurality of affinity reagents in an array on solid support. This strip could then be dipped into the test sample and then processed quickly through washes and detection steps to generate a measurable signal, such as a colored spot.

For separate or sequential assay of markers, suitable apparatuses include clinical laboratory analyzers such as the ElecSys (Roche), the AxSym (Abbott), the Access (Beckman), the ADVIA® CENTAUR® (Bayer) immunoassay systems, the NICHOLS ADVANTAGE® (Nichols Institute) immunoassay system, etc. Preferred apparatuses perform simultaneous assays of a plurality of markers using a single test device. Particularly useful physical formats comprise surfaces having a plurality of discrete, adressable locations for the detection of a plurality of different analytes. Such formats include protein microarrays, or "protein chips" (*see, e.g.,* Ng and Ilag, J. Cell Mol. Med. 6: 329-340 (2002)) and certain capillary devices (*see, e.g.,* U.S. Patent No. 6,019,944). In these embodiments, each discrete surface location may comprise antibodies to immobilize one or more analyte(s) (e.g., a marker) for detection at each location. Surfaces may alternatively comprise one or more discrete particles (*e.g.*, microparticles or nanoparticles) immobilized at discrete locations of a surface, where the microparticles comprise antibodies to immobilize one analyte (*e.g.*, a marker) for detection.

Preferred assay devices of the present invention will comprise, for one or more assays, a first antibody (or other affinity reagent such as an Affibody, Nanobody or Unibody) conjugated to a solid phase and a second antibody (or other affinity reagent such as an Affibody, Nanobody or Unibody) conjugated to a signal development element. Such assay devices are configured to perform a sandwich immunoassay for one or more analytes. These assay devices will preferably further comprise a sample application zone, and a flow path from the sample application zone to a second device region comprising the first antibody (or other affinity reagent such as an Affibody, Nanobody or Unibody) conjugated to a solid phase.

Flow of a sample along the flow path may be driven passively (*e.g.*, by capillary, hydrostatic, or other forces that do not require further manipulation of the device once sample is applied), actively (*e.g.*, by application of force generated via mechanical pumps, electroosmotic pumps, centrifugal force, increased air pressure, *etc.*), or by a combination of active and passive driving forces. Most preferably, sample applied to the sample application zone will contact both a first antibody (or other affinity reagent such as an Affibody, Nanobody or Unibody) conjugated to a solid phase and a second antibody (or other affinity reagent such as an Affibody, Nanobody or Unibody) conjugated to a signal development element along the flow path (sandwich assay format). Additional elements, such as filters to separate plasma or serum from blood, mixing chambers, *etc.,* may be included as required by the artisan. Exemplary devices are described in Chapter 41, entitled "Near Patient Tests: Triage® Cardiac System," in The Immunoassay Handbook, 2nd ed., David Wild, ed., Nature Publishing Group, 2001, which is hereby incorporated by reference in its entirety.

A panel consisting of the markers referenced above may be constructed to provide relevant information related to differential diagnosis. Such a panel may be constucted using 1, 2, 3, 4, 5, 6, 7, 8, 9, 10, 12, 15, 20 or more individual markers. The analysis of a single marker or subsets of markers comprising a larger panel of markers could be carried out by one skilled in the art to optimize clinical sensitivity or specificity in various clinical settings. These include, but are not limited to ambulatory, urgent care, critical care, intensive care, monitoring unit, inpatient, outpatient, physician office, medical clinic, and health screening settings. Furthermore, one skilled in the art can use a single marker or a subset of markers comprising a larger panel of markers in combination with an adjustment of the diagnostic threshold in each of the aforementioned settings to optimize clinical sensitivity and specificity. The clinical sensitivity of an assay is defined as the percentage of those with the disease that the assay correctly predicts, and the specificity of an assay is defined as the percentage of those without the disease that the assay correctly predicts (Tietz Textbook of Clinical Chemistry, 2nd edition, Carl Burtis and Edward Ashwood eds., W.B. Saunders and Company, p. 496).

The analysis of markers could be carried out in a variety of physical formats as well. For example, the use of microtiter plates or automation could be used to facilitate the processing of large numbers of test samples. Alternatively, single sample formats could be developed to facilitate immediate treatment and diagnosis in a timely fashion, for example, in ambulatory transport or emergency room settings.

In another embodiment, the present invention provides a kit for the analysis of markers. Such a kit preferably comprises devices and reagents for the analysis of at least one test sample and instructions for performing the assay. Optionally the kits may contain one or more means for using information obtained from immunoassays performed for a marker panel to rule in or out certain diagnoses. Other measurement strategies applicable to the methods described herein include chromatography (*e.g.*, HPLC), mass spectrometry, receptor-based assays, and combinations of the foregoing.

Production of Affinity Reagents to the CRCMPs

According to those in the art, there are three main types of affinity reagent - monoclonal antibodies, phage display antibodies and small molecules such as Affibodies, Domain Antibodies (dAbs), Nanobodies or Unibodies. In general in applications according to the present invention where the use of antibodies is stated, other affinity reagents (e.g. Affibodies, domain antibodies, Nanobodies or Unibodies) may be employed.

Production of Antibodies to the CRCMPs

According to the invention a CRCMP, a CRCMP analog, a CRCMP-related protein or a fragment or derivative of any of the foregoing may be used as an immunogen to generate antibodies which immunospecifically bind such an immunogen. Such immunogens can be isolated by any convenient means, including the methods described above. The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994) J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH2 domains; (iv) a Fv fragment consisting of the VL and VH domains of a single arm of an antibody, (v) a dAb fragment (Ward et al., (1989) Nature 341: 544-546), which consists of a VH domain; and (vi) an isolated complementarity determining region (CDR). Single chain antibodies are also included by reference in the term "antibody". Antibodies of the invention include, but are not limited to polyclonal, monoclonal, bispecific, humanized or chimeric antibodies, single chain antibodies, Fab fragments and F(ab')$_2$ fragments, fragments produced by a Fab expression library, anti-idiotypic (anti-Id) antibodies, and epitope-binding fragments of any of the above. The immunoglobulin molecules of the invention can be of any class (*e.g.* IgG, IgE, IgM, IgD and IgA) or subclass of immunoglobulin molecule.

The term "specifically binds" (or "immunospecifically binds") is not intended to indicate that an antibody binds exclusively to its intended target. Rather, an antibody "specifically binds" if its affinity for its intended target is about 5-fold greater when compared to its affinity for a non-target molecule. Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, specific binding between an antibody or other binding agent and an antigen means a binding affinity of at least $10^6$ M$^{-1}$. Preferred antibodies bind with affinities of at least about $10^7$ M$^{-1}$, and preferably between about $10^8$ M$^{-1}$ to about $10^9$ M$^{-1}$, about $10^9$ M$^{-1}$ to about $10^{10}$ M$^{-1}$, or about $10^{10}$ M$^{-1}$ to about $10^{11}$ M$^{-1}$.

Affinity is calculated as $K_d = k_{off}/k_{on}$ ($k_{off}$ is the dissociation rate constant, $k_{on}$ is the association rate constant and $K_d$ is the equilibrium constant. Affinity can be determined at equilibrium by measuring the fraction bound (r) of labeled ligand at various concentrations (c). The data are graphed using the Scatchard equation: r/c = K(n-r):

where

r = moles of bound ligand/mole of receptor at equilibrium;

c = free ligand concentration at equilibrium;

K = equilibrium association constant; and

n = number of ligand binding sites per receptor molecule

By graphical analysis, r/c is plotted on the Y-axis versus r on the X-axis thus producing a Scatchard plot. The affinity is the negative slope of the line. $k_{off}$ can be determined by competing bound labeled ligand with unlabeled excess ligand (see, e.g., U.S. Pat No. 6,316,409). The affinity of a targeting agent for its target molecule is preferably at least about $1 \times 10^{-6}$ moles/liter, is more preferably at least about $1 \times 10^{-7}$ moles/liter, is even more preferably at least about $1 \times 10^{-8}$ moles/liter, is yet even more preferably at least about $1 \times 10^{-9}$ moles/liter, and is most preferably at least about $1 \times 10^{-10}$ moles/liter. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

In one embodiment, antibodies that recognize gene products of genes encoding CRCMPs are publicly available. In another embodiment, methods known to those skilled in the art are used to produce antibodies that recognize a CRCMP, a CRCMP analog, a CRCMP-related polypeptide, or a fragment or derivative of any of the foregoing. One skilled in the art will recognize that many procedures are available for the production of antibodies, for example, as described in Antibodies, A Laboratory Manual, Ed Harlow and David Lane, Cold Spring Harbor Laboratory (1988), Cold Spring Harbor, N.Y. One skilled in the art will also appreciate that binding fragments or Fab fragments which mimic antibodies can also be prepared from genetic information by various procedures (Antibody Engineering: A Practical Approach (Borrebaeck, C., ed.), 1995, Oxford University Press, Oxford; J. Immunol. 149, 3914-3920 (1992)).

In one embodiment of the invention, antibodies to a specific domain of a CRCMP are produced. In a specific embodiment, hydrophilic fragments of a CRCMP are used as immunogens for antibody production.

In the production of antibodies, screening for the desired antibody can be accomplished by techniques known in the art, *e.g.* ELISA (enzyme-linked immunosorbent assay). For example, to select antibodies which recognize a specific domain of a CRCMP, one may assay generated hybridomas for a product which binds to a CRCMP fragment containing such domain. For selection of an antibody that specifically binds a first CRCMP homolog but which does not specifically bind to (or binds less avidly to) a second CRCMP homolog, one can select on the basis of positive binding to the first CRCMP homolog and a lack of binding to (or reduced binding to) the second CRCMP homolog. Similarly, for selection of an antibody that specifically binds a CRCMP but which does not specifically bind to (or binds less avidly to) a different isoform of the same protein (such as a different glycoform having the same core peptide as the CRCMP), one can select on the basis of positive binding to the CRCMP and a lack of binding to (or reduced binding to) the different isoform (*e.g.* a different glycoform). Thus, the present invention provides antibodies (preferably monoclonal antibodies) that bind with greater affinity (preferably at least 2-fold, more preferably at least 5-fold, still more preferably at least 10-fold greater affinity) to the CRCMPs than to a different isoform or isoforms (*e.g.* glycoforms) of the CRCMPs.

Polyclonal antibodies which may be used in the methods of the invention are heterogeneous populations of antibody molecules derived from the sera of immunized animals. Unfractionated immune serum can also be used. Various procedures known in the art may be used for the production of polyclonal antibodies to a CRCMP, a fragment of a CRCMP, a CRCMP-related polypeptide, or a fragment of a CRCMP-related polypeptide. For example, one way is to purify polypeptides of interest or to synthesize the polypeptides of interest using, *e.g.,* solid phase peptide synthesis methods well known in the art. *See, e.g.,* Guide to Proteins Purification, Murray P. Deutcher, ed., Meth. Enzymol. Vol 182 (1990); Solid Phase Peptide Synthesis, Greg B. Fields ed., Meth. Enzymol. Vol 289 (1997); Kiso et al., Chem. Pharm. Bull. (Tokyo) 38: 1192-99, 1990; Mostafavi et al., Biomed. Pept. Proteins Nucleic Acids 1: 255-60, 1995; Fujiwara et al., Chem. Pharm. Bull. (Tokyo) 44: 1326-31, 1996. The selected polypeptides may then be used to immunize by injection various host animals, including but not limited to rabbits, mice, rats, etc., to generate polyclonal or monoclonal antibodies. The Preferred Technology described herein provides isolated CRCMPs suitable for such immunization. If a CRCMP is purified by gel electrophoresis, the CRCMP can be used for immunization with or without prior extraction from the polyacrylamide gel. Various adjuvants (i.e. immunostimulants) may be used to enhance the immunological response, depending on the host species, including, but not limited to, complete or incomplete Freund's adjuvant, a mineral gel such as aluminum hydroxide, surface active substance such as lysolecithin, pluronic polyol, a polyanion, a peptide, an oil emulsion, keyhole limpet hemocyanin, dinitrophenol, and an adjuvant such as BCG (bacille Calmette-Guerin) or corynebacterium parvum. Additional adjuvants are also well known in the art.

For preparation of monoclonal antibodies (mAbs) directed toward a CRCMP, a fragment of a CRCMP, a CRCMP-related polypeptide, or a fragment of a CRCMP-related polypeptide, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in Monoclonal Antibodies and Cancer Therapy, Alan R. Liss, Inc., pp. 77-96). Such antibodies may be of any immunoglobulin class including IgG, IgM, IgE, IgA, IgD and any subclass thereof. The hybridoma producing the mAbs of the invention may be cultivated *in vitro* or *in vivo.* In an additional embodiment of the invention, monoclonal antibodies can be produced in germ-free animals utilizing known technology (PCT/US90/02545, incorporated herein by reference).

The monoclonal antibodies include but are not limited to human monoclonal antibodies and chimeric monoclonal antibodies (*e.g.* human-mouse chimeras). A chimeric antibody is a molecule in which different portions are derived from different animal species, such as those having a human immunoglobulin constant region and a variable region derived from a murine mAb. (See, *e.g.* Cabilly et al., U.S. Patent No. 4,816,567; and Boss et al., U.S. Patent No. 4,816397, which are incorporated herein by reference in their entirety.) Humanized antibodies are antibody molecules from non-human species having one or more complementarity determining regions (CDRs) from the non-human species and a framework region from a human immunoglobulin molecule. (See, *e.g.* Queen, U.S. Patent No. 5,585,089, which is incorporated herein by reference in its entirety.)

Chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314: 446-449; and Shaw et al., 1988, J. Natl. Cancer Inst. 80:1553-1559; Morrison, 1985, Science 229:1202-1207; Oi et al., 1986, Bio/Techniques 4:214; U.S. Patent 5,225,539; Jones et al., 1986, Nature 321:552-525; Verhoeyan et al. (1988) Science 239:1534; and Beidler et al., 1988, J. Immunol. 141:4053-4060.

Completely human antibodies are particularly desirable for therapeutic treatment of human subjects. Such antibodies can be produced using transgenic mice which are incapable of expressing endogenous immunoglobulin heavy and light chain genes, but which can express human heavy and light chain genes. The transgenic mice are immunized in the normal fashion with a selected antigen, *e.g.* all or a portion of a CRCMP. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, *see, e.g.* U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA) and Genpharm (San Jose, CA) can be engaged to provide human antibodies directed against a selected antigen using technology similar to that described above.

Completely human antibodies which recognize a selected epitope can be generated using a technique referred to as "guided selection". In this approach a selected non-human monoclonal antibody, *e.g.* a mouse antibody, is used to guide the selection of a completely human antibody recognizing the same epitope. (Jespers et al. (1994) Bio/technology 12:899-903).

The antibodies of the present invention can also be generated by the use of phage display technology to produce and screen libraries of polypeptides for binding to a selected target. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098, which is hereby incorporated in its entirety, including all tables, figures, and claims. In particular, such phage can be utilized to display antigen binding domains expressed from a repertoire or combinatorial antibody library (*e.g.* human or murine). Phage expressing an antigen binding domain that binds the antigen of interest can be selected or identified with antigen, *e.g.* using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Phage display methods that can be used to make the antibodies of the present invention include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT Application No. PCT/GB91/01134; PCT Publications WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108; each of which is incorporated herein by reference in its entirety.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g. as described in detail below. For example, techniques to recombinantly produce Fab, Fab' and F(ab')$_2$ fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240: 1041-1043 (1988) (said references incorporated by reference in their entireties).

Examples of techniques which can be used to produce single-chain Fvs and antibodies include those described in U.S. Patents 4,946,778 and 5,258,498; Huston et al., Methods in Enzymology 203:46-88 (1991); Shu et al., PNAS 90:7995-7999 (1993); and Skerra et al., Science 240:1038-1040 (1988).

The invention further provides for the use of bispecific antibodies, which can be made by methods known in the art. Traditional production of full length bispecific antibodies is based on the coexpression of two immunoglobulin heavy chain-light chain pairs, where the two chains have different specificities (Milstein et al., 1983, Nature 305:537-539). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, published 13 May 1993, and in Traunecker et al., 1991, EMBO J. 10:3655-3659.

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690 published March 3, 1994. For further details for generating bispecific antibodies see, for example, Suresh et al., Methods in Enzymology, 1986, 121:210.

The invention provides functionally active fragments, derivatives or analogs of the anti-CRCMP immunoglobulin molecules. Functionally active means that the fragment, derivative or analog is able to elicit anti-anti-idiotype antibodies (*i.e.*, tertiary antibodies) that recognize the same antigen that is recognized by the antibody from which the fragment, derivative or analog is derived. Specifically, in a preferred embodiment the antigenicity of the idiotype of the immunoglobulin molecule may be enhanced by deletion of framework and CDR sequences that are C-terminal to the CDR sequence that specifically recognizes the antigen. To determine which CDR sequences bind the antigen, synthetic peptides containing the CDR sequences can be used in binding assays with the antigen by any binding assay method known in the art.

The present invention provides antibody fragments such as, but not limited to, F(ab')$_2$ fragments and Fab fragments. Antibody fragments which recognize specific epitopes may be generated by known techniques. F(ab')$_2$ fragments consist of the variable region, the light chain constant region and the CH1 domain of the heavy chain and are generated by pepsin digestion of the antibody molecule. Fab fragments are generated by reducing the disulfide bridges of the F(ab')$_2$ fragments. The invention also provides heavy chain and light chain dimers of the antibodies of the invention, or any minimal fragment thereof such as Fvs or single chain antibodies (SCAs) (*e.g.* as described in U.S. Patent 4,946,778; Bird, 1988, Science 242:423-42; Huston et al., 1988, Proc. Natl. Acad. Sci. USA 85:5879-5883; and Ward et al., 1989, Nature 334:544-54), or any other molecule with the same specificity as the antibody of the invention. Single chain antibodies are formed by linking the heavy and light chain fragments of the Fv region via an amino acid bridge, resulting in a single chain polypeptide. Techniques for the assembly of functional Fv fragments in *E. coli* may be used (Skerra et al., 1988, Science 242:1038-1041).

In other embodiments, the invention provides fusion proteins of the immunoglobulins of the invention (or functionally active fragments thereof), for example in which the immunoglobulin is fused via a covalent bond (*e.g.* a peptide bond), at either the N-terminus or the C-terminus to an amino acid sequence of another protein (or portion thereof, preferably at least 10, 20 or 50 amino acid portion of the protein) that is not the immunoglobulin. Preferably the immunoglobulin, or fragment thereof, is covalently linked to the other protein at the N-terminus of the constant domain. As stated above, such fusion proteins may facilitate purification, increase half-life *in vivo,* and enhance the delivery of an antigen across an epithelial barrier to the immune system.

The immunoglobulins of the invention include analogs and derivatives that are modified, *i.e.,* by the covalent attachment of any type of molecule as long as such covalent attachment does not impair immunospecific binding. For example, but not by way of limitation, the derivatives and analogs of the immunoglobulins include those that have been further modified, *e.g.* by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, etc. Additionally, the analog or derivative may contain one or more non-classical amino acids.

The foregoing antibodies can be used in methods known in the art relating to the localization and activity of the CRCMPs, *e.g.* for imaging these proteins, measuring levels thereof in appropriate physiological samples, in diagnostic methods, etc.

Production of Affibodies to the CRCMPs

Affibody molecules represent a new class of affinity proteins based on a 58-amino acid residue protein domain, derived from one of the IgG-binding domains of staphylococcal protein A. This three helix bundle domain has been used as a scaffold for the construction of combinatorial phagemid libraries, from which Affibody variants that target the desired molecules can be selected using phage display technology (Nord K, Gunneriusson E, Ringdahl J, Stahl S, Uhlen M, Nygren PA, Binding proteins selected from combinatorial libraries of an $\alpha$-helical bacterial receptor domain, Nat Biotechnol 1997;15:772-7. Ronmark J, Gronlund H, Uhlen M, Nygren PA, Human immunoglobulin A (IgA)-specific ligands from combinatorial engineering of protein A, Eur J Biochem 2002;269:2647-55.). The simple, robust structure of Affibody molecules in combination with their low molecular weight (6 kDa), make them suitable for a wide variety of applications, for instance, as detection reagents (Ronmark J, Hansson M, Nguyen T, et al, Construction and characterization of affibody-Fc chimeras produced in Escherichia coli, J Immunol Methods 2002;261:199-211) and to inhibit receptor interactions (Sandstorm K, Xu Z, Forsberg G, Nygren PA, Inhibition of the CD28-CD80 co-stimulation signal by a CD28-binding Affibody ligand developed by combinatorial protein engineering, Protein Eng 2003;16:691-7). Further details of Affibodies and methods of production thereof may be obtained by reference to US Patent No 5831012 which is herein incorporated by reference in its entirety.

Labelled Affibodies may also be useful in imaging applications for determining abundance of Isoforms.

Production of Domain Antibodies to the CRCMPs

Domain Antibodies (dAbs) are the smallest functional binding units of antibodies, corresponding to the variable regions of either the heavy ($V_H$) or light ($V_L$) chains of human antibodies. Domain Antibodies have a molecular weight of approximately 13 kDa. Domantis has developed a series of large and highly functional libraries of fully human $V_H$ and $V_L$ dAbs (more than ten billion different sequences in each library), and uses these libraries to select dAbs that are specific to therapeutic targets. In contrast to many conventional antibodies, Domain Antibodies are well expressed in bacterial, yeast, and mammalian cell systems. Further details of domain antibodies and methods of production thereof may be obtained by reference to US Patent 6,291,158; 6,582,915; 6,593,081; 6,172,197; 6,696,245; US Serial No. 2004/0110941; European patent application No. 1433846 and European Patents 0368684 & 0616640; WO05/035572, WO04/101790, WO04/081026, WO04/058821, WO04/003019 and WO03/002609, each of which is herein incorporated by reference in its entirety.

Production of Nanobodies to the CRCMPs

Nanobodies are antibody-derived therapeutic proteins that contain the unique structural and functional properties of naturally-occurring heavy-chain antibodies. These heavy-chain antibodies contain a single variable domain (VHH) and two constant domains ($C_H2$ and $C_H3$). Importantly, the cloned and isolated VHH domain is a perfectly stable polypeptide harbouring the full antigen-binding capacity of the original heavy-chain antibody. Nanobodies have a high homology with the VH domains of human antibodies and can be further humanized without any loss of activity. Importantly, Nanobodies have a low immunogenic potential, which has been confirmed in primate studies with Nanobody lead compounds.

Nanobodies combine the advantages of conventional antibodies with important features of small molecule drugs. Like conventional antibodies, Nanobodies show high target specificity, high affinity for their target and low inherent toxicity. However, like small molecule drugs they can inhibit enzymes and readily access receptor clefts. Furthermore, Nanobodies are extremely stable, can be administered by means other than injection (see e.g. WO 04/041867, which is herein incorporated by reference in its entirety) and are easy to manufacture. Other advantages of Nanobodies include recognising uncommon or hidden epitopes as a result of their small size, binding into cavities or active sites of protein targets with high affinity and selectivity due to their unique 3-dimensional, drug format flexibility, tailoring of half-life and ease and speed of drug discovery.

Nanobodies are encoded by single genes and are efficiently produced in almost all prokaryotic and eukaryotic hosts e.g. *E. coli* (see e.g. US 6,765,087, which is herein incorporated by reference in its entirety), moulds (for example *Aspergillus* or *Trichoderma*) and yeast (for example *Saccharomyces, Kluyveromyces*, *Hansenula* or *Pichia)* (see e.g. US 6,838,254, which is herein incorporated by reference in its entirety). The production process is scalable and multi-kilogram quantities of Nanobodies have been produced. Because Nanobodies exhibit a superior stability compared with conventional antibodies, they can be formulated as a long shelf-life, ready-to-use solution.

The Nanoclone method (see e.g. WO 06/079372, which is herein incorporated by reference in its entirety) is a proprietary method for generating Nanobodies against a desired target, based on automated high-throughout selection of B-cells.

Production of Unibodies to the CRCMPs

UniBody is a new proprietary antibody technology that creates a stable, smaller antibody format with an anticipated longer therapeutic window than current small antibody formats. IgG4 antibodies are considered inert and thus do not interact with the immune system. Genmab modified fully human IgG4 antibodies by eliminating the hinge region of the antibody. Unlike the full size IgG4 antibody, the half molecule fragment is very stable and is termed a UniBody. Halving the IgG4 molecule left only one area on the UniBody that can bind to disease targets and the UniBody therefore binds univalently to only one site on target cells. This univalent binding does not stimulate cancer cells to grow like bivalent antibodies might and opens the door for treatment of some types of cancer which ordinary antibodies cannot treat.

The UniBody is about half the size of a regular IgG4 antibody. This small size can be a great benefit when treating some forms of cancer, allowing for better distribution of the molecule over larger solid tumors and potentially increasing efficacy.

Fabs typically do not have a very long half-life. UniBodies, however, were cleared at a similar rate to whole IgG4 antibodies and were able to bind as well as whole antibodies and antibody fragments in pre-clinical studies. Other antibodies primarily work by killing the targeted cells whereas UniBodies only inhibit or silence the cells.

Expression of Affinity Reagents

Expression of Antibodies

The antibodies of the invention can be produced by any method known in the art for the synthesis of antibodies, in particular, by chemical synthesis or by recombinant expression, and are preferably produced by recombinant expression techniques.

Recombinant expression of antibodies, or fragments, derivatives or analogs thereof, requires construction of a nucleic acid that encodes the antibody. If the nucleotide sequence of the antibody is known, a nucleic acid encoding the antibody may be assembled from chemically synthesized oligonucleotides (e.g. as described in Kutmeier et al., 1994, BioTechniques 17:242), which, briefly, involves the synthesis of overlapping oligonucleotides containing portions of the sequence encoding antibody, annealing and ligation of those oligonucleotides, and then amplification of the ligated oligonucleotides by PCR.

Alternatively, the nucleic acid encoding the antibody may be obtained by cloning the antibody. If a clone containing the nucleic acid encoding the particular antibody is not available, but the sequence of the antibody molecule is known, a nucleic acid encoding the antibody may be obtained from a suitable source (e.g. an antibody cDNA library, or cDNA library generated from any tissue or cells expressing the antibody) by PCR amplification using synthetic primers hybridizable to the 3' and 5' ends of the sequence or by cloning using an oligonucleotide probe specific for the particular gene sequence.

If an antibody molecule that specifically recognizes a particular antigen is not available (or a source for a cDNA library for cloning a nucleic acid encoding such an antibody), antibodies specific for a particular antigen may be generated by any method known in the art, for example, by immunizing an animal, such as a rabbit, to generate polyclonal antibodies or, more preferably, by generating monoclonal antibodies. Alternatively, a clone encoding at least the Fab portion of the antibody may be obtained by screening Fab expression libraries (e.g. as described in Huse et al., 1989, Science 246: 1275-1281) for clones of Fab fragments that bind the specific antigen or by screening antibody libraries (See, e.g. Clackson et al., 1991, Nature 352:624; Hane et al., 1997 Proc. Natl. Acad. Sci. USA 94:4937).

Once a nucleic acid encoding at least the variable domain of the antibody molecule is obtained, it may be introduced into a vector containing the nucleotide sequence encoding the constant region of the antibody molecule (see, e.g. PCT Publication WO 86/05807; PCT Publication WO 89/01036; and U.S. Patent No. 5,122,464). Vectors containing the complete light or heavy chain for co-expression with the nucleic acid to allow the expression of a complete antibody molecule are also available. Then, the nucleic acid encoding the antibody can be used to introduce the nucleotide substitution(s) or deletion(s) necessary to substitute (or delete) the one or more variable region cysteine residues participating in an intrachain disulfide bond with an amino acid residue that does not contain a sulfhydyl group. Such modifications can be carried out by any method known in the art for the introduction of specific mutations or deletions in a nucleotide sequence, for example, but not limited to, chemical mutagenesis, in vitro site directed mutagenesis (Hutchinson et al., 1978, J. Biol. Chem. 253:6551), PCT based methods, etc.

In addition, techniques developed for the production of "chimeric antibodies" (Morrison et al., 1984, Proc. Natl. Acad. Sci. 81:851-855; Neuberger et al., 1984, Nature 312:604-608; Takeda et al., 1985, Nature 314:452-454) by splicing genes from a mouse antibody molecule of appropriate antigen specificity together with genes from a human antibody molecule of appropriate biological activity can be used. As described *supra,* a chimeric antibody is a molecule in which different portions are derived from different animal species, such as those having a variable region derived from a murine mAb and a human antibody constant region, e.g. humanized antibodies.

Once a nucleic acid encoding an antibody molecule of the invention has been obtained, the vector for the production of the antibody molecule may be produced by recombinant DNA technology using techniques well known in the art. Thus, methods for preparing the proteins of the invention by expressing nucleic acid containing the antibody molecule sequences are described herein. Methods which are well known to those skilled in the art can be used to construct expression vectors containing an antibody molecule coding sequences and appropriate transcriptional and translational control signals. These methods include, for example, *in vitro* recombinant DNA techniques, synthetic techniques, and *in vivo* genetic recombination. See, for example, the techniques described in Sambrook et al. (1990, Molecular Cloning, A Laboratory Manual, 2nd Ed., Cold Spring Harbor Laboratory, Cold Spring Harbor, NY) and Ausubel et al. (eds., 1998, Current Protocols in Molecular Biology, John Wiley & Sons, NY).

The expression vector is transferred to a host cell by conventional techniques and the transfected cells are then cultured by conventional techniques to produce an antibody of the invention.

The host cells used to express a recombinant antibody of the invention may be either bacterial cells such as *Escherichia coli,* or, preferably, eukaryotic cells, especially for the expression of whole recombinant antibody molecule. In particular, mammalian cells such as Chinese hamster ovary cells (CHO), in conjunction with a vector such as the major intermediate early gene promoter element from human cytomegalovirus are an effective expression system for antibodies (Foecking et al., 1986, Gene 45:101; Cockett et al., 1990, Bio/Technology 8:2).

A variety of host-expression vector systems may be utilized to express an antibody molecule of the invention. Such host-expression systems represent vehicles by which the coding sequences of interest may be produced and subsequently purified, but also represent cells which may, when transformed or transfected with the appropriate nucleotide coding sequences, express the antibody molecule of the invention *in situ.* These include but are not limited to microorganisms such as bacteria (*e.g. E. coli, B. subtilis*) transformed with recombinant bacteriophage DNA, plasmid DNA or cosmid DNA expression vectors containing antibody coding sequences; yeast (*e.g. Saccharomyces, Pichia*) transformed with recombinant yeast expression vectors containing antibody coding sequences; insect cell systems infected with recombinant virus expression vectors (*e.g.* baculovirus) containing the antibody coding sequences; plant cell systems infected with recombinant virus expression vectors (*e.g.* cauliflower mosaic virus, CaMV; tobacco mosaic virus, TMV) or transformed with recombinant plasmid expression vectors (*e.g.* Ti plasmid) containing antibody coding sequences; or mammalian cell systems (*e.g.* COS, CHO, BHK, 293, 3T3 cells) harboring recombinant expression constructs containing promoters derived from the genome of mammalian cells (*e.g.* metallothionein promoter) or from mammalian viruses (*e.g.* the adenovirus late promoter; the vaccinia virus 7.5K promoter).

In bacterial systems, a number of expression vectors may be advantageously selected depending upon the use intended for the antibody molecule being expressed. For example, when a large quantity of such a protein is to be produced, for the generation of pharmaceutical compositions comprising an antibody molecule, vectors which direct the expression of high levels of fusion protein products that are readily purified may be desirable. Such vectors include, but are not limited, to the *E. coli* expression vector pUR278 (Ruther et al., 1983, EMBO J. 2:1791), in which the antibody coding sequence may be ligated individually into the vector in frame with the *lac Z* coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter). In mammalian host cells, a number of viral-based expression systems (e.g. an adenovirus expression system) may be utilized.

As discussed above, a host cell strain may be chosen which modulates the expression of the inserted sequences, or modifies and processes the gene product in the specific fashion desired. Such modifications (e.g. glycosylation) and processing (e.g. cleavage) of protein products may be important for the function of the protein.

For long-term, high-yield production of recombinant antibodies, stable expression is preferred. For example, cell lines that stably express an antibody of interest can be produced by transfecting the cells with an expression vector comprising the nucleotide sequence of the antibody and the nucleotide sequence of a selectable (e.g. neomycin or hygromycin), and selecting for expression of the selectable marker. Such engineered cell lines may be particularly useful in screening and evaluation of compounds that interact directly or indirectly with the antibody molecule.

The expression levels of the antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, Nature 322:52; Kohler, 1980, Proc. Natl. Acad. Sci. USA 77:2197). The coding sequences for the heavy and light chains may comprise cDNA or genomic DNA.

Once the antibody molecule of the invention has been recombinantly expressed, it may be purified by any method known in the art for purification of an antibody molecule, for example, by chromatography (e.g. ion exchange chromatography, affinity chromatography such as with protein A or specific antigen, and sizing column chromatography), centrifugation, differential solubility, or by any other standard technique for the purification of proteins.

Alternatively, any fusion protein may be readily purified by utilizing an antibody specific for the fusion protein being expressed. For example, a system described by Janknecht et al. allows for the ready purification of non-denatured fusion proteins expressed in human cell lines (Janknecht et al., 1991, Proc. Natl. Acad. Sci. USA 88:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with recombinant vaccinia virus are loaded onto $Ni^{2+}$ nitriloacetic acid-agarose columns and histidine-tagged proteins are selectively eluted with imidazole-containing buffers.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) is present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (*e.g.*, in sandwich assays) may interfere with one another sterically, *etc.*, assay performance of an antibody may be a more important measure than absolute affinity and specificity of an antibody.

Those skilled in the art will recognize that many approaches can be taken in producing antibodies or binding fragments and screening and selecting for affinity and specificity for the various polypeptides, but these approaches do not change the scope of the invention.

For therapeutic applications, antibodies (particularly monoclonal antibodies) may suitably be human or humanized animal (e.g. mouse) antibodies. Animal antibodies may be raised in animals using the human protein (e.g. a CRCMP) as immunogen. Humanisation typically involves grafting CDRs identified thereby into human framework regions. Normally some subsequent retromutation to optimize the conformation of chains is required. Such processes are known to persons skilled in the art.

Expression of Affibodies

The construction of affibodies has been described elsewhere (Ronnmark J, Gronlund H, Uhle' n, M., Nygren P.A°, Human immunoglobulin A (IgA)-specific ligands from combinatorial engineering of protein A, 2002, Eur. J. Biochem. 269, 2647-2655.), including the construction of affibody phage display libraries (Nord, K., Nilsson, J., Nilsson, B., Uhle' n, M. & Nygren, P.A°, A combinatorial library of an a-helical bacterial receptor domain, 1995, Protein Eng. 8, 601-608. Nord, K., Gunneriusson, E., Ringdahl, J., Sta° hl, S., Uhle' n, M. & Nygren, P.A°, Binding proteins selected from combinatorial libraries of an a-helical bacterial receptor domain, 1997, Nat. Biotechnol.15, 772-777.)

The biosensor analyses to investigate the optimal affibody variants using biosensor binding studies has also been described elsewhere (Ronnmark J, Gronlund H, Uhle' n, M., Nygren P.A°, Human immunoglobulin A (IgA)-specific ligands from combinatorial engineering of protein A, 2002, Eur. J. Biochem. 269, 2647-2655.).

Conjugated Affinity Reagents

In a preferred embodiment, anti-CRCMP affinity reagents such as antibodies or fragments thereof are conjugated to a diagnostic or therapeutic moiety. The antibodies can be used for diagnosis or to determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics according to the present invention. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include $^{125}I$, $^{131}I$, $^{111}In$ and $^{99}Tc$. $^{68}Ga$ may also be employed. Anti-CRCMP antibodies or fragments thereof can be conjugated to a therapeutic agent or drug moiety to modify a given biological response. The therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator, a thrombotic agent or an anti-angiogenic agent, *e.g.* angiostatin or endostatin; or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor.

Techniques for conjugating such therapeutic moiety to antibodies are well known, see, e.g. Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

Alternatively, an antibody can be conjugated to a second antibody to form an antibody heteroconjugate as described by Segal in U.S. Patent No. 4,676,980.

An antibody with or without a therapeutic moiety conjugated to it can be used as a therapeutic that is administered alone or in combination with cytotoxic factor(s) and/or cytokine(s).

Identification of Marker Panels

In accordance with the present invention, there are provided methods and systems for the identification of one or more markers useful in diagnosis, prognosis, and/or determining an appropriate therapeutic course. One skilled in the art will also recognize that univariate analysis of markers can be performed and the data from the univariate analyses of multiple markers can be combined to form panels of markers to differentiate different disease conditions in a variety of ways, including so-called "n-of-m" methods (for example, where if n markers (*e.g.*, 2) out of a total of m markers (*e.g.*, 3) meet some criteria, the test is considered positive), multiple linear regression, determining interaction terms, stepwise regression, *etc.*

Suitable methods for identifying markers useful for such purposes are also described in detail in U.S. Provisional Patent Application No. 60/436,392 filed December 24, 2002, PCT application US03/41426 filed December 23, 2003, U.S. Patent Application No. 10/331,127 filed December 27, 2002, and PCT application No. US03/41453, each of which is hereby incorporated by reference in its entirety, including all tables, figures, and claims. The following discussion provides an exemplary discussion of methods that may be used to provide the panels of the present invention.

In developing a panel of markers, data for a number of potential markers may be obtained from a group of subjects by testing for the presence or level of certain markers. The group of subjects is divided into two sets. The first set includes subjects who have been confirmed as having a disease, outcome, or, more generally, being in a first condition state. For example, this first set of patients may be those diagnosed with colorectal cancer that died as a result of that disease. Hereinafter, subjects in this first set will be referred to as "diseased."

The second set of subjects is simply those who do not fall within the first set. Subjects in this second set will hereinafter be referred to as "non-diseased". Preferably, the first set and the second set each have an approximately equal number of subjects. This set may be normal patients, and/or patients suffering from another cause of colorectal cancer, and/or patients that lived to a particular endpoint of interest.

The data obtained from subjects in these sets preferably includes levels of a plurality of markers. Preferably, data for the same set of markers is available for each patient. This set of markers may include all candidate markers that may be suspected as being relevant to the detection of a particular disease or condition. Actual known relevance is not required. Embodiments of the methods and systems described herein may be used to determine which of the candidate markers are most relevant to the diagnosis of the disease or condition. The levels of each marker in the two sets of subjects may be distributed across a broad range, *e.g.*, as a Gaussian distribution. However, no distribution fit is required.

As noted above, a single marker often is incapable of definitively identifying a subject as falling within a first or second group in a prospective fashion. For example, if a patient is measured as having a marker level that falls within an overlapping region in the distribution of diseased and non-diseased subjects, the results of the test may be useless in diagnosing the patient. An artificial cutoff may be used to distinguish between a positive and a negative test result for the detection of the disease or condition. Regardless of where the cutoff is selected, the effectiveness of the single marker as a diagnosis tool is unaffected. Changing the cutoff merely trades off between the number of false positives and the number of false negatives resulting from the use of the single marker. The effectiveness of a test having such an overlap is often expressed using a ROC (Receiver Operating Characteristic) curve. ROC curves are well known to those skilled in the art.

The horizontal axis of the ROC curve represents (1-specificity), which increases with the rate of false positives. The vertical axis of the curve represents sensitivity, which increases with the rate of true positives. Thus, for a particular cutoff selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

As discussed above, the measurement of the level of a single marker may have limited usefulness, *e.g.*, it may be non-specifically increased due to inflammation. The measurement of additional markers provides additional information, but the difficulty lies in properly combining the levels of two potentially unrelated measurements. In the methods and systems according to embodiments of the present invention, data relating to levels of various markers for the sets of diseased and non-diseased patients may be used to develop a panel of markers to provide a useful panel response. The data may be provided in a database such as Microsoft Access, Oracle, other SQL databases or simply in a data file. The database or data file may contain, for example, a patient identifier such as a name or number, the levels of the various markers present, and whether the patient is diseased or non-diseased.

Next, an artificial cutoff region may be initially selected for each marker. The location of the cutoff region may initially be selected at any point, but the selection may affect the optimization process described below. In this regard, selection near a suspected optimal location may facilitate faster convergence of the optimizer. In a preferred method, the cutoff region is initially centered about the center of the overlap region of the two sets of patients. In one embodiment, the cutoff region may simply be a cutoff point. In other embodiments, the cutoff region may have a length of greater than zero. In this regard, the cutoff region may be defined by a center value and a magnitude of length. In practice, the initial selection of the limits of the cutoff region may be determined according to a pre-selected percentile of each set of subjects. For example, a point above which a pre-selected percentile of diseased patients are measured may be used as the right (upper) end of the cutoff range.

Each marker value for each patient may then be mapped to an indicator. The indicator is assigned one value below the cutoff region and another value above the cutoff region. For example, if a marker generally has a lower value for non-diseased patients and a higher value for diseased patients, a zero indicator will be assigned to a low value for a particular marker, indicating a potentially low likelihood of a positive diagnosis. In other embodiments, the indicator may be calculated based on a polynomial. The coefficients of the polynomial may be determined based on the distributions of the marker values among the diseased and non-diseased subjects.

The relative importance of the various markers may be indicated by a weighting factor. The weighting factor may initially be assigned as a coefficient for each marker. As with the cutoff region, the initial selection of the weighting factor may be selected at any acceptable value, but the selection may affect the optimization process. In this regard, selection near a suspected optimal location may facilitate faster convergence of the optimizer. In a preferred method, acceptable weighting coefficients may range between zero and one, and an initial weighting coefficient for each marker may be assigned as 0.5. In a preferred embodiment, the initial weighting coefficient for each marker may be associated with the effectiveness of that marker by itself. For example, a ROC curve may be generated for the single marker, and the area under the ROC curve may be used as the initial weighting coefficient for that marker.

Next, a panel response may be calculated for each subject in each of the two sets. The panel response is a function of the indicators to which each marker level is mapped and the weighting coefficients for each marker. In a preferred embodiment, the panel response (R) for each subject (j) is expressed as:

$$R_j = \sum w_i I_{i,j,}$$

where i is the marker index, j is the subject index, $w_i$ is the weighting coefficient for marker i, I is the indicator value to which the marker level for marker i is mapped for subject j, and $\sum$ is the summation over all candidate markers i. This panel response value may be referred to as a "panel index."

One advantage of using an indicator value rather than the marker value is that an extraordinarily high or low marker levels do not change the probability of a diagnosis of diseased or non-diseased for that particular marker. Typically, a marker value above a certain level generally indicates a certain condition state. Marker values above that level indicate the condition state with the same certainty. Thus, an extraordinarily high marker value may not indicate an extraordinarily high probability of that condition state. The use of an indicator which is constant on one side of the cutoff region eliminates this concern.

The panel response may also be a general function of several parameters including the marker levels and other factors including, for example, race and gender of the patient. Other factors contributing to the panel response may include the slope of the value of a particular marker over time. For example, a patient may be measured when first arriving at the hospital for a particular marker. The same marker may be measured again an hour later, and the level of change may be reflected in the panel response. Further, additional markers may be derived from other markers and may contribute to the value of the panel response. For example, the ratio of values of two markers may be a factor in calculating the panel response.

Having obtained panel responses for each subject in each set of subjects, the distribution of the panel responses for each set may now be analyzed. An objective function may be defined to facilitate the selection of an effective panel. The objective function should generally be indicative of the effectiveness of the panel, as may be expressed by, for example, overlap of the panel responses of the diseased set of subjects and the panel responses of the non-diseased set of subjects. In this manner, the objective function may be optimized to maximize the effectiveness of the panel by, for example, minimizing the overlap.

In a preferred embodiment, the ROC curve representing the panel responses of the two sets of subjects may be used to define the objective function. For example, the objective function may reflect the area under the ROC curve. By maximizing the area under the curve, one may maximize the effectiveness of the panel of markers. In other embodiments, other features of the ROC curve may be used to define the objective function. For example, the point at which the slope of the ROC curve is equal to one may be a useful feature. In other embodiments, the point at which the product of sensitivity and specificity is a maximum, sometimes referred to as the "knee," may be used. In an embodiment, the sensitivity at the knee may be maximized. In further embodiments, the sensitivity at a predetermined specificity level may be used to define the objective function. Other embodiments may use the specificity at a predetermined sensitivity level may be used. In still other embodiments, combinations of two or more of these ROC-curve features may be used.

It is possible that one of the markers in the panel is specific to the disease or condition being diagnosed. When such markers are present at above or below a certain threshold, the panel response may be set to return a "positive" test result. When the threshold is not satisfied, however, the levels of the marker may nevertheless be used as possible contributors to the objective function.

An optimization algorithm may be used to maximize or minimize the objective function. Optimization algorithms are well-known to those skilled in the art and include several commonly available minimizing or maximizing functions including the Simplex method and other constrained optimization techniques. It is understood by those skilled in the art that some minimization functions are better than others at searching for global minimums, rather than local minimums. In the optimization process, the location and size of the cutoff region for each marker may be allowed to vary to provide at least two degrees of freedom per marker. Such variable parameters are referred to herein as independent variables. In a preferred embodiment, the weighting coefficient for each marker is also allowed to vary across iterations of the optimization algorithm. In various embodiments, any permutation of these parameters may be used as independent variables.

In addition to the above-described parameters, the sense of each marker may also be used as an independent variable. For example, in many cases, it may not be known whether a higher level for a certain marker is generally indicative of a diseased state or a non-diseased state. In such a case, it may be useful to allow the optimization process to search on both sides. In practice, this may be implemented in several ways. For example, in one embodiment, the sense may be a truly separate independent variable which may be flipped between positive and negative by the optimization process. Alternatively, the sense may be implemented by allowing the weighting coefficient to be negative.

The optimization algorithm may be provided with certain constraints as well. For example, the resulting ROC curve may be constrained to provide an area-under-curve of greater than a particular value. ROC curves having an area under the curve of 0.5 indicate complete randomness, while an area under the curve of 1.0 reflects perfect separation of the two sets. Thus, a minimum acceptable value, such as 0.75, may be used as a constraint, particularly if the objective function does not incorporate the area under the curve. Other constraints may include limitations on the weighting coefficients of particular markers. Additional constraints may limit the sum of all the weighting coefficients to a particular value, such as 1.0.

The iterations of the optimization algorithm generally vary the independent parameters to satisfy the constraints while minimizing or maximizing the objective function. The number of iterations may be limited in the optimization process. Further, the optimization process may be terminated when the difference in the objective function between two consecutive iterations is below a predetermined threshold, thereby indicating that the optimization algorithm has reached a region of a local minimum or a maximum.

Thus, the optimization process may provide a panel of markers including weighting coefficients for each marker and cutoff regions for the mapping of marker values to indicators. Certain markers may be then be changed or even eliminated from the panel, and the process repeated until a satisfactory result is obtained. The effective contribution of each marker in the panel may be determined to identify the relative importance of the markers. In one embodiment, the weighting coefficients resulting from the optimization process may be used to determine the relative importance of each marker. The markers with the lowest coefficients may be eliminated or replaced.

In certain cases, the lower weighting coefficients may not be indicative of a low importance. Similarly, a higher weighting coefficient may not be indicative of a high importance. For example, the optimization process may result in a high coefficient if the associated marker is irrelevant to the diagnosis. In this instance, there may not be any advantage that will drive the coefficient lower. Varying this coefficient may not affect the value of the objective function.

Evaluation of Marker Panels

To allow a determination of test accuracy, a "gold standard" test criterion may be selected which allows selection of subjects into two or more groups for comparison by the foregoing methods. In the case of colorectal cancer, this gold standard may be the carcinoembyonic antigen (CEA) test. This implies that those negative for the gold standard are free of colorectal cancer. Alternatively, an initial comparison of confirmed colorectal cancer subjects may be compared to normal healthy control subjects. In the case of a prognosis, mortality is a common test criterion.

The sensitivity and specificity of a diagnostic and/or prognostic test depends on more than just the analytical "quality" of the test - they also depend on the definition of what constitutes an abnormal result. In practice, Receiver Operating Characteristic curves, or "ROC" curves, are typically calculated by plotting the value of a variable versus its relative frequency in "normal" and "disease" populations. For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results don't necessarily give an accurate number. As long as one can rank results, one can create an ROC curve. For example, results of a test on "disease" samples might be ranked according to degree (say 1=low, 2=normal, and 3=high). This ranking can be correlated to results in the "normal" population, and a ROC curve created. These methods are well known in the art. *See*, *e.g.*, Hanley et al., Radiology 143: 29-36 (1982).

Measures of test accuracy may be obtained as described in Fischer et al., Intensive Care Med. 29: 1043-51, 2003, and used to determine the effectiveness of a given marker or panel of markers. These measures include sensitivity and specificity, predictive values, likelihood ratios, diagnostic odds ratios, and ROC curve areas. As discussed above, preferred tests and assays exhibit one or more of the following results on these various measures:

- at least 75% sensitivity, combined with at least 75% specificity;

- ROC curve area of at least 0.6, more preferably 0.7, still more preferably at least 0.8, even more preferably at least 0.9, and most preferably at least 0.95; and/or

- at least about 70% sensitivity, more preferably at least about 80% sensitivity, even more preferably at least about 85% sensitivity, still more preferably at least about 90% sensitivity, and most preferably at least about 95% sensitivity, combined with at least about 70% specificity, more preferably at least about 80% specificity, even more preferably at least about 85% specificity, still more preferably at least about 90% specificity, and most preferably at least about 95% specificity. In particularly preferred embodiments, both the sensitivity and specificity are at least about 75%, more preferably at least about 80%, even more preferably at least about 85%, still more preferably at least about 90%, and most preferably at least about 95%. The term "about" in this context refers to +/- 5% of a given measurement; and/or

- a positive likelihood ratio and/or a negative likelihood ratio of at least about 1.5 or more or about 0.67 or less, more preferably at least about 2 or more or about 0.5 or less, still more preferably at least about 5 or more or about 0.2 or less, even more preferably at least about 10 or more or about 0.1 or less, and most preferably at least about 20 or more or about 0.05 or less. The term "about" in this context refers to +/-5% of a given measurement. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group; and/or

- an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less. The term "about" in this context refers to +/- 5% of a given measurement. In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group; and/or

- a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less. The term "about" in this context refers to +/- 5% of a given measurement. In the case of a hazard ratio, a value of 1 indicates that the relative risk of an endpoint (*e.g.*, death) is equal in both the "diseased" and "control" groups; a value greater than 1 indicates that the risk is greater in the diseased group; and a value less than 1 indicates that the risk is greater in the control group.

Once a plurality of markers have been identified for use in a marker panel, such a panel may be used to evaluate an individual, *e.g.*, for diagnostic, prognostic, and/or therapeutic purposes. In certain embodiments, concentrations of the individual markers can each be compared to a level (a "threshold") that is preselected to rule in or out one or more particular diagnoses, prognoses, and/or therapy regimens. In these embodiments, correlating of each of the subject's selected marker level can comprise comparison to thresholds for each marker of interest that are indicative of a particular diagnosis. Similarly, by correlating the subject's marker levels to prognostic thresholds for each marker, the probability that the subject will suffer one or more future adverse outcomes may be determined.

In other embodiments, particular thresholds for one or more markers in a panel are not relied upon to determine if a profile of marker levels obtained from a subject are correlated to a particular diagnosis or prognosis. Rather, the present invention may utilize an evaluation of the entire profile of markers to provide a single result value (*e.g.*, a "panel response" value expressed either as a numeric score or as a percentage risk). In such embodiments, an increase, decrease, or other change (*e.g.*, slope over time) in a certain subset of markers may be sufficient to indicate a particular condition or future outcome in one patient, while an increase, decrease, or other change in a different subset of markers may be sufficient to indicate the same or a different condition or outcome in another patient.

In various embodiments, multiple determinations of one or more markers can be made, and a temporal change in the markers can be used to rule in or out one or more particular diagnoses and/or prognoses. For example, one or more markers may be determined at an initial time, and again at a second time, and the change (or lack thereof) in the marker level(s) over time determined. In such embodiments, an increase in the marker from the initial time to the second time may be indicative of a particular prognosis, of a particular diagnosis, *etc*. Likewise, a decrease in the marker from the initial time to the second time may be indicative of a particular prognosis, of a particular diagnosis, *etc.* In such a panel, the markers need not change in concert with one another. Temporal changes in one or more markers may also be used together with single time point marker levels to increase the discriminating power of marker panels. In yet another alternative, a "panel response" may be treated as a marker, and temporal changes in the panel response may be indicative of a particular prognosis, diagnosis, *etc.*

As discussed in detail herein, a plurality of markers may be combined, preferably to increase the predictive value of the analysis in comparison to that obtained from the markers individually. Such panels may comprise 2, 3, 4, 5, 6, 7, 8, 9, 10, 15, 20, or more or individual markers. The skilled artisan will also understand that diagnostic markers, differential diagnostic markers, prognostic markers, time of onset markers, *etc.*, may be combined in a single assay or device. For example, certain markers measured by a device or instrument may be used provide a prognosis, while a different set of markers measured by the device or instrument may rule in and/or out particular therapies; each of these sets of markers may comprise unique markers, or may include markers that overlap with one or both of the other sets. Markers may also be commonly used for multiple purposes by, for example, applying a different set of analysis parameters (*e.g.*, different midpoint, linear range window and/or weighting factor) to the marker(s) for the different purpose(s).

While exemplary panels are described herein, one or more markers may be replaced, added, or subtracted from these exemplary panels while still providing clinically useful results. Panels may comprise both specific markers of a condition of interest and/or non-specific markers (*e.g.*, markers that are increased or decreased due to a condition of interest, but are also increased in other conditions). While certain markers may not individually be definitive in the methods described herein, a particular "fingerprint" pattern of changes may, in effect, act as a specific indicator of disease state. As discussed above, that pattern of changes may be obtained from a single sample, or may optionally consider temporal changes in one or more members of the panel (or temporal changes in a panel response value).

Use in Conjunction With a Treatment Regimen

Just as the potential causes of any particular nonspecific symptom may be a large and diverse set of conditions, the appropriate treatments for these potential causes may be equally large and diverse. However, once a diagnosis is obtained, the clinician can readily select a treatment regimen that is compatible with the diagnosis. The skilled artisan is aware of appropriate treatments for numerous diseases discussed in relation to the methods of diagnosis described herein. *See, e.g.*, Merck Manual of Diagnosis and Therapy, 17th Ed. Merck Research Laboratories, Whitehouse Station, NJ, 1999.

In addition, since the methods and compositions described herein can provide prognostic information, the panels and markers of the present invention may be used to monitor a course of treatment. For example, improved or worsened prognostic state may indicate that a particular treatment is or is not efficacious. The term "theranostics" is used to describe the process of tailoring diagnostic therapy for an individual based on test results obtained for the particular individual. Theranostics go beyond traditional diagnosis, which is only concerned with identifying the presence of a disease. Theranostics can include one or more of predicting risks of disease, diagnosing disease, stratifying patients for risk, and monitoring therapeutic response. The diagnostic and/or prognostic methods of the present invention may be advantageously integrated into a therapy regimen so that the characteristics of treatment received by the individual is, at least in part, guided by the results of the methods, thereby individualizing and optimizing the therapeutic regimen of the individual.

Treatment and Prevention of Colorectal Cancer

Colorectal cancer is treated or prevented by administration to a subject suspected of having or known to have colorectal cancer or to be at risk of developing colorectal cancer of a compound that modulates (*i.e.,* increases or decreases) the level or activity (*i.e.,* function) of one or more CRCMPs that are differentially present in the serum of subjects having colorectal cancer compared with serum of subjects free from colorectal cancer. In one embodiment, colorectal cancer is treated or prevented by administering to a subject suspected of having or known to have colorectal cancer or to be at risk of developing colorectal cancer a compound that upregulates (*i.e.,* increases) the level or activity (*i.e.,* function) of one or more CRCMPs that are decreased in the serum of subjects having colorectal cancer. In another embodiment, a compound is administered that downregulates the level or activity (*i.e.*, function) of one or more CRCMPs that are increased in the serum of subjects having colorectal cancer. Examples of such a compound include but are not limited to: a CRCMP, CRCMP fragments and CRCMP-related polypeptides; nucleic acids encoding a CRCMP, a CRCMP fragment and a CRCMP-related polypeptide (e.g. for use in gene therapy); and, for those CRCMP or CRCMP-related polypeptides with enzymatic activity, compounds or molecules known to modulate that enzymatic activity. Other compounds that can be used, e.g. CRCMP agonists, can be identified using in *in vitro* assays.

Colorectal cancer is also treated or prevented by administration to a subject suspected of having or known to have colorectal cancer or to be at risk of developing colorectal cancer of a compound that downregulates the level or activity of one or more CRCMPs that are increased in the serum of subjects having colorectal cancer. In another embodiment, a compound is administered that upregulates the level or activity of one or more CRCMPs that are decreased in the serum of subjects having colorectal cancer. Examples of such a compound include, but are not limited to, CRCMP antisense oligonucleotides, ribozymes, antibodies (or other affinity reagents such as Affibodies, Nanobodies or Unibodies) directed against a CRCMP, and compounds that inhibit the enzymatic activity of a CRCMP. Other useful compounds e.g. CRCMP antagonists and small molecule CRCMP antagonists, can be identified using *in vitro* assays.

In a preferred embodiment, therapy or prophylaxis is tailored to the needs of an individual subj ect. Thus, in specific embodiments, compounds that promote the level or function of one or more CRCMPs are therapeutically or prophylactically administered to a subject suspected of having or known to have colorectal cancer, in whom the levels or functions of said one or more CRCMPs are absent or are decreased relative to a control or normal reference range. In further embodiments, compounds that promote the level or function of one or more CRCMPs are therapeutically or prophylactically administered to a subject suspected of having or known to have colorectal cancer in whom the levels or functions of said one or more CRCMPs are increased relative to a control or to a reference range. In further embodiments, compounds that decrease the level or function of one or more CRCMPs are therapeutically or prophylactically administered to a subject suspected of having or known to have colorectal cancer in whom the levels or functions of said one or more CRCMPs are increased relative to a control or to a reference range. In further embodiments, compounds that decrease the level or function of one or more CRCMPs are therapeutically or prophylactically administered to a subject suspected of having or known to have colorectal cancer in whom the levels or functions of said one or more CRCMPs are decreased relative to a control or to a reference range. The change in CRCMP function or level due to the administration of such compounds can be readily detected, *e.g.,* by obtaining a sample (*e.g.*, blood or urine) and assaying *in vitro* the levels or activities of said CRCMPs, or the levels of mRNAs encoding said CRCMPs, or any combination of the foregoing. Such assays can be performed before and after the administration of the compound as described herein.

The compounds of the invention include but are not limited to any compound, *e.g.*, a small organic molecule, protein, peptide, antibody (or other affinity reagent such as an Affibody, Nanobody or Unibody), nucleic acid, etc. that restores the CRCMP profile towards normal. The compounds of the invention may be given in combination with any other compound.

Immunotherapy and Prevention of Colorectal Cancer

CRCMPs may be useful in immunogenic compositions (suitably vaccines) for raising immune responses against proteins that may cause, sustain colorectal cancer or lead to metastases. Thus there is provided according to the invention a vaccine comprising one or more soluble polypeptides derived from a protein selected from the list consisting of proteins defined by SEQ ID Nos 1-18 and/or one or more antigenic or immunogenic fragments thereof.. There is also provided an immunogenic composition which comprises one or more soluble polypeptides derived from a protein selected from the list consisting of proteins defined by SEQ ID Nos 1-18 and/or one or more antigenic or immunogenic fragments thereof, and one or more suitable adjuvants. Such a composition is useful in inducing an immune response in a subject, e.g. a human. There is also provided the use of one or more soluble polypeptides derived from a protein selected from the list consisting of proteins defined by SEQ ID Nos 1-18 and/or one or more antigenic or immunogenic fragments thereof in the preparation of an immunogenic composition, preferably a vaccine. There is also provided a method for the treatment or prophylaxis of colorectal cancer in a subject, or of vaccinating a subject against colorectal cancer, which comprises the step of administering to the subject an effective amount of one or more soluble polypeptides derived from a protein selected from the list consisting of proteins defined by SEQ ID Nos 1-18 and/or one or more antigenic or immunogenic fragments thereof, preferably as a vaccine.

Suitable immunogenic fragments are at least 10 amino acids in length e.g. at least 12 amino acids in length suitably at least 15 amino acids in length.

Suitable adjuvants will be well known to a person skilled in the art (see Vaccine design - the subunit and adjuvant approach (1995) Plenum Press).

Determining Abundance of the CRCMPs by Imaging Technology

An advantage of determining abundance of the CRCMPs by imaging technology may be that such a method is non-invasive (save that reagents may need to be administered) and there is no need to extract a sample from the subject.

Suitable imaging technologies include positron emission tomography (PET) and single photon emission computed tomography (SPECT). Visualisation of the CRCMPs using such techniques requires incorporation or binding of a suitable label e.g. a radiotracer such as $^{18}F$, $^{11}C$ or $^{123}I$ (see e.g. NeuroRx - The Journal of the American Society for Experimental NeuroTherapeutics (2005) 2(2), 348-360 and *idem* pages 361-371 for further details of the techniques). Radiotracers or other labels may be incorporated into a CRCMP by administration to the subject (e.g. by injection) of a suitably labelled specific ligand. Alternatively they may be incorporated into a binding affinity reagent (antibody, Affibody, Nanobody, Unibody etc.) specific for the CRCMP which may be administered to the subject (e.g. by injection). For discussion of use of Affibodies for imaging see e.g. Orlova A, Magnusson M, Eriksson TL, Nilsson M, Larsson B, Hoiden-Guthenberg I, Widstrom C, Carlsson J, Tolmachev V, Stahl S, Nilsson FY, Tumor imaging using a picomolar affinity HER2 binding affibody molecule, Cancer Res. 2006 Apr 15;66(8):4339-48).

Diagnosis and Treatment of Colorectal Cancer using Immunohistochemistry

Immunohistochemistry is an excellent detection technique and may therefore be very useful in the diagnosis and treatment of colorectal cancer. Immunohistochemistry may be used to detect, diagnose, or monitor colorectal cancer through the localization of CRCMP antigens in tissue sections by the use of labeled antibodies (or other affinity reagents such as Affibodies, Nanobodies or Unibodies), derivatives and analogs thereof, which specifically bind to a CRCMP, as specific reagents through antigen-antibody interactions that are visualized by a marker such as fluorescent dye, enzyme, radioactive element or colloidal gold.

The advancement of monoclonal antibody technology has been of great significance in assuring the place of immunohistochemistry in the modern accurate microscopic diagnosis of human neoplasms. The identification of disseminated neoplastically transformed cells by immunohistochemistry allows for a clearer picture of cancer invasion and metastasis, as well as the evolution of the tumour cell associated immunophenotype towards increased malignancy. Future antineoplastic therapeutical approaches may include a variety of individualized immunotherapies, specific for the particular immunophenotypical pattern associated with each individual patient's neoplastic disease. For further discussion see e.g. Bodey B, The significance of immunohistochemistry in the diagnosis and therapy of neoplasms, Expert Opin Biol Ther. 2002 Apr;2(4):371-93.

Further aspects of the invention are represented by the following numbered clauses:

1. A method of diagnosing colorectal cancer in a subject, differentiating causes of colorectal cancer in a subject, guiding therapy in a subject suffering from colorectal cancer, assessing the risk of relapse in a subject suffering from colorectal cancer, or assigning a prognostic risk of one or more future clinical outcomes to a subject suffering from colorectal cancer, the method comprising:

(a) performing assays configured to detect a soluble polypeptide derived from a protein selected from the list consisting of proteins defined by SEQ ID Nos 1-18 as a marker in one or more samples obtained from said subject; and

(b) correlating the results of said assay(s) to the presence or absence of colorectal cancer in the subject, to a therapeutic regimen to be used in the subject, to a risk of relapse in the subject, or to the prognostic risk of one or more clinical outcomes for the subject suffering from colorectal cancer.

2. A method according to clause 1 wherein the soluble polypeptide detected in step (a) is derived from a protein defined by any one of SEQ ID Nos 4, 7, 8, 13 and 15.

3. A method according to clause 1 wherein step (b) involves determining that when the level of said detected marker is higher in the subject than a control level, said determination indicates the presence of colorectal cancer in the subject, indicates a greater risk of relapse in the subject, or indicates a worse prognosis for the subject.

4. A method according to any one of clauses 1 to 3 which is a method for diagnosing colorectal cancer in a subject.

5. A method according to any one of clauses 1 to 4 wherein the marker comprises an amino acid sequence recited in column 4 of Table 1, namely any one of SEQ ID Nos 34-35, 37-38, 40-42, 44, 47-56, 59-60, 62, 64-83, 85-87, 89-92, 95-127, 132-133, 137-141, 144-147, 149, 151-153, 155-161, 164-165, 167-175, 177-179, 182-187, 189-190, 193-195, 197-200, 202, 205-209, 211, 213-227, 229-241, 243.

6. A method according to any one of clauses 1 to 4 wherein the marker comprises an amino acid sequence recited in column 4 of Table 2, namely any one of SEQ ID Nos 36, 39-40, 42-43, 45-47, 57-58, 61, 63, 66, 75, 84, 88, 91, 93-94, 98, 100, 108, 111, 115, 121, 123-124, 126, 128-131, 134-136, 140, 142-143, 147-150, 152-154, 160-163, 166, 168, 172, 174-176, 180-181, 188, 190-192, 196, 200-201, 203-204, 212, 214, 216, 218, 224, 228, 238-239, 242, 244-245.

7. A method according to any one of clauses 1 to 4 and 6 wherein the marker is derived from a protein in an isoform characterized by a pI and MW as listed in columns 2 and 3 of Table 2.

8. A method according to any one of clauses 1 to 7 wherein the marker sequence overlaps with or is preferably within a sequence corresponding to an extracellular portion of a protein having a sequence selected from any one of SEQ ID Nos 1-18 (i.e. overlaps with or is preferably within a sequence corresponding to a sequence selected from SEQ ID Nos 19, 21, 22, 25, 27, 29, 30 and 32).

9. A method according to clause 8 wherein the marker sequence overlaps with or is preferably within a sequence corresponding to an extracellular portion of a protein having a sequence selected from any one of SEQ ID Nos 4, 7, 8, 13 and 15.

10. A method according to any one of clauses 1 to 9, wherein the method comprises performing assays configured to detect two or more said markers.

11. A method according to clause 10 wherein the two or more said markers are derived from at least two different proteins.

12. A method according to any one of clauses 1 to 9, wherein the method comprises performing assays configured to detect three or more said markers.

13. A method according to clause 12 wherein the three or more said markers are derived from at least three different proteins.

14. A method according to any one of clauses 1 to 9, wherein the method comprises performing assays configured to detect four or more said markers.

15. A method according to clause 14 wherein the four or more said markers are derived from at least four different proteins.

16. A method according to any one of clauses 1 to 9, wherein the method comprises performing assays configured to detect five or more said markers.

17. A method according to clause 16 wherein the five or more said markers are derived from at least five different proteins.

18. A method according to any one of clauses 1 to 17, wherein the method comprises performing one or more additional assays configured to detect one or more additional markers in addition to the soluble polypeptide derived from a protein selected from the list consisting of proteins defined by SEQ ID Nos 1-18 and wherein said correlating step comprises correlating the results of said assay(s) and the results of said additional assay(s) to the presence or absence of colorectal cancer in the subject, to a risk of relapse in the subject, or to the prognostic risk of one or more clinical outcomes for the subject suffering from colorectal cancer.

19. A method according to clause 18 wherein the soluble polypeptide is derived from a protein defined by any one of SEQ ID Nos 4, 7, 8, 13 and 15.

20. A method according to any one of clauses 1 to 19, wherein the subject is a human.

21. A method according to any one of clauses 1 to 19, wherein one or more of said assay(s) is an immunoassay.

22. An antibody or other affinity reagent such as an Affibody, Nanobody or Unibody capable of immunospecific binding to a soluble polypeptide derived from a protein selected from the list consisting of proteins defined by SEQ ID Nos 1-18.

23. An antibody according to clause 22 wherein the soluble polypeptide is derived from a protein defined by any one of SEQ ID Nos 4, 7, 8, 13 and 15.

24. A kit comprising an antibody or other affinity reagent such as an Affibody, Nanobody or Unibody as defined in clause 22.

25. A kit comprising a plurality of distinct antibodies or other affinity reagents such as Affibodies, Nanobodies or Unibodies as defined in clause 22.

26. Use of an antibody or other affinity reagent such as an Affibody, Nanobody or Unibody according to clause 22 or a kit according to clause 24 or clause 25 for diagnosing colorectal cancer in a subject, differentiating causes of colorectal cancer in a subject, guiding therapy in a subject suffering from colorectal cancer, assessing the risk of relapse in a subject suffering from colorectal cancer, or assigning a prognostic risk of one or more future clinical outcomes to a subject suffering from colorectal cancer.

27. A method for identifying the presence or absence of colorectal cancer cells in a biological sample obtained from a human subject, which comprises the step of identifying the presence or absence of one or more soluble polypeptides derived from a protein selected from the list consisting of proteins defined by any one of SEQ ID Nos 1-18.

28. A method according to clause 27 wherein the soluble polypeptide is derived from a protein defined by any one of SEQ ID Nos 4, 7, 8, 13 and 15.

29. A method of detecting, diagnosing colorectal cancer in a subject, differentiating causes of colorectal cancer in a subject, guiding therapy in a subject suffering from colorectal cancer, assessing the risk of relapse in a subject suffering from colorectal cancer, or assigning a prognostic risk of one or more future clinical outcomes to a subject suffering from colorectal cancer, the method comprising:

(a) bringing into contact with a sample to be tested from said subject one or more antibodies, or other affinity reagents such as Affibodies, Nanobodies or Unibodies, capable of specific binding to a soluble polypeptides derived from a protein selected from the list consisting of proteins defined by SEQ ID Nos 1-18; and
(b) thereby detecting the presence of one or more soluble polypeptides derived from a protein selected from the list consisting of proteins defined by any one of SEQ ID Nos 1-18 in the sample.

30. A method according to clause 29 wherein the soluble polypeptide is derived from a protein defined by any one of SEQ ID Nos 4, 7, 8, 13 and 15.

31. A method of detecting colorectal cancer in a patient according to clause 29 or clause 30 wherein the presence of one or more said soluble polypeptides indicates the presence of colorectal cancer in the patient.

32. A method for identifying the presence of colorectal cancer in a subject which comprises the step of carrying out a whole body scan of said subject to determine the localisation of colorectal cancer cells, particularly metastatic colorectal cancer cells, in order to determine presence or amount of one or more soluble polypeptides derived from a protein selected from the list consisting of proteins defined by SEQ ID Nos 1-18, wherein the presence or amount of one or more of said soluble polypeptides indicates the presence of colorectal cancer in the subj ect.

33. A method according to clause 32 wherein the soluble polypeptide is derived from a protein defined by any one of SEQ ID Nos 4, 7, 8, 13 and 15.

34. A method for identifying the presence of colorectal cancer in a subject which comprises determining the localisation of colorectal cancer cells by reference to a whole body scan of said subject, which scan indicates the presence or amount of one or more soluble polypeptides derived from a protein selected from the list consisting of proteins defined by SEQ ID Nos 1-18, wherein the presence or amount of one or more of said soluble polypeptides indicates the presence of colorectal cancer in the subject.

35. A method according to clause 34 wherein the soluble polypeptide is derived from a protein defined by any one of SEQ ID Nos 4, 7, 8, 13 and 15.

36. A method as described in any of clauses 32-35, wherein labelled antibodies, or other affinity reagents such as Affibodies, Nanobodies or Unibodies, are employed to determine the presence of one or more said soluble polypeptides.

37. A diagnostic kit comprising one or more reagents for use in the detection and/or determination of one or more soluble polypeptides derived from a protein selected from the list consisting of proteins defined by SEQ ID Nos 1-18.

38. A kit as described in clause 37 wherein the soluble polypeptide is particularly derived from a protein defined by any one of SEQ ID Nos 4, 7, 8, 13 and 15.

39. A kit as described in clause 37 or clause 38, which comprises one or more containers with one or more antibodies, or other affinity reagents such as Affibodies, Nanobodies or Unibodies, against one or more said soluble polypeptides.

40. A kit as described in clause 39, which further comprises a labelled binding partner to the or each antibody, or other affinity reagent such as an Affibody, Nanobody or Unibody, and/or a solid phase, such as a reagent strip, upon which the or each antibody, or other affinity reagent such as an Affibody, Nanobody or Unibody, is/are immobilised.

41. A method of detecting, diagnosing colorectal cancer in a subject, differentiating causes of colorectal cancer in a subject, guiding therapy in a subject suffering from colorectal cancer, assessing the risk of relapse in a subject suffering from colorectal cancer, or assigning a prognostic risk of one or more future clinical outcomes to a subject suffering from colorectal cancer, the method comprising:

(a) bringing into contact with a sample to be tested one or more soluble polypeptides derived from a protein selected from the list consisting of proteins defined by SEQ ID Nos 1-18 or one or more antigenic or immunogenic fragments thereof; and
(b) detecting the presence of antibodies, or other affinity reagents such as Affibodies, Nanobodies or Unibodies, in the subject capable of specific binding to one or more of said polypeptides, or antigenic or immunogenic fragments thereof.

42. A method according to clause 41 wherein the soluble polypeptide is derived from a protein defined by any one of SEQ ID Nos 4, 7, 8, 13 and 15.

43. A kit for use in the detection, diagnosis of colorectal cancer in a subject, for differentiating causes of colorectal cancer in a subject, for guiding therapy in a subject suffering from colorectal cancer, for assessing the risk of relapse in a subject suffering from colorectal cancer, or for assigning a prognostic risk of one or more future clinical outcomes to a subject suffering from colorectal cancer, which kit comprises one or more soluble polypeptides derived from a protein selected from the list consisting of proteins defined by SEQ ID Nos 1-18 and/or one or more antigenic or immunogenic fragments thereof.

44. A kit as described in clause 43 wherein the soluble polypeptide is derived from a protein defined by any one of SEQ ID Nos 4, 7, 8, 13 and 15.

45. A vaccine comprising one or more soluble polypeptides derived from a protein selected from the list consisting of proteins defined by SEQ ID Nos 1-18 and/or one or more antigenic or immunogenic fragments thereof.

46. A vaccine as described in clause 45 wherein the soluble polypeptide is derived from a protein defined by any one of SEQ ID Nos 4, 7, 8, 13 and 15.

47. An immunogenic composition which comprises one or more soluble polypeptides derived from a protein selected from the list consisting of proteins defined by SEQ ID Nos 1-18 and/or one or more antigenic or immunogenic fragments thereof, and one or more suitable adjuvants.

48. An immunogenic composition as described in clause 47 wherein the soluble polypeptide is derived from a protein defined by any one of SEQ ID Nos 4, 7, 8, 13 and 15.

49. The use of a composition as described in clause 47 or clause 48 in inducing an immune response in a subj ect.

50. The use of one or more soluble polypeptides derived from a protein selected from the list consisting of proteins defined by SEQ ID Nos 1-18 and/or one or more antigenic or immunogenic fragments thereof in the preparation of an immunogenic composition, preferably a vaccine.

51. The use of one or more soluble polypeptides as described in clause 50 wherein the soluble polypeptide is derived from a protein defined by any one of SEQ ID Nos 4, 7, 8, 13 and 15.

52. A method for the treatment or prophylaxis of colorectal cancer in a subject, or of vaccinating a subject against colorectal cancer, which comprises the step of administering to the subject an effective amount of one or more soluble polypeptides derived from a protein selected from the list consisting of proteins defined by SEQ ID Nos 1-18 and/or one or more antigenic or immunogenic fragments thereof, preferably as a vaccine.

53. A method according to clause 52 wherein the soluble polypeptide is derived from a protein defined by any one of SEQ ID Nos 4, 7, 8, 13 and 15.

54. A method according to any one of clauses 1-21 wherein the soluble polypeptide derived from a protein selected from the list consisting of proteins defined by SEQ ID Nos 1-18, is detected by a method which involves use of an imaging technology.

55. A method according to clause 54 wherein the imaging technology involves use of labelled Affibodies.

56. A method according to clause 54 wherein the imaging technology involves use of labelled antibodies.

57. A method for identifying the presence of colorectal cancer in a subject which comprises the step of carrying out immunohistochemistry to determine the localisation of colorectal cancer cells, particularly metastatic colorectal cancer cells, in tissue sections, by the use of labeled antibodies, or other affinity reagents such as Affibodies, Nanobodies or Unibodies, derivatives and analogs thereof, capable of specific binding to one or more soluble polypeptides derived from a protein selected from the list consisting of proteins defined by SEQ ID Nos 1-18 or one or more antigenic or immunogenic fragments thereof, in order to determine presence or amount of one or more of said soluble polypeptides, wherein the presence or amount of one or more of said soluble polypeptides indicates the presence of colorectal cancer in the subject.

58. A method according to clause 57 wherein the soluble polypeptide is derived from a protein defined by any one of SEQ ID Nos 4, 7, 8, 13 and 15.

Preferred features of each aspect of the invention are as for each of the other aspects *mutatis mutandis.* The prior art documents mentioned herein are incorporated to the fullest extent permitted by law.

EXAMPLE 1: IDENTIFICATION OF MEMBRANE PROTEINS EXPRESSED IN COLORECTAL CANCER TISSUE SAMPLES

Using the following Reference Protocol, membrane proteins extracted from colorectal tissue samples were separated by 1D gel and analysed.

### 1.1 MATERIALS AND METHODS

*1.1.1 - Plasma Membrane Fractionation*

The cells recovered from the epithelium of a colorectal adenocarcinoma were lysed and submitted to centrifugation at 1000G. The supernatant was taken, and it was subsequently centrifuged at 3000G. Once again, the supernatant was taken, and it was then centrifuged at 100 000G.
The resulting pellet was recovered and put on 15-60% sucrose gradient.
A Western blot was used to identify sub cellular markers, and the Plasma Membrane fractions were pooled.
The pooled solution was either run directly on 1D gels (see section 1.1.4 below), or further fractionnated into heparin binding and nucleotide binding fractions as described below.

*1.1.2 - Plasma Membrane Heparin-binding Fraction*

The pooled solution from 1a above was applied to an Heparin column, eluted from column and run on 1D gels (see section 1D below).

*1.1.3 - Plasma Nucleotide-binding Fraction*

The pooled solution from 1.1.1 above was applied to a Cibacrom Blue 3GA column, eluted from column and run on 1D gels (see section 1.1.4 below).

*1.1.4 - 1D gel technology*

Protein or membrane pellets were solubilised in 1D sample buffer (1-2 μg/μl). The sample buffer and protein mixture was then heated to 95°C for 3 min.

A 9-16% acrylamide gradient gel was cast with a stacking gel and a stacking comb according to the procedure described in Ausubel F.M. et al., eds., 1989, Current Protocols in Molecular Biology, Vol. II, Green Publishing Associates, Inc., and John Wiley & Sons, Inc., New York, section 10.2, incorporated herein by reference in its entirety.

30-50 micrograms of the protein mixtures obtained from detergent and the molecular weight standards (66, 45 ,31, 21,14 kDa) were added to the stacking gel wells using a 10 microlitre pipette tip and the samples run at 40mA for 5 hours.

The plates were then prised open, the gel placed in a tray of fixer (10% acetic acid, 40% ethanol, 50% water) and shaken overnight. Following this, the gel was primed by 30 minutes shaking in a primer solution (7.5% acetic acid (75mls), 0.05% SDS (5mls of 10%)). The gel was then incubated with a fluorescent dye (7.5% acetic acid, 0.06% OGS in-house dye (600μl) with shaking for 3 hrs. Sypro Red (Molecular Probes, Inc., Eugene, Oregon) is a suitable dye for this purpose. A preferred fluorescent dye is disclosed in U.S. Application No. 09/412,168, filed on October 5, 1999, which is incorporated herein by reference in its entirety.

A computer-readable output was produced by imaging the fluorescently stained gels with an Apollo 3 scanner (Oxford Glycosciences, Oxford, UK). This scanner is developed from the scanner described in WO 96/36882 and in the Ph.D. thesis of David A. Basiji, entitled "Development of a High-throughput Fluorescence Scanner Employing Internal Reflection Optics and Phase-sensitive Detection (Total Internal Reflection, Electrophoresis)", University of Washington (1997), Volume 58/12-B of Dissertation Abstracts International, page 6686, the contents of each of which are incorporated herein by reference. The latest embodiment of this instrument incudes the following improvements: The gel is transported through the scanner on a precision lead-screw drive system. This is preferrable to laying the glass plate on the belt-driven system that is defined in the Basiji thesis as it provides a reproducible means of accurately transporting the gel past the imaging optics.

The gel is secured into the scanner against three alignment stops that rigidly hold the glass plate in a known position. By doing this in conjunction with the above precision transport system and the fact that the gel is bound to the glass plate, the absolute position of the gel can be predicted and recorded. This ensures that accurate co-ordinates of each feature on the gel can be communicated to the cutting robot for excision. This cutting robot has an identical mounting arrangement for the glass plate to preserve the positional accuracy.

The carrier that holds the gel in place has integral fluorescent markers (Designated M1, M2, M3) that are used to correct the image geometry and are a quality control feature to confirm that the scanning has been performed correctly.

The optical components of the system have been inverted. The laser, mirror, waveguide and other optical components are now above the glass plate being scanned. The embodiment of the Basiji thesis has these underneath. The glass plate is therefore mounted onto the scanner gel side down, so that the optical path remains through the glass plate. By doing this, any particles of gel that may break away from the glass plate will fall onto the base of the instrument rather than into the optics.

In scanning the gels, they were removed from the stain, rinsed with water and allowed to air dry briefly and imaged on the Apollo 3. After imaging, the gels were sealed in polyethylene bags containing a small volume of staining solution, and then stored at 4°C.

Apparent molecular weights were calculated by interpolation from a set of known molecular weight markers run alongside the samples.

*1.1.5 - Recovery and analysis of selected proteins*

Proteins were robotically excised from the gels by the process described in U.S. Patent No. 6,064,754, Sections 5.4 and 5.6, 5.7, 5.8 (incorporated herein by reference), as is applicable to 1D-electrophoresis, with modification to the robotic cutter as follows: the cutter begins at the top of the lane, and cuts a gel disc 1.7mm in diameter from the left edge of the lane. The cutter then moves 2mm to the right, and 0.7mm down and cuts a further disc. This is then repeated. The cutter then moves back to a position directly underneath the first gel cut, but offset by 2.2mm downwards, and the pattern of three diagonal cuts are repeated. This is continued for the whole length of the gel.

NOTE: If the lane is observed to broaden significantly then a correction can be made also sideways i.e instead of returning to a position directly underneath a previous gel cut, the cut can be offset slightly to the left (on the left of the lane) and/or the right (on the right of the lane). The proteins contained within the gel fragments were processed to generate tryptic peptides; partial amino acid sequences of these peptides were determined by mass spectroscopy as described in WO98/53323 and Application No. 09/094,996, filed June 15, 1998.

Proteins were processed to generate tryptic digest peptides. Tryptic peptides were analyzed by mass spectrometry using a PerSeptive Biosystems Voyager- DETM STR Matrix-Assisted Laser Desorption Ionization Time-of-Flight (MALDI-TOF) mass spectrometer, and selected tryptic peptides were analyzed by tandem mass spectrometry (MS/MS) using a Micromass Quadrupole Time-of-Flight (Q-TOF) mass spectrometer (Micromass, Altrincham, U.K.) equipped with a nanoflowTM electrospray Z-spray source. For partial amino acid sequencing and identification of CRCMPs uninterpreted tandem mass spectra of tryptic peptides were searched using the SEQUEST search program (Eng et al., 1994, J. Am. Soc. Mass Spectrom. 5:976-989), version v.C.1. Criteria for database identification included: the cleavage specificity of trypsin; the detection of a suite of a, b and y ions in peptides returned from the database, and a mass increment for all Cys residues to account for carbamidomethylation. The database searched was a database constructed of protein entries in the non-redundant database held by the National Centre for Biotechnology Information (NCBI) which is accessible at http://www.ncbi.nlm.nih.gov/. Following identification of proteins through spectral-spectral correlation using the SEQUEST program, masses detected in MALDI-TOF mass spectra were assigned to tryptic digest peptides within the proteins identified. In cases where no amino acid sequences could be identified through searching with uninterpreted MS/MS spectra of tryptic digest peptides using the SEQUEST program, tandem mass spectra of the peptides were interpreted manually, using methods known in the art. (In the case of interpretation of low-energy fragmentation mass spectra of peptide ions see Gaskell et al., 1992, Rapid Commun. Mass Spectrom. 6:658-662).

*1.1.6 - Discrimination of colorectal cancer associated proteins*

The process to identify the CRCMPs uses the peptide sequences obtained experimentally by mass spectrometry described above of naturally occurring human proteins to identify and organize coding exons in the published human genome sequence.

Recent dramatic advances in defining the chemical sequence of the human genome have led to the near completion of this immense task (Venter, J.C. et al. (2001). The sequence of the human genome. Science 16: 1304-51; International Human Genome Sequencing Consortium. (2001). Initial sequencing and analysis of the human genome Nature 409: 860-921). There is little doubt that this sequence information will have a substantial impact on our understanding of many biological processes, including molecular evolution, comparative genomics, pathogenic mechanisms and molecular medicine. For the full medical value inherent in the sequence of the human genome to be realised, the genome needs to be 'organised' and annotated. By this, is meant at least the following three things. (i) The assembly of the sequences of the individual portions of the genome into a coherent, continuous sequence for each chromosome. (ii) The unambiguous identification of those regions of each chromosome that contain genes. (iii) Determination of the fine structure of the genes and the properties of its mRNA and protein products. While the definition of a 'gene' is an increasingly complex issue (H Pearson: What is a gene? Nature (2006) 24: 399 - 401)), what is of immediate interest for drug discovery and development is a catalogue of those genes that encode functional, expressed proteins. A subset of these genes will be involved in the molecular basis of most if not all pathologies. Therefore an important and immediate goal for the pharmaceutical industry is to identify all such genes in the human genome and describe their fine structure.

Processing and integration of peptide masses, peptide signatures, ESTs and Public Domain Genomic Sequence Data to form OGAP® database

Discrete genetic units (exons, transcripts and genes) were identified using the following sequential steps:

1. A 'virtual transcriptome' is generated, containing the tryptic peptides which map to the human genome by combining the gene identifications available from Ensembl and various gene prediction programs. This also incorporates SNP data (from dbSNP) and all alternate splicing of gene identifications. Known contaminants were also added to the virtual transcriptome.

2. All tandem spectra in the OGeS Mass Spectrometry Database are interpreted in order to produce a peptide that can be mapped to one in the virtual transcriptome. A set of automated spectral interpretation algorithms were used to produce the peptide identifications.

3. The set of all mass-matched peptides in the OGeS Mass Spectrometry Database is generated by searching all peptides from transcripts hit by the tandem peptides using a tolerance based on the mass accuracy of the mass spectrometer, typically 20ppm.

4. All tandem and mass-matched peptides are combined in the form of "protein clusters". This is done using a recursive process which groups sequences into clusters based on common peptide hits. Biological sequences are considered to belong to the same cluster if they share one or more tandem or mass-matched peptide.

5. After initial filtering to screen out incorrectly identified peptides, the resulting clusters are then mapped on the human genome.

6. The protein clusters are then aggregated into regions that define preliminary gene boundaries using their proximity and the co-observation of peptides within protein clusters. Proximity is defined as the peptide being within 80,000 nucleotides on the same strand of the same chromosome. Various elimination rules, based on cluster observation scoring and multiple mapping to the genome are used to refine the output. The resulting 'confirmed genes' are those which best account for the peptides and masses observed by mass spectrometry in each cluster. Nominal co-ordinates for the gene are also an output of this stage.

7. The best set of transcripts for each confirmed gene are created from the protein clusters, peptides, ESTs, candidate exons and molecular weight of the original protein spot.

8. Each identified transcript was linked to the sample providing the observed peptides

9. Use of an application for viewing and mining the data. The result of steps 1 - 8 was a database containing genes, each of which consisted of a number of exons and one or more transcripts. An application was written to display and search this integrated genome / proteome data. Any features (OMIM disease locus, InterPro etc.) that had been mapped to the same Golden Path co-ordinate system by Ensembl could be cross-referenced to these genes by coincidence of location and fine structure.

Results

The process was used to generate approximately 1 million peptide sequences to identify protein-coding genes and their exons resulted in the identification of protein sequences for 18083 genes across 67 different tissues and 57 diseases including 506 genes in Bladder cancer, 4,713 genes in Breast cancer, 767 genes in Burkitt's lymphoma, 1,372 genes in Cervical cancer, 949 genes in colorectal cancer, 1,783 genes in Hepatocellular cancer, 2,425 genes in CLL, 978 genes in Lung cancer, 1,764 genes in Melanoma, 1,033 genes in Ovarian Cancer, 2,961 genes in Pancreatic cancer and 3,308 genes in Prostate cancer illustrated here by the list of proteins isolated and identified from colorectal cancer samples. Following comparison of the experimentally determined sequences with sequences in the OGAP® database, the CRCMPs listed in the tables showed a high degree of specificity to colorectal cancer indicative of the prognostic and diagnostic nature.

*1.2 RESULTS*

These experiments identified Colorectal Cancer-associated features corresponding to 18 different genes, as listed in Table 1. The source of each feature according to the fractionation protocols described above is detailed in Table 4 below.

**Table 4: Origins of the Features detected by 1 D gel**

| CRCMP# | Plasma Membrane Fractionation 1D | Plasma Membrane Heparin binding fraction | Plasma Membrane Nucleotide binding fraction |
|---|---|---|---|
| 1 | √ | | √ |
| 2 | √ | | √ |
| 5 | | | √ |
| 6 | √ | | |
| 7 | √ | | |
| 8 | | √ | |
| 9 | √ | | |
| 10 | √ | | |
| 12 | | √ | √ |
| 14 | | √ | |

(continued)

| CRCMP# | Plasma Membrane Fractionation 1D | Plasma Membrane Heparin binding fraction | Plasma Membrane Nucleotide binding fraction |
|---|---|---|---|
| 17 | √ | | √ |
| 18 | √ | √ | √ |
| 19 | √ | | |
| 20 | √ | | |
| 22 | √ | √ | √ |
| 23 | | | √ |
| 25 | √ | √ | √ |
| 26 | √ | | |

EXAMPLE 2: IDENTIFICATION OF THE SOLUBLE FORMS OF THE MEMBRANE PROTEINS EXPRESSED IN COLORECTAL CANCER TISSUE SAMPLES

Using the following exemplary and non-limiting procedure, serum was analysed by isoelectric focusing followed by SDS-PAGE and the proteins corresponding to the features identified in Example 1 above were characterised in their circulating forms.

***2.1 MATERIALS AND METHODS***

2.1.1 Sample Preparation

A protein assay (Pierce BCA Cat # 23225) was performed on each serum sample as received. Prior to protein separation, each sample was processed for selective depletion of certain proteins, in order to enhance and simplify protein separation and facilitate analysis by removing proteins that may interfere with or limit analysis of proteins of interest. See International Patent Application No. PCT/GB99/01742, filed June 1, 1999, which is incorporated by reference in its entirety, with particular reference to pages 3 and 6.

Removal of albumin, haptoglobin, transferrin and immunoglobin G (IgG) from serum ("serum depletion") was achieved by an affinity chromatography purification step in which the sample was passed through a series of >Hi-Trap' columns containing immobilized antibodies for selective removal of albumin, haptoglobin and transferrin, and protein G for selective removal of immunoglobin G. Two affinity columns in a tandem assembly were prepared by coupling antibodies to protein G-sepharose contained in Hi-Trap columns (Protein G-Sepharose Hi-Trap columns (1 ml) Pharmacia Cat. No. 17-0404-01). This was done by circulating the following solutions sequentially through the columns: (1) Dulbecco's Phosphate Buffered Saline (Gibco BRL Cat. No. 14190-094); (2) concentrated antibody solution; (3) 200 mM sodium carbonate buffer, pH 8.35; (4) cross-linking solution (200 mM sodium carbonate buffer, pH 8.35, 20 mM dimethylpimelimidate); and (5) 500 mM ethanolamine, 500 mM NaCl. A third (un-derivatised) protein G Hi-Trap column was then attached to the lower end of the tandem column assembly.

The chromatographic procedure was automated using an Akta Fast Protein Liquid Chromatography (FPLC) System such that a series of up to seven runs could be performed sequentially. The samples were passed through the series of 3 Hi-Trap columns in which the affinity chromatography media selectively bind the above proteins thereby removing them from the sample. Fractions (typically 3 ml per tube) were collected of unbound material ("Flowthrough fractions") that eluted through the column during column loading and washing stages and of bound proteins ("Bound/Eluted fractions") that were eluted by step elution with Immunopure Gentle Ag/Ab Elution Buffer (Pierce Cat. No. 21013). The eluate containing unbound material was collected in fractions which were pooled, desalted/concentrated by centrifugal ultrafiltration and stored to await further analysis by 2D PAGE.

A volume of depleted serum containing approximately 300 μg of total protein was aliquoted and an equal volume of 10% (w/v) SDS (Fluka 71729), 2.3% (w/v) dithiothreitol (BDH 443852A) was added. The sample was heated at 95°C for 5 mins, and then allowed to cool to 20°C. 125μl of the following buffer was then added to the sample:

8M urea (BDH 452043w)
4% CHAPS (Sigma C3023)
65mM dithiotheitol (DTT)

2% (v/v) Resolytes 3.5-10 (BDH 44338 2x)

This mixture was vortexed, and centrifuged at 13000 rpm for 5 mins at 15°C, and the supernatant was separated by isoelectric focusing as described below.

2.1.2 Isoelectric Focusing

Isoelectric focusing (IEF), was performed using the Immobiline7 DryStrip Kit (Pharmacia BioTech), following the procedure described in the manufacturer's instructions, see Instructions for Immobiline7 DryStrip Kit, Pharmacia, # 18-1038-63, Edition AB (incorporated herein by reference in its entirety). Immobilized pH Gradient (IPG) strips (18cm, pH 3-10 non-linear strips; Pharmacia Cat. # 17-1235-01) were rehydrated overnight at 20°C in a solution of 8M urea, 2% (w/v) CHAPS, 10mM DTT, 2% (v/v) Resolytes 3.5-10, as described in the Immobiline DryStrip Users Manual. For IEF, 50µl of supernatant (prepared as above) was loaded onto a strip, with the cup-loading units being placed at the basic end of the strip. The loaded gels were then covered with mineral oil (Pharmacia 17-3335-01) and a voltage was immediately applied to the strips according to the following profile, using a Pharmacia EPS3500XL power supply (Cat 19-3500-01):

Initial voltage = 300V for 2 hrs
Linear Ramp from 300V to 3500V over 3hrs
Hold at 3500V for 19hrs

For all stages of the process, the current limit was set to 10mA for 12 gels, and the wattage limit to 5W. The temperature was held at 20°C throughout the run.

2.1.3 Gel Equilibration and SDS-PAGE

After the final 19hr step, the strips were immediately removed and immersed for 10 mins at 20°C in a first solution of the following composition: 6M urea; 2% (w/v) DTT; 2% (w/v) SDS; 30% (v/v) glycerol (Fluka 49767); 0.05M Tris/HCl, pH 6.8 (Sigma Cat T-1503). The strips were removed from the first solution and immersed for 10 mins at 20°C in a second solution of the following composition: 6M urea; 2% (w/v) iodoacetamide (Sigma 1-6125); 2% (w/v) SDS; 30% (v/v) glycerol; 0.05M Tris/HCl, pH 6.8. After removal from the second solution, the strips were loaded onto supported gels for SDS-PAGE according to Hochstrasser et al., 1988, Analytical Biochemistry 173: 412-423 (incorporated herein by reference in its entirety), with modifications as specified below.

2.1.4 Preparation of supported gels

The gels were cast between two glass plates of the following dimensions: 23cm wide x 24cm long (back plate); 23cm wide x 24cm long with a 2cm deep notch in the central 19cm (front plate). To promote covalent attachment of SDS-PAGE gels, the back plate was treated with a 0.4% solution of y-methacryl-oxypropyltrimethoxysilane in ethanol (BindSilaneJ; Pharmacia Cat. # 17-1330-01). The front plate was treated with (RepelSilaneJ Pharmacia Cat. # 17-1332-01) to reduce adhesion of the gel. Excess reagent was removed by washing with water, and the plates were allowed to dry. At this stage, both as identification for the gel, and as a marker to identify the coated face of the plate, an adhesive bar-code was attached to the back plate in a position such that it would not come into contact with the gel matrix.

The dried plates were assembled into a casting box with a capacity of 13 gel sandwiches. The front and back plates of each sandwich were spaced by means of 1mm thick spacers, 2.5 cm wide. The sandwiches were interleaved with acetate sheets to facilitate separation of the sandwiches after gel polymerization. Casting was then carried out according to Hochstrasser et al., op. cit.

A 9-16% linear polyacrylamide gradient was cast, extending up to a point 2cm below the level of the notch in the front plate, using the Angelique gradient casting system (Large Scale Biology). Stock solutions were as follows. Acrylamide (40% in water) was from Serva (Cat. # 10677). The cross-linking agent was PDA (BioRad 161-0202), at a concentration of 2.6% (w/w) of the total starting monomer content. The gel buffer was 0.375M Tris/HCl, pH 8.8. The polymerization catalyst was 0.05% (v/v) TEMED (BioRad 161-0801), and the initiator was 0.1% (w/v) APS (BioRad 161-0700). No SDS was included in the gel and no stacking gel was used. The cast gels were allowed to polymerize at 20°C overnight, and then stored individually at 4°C in sealed polyethylene bags with 6ml of gel buffer, and were used within 4 weeks.

2.1.5 SDS-PAGE

A solution of 0.5% (w/v) agarose (Fluka Cat 05075) was prepared in running buffer (0.025M Tris, 0.198M glycine (Fluka 50050), 1% (w/v) SDS, supplemented by a trace of bromophenol blue). The agarose suspension was heated to 70°C with stirring, until the agarose had dissolved. The top of the supported 2nd D gel was filled with the agarose solution, and the equilibrated strip was placed into the agarose, and tapped gently with a palette knife until the gel was intimately in contact with the 2nd D gel. The gels were placed in the 2nd D running tank, as described by Amess et al., 1995, Electrophoresis 16: 1255-1267 (incorporated herein by reference in its entirety). The tank was filled with running buffer (as above) until the level of the buffer was just higher than the top of the region of the 2nd D gels which contained polyacrylamide, so as to achieve efficient cooling of the active gel area. Running buffer was added to the top buffer compartments formed by the gels, and then voltage was applied immediately to the gels using a Consort E-833 power supply. For 1 hour, the gels were run at 20mA/gel. The wattage limit was set to 150W, for a tank containing 6 gels, and the voltage limit was set to 600V. After 1 hour, the gels were then run at 40mA/gel, with the same voltage and wattage limits as before, until the bromophenol blue line was 0.5cm from the bottom of the gel. The temperature of the buffer was held at 16°C throughout the run.

2.1.6 Staining

Upon completion of the electrophoresis run, the gels were immediately removed from the tank for fixation. The top plate of the gel cassette was carefully removed, leaving the gel bonded to the bottom plate. The bottom plate with its attached gel was then placed into a staining apparatus, which can accommodate 12 gels. The gels were completely immersed in fixative solution of 40% (v/v) ethanol (BDH 28719), 10% (v/v) acetic acid (BDH 100016X), 50% (v/v) water (MilliQ-Millipore), which was continuously circulated over the gels. After an overnight incubation, the fixative was drained from the tank, and the gels were primed by immersion in 7.5% (v/v) acetic acid, 0.05% (w/v) SDS, 92.5% (v/v) water for 30 mins. The priming solution was then drained, and the gels were stained by complete immersion for 4 hours in a staining solution of Sypro Red (Molecular Probes, Inc., Eugene, Oregon). Alternative dyes which can be used for this purpose are described in US patent application number 09/412168, filed October 5 1999, and incorporated herein by reference in its entirety.

2.1.7 Imaging of the gel

A computer-readable output was produced by imaging the fluorescently stained gels with the Apollo 2 scanner (Oxford Glycosciences, Oxford, UK). This scanner has a gel carrier with four integral fluorescent markers (Designated M1, M2, M3, M4) that are used to correct the image geometry and are a quality control feature to confirm that the scanning has been performed correctly.
For scanning, the gels were removed from the stain, rinsed with water and allowed to air dry briefly, and imaged on the Apollo 2. After imaging, the gels were sealed in polyethylene bags containing a small volume of staining solution, and then stored at 4°C.

2.1.8 Digital Analysis of the Data

The data were processed as described in U.S. Patent No 6,064,654, (published as WO 98/23950) at Sections 5.4 and 5.5 (incorporated herein by reference), as set forth more particularly below.
The output from the scanner was first processed using the MELANIE 7 II 2D PAGE analysis program (Release 2.2, 1997, BioRad Laboratories, Hercules, California, Cat. # 170-7566) to autodetect the registration points, M1, M2, M3 and M4; to autocrop the images (i.e., to eliminate signals originating from areas of the scanned image lying outside the boundaries of the gel, e.g. the reference frame); to filter out artifacts due to dust; to detect and quantify features; and to create image files in GIF format. Features were detected using the following parameters:

Smooths =2
Laplacian threshold 50
Partials threshold 1
Saturation = 100
Peakedness = 0
Minimum Perimeter = 10

2.1.9 Assignment of pl and MW Values

Landmark identification was used to determine the pI and MW of features detected in the images. Sixteen landmark features were identified in a standard serum image.

As many of these landmarks as possible were identified in each gel image of the dataset. Each feature in the study gels was then assigned a pI value by linear interpolation or extrapolation (using the MELANIE7-II software) to the two nearest landmarks, and was assigned a MW value by linear interpolation or extrapolation (using the MELANIE7-II software) to the two nearest landmarks.

2.1.10 Matching With Primary Master Image

Images were edited to remove gross artifacts such as dust, to reject images which had gross abnormalities such as smearing of protein features, or were of too low a loading or overall image intensity to allow identification of more than the most intense features, or were of too poor a resolution to allow accurate detection of features. Images were then compared by pairing with one common image from the whole sample set. This common image, the "primary master image", was selected on the basis of protein load (maximum load consistent with maximum feature detection), a well resolved myoglobin region, (myoglobin was used as an internal standard), and general image quality. Additionally, the primary master image was chosen to be an image which appeared to be generally representative of all those to be included in the analysis. (This process by which a primary master gel was judged to be representative of the study gels was rechecked by the method described below and in the event that the primary master gel was seen to be unrepresentative, it was rejected and the process repeated until a representative primary master gel was found.)

Each of the remaining study gel images was individually matched to the primary master image such that common protein features were paired between the primary master image and each individual study gel image as described below.

2.1.11 Cross-matching Between Samples

The geometry of each study gel was adjusted for maximum alignment between its pattern of protein features, and that of the primary master, as follows. Each of the study gel images was individually transformed into the geometry of the primary master image using a multi-resolution warping procedure. This procedure corrects the image geometry for the distortions brought about by small changes in the physical parameters of the electrophoresis separation process from one sample to another. The observed changes are such that the distortions found are not simple geometric distortions, but rather a smooth flow, with variations at both local and global scale.

The fundamental principle in multi-resolution modeling is that smooth signals may be modeled as an evolution through 'scale space', in which details at successively finer scales are added to a low resolution approximation to obtain the high resolution signal. This type of model is applied to the flow field of vectors (defined at each pixel position on the reference image) and allows flows of arbitrary smoothness to be modeled with relatively few degrees of freedom. Each image is first reduced to a stack, or pyramid, of images derived from the initial image, but smoothed and reduced in resolution by a factor of 2 in each direction at every level (Gaussian pyramid) and a corresponding difference image is also computed at each level, representing the difference between the smoothed image and its progenitor (Laplacian pyramid). Thus the Laplacian images represent the details in the image at different scales.

To estimate the distortion between any 2 given images, a calculation was performed at level 7 in the pyramid (i.e. after 7 successive reductions in resolution). The Laplacian images were segmented into a grid of 16x16 pixels, with 50% overlap between adjacent grid positions in both directions, and the cross correlation between corresponding grid squares on the reference and the test images was computed. The distortion displacement was then given by the location of the maximum in the correlation matrix. After all displacements had been calculated at a particular level, they were interpolated to the next level in the pyramid, applied to the test image, and then further corrections to the displacements were calculated at the next scale.

The warping process brought about good alignment between the common features in the primary master image, and the images for the other samples. The MELANIE 7 II 2D PAGE analysis program was used to calculate and record approximately 500-700 matched feature pairs between the primary master and each of the other images. The accuracy of, this program was significantly enhanced by the alignment of the images in the manner described above. To improve accuracy still further, all pairings were finally examined by eye in the MelView interactive editing program and residual recognizably incorrect pairings were removed. Where the number of such recognizably incorrect pairings exceeded the overall reproducibility of the technology (as measured by repeat analysis of the same biological sample) the gel selected to be the primary master gel was judged to be insufficiently representative of the study gels to serve as a primary master gel. In that case, the gel chosen as the primary master gel was rejected, and different gel was selected as the primary master gel, and the process was repeated.

All the images were then added together to create a composite master image, and the positions and shapes of all the gel features of all the component images were super-imposed onto this composite master as described below.

Once all the initial pairs had been computed, corrected and saved, a second pass was performed whereby the original (unwarped) images were transformed a second time to the geometry of the primary master, this time using a flow field computed by smooth interpolation of the multiple tie-points defined by the centroids of the paired gel features. A composite master image was thus generated by initializing the primary master image with its feature descriptors. As each image was transformed into the primary master geometry, it was digitally summed pixel by pixel into the composite master image, and the features that had not been paired by the procedure outlined above were likewise added to the composite master image description, with their centroids adjusted to the master geometry using the flow field correction.

The final stage of processing was applied to the composite master image and its feature descriptors, which now represent all the features from all the images in the study transformed to a common geometry. The features were grouped together into linked sets or "clusters", according to the degree of overlap between them. Each cluster was then given a unique identifying index, the molecular cluster index (MCI).

An MCI identifies a set of matched features on different images. Thus an MCI represents a protein or proteins eluting at equivalent positions in the 2D separation in different samples.

2.1.12. Construction of Profiles

After matching all component gels in the study to the final composite master image, the intensity of each feature was measured and stored. The end result of this analysis was the generation of a digital profile which contained, for each identified feature:, 1) a unique identification code relative to corresponding feature within the composite master image (MCI), 2) the x, y coordinates of the features within the gel, 3) the isoelectric point (pI) of the Protein Isoforms, 4) the apparent molecular weight (MW) of the Protein Isoforms, 5) the signal value, 6) the standard deviation for each of the preceding measurements, and 7) a method of linking the MCI of each feature to the master gel to which this feature was matched. By virtue of a Laboratory Information Management System (LIMS), this MCI profile was traceable to the actual stored gel from which it was generated, so that proteins identified by computer analysis of gel profile databases could be retrieved. The LIMS also permitted the profile to be traced back to an original sample or patient.

2.1.13. Recovery and analysis of selected proteins

Protein Isoforms were robotically excised and processed to generate tryptic digest peptides. Tryptic peptides were analyzed by mass spectrometry using a PerSeptive Biosystems Voyager- DETM STR Matrix-Assisted Laser Desorption Ionization Time-of-Flight

(MALDI-TOF) mass spectrometer, and selected tryptic peptides were analyzed by tandem mass spectrometry (MS/MS) using a Micromass Quadrupole Time-of-Flight (Q-TOF) mass spectrometer (Micromass, Altrincham, U.K.), equipped with a nanoflowTM electrospray Z-spray source. For partial amino acid sequencing and identification of Protein Isoforms uninterpreted tandem mass spectra of tryptic peptides were searched using the SEQUEST search program (Eng et al., 1994, J. Am. Soc. Mass Spectrom. 5:976-989), version v.C.1. Criteria for database identification included: the cleavage specificity of trypsin; the detection of a suite of a, b and y ions in peptides returned from the database, and a mass increment for all Cys residues to account for carbamidomethylation. The database searched was a database constructed of protein entries in the non-redundant database held by the National Centre for Biotechnology Information (NCBI) which is accessible at http://www.ncbi.nlm.nih.gov/. Following identification of proteins through spectral-spectral correlation using the SEQUEST program, masses detected in MALDI-TOF mass spectra were assigned to tryptic digest peptides within the proteins identified. In cases where no amino acid sequences could be identified through searching with uninterpreted MS/MS spectra of tryptic digest peptides using the SEQUEST program, tandem mass spectra of the peptides were interpreted manually, using methods known in the art. (In the case of interpretation of low-energy fragmentation mass spectra of peptide ions see Gaskell et al., 1992, Rapid Commun. Mass Spectrom. 6:658-662).

2.1.14 - Discrimination of colorectal cancer associated proteins The process described in Example 1 section 1.1.6 was employed to discriminate the colorectal cancer associated proteins in the experimental samples.

*2.2 RESULTS*

These experiments identified the CRCMPs which are listed in Table 2.

EXAMPLE 3: EVALUATION OF COLORECTAL CANCER MARKER PROTEINS IN SANDWICH ELISA

Using the following Reference Protocol, the Colorectal Cancer Marker Proteins (CRCMPs) listed in Tables 1 and 2 were evaluated in a sandwich ELISA.

## *3.1 MATERIALS AND METHODS*

Antibodies for the sandwich ELISAs were developed at Biosite. Biotinylated antibody (primary antibody) was diluted into assay buffer (10 mM Tris, 150 mM NaCl, 1% BSA) to 2 ug/ml and added to 384 well neutravidin coated plate (Pierce Chemical Company, Rockford IL) and allowed to incubate at room temperature for 1 hour. Wells were then washed with wash buffer (20mM Borate, 150 mM NaCl, 0.2% Tween 20). Samples and standards were added and allowed to incubate at room temperature for 1 hour. Wells again were washed. An antibody conjugated to fluorscein (secondary antibody) was diluted into assay buffer to 2 ug/ml and was then added to the plate and allowed to incubate at room temperature for 1 hour. Wells again were washed. Anti-fluorscein antibody conjugated to alkaline phosphatase, diluted 1/2338 into assay buffer, was added and allowed to incubate at room temperature for 1 hour. Final wash was then performed. Finally substrate (Promega Attophos Product#S1011, Promega Corporation, Madison, WI) was added and the plate was read immediately. All additions were 10 ul/well. The plate was washed 3 times between each addition and final wash was 9 times prior to the addition of substrate. Standards were prepared by spiking specific antigen into a normal serum patient pool. Reading was performed using a Tecan Spectrafluor plus (Tecan Inc, Mannedorf, Switzerland) in kinetic mode for 6 read cycles with excitation filter of 430nm and an emission filter 570nm emission. Slope of RFU/seconds was determined.

Final Box and ROC results were analyzed using Analyse-it General + Clinical Laboratory 1.73 (Analyse-it Software Ltd., Leeds England).

## *3.2 RESULTS*

These experiments identified CRCMPs of particular interest including, but not limited to, CRCMP#19 (SEQ ID No: 13), CRCMP#9 (SEQ ID No: 7), CRCMP#6 (SEQ ID No: 4), CRCMP#22 (SEQ ID No: 15) and CRCMP#10 (SEQ ID No: 8).

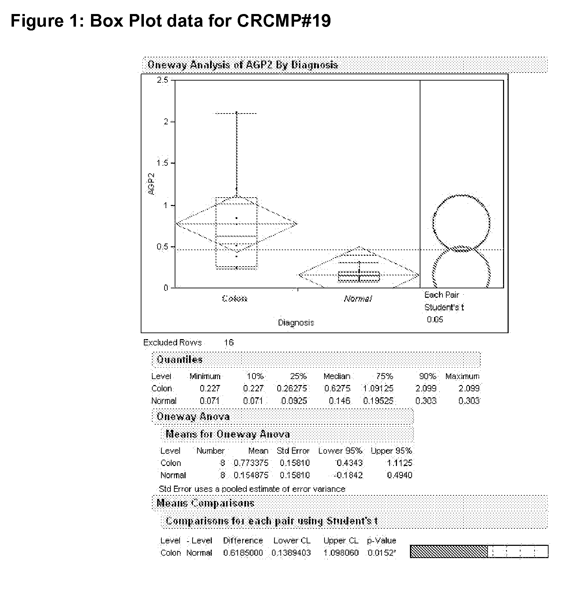

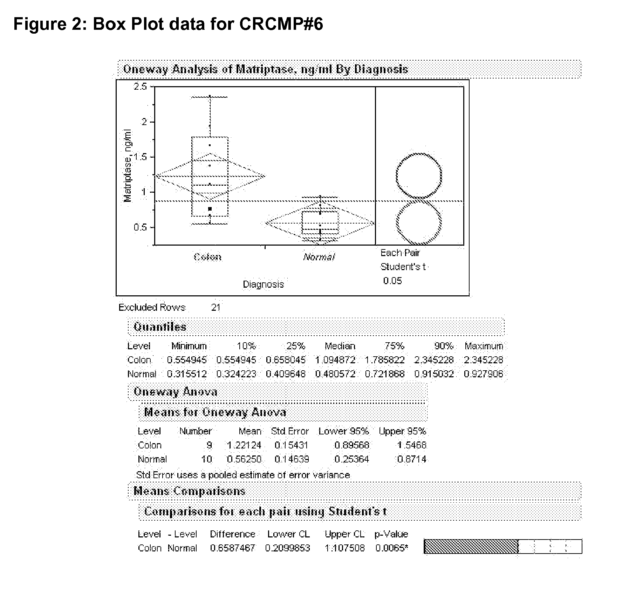

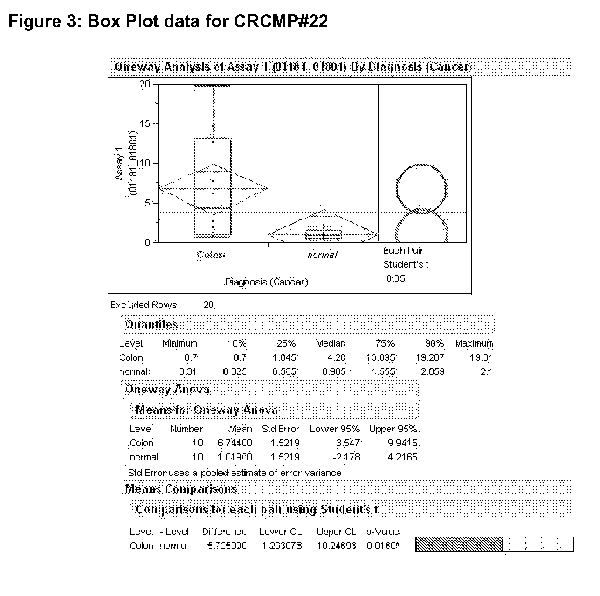

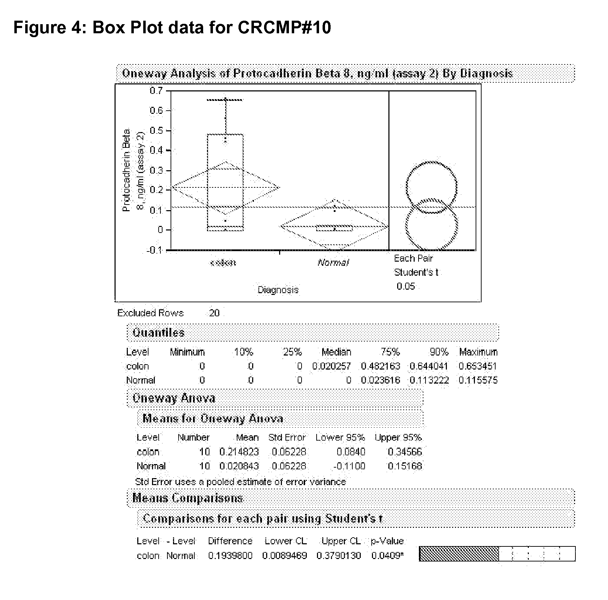

Figures 1-4 show Box plot data for CRCMP#19, CRCMP#6, CRCMP#22 and CRCMP#10 respectively. The vertical axes on these graphs are concentration of the CRCMP in ng/ml, except for Figure 3 where the vertical axis is signal response. These data all show higher concentration of the CRCMP in colorectal cancer samples compared to normal samples, with significant p values, thereby indicating that CRCMP#19, CRCMP#6, CRCMP#22 and CRCMP#10 discriminate well between colorectal cancer and normal, making them good potential markers for colorectal cancer.

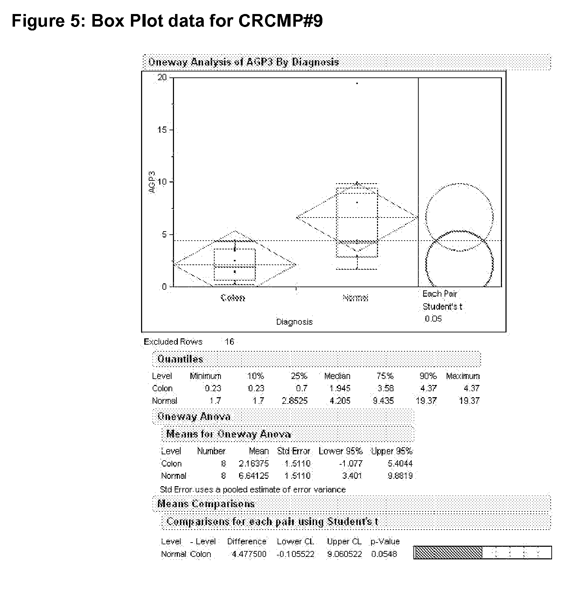

Figure 5 shows Box plot data for CRCMP#9. The vertical axis on this graph is concentration of CRCMP#9 in ng/ml. These data show decreased concentration of CRCMP#9 in colorectal cancer samples compared to normal samples, with an almost significant p value, thereby indicating that CRCMP#9 discriminates well between colorectal cancer and normal, making it a good potential marker for colorectal cancer.

EXAMPLE 4: EVALUATION OF COLORECTAL CANCER MARKER PROTEINS IN MULTIPLEX ASSAY USING LUMINEX TECHNOLOGY

Using the following Reference Protocol, Colorectal Cancer Marker Proteins (CRCMPs) listed in Tables 1 and 2 were evaluated in a multiplex assay using the Luminex technology.

## *4.1 MATERIALS AND METHODS*

Each primary antibody was conjugated to a unique Luminex magnetic microsphere (Mug beads, Luminex Corporation, Austin, TX). Mag bead cocktail (50ul) was added to a 96 black well round bottom Costar plate (Coming Incorporated, Coming NY). Using a 96 well magnetic ring stand, the Mag beads were pulled down for 1 minute and washed with wash/assay buffer (PBS with 1% BSA and 0.02% Tween 20). 50ul of sample or standard was added along with an additional 50ul of wash/assay buffer and allowed to incubate on a shaker for 1 hour at room temperature. Plate was placed on magnetic ring stand and allowed to sit for 1 minute. Mag beads were then washed again. Biotin labeled antibody was then added at 50ul per well with an additional 50ul of wash/assay buffer and allowed to incubate on a shaker for 1 hour at room temperature. The plate again was placed on a magnetic stand and the Mag beads were washed. Streptavidin-RPE (Prozyme, San Leandro, CA, Phycolin, Code#PJ31S) was diluted to 1ug/ml in wash/assay buffer and 50ul was added to each well along with an additional 50ul of wash/assay buffer and allowed to incubate on a shaker for 1 hour at room temperature. Final wash was performed and the beads were re-suspended with 100 ul of wash/assay buffer and each well was then read in a Luminex 200 reader using Xponent software 3.0. All reagent dilutions were made in wash/assay buffer. Biotin-antibody varied for each assay to optimal concentration. Initial Mag bead amounts added were approximately 50,000 for each assay. Magnetic beads were allowed 1 minute pull down time prior to each wash. Each wash step was 3 times washed with 100ul of wash/assay buffer. Assay standard curves were made in a normal donor patient serum pool. Luminex reader and Mag beads were used and prepared according to manufacturer guidelines. Standard curves were calculated using a 5 parameter log-logistic fit and each sample concentration was determined from this curve fit.

Final Box and ROC results were analyzed using Analyse-it General + Clinical Laboratory 1.73 (Analyse-it Software Ltd., Leeds England).

## 4.2 RESULTS

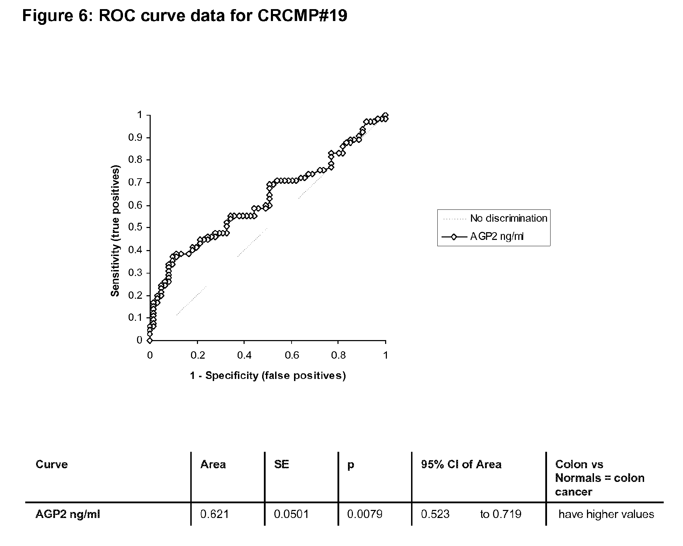

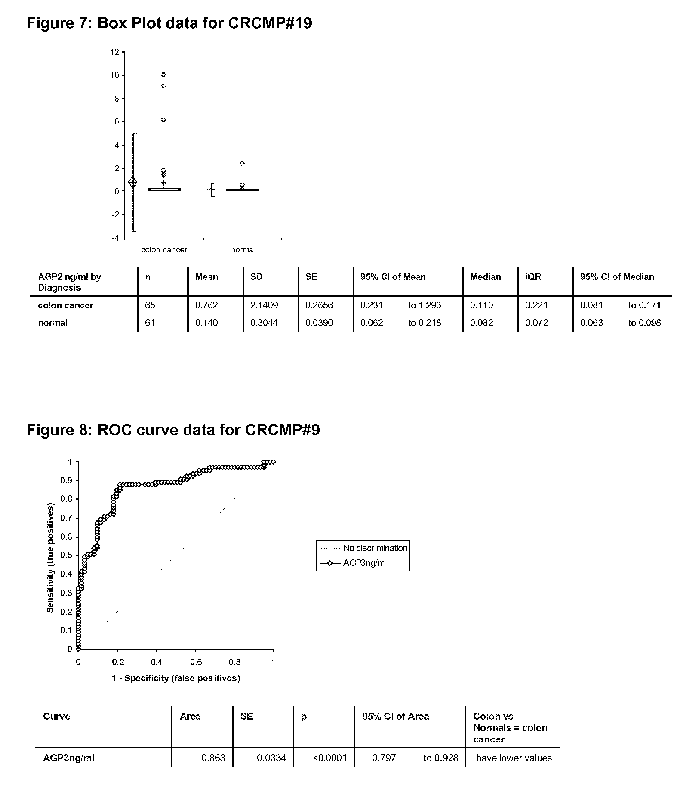

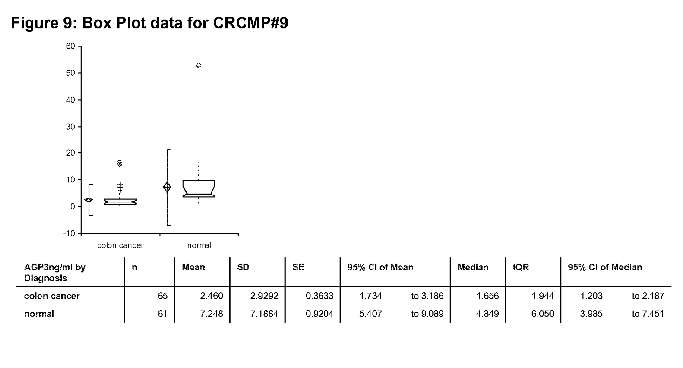

Experiments using 61 normal samples and 65 colorectal cancer samples resulted in further evidence for some of the CRCMPs of interest identified in Example 3 above, including, but not limited to, CRCMP#19 (SEQ ID No: 13) and CRCMP#9 (SEQ ID No: 7). Figure 6 shows ROC curve data for CRCMP#19 and Figure 7 shows Box plot data for CRCMP#19. Figure 8 shows ROC curve data for CRCMP#9 and Figure 9 shows Box plot data for CRCMP#9.

The ROC curves plot sensitivity (true positives) against 1-specificity (false positives). The area under the ROC curve is a measure of the probability that the measured marker level will allow correct identification of a disease or condition. An area of greater than 0.5 indicates that the marker can discriminate between disease and normal. This is the case in the data shown in Figure 6 and Figure 8 therefore indicating that both CRCMP#19 and CRCMP#9 are good potential markers to discriminate between colorectal cancer and normal. CRCMP#9 in particular has a high area under the curve and a very low p value indicating that it may be a particularly good marker for colorectal cancer.

The vertical axes on the box plots in Figure 7 and Figure 9 is concentration of the CRCMP in ng/ml. Figure 7 shows higher concentration of CRCMP#19 in colorectal cancer samples than in normal samples whereas Figure 9 shows lower concentration of CRCMP#9 in colorectal cancer samples than in normal samples. Both CRCMP#19 and CRCMP#9 show good discrimination between colorectal cancer and normal, indicating that these are both good potential markers for colorectal cancer.

All references referred to in this application, including patent and patent applications, are incorporated herein by reference to the fullest extent possible.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation, the following claims:

SEQUENCE LISTING

<110> Oxford Genome Sciences (UK) Ltd

<120> PROTEINS

<130> OGL-P104-PCT

<160> 245

<170> PatentIn version 3.3

<210> 1
<211> 832
<212> PRT
<213> Homo Sapiens


<220>
<221> MISC_FEATURE
<222> (1)..(832)
<223> Accession No: Q12864

<400> 1

Met Ile Leu Gln Ala His Leu His Ser Leu Cys Leu Leu Met Leu Tyr
1               5                   10                  15


Leu Ala Thr Gly Tyr Gly Gln Glu Gly Lys Phe Ser Gly Pro Leu Lys
            20                  25                  30


Pro Met Thr Phe Ser Ile Tyr Glu Gly Gln Glu Pro Ser Gln Ile Ile
        35                  40                  45


Phe Gln Phe Lys Ala Asn Pro Pro Ala Val Thr Phe Glu Leu Thr Gly
        50                  55                  60


Glu Thr Asp Asn Ile Phe Val Ile Glu Arg Glu Gly Leu Leu Tyr Tyr
65                  70                  75                  80


Asn Arg Ala Leu Asp Arg Glu Thr Arg Ser Thr His Asn Leu Gln Val
                85                  90                  95


Ala Ala Leu Asp Ala Asn Gly Ile Ile Val Glu Gly Pro Val Pro Ile
            100                 105                 110


Thr Ile Lys Val Lys Asp Ile Asn Asp Asn Arg Pro Thr Phe Leu Gln
            115                 120                 125


Ser Lys Tyr Glu Gly Ser Val Arg Gln Asn Ser Arg Pro Gly Lys Pro
        130                 135                 140


Phe Leu Tyr Val Asn Ala Thr Asp Leu Asp Asp Pro Ala Thr Pro Asn

145                150                155                160

Gly Gln Leu Tyr Tyr Gln Ile Val Ile Gln Leu Pro Met Ile Asn Asn
              165              170              175

Val Met Tyr Phe Gln Ile Asn Asn Lys Thr Gly Ala Ile Ser Leu Thr
              180              185              190

Arg Glu Gly Ser Gln Glu Leu Asn Pro Ala Lys Asn Pro Ser Tyr Asn
              195              200              205

Leu Val Ile Ser Val Lys Asp Met Gly Gly Gln Ser Glu Asn Ser Phe
              210              215              220

Ser Asp Thr Thr Ser Val Asp Ile Ile Val Thr Glu Asn Ile Trp Lys
225                230              235              240

Ala Pro Lys Pro Val Glu Met Val Glu Asn Ser Thr Asp Pro His Pro
              245              250              255

Ile Lys Ile Thr Gln Val Arg Trp Asn Asp Pro Gly Ala Gln Tyr Ser
              260              265              270

Leu Val Asp Lys Glu Lys Leu Pro Arg Phe Pro Phe Ser Ile Asp Gln
              275              280              285

Glu Gly Asp Ile Tyr Val Thr Gln Pro Leu Asp Arg Glu Glu Lys Asp
              290              295              300

Ala Tyr Val Phe Tyr Ala Val Ala Lys Asp Glu Tyr Gly Lys Pro Leu
305                310              315              320

Ser Tyr Pro Leu Glu Ile His Val Lys Val Lys Asp Ile Asn Asp Asn
              325              330              335

Pro Pro Thr Cys Pro Ser Pro Val Thr Val Phe Glu Val Gln Glu Asn
              340              345              350

Glu Arg Leu Gly Asn Ser Ile Gly Thr Leu Thr Ala His Asp Arg Asp
              355              360              365

Glu Glu Asn Thr Ala Asn Ser Phe Leu Asn Tyr Arg Ile Val Glu Gln
              370              375              380

Thr Pro Lys Leu Pro Met Asp Gly Leu Phe Leu Ile Gln Thr Tyr Ala

385              390              395              400

Gly Met Leu Gln Leu Ala Lys Gln Ser Leu Lys Lys Gln Asp Thr Pro
405              410              415

Gln Tyr Asn Leu Thr Ile Glu Val Ser Asp Lys Asp Phe Lys Thr Leu
420              425              430

Cys Phe Val Gln Ile Asn Val Ile Asp Ile Asn Asp Gln Ile Pro Ile
435              440              445

Phe Glu Lys Ser Asp Tyr Gly Asn Leu Thr Leu Ala Glu Asp Thr Asn
450              455              460

Ile Gly Ser Thr Ile Leu Thr Ile Gln Ala Thr Asp Ala Asp Glu Pro
465              470              475              480

Phe Thr Gly Ser Ser Lys Ile Leu Tyr His Ile Ile Lys Gly Asp Ser
485              490              495

Glu Gly Arg Leu Gly Val Asp Thr Asp Pro His Thr Asn Thr Gly Tyr
500              505              510

Val Ile Ile Lys Lys Pro Leu Asp Phe Glu Thr Ala Ala Val Ser Asn
515              520              525

Ile Val Phe Lys Ala Glu Asn Pro Glu Pro Leu Val Phe Gly Val Lys
530              535              540

Tyr Asn Ala Ser Ser Phe Ala Lys Phe Thr Leu Ile Val Thr Asp Val
545              550              555              560

Asn Glu Ala Pro Gln Phe Ser Gln His Val Phe Gln Ala Lys Val Ser
565              570              575

Glu Asp Val Ala Ile Gly Thr Lys Val Gly Asn Val Thr Ala Lys Asp
580              585              590

Pro Glu Gly Leu Asp Ile Ser Tyr Ser Leu Arg Gly Asp Thr Arg Gly
595              600              605

Trp Leu Lys Ile Asp His Val Thr Gly Glu Ile Phe Ser Val Ala Pro
610              615              620

Leu Asp Arg Glu Ala Gly Ser Pro Tyr Arg Val Gln Val Val Ala Thr

625 630 635 640

Glu Val Gly Gly Ser Ser Leu Ser Ser Val Ser Glu Phe His Leu Ile
                  645              650              655

Leu Met Asp Val Asn Asp Asn Pro Pro Arg Leu Ala Lys Asp Tyr Thr
                  660              665              670

Gly Leu Phe Phe Cys His Pro Leu Ser Ala Pro Gly Ser Leu Ile Phe
                  675              680              685

Glu Ala Thr Asp Asp Asp Gln His Leu Phe Arg Gly Pro His Phe Thr
        690              695              700

Phe Ser Leu Gly Ser Gly Ser Leu Gln Asn Asp Trp Glu Val Ser Lys
705              710              715              720

Ile Asn Gly Thr His Ala Arg Leu Ser Thr Arg His Thr Glu Phe Glu
                  725              730              735

Glu Arg Glu Tyr Val Val Leu Ile Arg Ile Asn Asp Gly Gly Arg Pro
             740              745              750

Pro Leu Glu Gly Ile Val Ser Leu Pro Val Thr Phe Cys Ser Cys Val
             755              760              765

Glu Gly Ser Cys Phe Arg Pro Ala Gly His Gln Thr Gly Ile Pro Thr
        770              775              780

Val Gly Met Ala Val Gly Ile Leu Leu Thr Thr Leu Leu Val Ile Gly
785              790              795              800

Ile Ile Leu Ala Val Val Phe Ile Arg Ile Lys Lys Asp Lys Gly Lys
                  805              810              815

Asp Asn Val Glu Ser Ala Gln Ala Ser Glu Val Lys Pro Leu Arg Ser
             820              825              830

<210> 2
<211> 319
<212> PRT
<213> Homo Sapiens


<220>
<221> MISC_FEATURE
<222> (1)..(319)

<223> Accession No: Q99795

<400> 2

Met Val Gly Lys Met Trp Pro Val Leu Trp Thr Leu Cys Ala Val Arg
1               5                   10                  15

Val Thr Val Asp Ala Ile Ser Val Glu Thr Pro Gln Asp Val Leu Arg
                20                  25                  30

Ala Ser Gln Gly Lys Ser Val Thr Leu Pro Cys Thr Tyr His Thr Ser
        35                  40                  45

Thr Ser Ser Arg Glu Gly Leu Ile Gln Trp Asp Lys Leu Leu Leu Thr
        50                  55                  60

His Thr Glu Arg Val Val Ile Trp Pro Phe Ser Asn Lys Asn Tyr Ile
65                  70                  75                  80

His Gly Glu Leu Tyr Lys Asn Arg Val Ser Ile Ser Asn Asn Ala Glu
                85                  90                  95

Gln Ser Asp Ala Ser Ile Thr Ile Asp Gln Leu Thr Met Ala Asp Asn
            100                 105                 110

Gly Thr Tyr Glu Cys Ser Val Ser Leu Met Ser Asp Leu Glu Gly Asn
            115                 120                 125

Thr Lys Ser Arg Val Arg Leu Leu Val Leu Val Pro Pro Ser Lys Pro
        130                 135                 140

Glu Cys Gly Ile Glu Gly Glu Thr Ile Ile Gly Asn Asn Ile Gln Leu
145                 150                 155                 160

Thr Cys Gln Ser Lys Glu Gly Ser Pro Thr Pro Gln Tyr Ser Trp Lys
                165                 170                 175

Arg Tyr Asn Ile Leu Asn Gln Glu Gln Pro Leu Ala Gln Pro Ala Ser
            180                 185                 190

Gly Gln Pro Val Ser Leu Lys Asn Ile Ser Thr Asp Thr Ser Gly Tyr
            195                 200                 205

Tyr Ile Cys Thr Ser Ser Asn Glu Glu Gly Thr Gln Phe Cys Asn Ile
        210                 215                 220

Thr Val Ala Val Arg Ser Pro Ser Met Asn Val Ala Leu Tyr Val Gly

```
            225                   230                   235                   240


      Ile Ala Val Gly Val Val Ala Ala Leu Ile Ile Ile Gly Ile Ile Ile
                      245                   250                   255


      Tyr Cys Cys Cys Cys Arg Gly Lys Asp Asp Asn Thr Glu Asp Lys Glu
                      260                   265                   270


      Asp Ala Arg Pro Asn Arg Glu Ala Tyr Glu Glu Pro Pro Glu Gln Leu
                      275                   280                   285


      Arg Glu Leu Ser Arg Glu Arg Glu Glu Glu Asp Asp Tyr Arg Gln Glu
                      290                   295                   300


      Glu Gln Arg Ser Thr Gly Arg Glu Ser Pro Asp His Leu Asp Gln
      305                   310                   315



      <210>  3
      <211>  1055
      <212>  PRT
      <213>  Homo Sapiens


      <220>
      <221>  MISC_FEATURE
      <222>  (1)..(1055)
      <223>  Accession No: P29323


      <400>  3

      Met Ala Leu Arg Arg Leu Gly Ala Ala Leu Leu Leu Leu Pro Leu Leu
      1                   5                   10                  15


      Ala Ala Val Glu Glu Thr Leu Met Asp Ser Thr Thr Ala Thr Ala Glu
                      20                  25                  30


      Leu Gly Trp Met Val His Pro Pro Ser Gly Trp Glu Glu Val Ser Gly
                      35                  40                  45


      Tyr Asp Glu Asn Met Asn Thr Ile Arg Thr Tyr Gln Val Cys Asn Val
                      50                  55                  60


      Phe Glu Ser Ser Gln Asn Asn Trp Leu Arg Thr Lys Phe Ile Arg Arg
      65                  70                  75                  80


      Arg Gly Ala His Arg Ile His Val Glu Met Lys Phe Ser Val Arg Asp
                      85                  90                  95


      Cys Ser Ser Ile Pro Ser Val Pro Gly Ser Cys Lys Glu Thr Phe Asn
```

                    100                    105                    110

Leu Tyr Tyr Tyr Glu Ala Asp Phe Asp Ser Ala Thr Lys Thr Phe Pro
    115                 120                 125

Asn Trp Met Glu Asn Pro Trp Val Lys Val Asp Thr Ile Ala Ala Asp
    130                 135                 140

Glu Ser Phe Ser Gln Val Asp Leu Gly Gly Arg Val Met Lys Ile Asn
145                 150                 155                 160

Thr Glu Val Arg Ser Phe Gly Pro Val Ser Arg Ser Gly Phe Tyr Leu
                165                 170                 175

Ala Phe Gln Asp Tyr Gly Gly Cys Met Ser Leu Ile Ala Val Arg Val
                180                 185                 190

Phe Tyr Arg Lys Cys Pro Arg Ile Ile Gln Asn Gly Ala Ile Phe Gln
                195                 200                 205

Glu Thr Leu Ser Gly Ala Glu Ser Thr Ser Leu Val Ala Ala Arg Gly
    210                 215                 220

Ser Cys Ile Ala Asn Ala Glu Glu Val Asp Val Pro Ile Lys Leu Tyr
225                 230                 235                 240

Cys Asn Gly Asp Gly Glu Trp Leu Val Pro Ile Gly Arg Cys Met Cys
                245                 250                 255

Lys Ala Gly Phe Glu Ala Val Glu Asn Gly Thr Val Cys Arg Gly Cys
                260                 265                 270

Pro Ser Gly Thr Phe Lys Ala Asn Gln Gly Asp Glu Ala Cys Thr His
                275                 280                 285

Cys Pro Ile Asn Ser Arg Thr Thr Ser Glu Gly Ala Thr Asn Cys Val
    290                 295                 300

Cys Arg Asn Gly Tyr Tyr Arg Ala Asp Leu Asp Pro Leu Asp Met Pro
305                 310                 315                 320

Cys Thr Thr Ile Pro Ser Ala Pro Gln Ala Val Ile Ser Ser Val Asn
                325                 330                 335

Glu Thr Ser Leu Met Leu Glu Trp Thr Pro Pro Arg Asp Ser Gly Gly

```
                    340                    345                    350


Arg Glu Asp Leu Val Tyr Asn Ile Ile Cys Lys Ser Cys Gly Ser Gly
    355                    360                    365


Arg Gly Ala Cys Thr Arg Cys Gly Asp Asn Val Gln Tyr Ala Pro Arg
    370                    375                    380


Gln Leu Gly Leu Thr Glu Pro Arg Ile Tyr Ile Ser Asp Leu Leu Ala
385                    390                    395                    400


His Thr Gln Tyr Thr Phe Glu Ile Gln Ala Val Asn Gly Val Thr Asp
                405                    410                    415


Gln Ser Pro Phe Ser Pro Gln Phe Ala Ser Val Asn Ile Thr Thr Asn
            420                    425                    430


Gln Ala Ala Pro Ser Ala Val Ser Ile Met His Gln Val Ser Arg Thr
        435                    440                    445


Val Asp Ser Ile Thr Leu Ser Trp Ser Gln Pro Asp Gln Pro Asn Gly
    450                    455                    460


Val Ile Leu Asp Tyr Glu Leu Gln Tyr Tyr Glu Lys Glu Leu Ser Glu
465                    470                    475                    480


Tyr Asn Ala Thr Ala Ile Lys Ser Pro Thr Asn Thr Val Thr Val Gln
                485                    490                    495


Gly Leu Lys Ala Gly Ala Ile Tyr Val Phe Gln Val Arg Ala Arg Thr
            500                    505                    510


Val Ala Gly Tyr Gly Arg Tyr Ser Gly Lys Met Tyr Phe Gln Thr Met
            515                    520                    525


Thr Glu Ala Glu Tyr Gln Thr Ser Ile Gln Glu Lys Leu Pro Leu Ile
        530                    535                    540


Ile Gly Ser Ser Ala Ala Gly Leu Val Phe Leu Ile Ala Val Val Val
545                    550                    555                    560


Ile Ala Ile Val Cys Asn Arg Arg Gly Phe Glu Arg Ala Asp Ser Glu
                565                    570                    575


Tyr Thr Asp Lys Leu Gln His Tyr Thr Ser Gly His Met Thr Pro Gly
```

```
                580                    585                    590

Met Lys Ile Tyr Ile Asp Pro Phe Thr Tyr Glu Asp Pro Asn Glu Ala
        595              600              605

Val Arg Glu Phe Ala Lys Glu Ile Asp Ile Ser Cys Val Lys Ile Glu
        610              615              620

Gln Val Ile Gly Ala Gly Glu Phe Gly Glu Val Cys Ser Gly His Leu
625              630              635              640

Lys Leu Pro Gly Lys Arg Glu Ile Phe Val Ala Ile Lys Thr Leu Lys
              645              650              655

Ser Gly Tyr Thr Glu Lys Gln Arg Arg Asp Phe Leu Ser Glu Ala Ser
            660              665              670

Ile Met Gly Gln Phe Asp His Pro Asn Val Ile His Leu Glu Gly Val
            675              680              685

Val Thr Lys Ser Thr Pro Val Met Ile Ile Thr Glu Phe Met Glu Asn
        690              695              700

Gly Ser Leu Asp Ser Phe Leu Arg Gln Asn Asp Gly Gln Phe Thr Val
705              710              715              720

Ile Gln Leu Val Gly Met Leu Arg Gly Ile Ala Ala Gly Met Lys Tyr
              725              730              735

Leu Ala Asp Met Asn Tyr Val His Arg Asp Leu Ala Ala Arg Asn Ile
            740              745              750

Leu Val Asn Ser Asn Leu Val Cys Lys Val Ser Asp Phe Gly Leu Ser
            755              760              765

Arg Phe Leu Glu Asp Asp Thr Ser Asp Pro Thr Tyr Thr Ser Ala Leu
        770              775              780

Gly Gly Lys Ile Pro Ile Arg Trp Thr Ala Pro Glu Ala Ile Gln Tyr
785              790              795              800

Arg Lys Phe Thr Ser Ala Ser Asp Val Trp Ser Tyr Gly Ile Val Met
              805              810              815

Trp Glu Val Met Ser Tyr Gly Glu Arg Pro Tyr Trp Asp Met Thr Asn
```

```
                    820                 825                 830

Gln Asp Val Ile Asn Ala Ile Glu Gln Asp Tyr Arg Leu Pro Pro Pro
        835                 840                 845

Met Asp Cys Pro Ser Ala Leu His Gln Leu Met Leu Asp Cys Trp Gln
        850                 855                 860

Lys Asp Arg Asn His Arg Pro Lys Phe Gly Gln Ile Val Asn Thr Leu
865                 870                 875                 880

Asp Lys Met Ile Arg Asn Pro Asn Ser Leu Lys Ala Met Ala Pro Leu
                885                 890                 895

Ser Ser Gly Ile Asn Leu Pro Leu Leu Asp Arg Thr Ile Pro Asp Tyr
            900                 905                 910

Thr Ser Phe Asn Thr Val Asp Glu Trp Leu Glu Ala Ile Lys Met Gly
        915                 920                 925

Gln Tyr Lys Glu Ser Phe Ala Asn Ala Gly Phe Thr Ser Phe Asp Val
        930                 935                 940

Val Ser Gln Met Met Met Glu Asp Ile Leu Arg Val Gly Val Thr Leu
945                 950                 955                 960

Ala Gly His Gln Lys Lys Ile Leu Asn Ser Ile Gln Val Met Arg Ala
                965                 970                 975

Gln Met Asn Gln Ile Gln Ser Val Glu Gly Gln Pro Leu Ala Arg Arg
            980                 985                 990

Pro Arg Ala Thr Gly Arg Thr Lys  Arg Cys Gln Pro Arg  Asp Val Thr
        995                 1000                1005

Lys Lys  Thr Cys Asn Ser Asn  Asp Gly Lys Lys Lys  Gly Met Gly
        1010                1015                1020

Lys Lys  Lys Thr Asp Pro Gly  Arg Gly Arg Glu Ile  Gln Gly Ile
        1025                1030                1035

Phe Phe  Lys Glu Asp Ser His  Lys Glu Ser Asn Asp  Cys Ser Cys
        1040                1045                1050

Gly Gly
```

1055

```
<210>  4
<211>  855
<212>  PRT
<213>  Homo Sapiens


<220>
<221>  MISC_FEATURE
<222>  (1)..(855)
<223>  Accession No: Q9Y5Y6

<400>  4

Met Gly Ser Asp Arg Ala Arg Lys Gly Gly Gly Pro Lys Asp Phe
1               5                   10                  15


Gly Ala Gly Leu Lys Tyr Asn Ser Arg His Glu Lys Val Asn Gly Leu
            20                  25                  30


Glu Glu Gly Val Glu Phe Leu Pro Val Asn Asn Val Lys Lys Val Glu
            35                  40                  45


Lys His Gly Pro Gly Arg Trp Val Val Leu Ala Ala Val Leu Ile Gly
            50                  55                  60


Leu Leu Leu Val Leu Leu Gly Ile Gly Phe Leu Val Trp His Leu Gln
65                  70                  75                  80


Tyr Arg Asp Val Arg Val Gln Lys Val Phe Asn Gly Tyr Met Arg Ile
                85                  90                  95


Thr Asn Glu Asn Phe Val Asp Ala Tyr Glu Asn Ser Asn Ser Thr Glu
            100                 105                 110


Phe Val Ser Leu Ala Ser Lys Val Lys Asp Ala Leu Lys Leu Leu Tyr
            115                 120                 125


Ser Gly Val Pro Phe Leu Gly Pro Tyr His Lys Glu Ser Ala Val Thr
            130                 135                 140


Ala Phe Ser Glu Gly Ser Val Ile Ala Tyr Tyr Trp Ser Glu Phe Ser
145                 150                 155                 160


Ile Pro Gln His Leu Val Glu Glu Ala Glu Arg Val Met Ala Glu Glu
                165                 170                 175


Arg Val Val Met Leu Pro Pro Arg Ala Arg Ser Leu Lys Ser Phe Val
```

```
                  180                    185                      190


Val Thr Ser Val Val Ala Phe Pro Thr Asp Ser Lys Thr Val Gln Arg
        195                 200                 205


Thr Gln Asp Asn Ser Cys Ser Phe Gly Leu His Ala Arg Gly Val Glu
    210                 215                 220


Leu Met Arg Phe Thr Thr Pro Gly Phe Pro Asp Ser Pro Tyr Pro Ala
225                 230                 235                 240


His Ala Arg Cys Gln Trp Ala Leu Arg Gly Asp Ala Asp Ser Val Leu
                245                 250                 255


Ser Leu Thr Phe Arg Ser Phe Asp Leu Ala Ser Cys Asp Glu Arg Gly
            260                 265                 270


Ser Asp Leu Val Thr Val Tyr Asn Thr Leu Ser Pro Met Glu Pro His
            275                 280                 285


Ala Leu Val Gln Leu Cys Gly Thr Tyr Pro Pro Ser Tyr Asn Leu Thr
        290                 295                 300


Phe His Ser Ser Gln Asn Val Leu Leu Ile Thr Leu Ile Thr Asn Thr
305                 310                 315                 320


Glu Arg Arg His Pro Gly Phe Glu Ala Thr Phe Phe Gln Leu Pro Arg
                325                 330                 335


Met Ser Ser Cys Gly Gly Arg Leu Arg Lys Ala Gln Gly Thr Phe Asn
            340                 345                 350


Ser Pro Tyr Tyr Pro Gly His Tyr Pro Pro Asn Ile Asp Cys Thr Trp
        355                 360                 365


Asn Ile Glu Val Pro Asn Asn Gln His Val Lys Val Arg Phe Lys Phe
    370                 375                 380


Phe Tyr Leu Leu Glu Pro Gly Val Pro Ala Gly Thr Cys Pro Lys Asp
385                 390                 395                 400


Tyr Val Glu Ile Asn Gly Glu Lys Tyr Cys Gly Glu Arg Ser Gln Phe
            405                 410                 415


Val Val Thr Ser Asn Ser Asn Lys Ile Thr Val Arg Phe His Ser Asp
```

                    420                         425                         430

Gln Ser Tyr Thr Asp Thr Gly Phe Leu Ala Glu Tyr Leu Ser Tyr Asp
        435                 440                 445

Ser Ser Asp Pro Cys Pro Gly Gln Phe Thr Cys Arg Thr Gly Arg Cys
        450                 455                 460

Ile Arg Lys Glu Leu Arg Cys Asp Gly Trp Ala Asp Cys Thr Asp His
465                 470                 475                 480

Ser Asp Glu Leu Asn Cys Ser Cys Asp Ala Gly His Gln Phe Thr Cys
                485                 490                 495

Lys Asn Lys Phe Cys Lys Pro Leu Phe Trp Val Cys Asp Ser Val Asn
        500                 505                 510

Asp Cys Gly Asp Asn Ser Asp Glu Gln Gly Cys Ser Cys Pro Ala Gln
        515                 520                 525

Thr Phe Arg Cys Ser Asn Gly Lys Cys Leu Ser Lys Ser Gln Gln Cys
        530                 535                 540

Asn Gly Lys Asp Asp Cys Gly Asp Gly Ser Asp Glu Ala Ser Cys Pro
545                 550                 555                 560

Lys Val Asn Val Val Thr Cys Thr Lys His Thr Tyr Arg Cys Leu Asn
                565                 570                 575

Gly Leu Cys Leu Ser Lys Gly Asn Pro Glu Cys Asp Gly Lys Glu Asp
        580                 585                 590

Cys Ser Asp Gly Ser Asp Glu Lys Asp Cys Asp Cys Gly Leu Arg Ser
        595                 600                 605

Phe Thr Arg Gln Ala Arg Val Val Gly Gly Thr Asp Ala Asp Glu Gly
        610                 615                 620

Glu Trp Pro Trp Gln Val Ser Leu His Ala Leu Gly Gln Gly His Ile
625                 630                 635                 640

Cys Gly Ala Ser Leu Ile Ser Pro Asn Trp Leu Val Ser Ala Ala His
                645                 650                 655

Cys Tyr Ile Asp Asp Arg Gly Phe Arg Tyr Ser Asp Pro Thr Gln Trp

```
                660                   665                   670


Thr Ala Phe Leu Gly Leu His Asp Gln Ser Gln Arg Ser Ala Pro Gly
    675                 680                 685


Val Gln Glu Arg Arg Leu Lys Arg Ile Ile Ser His Pro Phe Phe Asn
    690                 695                 700


Asp Phe Thr Phe Asp Tyr Asp Ile Ala Leu Leu Glu Leu Glu Lys Pro
705                 710                 715                 720


Ala Glu Tyr Ser Ser Met Val Arg Pro Ile Cys Leu Pro Asp Ala Ser
                725                 730                 735


His Val Phe Pro Ala Gly Lys Ala Ile Trp Val Thr Gly Trp Gly His
            740                 745                 750


Thr Gln Tyr Gly Gly Thr Gly Ala Leu Ile Leu Gln Lys Gly Glu Ile
        755                 760                 765


Arg Val Ile Asn Gln Thr Thr Cys Glu Asn Leu Leu Pro Gln Gln Ile
    770                 775                 780


Thr Pro Arg Met Met Cys Val Gly Phe Leu Ser Gly Gly Val Asp Ser
785                 790                 795                 800


Cys Gln Gly Asp Ser Gly Gly Pro Leu Ser Ser Val Glu Ala Asp Gly
                805                 810                 815


Arg Ile Phe Gln Ala Gly Val Val Ser Trp Gly Asp Gly Cys Ala Gln
            820                 825                 830


Arg Asn Lys Pro Gly Val Tyr Thr Arg Leu Pro Leu Phe Arg Asp Trp
        835                 840                 845


Ile Lys Glu Asn Thr Gly Val
    850                 855


<210>   5
<211>   802
<212>   PRT
<213>   Homo Sapiens


<220>
<221>   MISC_FEATURE
<222>   (1)..(802)
```

```
<223>   Accession No: P18433

<400>   5

Met Asp Ser Trp Phe Ile Leu Val Leu Leu Gly Ser Gly Leu Ile Cys
1               5               10              15

Val Ser Ala Asn Asn Ala Thr Thr Val Ala Pro Ser Val Gly Ile Thr
            20              25              30

Arg Leu Ile Asn Ser Ser Thr Ala Glu Pro Val Lys Glu Glu Ala Lys
        35              40              45

Thr Ser Asn Pro Thr Ser Ser Leu Thr Ser Leu Ser Val Ala Pro Thr
    50              55              60

Phe Ser Pro Asn Ile Thr Leu Gly Pro Thr Tyr Leu Thr Thr Val Asn
65              70              75              80

Ser Ser Asp Ser Asp Asn Gly Thr Thr Arg Thr Ala Ser Thr Asn Ser
            85              90              95

Ile Gly Ile Thr Ile Ser Pro Asn Gly Thr Trp Leu Pro Asp Asn Gln
        100             105             110

Phe Thr Asp Ala Arg Thr Glu Pro Trp Glu Gly Asn Ser Ser Thr Ala
        115             120             125

Ala Thr Thr Pro Glu Thr Phe Pro Pro Ser Asp Glu Thr Pro Ile Ile
    130             135             140

Ala Val Met Val Ala Leu Ser Ser Leu Leu Val Ile Val Phe Ile Ile
145             150             155             160

Ile Val Leu Tyr Met Leu Arg Phe Lys Lys Tyr Lys Gln Ala Gly Ser
            165             170             175

His Ser Asn Ser Lys Gln Ala Gly Ser His Ser Asn Ser Phe Arg Leu
        180             185             190

Ser Asn Gly Arg Thr Glu Asp Val Glu Pro Gln Ser Val Pro Leu Leu
        195             200             205

Ala Arg Ser Pro Ser Thr Asn Arg Lys Tyr Pro Pro Leu Pro Val Asp
    210             215             220

Lys Leu Glu Glu Glu Ile Asn Arg Arg Met Ala Asp Asp Asn Lys Leu
```

225                230                235                240

Phe Arg Glu Glu Phe Asn Ala Leu Pro Ala Cys Pro Ile Gln Ala Thr
                245                250                255

Cys Glu Ala Ala Ser Lys Glu Glu Asn Lys Glu Lys Asn Arg Tyr Val
                260                265                270

Asn Ile Leu Pro Tyr Asp His Ser Arg Val His Leu Thr Pro Val Glu
                275                280                285

Gly Val Pro Asp Ser Asp Tyr Ile Asn Ala Ser Phe Ile Asn Gly Tyr
                290                295                300

Gln Glu Lys Asn Lys Phe Ile Ala Ala Gln Gly Pro Lys Glu Glu Thr
305                310                315                320

Val Asn Asp Phe Trp Arg Met Ile Trp Glu Gln Asn Thr Ala Thr Ile
                325                330                335

Val Met Val Thr Asn Leu Lys Glu Arg Lys Glu Cys Lys Cys Ala Gln
                340                345                350

Tyr Trp Pro Asp Gln Gly Cys Trp Thr Tyr Gly Asn Ile Arg Val Ser
                355                360                365

Val Glu Asp Val Thr Val Leu Val Asp Tyr Thr Val Arg Lys Phe Cys
                370                375                380

Ile Gln Gln Val Gly Asp Met Thr Asn Arg Lys Pro Gln Arg Leu Ile
385                390                395                400

Thr Gln Phe His Phe Thr Ser Trp Pro Asp Phe Gly Val Pro Phe Thr
                405                410                415

Pro Ile Gly Met Leu Lys Phe Leu Lys Lys Val Lys Ala Cys Asn Pro
                420                425                430

Gln Tyr Ala Gly Ala Ile Val Val His Cys Ser Ala Gly Val Gly Arg
                435                440                445

Thr Gly Thr Phe Val Val Ile Asp Ala Met Leu Asp Met Met His Thr
                450                455                460

Glu Arg Lys Val Asp Val Tyr Gly Phe Val Ser Arg Ile Arg Ala Gln

67

```
       465                    470                    475                    480

Arg Cys Gln Met Val Gln Thr Asp Met Gln Tyr Val Phe Ile Tyr Gln
                485                    490                    495

Ala Leu Leu Glu His Tyr Leu Tyr Gly Asp Thr Glu Leu Glu Val Thr
                500                    505                    510

Ser Leu Glu Thr His Leu Gln Lys Ile Tyr Asn Lys Ile Pro Gly Thr
                515                    520                    525

Ser Asn Asn Gly Leu Glu Glu Glu Phe Lys Lys Leu Thr Ser Ile Lys
                530                    535                    540

Ile Gln Asn Asp Lys Met Arg Thr Gly Asn Leu Pro Ala Asn Met Lys
545                    550                    555                    560

Lys Asn Arg Val Leu Gln Ile Ile Pro Tyr Glu Phe Asn Arg Val Ile
                565                    570                    575

Ile Pro Val Lys Arg Gly Glu Glu Asn Thr Asp Tyr Val Asn Ala Ser
                580                    585                    590

Phe Ile Asp Gly Tyr Arg Gln Lys Asp Ser Tyr Ile Ala Ser Gln Gly
                595                    600                    605

Pro Leu Leu His Thr Ile Glu Asp Phe Trp Arg Met Ile Trp Glu Trp
                610                    615                    620

Lys Ser Cys Ser Ile Val Met Leu Thr Glu Leu Glu Glu Arg Gly Gln
625                    630                    635                    640

Glu Lys Cys Ala Gln Tyr Trp Pro Ser Asp Gly Leu Val Ser Tyr Gly
                645                    650                    655

Asp Ile Thr Val Glu Leu Lys Lys Glu Glu Glu Cys Glu Ser Tyr Thr
                660                    665                    670

Val Arg Asp Leu Leu Val Thr Asn Thr Arg Glu Asn Lys Ser Arg Gln
                675                    680                    685

Ile Arg Gln Phe His Phe His Gly Trp Pro Glu Val Gly Ile Pro Ser
                690                    695                    700

Asp Gly Lys Gly Met Ile Ser Ile Ile Ala Ala Val Gln Lys Gln Gln
```

705                    710                    715                    720

Gln Gln Ser Gly Asn His Pro Ile Thr Val His Cys Ser Ala Gly Ala
              725                    730                    735

Gly Arg Thr Gly Thr Phe Cys Ala Leu Ser Thr Val Leu Glu Arg Val
              740                    745                    750

Lys Ala Glu Gly Ile Leu Asp Val Phe Gln Thr Val Lys Ser Leu Arg
          755                    760                    765

Leu Gln Arg Pro His Met Val Gln Thr Leu Glu Gln Tyr Glu Phe Cys
          770                    775                    780

Tyr Lys Val Val Gln Glu Tyr Ile Asp Ala Phe Ser Asp Tyr Ala Asn
785                    790                    795                    800

Phe Lys


<210>  6
<211>  1015
<212>  PRT
<213>  Homo Sapiens


<220>
<221>  MISC_FEATURE
<222>  (1)..(1015)
<223>  Accesssion No: Q6PIM3

<400>  6

Met Arg Arg Phe Leu Arg Pro Gly His Asp Pro Val Arg Glu Arg Leu
1               5                    10                    15

Lys Arg Asp Leu Phe Gln Phe Asn Lys Thr Val Glu His Gly Phe Pro
              20                    25                    30

His Gln Pro Ser Ala Leu Gly Tyr Ser Pro Ser Leu Arg Ile Leu Ala
          35                    40                    45

Ile Gly Thr Arg Ser Gly Ala Ile Lys Leu Tyr Gly Ala Pro Gly Val
      50                    55                    60

Glu Phe Met Gly Leu His Gln Glu Asn Asn Ala Val Thr Gln Ile His
65                    70                    75                    80

Leu Leu Pro Gly Gln Cys Gln Leu Val Thr Leu Leu Asp Asp Asn Ser

```
                    85                   90                      95

Leu His Leu Trp Ser Leu Lys Val Lys Gly Gly Ala Ser Glu Leu Gln
            100                 105             110

Glu Asp Glu Ser Phe Thr Leu Arg Gly Pro Pro Gly Ala Ala Pro Ser
            115                 120             125

Ala Thr Gln Ile Thr Val Val Leu Pro His Ser Ser Cys Glu Leu Leu
        130             135             140

Tyr Leu Gly Thr Glu Ser Gly Asn Val Phe Val Val Gln Leu Pro Ala
145             150             155             160

Phe Arg Ala Leu Glu Asp Arg Thr Ile Ser Ser Asp Ala Val Leu Gln
            165             170             175

Arg Leu Pro Glu Glu Ala Arg His Arg Arg Val Phe Glu Met Val Glu
            180             185             190

Ala Leu Gln Glu His Pro Arg Asp Pro Asn Gln Ile Leu Ile Gly Tyr
            195             200             205

Ser Arg Gly Leu Val Val Ile Trp Asp Leu Gln Gly Ser Arg Val Leu
            210             215             220

Tyr His Phe Leu Ser Ser Gln Gln Leu Glu Asn Ile Trp Trp Gln Arg
225             230             235             240

Asp Gly Arg Leu Leu Val Ser Cys His Ser Asp Gly Ser Tyr Cys Gln
            245             250             255

Trp Pro Val Ser Ser Glu Ala Gln Gln Pro Glu Pro Leu Arg Ser Leu
            260             265             270

Val Pro Tyr Gly Pro Phe Pro Cys Lys Ala Ile Thr Arg Ile Leu Trp
            275             280             285

Leu Thr Thr Arg Gln Gly Leu Pro Phe Thr Ile Phe Gln Gly Gly Met
            290             295             300

Pro Arg Ala Ser Tyr Gly Asp Arg His Cys Ile Ser Val Ile His Asp
305             310             315             320

Gly Gln Gln Thr Ala Phe Asp Phe Thr Ser Arg Val Ile Gly Phe Thr
```

325                       330                       335

Val Leu Thr Glu Ala Asp Pro Ala Ala Thr Phe Asp Asp Pro Tyr Ala
         340              345             350

Leu Val Val Leu Ala Glu Glu Glu Leu Val Val Ile Asp Leu Gln Thr
         355              360             365

Ala Gly Trp Pro Pro Val Gln Leu Pro Tyr Leu Ala Ser Leu His Cys
       370              375             380

Ser Ala Ile Thr Cys Ser His His Val Ser Asn Ile Pro Leu Lys Leu
385             390              395             400

Trp Glu Arg Ile Ile Ala Ala Gly Ser Arg Gln Asn Ala His Phe Ser
           405              410             415

Thr Met Glu Trp Pro Ile Asp Gly Gly Thr Ser Leu Thr Pro Ala Pro
         420              425             430

Pro Gln Arg Asp Leu Leu Leu Thr Gly His Glu Asp Gly Thr Val Arg
         435              440             445

Phe Trp Asp Ala Ser Gly Val Cys Leu Arg Leu Leu Tyr Lys Leu Ser
         450              455             460

Thr Val Arg Val Phe Leu Thr Asp Thr Asp Pro Asn Glu Asn Phe Ser
465             470              475             480

Ala Gln Gly Glu Asp Glu Trp Pro Pro Leu Arg Lys Val Gly Ser Phe
           485              490             495

Asp Pro Tyr Ser Asp Asp Pro Arg Leu Gly Ile Gln Lys Ile Phe Leu
         500              505             510

Cys Lys Tyr Ser Gly Tyr Leu Ala Val Ala Gly Thr Ala Gly Gln Val
         515              520             525

Leu Val Leu Glu Leu Asn Asp Glu Ala Ala Glu Gln Ala Val Glu Gln
         530              535             540

Val Glu Ala Asp Leu Leu Gln Asp Gln Glu Gly Tyr Arg Trp Lys Gly
545             550              555             560

His Glu Arg Leu Ala Ala Arg Ser Gly Pro Val Arg Phe Glu Pro Gly

565                     570                     575

Phe Gln Pro Phe Val Leu Val Gln Cys Gln Pro Pro Ala Val Val Thr
        580                     585                     590

Ser Leu Ala Leu His Ser Glu Trp Arg Leu Val Ala Phe Gly Thr Ser
        595                     600                     605

His Gly Phe Gly Leu Phe Asp His Gln Gln Arg Arg Gln Val Phe Val
        610                     615                     620

Lys Cys Thr Leu His Pro Ser Asp Gln Leu Ala Leu Glu Gly Pro Leu
625                     630                     635                     640

Ser Arg Val Lys Ser Leu Lys Lys Ser Leu Arg Gln Ser Phe Arg Arg
                        645                     650                     655

Met Arg Arg Ser Arg Val Ser Ser Arg Lys Arg His Pro Ala Gly Pro
        660                     665                     670

Pro Gly Glu Ala Gln Glu Gly Ser Ala Lys Ala Glu Arg Pro Gly Leu
        675                     680                     685

Gln Asn Met Glu Leu Ala Pro Val Gln Arg Lys Ile Glu Ala Arg Ser
        690                     695                     700

Ala Glu Asp Ser Phe Thr Gly Phe Val Arg Thr Leu Tyr Phe Ala Asp
705                     710                     715                     720

Thr Tyr Leu Lys Asp Ser Ser Arg His Cys Pro Ser Leu Trp Ala Gly
                        725                     730                     735

Thr Asn Gly Gly Thr Ile Tyr Ala Phe Ser Leu Arg Val Pro Pro Ala
        740                     745                     750

Glu Arg Arg Met Asp Glu Pro Val Arg Ala Glu Gln Ala Lys Glu Ile
        755                     760                     765

Gln Leu Met His Arg Ala Pro Val Val Gly Ile Leu Val Leu Asp Gly
        770                     775                     780

His Ser Val Pro Leu Pro Glu Pro Leu Glu Val Ala His Asp Leu Ser
785                     790                     795                     800

Lys Ser Pro Asp Met Gln Gly Ser His Gln Leu Leu Val Val Ser Glu

**72**

```
                    805                     810                        815


Glu Gln Phe Lys Val Phe Thr Leu Pro Lys Val Ser Ala Lys Leu Lys
          820                   825                   830


Leu Lys Leu Thr Ala Leu Glu Gly Ser Arg Val Arg Arg Val Ser Val
          835                   840                   845


Ala His Phe Gly Ser Arg Arg Ala Glu Asp Tyr Gly Glu His His Leu
      850                   855                   860


Ala Val Leu Thr Asn Leu Gly Asp Ile Gln Val Val Ser Leu Pro Leu
865                   870                   875                   880


Leu Lys Pro Gln Val Arg Tyr Ser Cys Ile Arg Arg Glu Asp Val Ser
                885                   890                   895


Gly Ile Ala Ser Cys Val Phe Thr Lys Tyr Gly Gln Gly Phe Tyr Leu
          900                   905                   910


Ile Ser Pro Ser Glu Phe Glu Arg Phe Ser Leu Ser Thr Lys Trp Leu
          915                   920                   925


Val Glu Pro Arg Cys Leu Val Asp Ser Ala Glu Thr Lys Asn His Arg
      930                   935                   940


Pro Gly Asn Gly Ala Gly Pro Lys Lys Ala Pro Ser Arg Ala Arg Asn
945                   950                   955                   960


Ser Gly Thr Gln Ser Asp Gly Glu Glu Lys Gln Pro Gly Leu Val Met
                965                   970                   975


Glu Arg Ala Leu Leu Ser Asp Glu Arg Ala Ala Thr Gly Val His Ile
          980                   985                   990


Glu Pro Pro Trp Gly Ala Ala Ser  Ala Met Ala Glu Gln  Ser Glu Trp
          995                   1000                   1005


Leu Ser  Val Gln Ala Ala Arg
    1010                   1015


<210>   7
<211>   166
<212>   PRT
<213>   Homo Sapiens


<220>
```

```
<221>   MISC_FEATURE
<222>   (1)..(166)
<223>   Accession No: Q8TD06

<400>   7

Met Met Leu His Ser Ala Leu Gly Leu Cys Leu Leu Leu Val Thr Val
1               5                   10                  15


Ser Ser Asn Leu Ala Ile Ala Ile Lys Lys Glu Lys Arg Pro Pro Gln
            20                  25                  30


Thr Leu Ser Arg Gly Trp Gly Asp Asp Ile Thr Trp Val Gln Thr Tyr
            35                  40                  45


Glu Glu Gly Leu Phe Tyr Ala Gln Lys Ser Lys Lys Pro Leu Met Val
            50                  55                  60


Ile His His Leu Glu Asp Cys Gln Tyr Ser Gln Ala Leu Lys Lys Val
65                  70                  75                  80


Phe Ala Gln Asn Glu Glu Ile Gln Glu Met Ala Gln Asn Lys Phe Ile
                85                  90                  95


Met Leu Asn Leu Met His Glu Thr Thr Asp Lys Asn Leu Ser Pro Asp
                100                 105                 110


Gly Gln Tyr Val Pro Arg Ile Met Phe Val Asp Pro Ser Leu Thr Val
                115                 120                 125


Arg Ala Asp Ile Ala Gly Arg Tyr Ser Asn Arg Leu Tyr Thr Tyr Glu
                130                 135                 140


Pro Arg Asp Leu Pro Leu Leu Ile Glu Asn Met Lys Lys Ala Leu Arg
145                 150                 155                 160


Leu Ile Gln Ser Glu Leu
                165


<210>   8
<211>   801
<212>   PRT
<213>   Homo Sapiens


<220>
<221>   MISC_FEATURE
<222>   (1)..(801)
<223>   Accession No: Q9UN66
```

<400> 8

Met Glu Ala Ser Gly Lys Leu Ile Cys Arg Gln Arg Gln Val Leu Phe
1               5                   10                  15

Ser Phe Leu Leu Leu Gly Leu Ser Leu Ala Gly Ala Ala Glu Pro Arg
            20                  25                  30

Ser Tyr Ser Val Val Glu Glu Thr Glu Gly Ser Ser Phe Val Thr Asn
        35                  40                  45

Leu Ala Lys Asp Leu Gly Leu Glu Gln Arg Glu Phe Ser Arg Arg Gly
        50                  55                  60

Val Arg Val Val Ser Arg Gly Asn Lys Leu His Leu Gln Leu Asn Gln
65                  70                  75                  80

Glu Thr Ala Asp Leu Leu Leu Asn Glu Lys Leu Asp Arg Glu Asp Leu
                85                  90                  95

Cys Gly His Thr Glu Pro Cys Val Leu Arg Phe Gln Val Leu Leu Glu
            100                 105                 110

Ser Pro Phe Glu Phe Phe Gln Ala Glu Leu Gln Val Ile Asp Ile Asn
        115                 120                 125

Asp His Ser Pro Val Phe Leu Asp Lys Gln Met Leu Val Lys Val Ser
        130                 135                 140

Glu Ser Ser Pro Pro Gly Thr Ala Phe Pro Leu Lys Asn Ala Glu Asp
145                 150                 155                 160

Leu Asp Ile Gly Gln Asn Asn Ile Glu Asn Tyr Ile Ile Ser Pro Asn
                165                 170                 175

Ser Tyr Phe Arg Val Leu Thr Arg Lys Arg Ser Asp Gly Arg Lys Tyr
            180                 185                 190

Pro Glu Leu Val Leu Asp Asn Ala Leu Asp Arg Glu Glu Glu Ala Glu
        195                 200                 205

Leu Arg Leu Thr Leu Thr Ala Leu Asp Gly Gly Ser Pro Pro Arg Ser
        210                 215                 220

Gly Thr Ala Gln Val Tyr Ile Glu Val Val Asp Val Asn Asp Asn Ala

```
            225                    230                    235                    240
```

Pro Glu Phe Gln Gln Pro Phe Tyr Arg Val Gln Ile Ser Glu Asp Ser
                245                    250                    255

Pro Ile Ser Phe Leu Val Val Lys Val Ser Ala Thr Asp Val Asp Thr
                260                    265                    270

Gly Val Asn Gly Glu Ile Ser Tyr Ser Leu Phe Gln Ala Ser Asp Glu
                275                    280                    285

Ile Ser Lys Thr Phe Lys Val Asp Phe Leu Thr Gly Glu Ile Arg Leu
                290                    295                    300

Lys Lys Gln Leu Asp Phe Glu Lys Phe Gln Ser Tyr Glu Val Asn Ile
305                    310                    315                    320

Glu Ala Arg Asp Ala Gly Gly Phe Ser Gly Lys Cys Thr Val Leu Ile
                325                    330                    335

Gln Val Ile Asp Val Asn Asp His Ala Pro Glu Val Thr Met Ser Ala
                340                    345                    350

Phe Thr Ser Pro Ile Pro Glu Asn Ala Pro Glu Thr Val Val Ala Leu
                355                    360                    365

Phe Ser Val Ser Asp Leu Asp Ser Gly Glu Asn Gly Lys Ile Ser Cys
    370                    375                    380

Ser Ile Gln Glu Asp Leu Pro Phe Leu Leu Lys Ser Ser Val Gly Asn
385                    390                    395                    400

Phe Tyr Thr Leu Leu Thr Glu Thr Pro Leu Asp Arg Glu Ser Arg Ala
                405                    410                    415

Glu Tyr Asn Val Thr Ile Thr Val Thr Asp Leu Gly Thr Pro Arg Leu
                420                    425                    430

Thr Thr His Leu Asn Met Thr Val Leu Val Ser Asp Val Asn Asp Asn
    435                    440                    445

Ala Pro Ala Phe Thr Gln Thr Ser Tyr Thr Leu Phe Val Arg Glu Asn
    450                    455                    460

Asn Ser Pro Ala Leu His Ile Gly Ser Val Ser Ala Thr Asp Arg Asp

```
             465                  470                  475                  480


    Ser Gly Thr Asn Ala Gln Val Thr Tyr Ser Leu Leu Pro Pro Gln Asp
                    485                  490                  495


    Pro His Leu Pro Leu Ala Ser Leu Val Ser Ile Asn Thr Asp Asn Gly
                    500                  505                  510


    His Leu Phe Ala Leu Arg Ser Leu Asp Tyr Glu Ala Leu Gln Ala Phe
                    515                  520                  525


    Glu Phe Arg Val Gly Ala Ser Asp Arg Gly Ser Pro Ala Leu Ser Ser
                530                  535                  540


    Glu Ala Leu Val Arg Val Leu Val Leu Asp Ala Asn Asp Asn Ser Pro
    545                  550                  555                  560


    Phe Val Leu Tyr Pro Leu Gln Asn Gly Ser Ala Pro Cys Thr Glu Leu
                    565                  570                  575


    Val Pro Arg Ala Ala Glu Pro Gly Tyr Leu Val Thr Lys Val Val Ala
                    580                  585                  590


    Val Asp Gly Asp Ser Gly Gln Asn Ala Trp Leu Ser Tyr Gln Leu Leu
                    595                  600                  605


    Lys Ala Thr Glu Pro Gly Leu Phe Gly Val Trp Ala His Asn Gly Glu
                610                  615                  620


    Val Arg Thr Ala Arg Leu Leu Ser Glu Arg Asp Ala Ala Lys Gln Arg
    625                  630                  635                  640


    Leu Val Val Leu Val Lys Asp Asn Gly Glu Pro Pro Cys Ser Ala Thr
                    645                  650                  655


    Ala Thr Leu His Leu Leu Leu Val Asp Gly Phe Ser Gln Pro Tyr Leu
                    660                  665                  670


    Pro Leu Pro Glu Ala Ala Pro Ala Gln Gly Gln Ala Asp Ser Leu Thr
                    675                  680                  685


    Val Tyr Leu Val Val Ala Leu Ala Ser Val Ser Ser Leu Phe Leu Phe
                690                  695                  700


    Ser Val Leu Leu Phe Val Ala Val Leu Leu Cys Arg Arg Ser Arg Ala
```

```
                705                  710                  715                  720
```

```
Ala Ser Val Gly Arg Cys Ser Val Pro Glu Gly Pro Phe Pro Gly His
                    725              730                  735
```

```
Leu Val Asp Val Arg Gly Thr Gly Ser Leu Ser Gln Asn Tyr Gln Tyr
                740              745                  750
```

```
Glu Val Cys Leu Ala Gly Gly Ser Gly Thr Asn Glu Phe Gln Phe Leu
            755                  760                  765
```

```
Lys Pro Val Leu Pro Asn Ile Gln Gly His Ser Phe Gly Pro Glu Met
        770                  775                  780
```

```
Glu Gln Asn Ser Asn Phe Arg Asn Gly Phe Gly Phe Ser Leu Gln Leu
785                  790                  795                  800
```

```
Lys
```

```
<210>  9
<211>  314
<212>  PRT
<213>  Homo Sapiens
```

```
<220>
<221>  MISC_FEATURE
<222>  (1)..(314)
<223>  Accession No: P16422
```

```
<400>  9
```

```
Met Ala Pro Pro Gln Val Leu Ala Phe Gly Leu Leu Leu Ala Ala Ala
1               5               10                  15
```

```
Thr Ala Thr Phe Ala Ala Ala Gln Glu Glu Cys Val Cys Glu Asn Tyr
                20              25                  30
```

```
Lys Leu Ala Val Asn Cys Phe Val Asn Asn Asn Arg Gln Cys Gln Cys
            35              40                  45
```

```
Thr Ser Val Gly Ala Gln Asn Thr Val Ile Cys Ser Lys Leu Ala Ala
        50              55                  60
```

```
Lys Cys Leu Val Met Lys Ala Glu Met Asn Gly Ser Lys Leu Gly Arg
65                  70              75                  80
```

```
Arg Ala Lys Pro Glu Gly Ala Leu Gln Asn Asn Asp Gly Leu Tyr Asp
```

                            85                      90                      95

Pro Asp Cys Asp Glu Ser Gly Leu Phe Lys Ala Lys Gln Cys Asn Gly
            100                 105                 110

Thr Ser Met Cys Trp Cys Val Asn Thr Ala Gly Val Arg Arg Thr Asp
            115                 120                 125

Lys Asp Thr Glu Ile Thr Cys Ser Glu Arg Val Arg Thr Tyr Trp Ile
    130                 135                 140

Ile Ile Glu Leu Lys His Lys Ala Arg Glu Lys Pro Tyr Asp Ser Lys
145                 150                 155                 160

Ser Leu Arg Thr Ala Leu Gln Lys Glu Ile Thr Thr Arg Tyr Gln Leu
                165                 170                 175

Asp Pro Lys Phe Ile Thr Ser Ile Leu Tyr Glu Asn Asn Val Ile Thr
            180                 185                 190

Ile Asp Leu Val Gln Asn Ser Ser Gln Lys Thr Gln Asn Asp Val Asp
            195                 200                 205

Ile Ala Asp Val Ala Tyr Tyr Phe Glu Lys Asp Val Lys Gly Glu Ser
    210                 215                 220

Leu Phe His Ser Lys Lys Met Asp Leu Thr Val Asn Gly Glu Gln Leu
225                 230                 235                 240

Asp Leu Asp Pro Gly Gln Thr Leu Ile Tyr Tyr Val Asp Glu Lys Ala
                245                 250                 255

Pro Glu Phe Ser Met Gln Gly Leu Lys Ala Gly Val Ile Ala Val Ile
            260                 265                 270

Val Val Val Val Ile Ala Val Val Ala Gly Ile Val Val Leu Val Ile
            275                 280                 285

Ser Arg Lys Lys Arg Met Ala Lys Tyr Glu Lys Ala Glu Ile Lys Glu
    290                 295                 300

Met Gly Glu Met His Arg Glu Leu Asn Ala
305                 310

<210> 10

```
<211>   768
<212>   PRT
<213>   Homo Sapiens


<220>
<221>   MISC_FEATURE
<222>   (1)..(768)
<223>   Accession No: ENST00000322765

<400>   10

Met Leu Cys Gly Arg Trp Arg Arg Cys Arg Arg Pro Pro Glu Glu Pro
1               5                   10                  15


Pro Val Ala Ala Gln Val Ala Ala Gln Val Ala Ala Pro Val Ala Leu
            20                  25                  30


Pro Ser Pro Pro Thr Pro Ser Asp Gly Gly Thr Lys Arg Pro Gly Leu
        35                  40                  45


Arg Ala Leu Lys Lys Met Gly Leu Thr Glu Asp Glu Asp Val Arg Ala
        50                  55                  60


Met Leu Arg Gly Ser Arg Leu Arg Lys Ile Arg Ser Arg Thr Trp His
65                  70                  75                  80


Lys Glu Arg Leu Tyr Arg Leu Gln Glu Asp Gly Leu Ser Val Trp Phe
                85                  90                  95


Gln Arg Arg Ile Pro Arg Ala Pro Ser Gln His Ile Phe Phe Val Gln
                100                 105                 110


His Ile Glu Ala Val Arg Glu Gly His Gln Ser Glu Gly Leu Arg Arg
            115                 120                 125


Phe Gly Gly Ala Phe Ala Pro Ala Arg Cys Leu Thr Ile Ala Phe Lys
        130                 135                 140


Gly Arg Arg Lys Asn Leu Asp Leu Ala Ala Pro Thr Ala Glu Glu Ala
145                 150                 155                 160


Gln Arg Trp Val Arg Ala Ser Tyr Leu Arg Ala Gly Gly Ser Leu Ala
                165                 170                 175


Cys Cys Cys Tyr Phe Leu Ser Thr His Thr Trp Ile His Ser Tyr Leu
                180                 185                 190


His Arg Ala Asp Ser Asn Gln Asp Ser Lys Met Ser Phe Lys Glu Ile
```

195                    200                    205

Lys Ser Leu Leu Arg Met Val Asn Val Asp Met Asn Asp Met Tyr Ala
    210                215                220

Tyr Leu Leu Phe Lys Glu Cys Asp His Ser Asn Asn Asp Arg Leu Glu
225                230                235                240

Gly Ala Glu Ile Glu Glu Phe Leu Arg Arg Leu Leu Lys Arg Pro Glu
                245                250                255

Leu Glu Glu Ile Phe His Gln Tyr Ser Gly Glu Asp Arg Val Leu Ser
                260                265                270

Ala Pro Glu Leu Leu Glu Phe Leu Glu Asp Gln Gly Glu Glu Gly Ala
            275                280                285

Thr Leu Ala Arg Ala Gln Gln Leu Ile Gln Thr Tyr Glu Leu Asn Glu
    290                295                300

Thr Ala Lys Gln His Glu Leu Met Thr Leu Asp Gly Phe Met Met Tyr
305                310                315                320

Leu Leu Ser Pro Glu Gly Ala Ala Leu Asp Asn Thr His Thr Cys Val
                325                330                335

Phe Gln Asp Met Asn Gln Pro Leu Ala His Tyr Phe Ile Ser Ser Ser
                340                345                350

His Asn Thr Tyr Leu Thr Asp Ser Gln Ile Gly Gly Pro Ser Ser Thr
                355                360                365

Glu Ala Tyr Val Arg Ala Phe Ala Gln Gly Cys Arg Cys Val Glu Leu
    370                375                380

Asp Cys Trp Glu Gly Pro Gly Gly Glu Pro Val Ile Tyr His Gly His
385                390                395                400

Thr Leu Thr Ser Lys Ile Leu Phe Arg Asp Val Val Gln Ala Val Arg
                405                410                415

Asp His Ala Phe Thr Leu Ser Pro Tyr Pro Val Ile Leu Ser Leu Glu
            420                425                430

Asn His Cys Gly Leu Glu Gln Gln Ala Ala Met Ala Arg His Leu Cys

```
                 435                    440                    445
```

Thr Ile Leu Gly Asp Met Leu Val Thr Gln Ala Leu Asp Ser Pro Asn
    450                 455                 460

Pro Glu Glu Leu Pro Ser Pro Glu Gln Leu Lys Gly Arg Val Leu Val
465                 470                 475                 480

Lys Gly Lys Lys Leu Pro Ala Ala Arg Ser Glu Asp Gly Arg Ala Leu
                585                 490                 495

Ser Asp Arg Glu Glu Glu Glu Glu Asp Asp Glu Glu Glu Glu Glu Glu
                500                 505                 510

Val Glu Ala Ala Ala Gln Arg Arg Leu Leu His Pro Ala Pro Asn Ala
                515                 520                 525

Pro Gln Pro Cys Gln Val Ser Ser Leu Ser Glu Arg Lys Ala Lys Lys
    530                 535                 540

Leu Ile Arg Glu Ala Gly Asn Ser Phe Val Arg His Asn Ala Arg Gln
545                 550                 555                 560

Leu Thr Arg Val Tyr Pro Leu Gly Leu Arg Met Asn Ser Ala Asn Tyr
                565                 570                 575

Ser Pro Gln Glu Met Trp Asn Ser Gly Cys Gln Leu Val Ala Leu Asn
                580                 585                 590

Phe Gln Thr Pro Gly Tyr Glu Met Asp Leu Asn Ala Gly Arg Phe Leu
    595                 600                 605

Val Asn Gly Gln Cys Gly Tyr Val Leu Lys Pro Ala Cys Leu Arg Gln
    610                 615                 620

Pro Asp Ser Thr Phe Asp Pro Glu Tyr Pro Gly Pro Pro Arg Thr Thr
625                 630                 635                 640

Leu Ser Ile Gln Val Leu Thr Ala Gln Gln Leu Pro Lys Leu Asn Ala
                645                 650                 655

Glu Lys Pro His Ser Ile Val Asp Pro Leu Val Arg Ile Glu Ile His
                660                 665                 670

Gly Val Pro Ala Asp Cys Ala Arg Gln Glu Thr Asp Tyr Val Leu Asn

```
                  675                    680                      685


Asn Gly Phe Asn Pro Arg Trp Gly Gln Thr Leu Gln Phe Gln Leu Arg
        690                 695                 700


Ala Pro Glu Leu Ala Leu Val Arg Phe Val Val Glu Asp Tyr Asp Ala
705                 710                 715                 720


Thr Ser Pro Asn Asp Phe Val Gly Gln Phe Thr Leu Pro Leu Ser Ser
                725                 730                 735


Leu Lys Gln Gly Tyr Arg His Ile His Leu Leu Ser Lys Asp Gly Ala
            740                 745                 750


Ser Leu Ser Pro Ala Thr Leu Phe Ile Gln Ile Arg Ile Gln Arg Ser
            755                 760                 765


<210>  11
<211>  517
<212>  PRT
<213>  Homo Sapiens


<220>
<221>  MISC_FEATURE
<222>  (1)..(517)
<223>  Accession No: O00515


<400>  11

Met Ala Val Ser Arg Lys Asp Trp Ser Ala Leu Ser Ser Leu Ala Arg
1               5                   10                  15


Gln Arg Thr Leu Glu Asp Glu Glu Glu Gln Glu Arg Glu Arg Arg Arg
            20                  25                  30


Arg His Arg Asn Leu Ser Ser Thr Thr Asp Asp Glu Ala Pro Arg Leu
        35                  40                  45


Ser Gln Asn Gly Asp Arg Gln Ala Ser Ala Ser Glu Arg Leu Pro Ser
    50                  55                  60


Val Glu Glu Ala Glu Val Pro Lys Pro Leu Pro Pro Ala Ser Lys Asp
65                  70                  75                  80


Glu Asp Glu Asp Ile Gln Ser Ile Leu Arg Thr Arg Gln Glu Arg Arg
                85                  90                  95


Gln Arg Arg Gln Val Val Glu Ala Ala Gln Ala Pro Ile Gln Glu Arg
```

83

```
                           100                     105                     110

         Leu Glu Ala Glu Glu Gly Arg Asn Ser Leu Ser Pro Val Gln Ala Thr
                         115                 120                 125

         Gln Lys Pro Leu Val Ser Lys Lys Glu Leu Glu Ile Pro Pro Arg Arg
                         130                 135                 140

         Arg Leu Ser Arg Glu Gln Arg Gly Pro Trp Pro Leu Glu Glu Glu Ser
         145                 150                 155                 160

         Leu Val Gly Arg Glu Pro Glu Glu Arg Lys Lys Gly Val Pro Glu Lys
                             165                 170                 175

         Ser Pro Val Leu Glu Lys Ser Ser Met Pro Lys Lys Thr Ala Pro Glu
                         180                 185                 190

         Lys Ser Leu Val Ser Asp Lys Thr Ser Ile Ser Glu Lys Val Leu Ala
                         195                 200                 205

         Ser Glu Lys Thr Ser Leu Ser Glu Lys Ile Ala Val Ser Glu Lys Arg
                         210                 215                 220

         Asn Ser Ser Glu Lys Lys Ser Val Leu Glu Lys Thr Ser Val Ser Glu
         225                 230                 235                 240

         Lys Ser Leu Ala Pro Gly Met Ala Leu Gly Ser Gly Arg Arg Leu Val
                             245                 250                 255

         Ser Glu Lys Ala Ser Ile Phe Glu Lys Ala Leu Ala Ser Glu Lys Ser
                         260                 265                 270

         Pro Thr Ala Asp Ala Lys Pro Ala Pro Lys Arg Ala Thr Ala Ser Glu
                         275                 280                 285

         Gln Pro Leu Ala Gln Glu Pro Pro Ala Ser Gly Gly Ser Pro Ala Thr
                         290                 295                 300

         Thr Lys Glu Gln Arg Gly Arg Ala Leu Pro Gly Lys Asn Leu Pro Ser
         305                 310                 315                 320

         Leu Ala Lys Gln Gly Ala Ser Asp Pro Pro Thr Val Ala Ser Arg Leu
                             325                 330                 335

         Pro Pro Val Thr Leu Gln Val Lys Ile Pro Ser Lys Glu Glu Glu Ala
```

84

```
                    340                    345                    350


Asp Met Ser Ser Pro Thr Gln Arg Thr Tyr Ser Ser Ser Leu Lys Arg
        355                360                365


Ser Ser Pro Arg Thr Ile Ser Phe Arg Met Lys Pro Lys Lys Glu Asn
    370                375                380


Ser Glu Thr Thr Leu Thr Arg Ser Ala Ser Met Lys Leu Pro Asp Asn
385                390                395                400


Thr Val Lys Leu Gly Glu Lys Leu Glu Arg Tyr His Thr Ala Ile Arg
                405                410                415


Arg Ser Glu Ser Val Lys Ser Arg Gly Leu Pro Cys Thr Glu Leu Phe
            420                425                430


Val Ala Pro Val Gly Val Ala Ser Lys Arg His Leu Phe Glu Lys Glu
            435                440                445


Leu Ala Gly Gln Ser Arg Ala Glu Pro Ala Ser Ser Arg Lys Glu Asn
        450                455                460


Leu Arg Leu Ser Gly Val Val Thr Ser Arg Leu Asn Leu Trp Ile Ser
465                470                475                480


Arg Thr Gln Glu Ser Gly Asp Gln Asp Pro Gln Glu Ala Gln Lys Ala
                485                490                495


Ser Ser Ala Thr Glu Arg Thr Gln Trp Gly Gln Lys Ser Asp Ser Ser
            500                505                510


Leu Asp Ala Glu Val
        515


<210>   12
<211>   733
<212>   PRT
<213>   Homo Sapiens


<220>
<221>   MISC_FEATURE
<222>   (1)..(733)
<223>   Accession No: Q96TA1


<400>   12

Met Gly Trp Met Gly Glu Lys Thr Gly Lys Ile Leu Thr Glu Phe Leu
```

```
           1                    5                         10                         15

Gln Phe Tyr Glu Asp Gln Tyr Gly Val Ala Leu Phe Asn Ser Met Arg
            20                      25                      30

His Glu Ile Glu Gly Thr Gly Leu Pro Gln Ala Gln Leu Leu Trp Arg
            35                      40                      45

Lys Val Pro Leu Asp Glu Arg Ile Val Phe Ser Gly Asn Leu Phe Gln
            50                      55                      60

His Gln Glu Asp Ser Lys Lys Trp Arg Asn Arg Phe Ser Leu Val Pro
65                      70                      75                      80

His Asn Tyr Gly Leu Val Leu Tyr Glu Asn Lys Ala Ala Tyr Glu Arg
                    85                      90                      95

Gln Val Pro Pro Arg Ala Val Ile Asn Ser Ala Gly Tyr Lys Ile Leu
            100                     105                     110

Thr Ser Val Asp Gln Tyr Leu Glu Leu Ile Gly Asn Ser Leu Pro Gly
            115                     120                     125

Thr Thr Ala Lys Ser Gly Ser Ala Pro Ile Leu Lys Cys Pro Thr Gln
            130                     135                     140

Phe Pro Leu Ile Leu Trp His Pro Tyr Ala Arg His Tyr Tyr Phe Cys
145                     150                     155                     160

Met Met Thr Glu Ala Glu Gln Asp Lys Trp Gln Ala Val Leu Gln Asp
                    165                     170                     175

Cys Ile Arg His Cys Asn Asn Gly Ile Pro Glu Asp Ser Lys Val Glu
                    180                     185                     190

Gly Pro Ala Phe Thr Asp Ala Ile Arg Met Tyr Arg Gln Ser Lys Glu
            195                     200                     205

Leu Tyr Gly Thr Trp Glu Met Leu Cys Gly Asn Glu Val Gln Ile Leu
            210                     215                     220

Ser Asn Leu Val Met Glu Glu Leu Gly Pro Glu Leu Lys Ala Glu Leu
225                     230                     235                     240

Gly Pro Arg Leu Lys Gly Lys Pro Gln Glu Arg Gln Arg Gln Trp Ile
```

```
                    245                250                255

Gln Ile Ser Asp Ala Val Tyr His Met Val Tyr Glu Gln Ala Lys Ala
            260                265                270

Arg Phe Glu Glu Val Leu Ser Lys Val Gln Gln Val Gln Pro Ala Met
            275                280                285

Gln Ala Val Ile Arg Thr Asp Met Asp Gln Ile Ile Thr Ser Lys Glu
        290                295                300

His Leu Ala Ser Lys Ile Arg Ala Phe Ile Leu Pro Lys Ala Glu Val
305                310                315                320

Cys Val Arg Asn His Val Gln Pro Tyr Ile Pro Ser Ile Leu Glu Ala
            325                330                335

Leu Met Val Pro Thr Ser Gln Gly Phe Thr Glu Val Arg Asp Val Phe
            340                345                350

Phe Lys Glu Val Thr Asp Met Asn Leu Asn Val Ile Asn Glu Gly Gly
            355                360                365

Ile Asp Lys Leu Gly Glu Tyr Met Glu Lys Leu Ser Arg Leu Ala Tyr
        370                375                380

His Pro Leu Lys Met Gln Ser Cys Tyr Glu Lys Met Glu Ser Leu Arg
385                390                395                400

Leu Asp Gly Leu Gln Gln Arg Phe Asp Val Ser Ser Thr Ser Val Phe
            405                410                415

Lys Gln Arg Ala Gln Ile His Met Arg Glu Gln Met Asp Asn Ala Val
            420                425                430

Tyr Thr Phe Glu Thr Leu Leu His Gln Glu Leu Gly Lys Gly Pro Thr
            435                440                445

Lys Glu Glu Leu Cys Lys Ser Ile Gln Arg Val Leu Glu Arg Val Leu
        450                455                460

Lys Lys Tyr Asp Tyr Asp Ser Ser Ser Val Arg Lys Arg Phe Phe Arg
465                470                475                480

Glu Ala Leu Leu Gln Ile Ser Ile Pro Phe Leu Leu Lys Lys Leu Ala
```

EP 2 375 255 A1

                        485                 490                 495

Pro Thr Cys Lys Ser Glu Leu Pro Arg Phe Gln Glu Leu Ile Phe Glu
            500                 505                 510

Asp Phe Ala Arg Phe Ile Leu Val Glu Asn Thr Tyr Glu Glu Val Val
            515                 520                 525

Leu Gln Thr Val Met Lys Asp Ile Leu Gln Ala Val Lys Glu Ala Ala
        530                 535                 540

Val Gln Arg Lys His Asn Leu Tyr Arg Asp Ser Met Val Met His Asn
545                 550                 555                 560

Ser Asp Pro Asn Leu His Leu Leu Ala Glu Gly Ala Pro Ile Asp Trp
                565                 570                 575

Gly Glu Glu Tyr Ser Asn Ser Gly Gly Gly Ser Pro Ser Pro Ser
            580                 585                 590

Thr Pro Glu Ser Ala Thr Leu Ser Glu Lys Arg Arg Arg Ala Lys Gln
            595                 600                 605

Val Val Ser Val Val Gln Asp Glu Glu Val Gly Leu Pro Phe Glu Ala
        610                 615                 620

Ser Pro Glu Ser Pro Pro Pro Ala Ser Pro Asp Gly Val Thr Glu Ile
625                 630                 635                 640

Arg Gly Leu Leu Ala Gln Gly Leu Arg Pro Glu Ser Pro Pro Pro Ala
                645                 650                 655

Gly Pro Leu Leu Asn Gly Ala Pro Ala Gly Glu Ser Pro Gln Pro Lys
                660                 665                 670

Ala Ala Pro Glu Ala Ser Ser Pro Pro Ala Ser Pro Leu Gln His Leu
            675                 680                 685

Leu Pro Gly Lys Ala Val Asp Leu Gly Pro Pro Lys Pro Ser Asp Gln
            690                 695                 700

Glu Thr Gly Glu Gln Val Ser Ser Pro Ser Ser His Pro Ala Leu His
705                 710                 715                 720

Thr Thr Thr Glu Asp Ser Ala Gly Val Gln Thr Glu Phe

88

725                          730


<210>  13
<211>  175
<212>  PRT
<213>  Homo Sapiens


<220>
<221>  MISC_FEATURE
<222>  (1)..(175)
<223>  Accession No: O95994

<400>  13

Met Glu Lys Ile Pro Val Ser Ala Phe Leu Leu Leu Val Ala Leu Ser
1               5                   10                  15


Tyr Thr Leu Ala Arg Asp Thr Thr Val Lys Pro Gly Ala Lys Lys Asp
                20                  25                  30


Thr Lys Asp Ser Arg Pro Lys Leu Pro Gln Thr Leu Ser Arg Gly Trp
                35                  40                  45


Gly Asp Gln Leu Ile Trp Thr Gln Thr Tyr Glu Glu Ala Leu Tyr Lys
                50                  55                  60


Ser Lys Thr Ser Asn Lys Pro Leu Met Ile Ile His His Leu Asp Glu
65                  70                  75                  80


Cys Pro His Ser Gln Ala Leu Lys Lys Val Phe Ala Glu Asn Lys Glu
                85                  90                  95


Ile Gln Lys Leu Ala Glu Gln Phe Val Leu Leu Asn Leu Val Tyr Glu
                100                 105                 110


Thr Thr Asp Lys His Leu Ser Pro Asp Gly Gln Tyr Val Pro Arg Ile
                115                 120                 125


Met Phe Val Asp Pro Ser Leu Thr Val Arg Ala Asp Ile Thr Gly Arg
                130                 135                 140


Tyr Ser Asn Arg Leu Tyr Ala Tyr Glu Pro Ala Asp Thr Ala Leu Leu
145                 150                 155                 160


Leu Asp Asn Met Lys Lys Ala Leu Lys Leu Leu Lys Thr Glu Leu
                165                 170                 175


<210>  14

```
<211>  1383
<212>  PRT
<213>  Homo Sapiens


<220>
<221>  MISC_FEATURE
<222>  (1)..(1383)
<223>  Accession No: Q9UHN6


<400>  14

Met Tyr Ala Thr Asp Ser Arg Gly His Ser Pro Ala Phe Leu Gln Pro
1               5                   10                  15


Gln Asn Gly Asn Ser Arg His Pro Ser Gly Tyr Val Pro Gly Lys Val
            20                  25                  30


Val Pro Leu Arg Pro Pro Pro Pro Lys Ser Gln Ala Ser Ala Lys
        35                  40                  45


Phe Thr Ser Ile Arg Arg Glu Asp Arg Ala Thr Phe Ala Phe Ser Pro
        50                  55                  60


Glu Glu Gln Gln Ala Gln Arg Glu Ser Gln Lys Gln Lys Arg His Lys
65                  70                  75                  80


Asn Thr Phe Ile Cys Phe Ala Ile Thr Ser Phe Ser Phe Phe Ile Ala
                85                  90                  95


Leu Ala Ile Ile Leu Gly Ile Ser Ser Lys Tyr Ala Pro Asp Glu Asn
            100                 105                 110


Cys Pro Asp Gln Asn Pro Arg Leu Arg Asn Trp Asp Pro Gly Gln Asp
            115                 120                 125


Ser Ala Lys Gln Val Val Ile Lys Glu Gly Asp Met Leu Arg Leu Thr
        130                 135                 140


Ser Asp Ala Thr Val His Ser Ile Val Ile Gln Asp Gly Gly Leu Leu
145                 150                 155                 160


Val Phe Gly Asp Asn Lys Asp Gly Ser Arg Asn Ile Thr Leu Arg Thr
            165                 170                 175


His Tyr Ile Leu Ile Gln Asp Gly Gly Ala Leu His Ile Gly Ala Glu
            180                 185                 190


Lys Cys Arg Tyr Lys Ser Lys Ala Thr Ile Thr Leu Tyr Gly Lys Ser
```

195　　　　　　　　　200　　　　　　　　　205

Asp Glu Gly Glu Ser Met Pro Thr Phe Gly Lys Lys Phe Ile Gly Val
　　　210　　　　　　　　215　　　　　　　　220

Glu Ala Gly Gly Thr Leu Glu Leu His Gly Ala Arg Lys Ala Ser Trp
225　　　　　　　　230　　　　　　　　235　　　　　　　　240

Thr Leu Leu Ala Arg Thr Leu Asn Ser Ser Gly Leu Pro Phe Gly Ser
　　　　　　245　　　　　　　　250　　　　　　　　255

Tyr Thr Phe Glu Lys Asp Phe Ser Arg Gly Leu Asn Val Arg Val Ile
　　　　　260　　　　　　　　265　　　　　　　270

Asp Gln Asp Thr Ala Lys Ile Leu Glu Ser Glu Arg Phe Asp Thr His
　　　　275　　　　　　　　280　　　　　　　285

Glu Tyr Arg Asn Glu Ser Arg Arg Leu Gln Glu Phe Leu Arg Phe Gln
　　　290　　　　　　　　295　　　　　　　　300

Asp Pro Gly Arg Ile Val Ala Ile Ala Val Gly Asp Ser Ala Ala Lys
305　　　　　　　　310　　　　　　　　315　　　　　　　　320

Ser Leu Leu Gln Gly Thr Ile Gln Met Ile Gln Glu Arg Leu Gly Ser
　　　　　　325　　　　　　　　330　　　　　　　　335

Glu Leu Ile Gln Gly Leu Gly Tyr Arg Gln Ala Trp Ala Leu Val Gly
　　　　340　　　　　　　　345　　　　　　　350

Val Ile Asp Gly Gly Ser Thr Ser Cys Asn Glu Ser Val Arg Asn Tyr
　　　　355　　　　　　　　360　　　　　　　365

Glu Asn His Ser Ser Gly Gly Lys Ala Leu Ala Gln Arg Glu Phe Tyr
　　　370　　　　　　　　375　　　　　　　　380

Thr Val Asp Gly Gln Lys Phe Ser Val Thr Ala Tyr Ser Glu Trp Ile
385　　　　　　　　390　　　　　　　　395　　　　　　　　400

Glu Gly Val Ser Leu Ser Gly Phe Arg Val Glu Val Val Asp Gly Val
　　　　　　405　　　　　　　　410　　　　　　　　415

Lys Leu Asn Leu Leu Asp Asp Val Ser Ser Trp Lys Pro Gly Asp Gln
　　　　　　420　　　　　　　　425　　　　　　　　430

Ile Val Val Ala Ser Thr Asp Tyr Ser Met Tyr Gln Ala Glu Glu Phe

```
                    435                 440                 445


Thr Leu Leu Pro Cys Ser Glu Cys Ser His Phe Gln Val Lys Val Lys
    450                 455                 460


Glu Thr Pro Gln Phe Leu His Met Gly Glu Ile Ile Asp Gly Val Asp
465                 470                 475                 480


Met Arg Ala Glu Val Gly Ile Leu Thr Arg Asn Ile Val Ile Gln Gly
                485                 490                 495


Glu Val Glu Asp Ser Cys Tyr Ala Glu Asn Gln Cys Gln Phe Phe Asp
                500                 505                 510


Tyr Asp Thr Phe Gly Gly His Ile Met Ile Met Lys Asn Phe Thr Ser
            515                 520                 525


Val His Leu Ser Tyr Val Glu Leu Lys His Met Gly Gln Gln Gln Met
    530                 535                 540


Gly Arg Tyr Pro Val His Phe His Leu Cys Gly Asp Val Asp Tyr Lys
545                 550                 555                 560


Gly Gly Tyr Arg His Ala Thr Phe Val Asp Gly Leu Ser Ile His His
                565                 570                 575


Ser Phe Ser Arg Cys Ile Thr Val His Gly Thr Asn Gly Leu Leu Ile
            580                 585                 590


Lys Asp Thr Ile Gly Phe Asp Thr Leu Gly His Cys Phe Phe Leu Glu
            595                 600                 605


Asp Gly Ile Glu Gln Arg Asn Thr Leu Phe His Asn Leu Gly Leu Leu
            610                 615                 620


Thr Lys Pro Gly Thr Leu Leu Pro Thr Asp Arg Asn Asn Ser Met Cys
625                 630                 635                 640


Thr Thr Met Arg Asp Lys Val Phe Gly Asn Tyr Ile Pro Val Pro Ala
                645                 650                 655


Thr Asp Cys Met Ala Val Ser Thr Phe Trp Ile Ala His Pro Asn Asn
            660                 665                 670


Asn Leu Ile Asn Asn Ala Ala Ala Gly Ser Gln Asp Ala Gly Ile Trp
```

                    675                      680                      685

Tyr Leu Phe His Lys Glu Pro Thr Gly Glu Ser Ser Gly Leu Gln Leu
    690                  695                  700

Leu Ala Lys Pro Glu Leu Thr Pro Leu Gly Ile Phe Tyr Asn Asn Arg
705                  710                  715                  720

Val His Ser Asn Phe Lys Ala Gly Leu Phe Ile Asp Lys Gly Val Lys
                725                  730                  735

Thr Thr Asn Ser Ser Ala Ala Asp Pro Arg Glu Tyr Leu Cys Leu Asp
                740                  745                  750

Asn Ser Ala Arg Phe Arg Pro His Gln Asp Ala Asn Pro Glu Lys Pro
                755                  760                  765

Arg Val Ala Ala Leu Ile Asp Arg Leu Ile Ala Phe Lys Asn Asn Asp
    770                  775                  780

Asn Gly Ala Trp Val Arg Gly Gly Asp Ile Ile Val Gln Asn Ser Ala
785                  790                  795                  800

Phe Ala Asp Asn Gly Ile Gly Leu Thr Phe Ala Ser Asp Gly Ser Phe
                805                  810                  815

Pro Ser Asp Glu Gly Ser Ser Gln Glu Val Ser Glu Ser Leu Phe Val
                820                  825                  830

Gly Glu Ser Arg Asn Tyr Gly Phe Gln Gly Gly Gln Asn Lys Tyr Val
                835                  840                  845

Gly Thr Gly Gly Ile Asp Gln Lys Pro Arg Thr Leu Pro Arg Asn Arg
    850                  855                  860

Thr Phe Pro Ile Arg Gly Phe Gln Ile Tyr Asp Gly Pro Ile His Leu
865                  870                  875                  880

Thr Arg Ser Thr Phe Lys Lys Tyr Val Pro Thr Pro Asp Arg Tyr Ser
                885                  890                  895

Ser Ala Ile Gly Phe Leu Met Lys Asn Ser Trp Gln Ile Thr Pro Arg
                900                  905                  910

Asn Asn Ile Ser Leu Val Lys Phe Gly Pro His Val Ser Leu Asn Val

915                    920                    925

Phe Phe Gly Lys Pro Gly Pro Trp Phe Glu Asp Cys Glu Met Asp Gly
        930                    935                    940

Asp Lys Asn Ser Ile Phe His Asp Ile Asp Gly Ser Val Thr Gly Tyr
945                    950                    955                    960

Lys Asp Ala Tyr Val Gly Arg Met Asp Asn Tyr Leu Ile Arg His Pro
                965                    970                    975

Ser Cys Val Asn Val Ser Lys Trp Asn Ala Val Ile Cys Ser Gly Thr
            980                    985                    990

Tyr Ala Gln Val Tyr Val Gln Thr  Trp Ser Thr Gln Asn  Leu Ser Met
        995                    1000                    1005

Thr Ile  Thr Arg Asp Glu Tyr  Pro Ser Asn Pro Met  Val Leu Arg
    1010                    1015                    1020

Gly Ile  Asn Gln Lys Ala Ala  Phe Pro Gln Tyr Gln  Pro Val Val
    1025                    1030                    1035

Met Leu  Glu Lys Gly Tyr Thr  Ile His Trp Asn Gly  Pro Ala Pro
    1040                    1045                    1050

Arg Thr  Thr Phe Leu Tyr Leu  Val Asn Phe Asn Lys  Asn Asp Trp
    1055                    1060                    1065

Ile Arg  Val Gly Leu Cys Tyr  Pro Ser Asn Thr Ser  Phe Gln Val
    1070                    1075                    1080

Thr Phe  Gly Tyr Leu Gln Arg  Gln Asn Gly Ser Leu  Ser Lys Ile
    1085                    1090                    1095

Glu Glu  Tyr Glu Pro Val His  Ser Leu Glu Glu Leu  Gln Arg Lys
    1100                    1105                    1110

Gln Ser  Glu Arg Lys Phe Tyr  Phe Asp Ser Ser Thr  Gly Leu Leu
    1115                    1120                    1125

Phe Leu  Tyr Leu Lys Ala Lys  Ser His Arg His Gly  His Ser Tyr
    1130                    1135                    1140

Cys Ser  Ser Gln Gly Cys Glu  Arg Val Lys Ile Gln  Ala Ala Thr

```
        1145                    1150                    1155


Asp Ser  Lys Asp Ile Ser Asn  Cys Met Ala Lys Ala  Tyr Pro Gln
    1160                    1165                    1170


Tyr Tyr  Arg Lys Pro Ser Val  Val Lys Arg Met Pro  Ala Met Leu
    1175                    1180                    1185


Thr Gly  Leu Cys Gln Gly Cys  Gly Thr Arg Gln Val  Val Phe Thr
    1190                    1195                    1200


Ser Asp  Pro His Lys Ser Tyr  Leu Pro Val Gln Phe  Gln Ser Pro
    1205                    1210                    1215


Asp Lys  Ala Glu Thr Gln Arg  Gly Asp Pro Ser Val  Ile Ser Val
    1220                    1225                    1230


Asn Gly  Thr Asp Phe Thr Phe  Arg Ser Ala Gly Val  Leu Leu Leu
    1235                    1240                    1245


Val Val  Asp Pro Cys Ser Val  Pro Phe Arg Leu Thr  Glu Lys Thr
    1250                    1255                    1260


Val Phe  Pro Leu Ala Asp Val  Ser Arg Ile Glu Glu  Tyr Leu Lys
    1265                    1270                    1275


Thr Gly  Ile Pro Pro Arg Ser  Ile Val Leu Leu Ser  Thr Arg Gly
    1280                    1285                    1290


Glu Ile  Lys Gln Leu Asn Ile  Ser His Leu Leu Val  Pro Leu Gly
    1295                    1300                    1305


Leu Ala  Lys Pro Ala His Leu  Tyr Asp Lys Gly Ser  Thr Ile Phe
    1310                    1315                    1320


Leu Gly  Phe Ser Gly Asn Phe  Lys Pro Ser Trp Thr  Lys Leu Phe
    1325                    1330                    1335


Thr Ser  Pro Ala Gly Gln Gly  Leu Gly Val Leu Glu  Gln Phe Ile
    1340                    1345                    1350


Pro Leu  Gln Leu Asp Glu Tyr  Gly Cys Pro Arg Ala  Thr Thr Val
    1355                    1360                    1365


Arg Arg  Arg Asp Leu Glu Leu  Leu Lys Gln Ala Ser  Lys Ala His
```

95

```
                1370                    1375                    1380


<210>  15
<211>  764
<212>  PRT
<213>  Homo Sapiens


<220>
<221>  MISC_FEATURE
<222>  (1)..(764)
<223>  Accession No: P01833


<400>  15

Met Leu Leu Phe Val Leu Thr Cys Leu Leu Ala Val Phe Pro Ala Ile
1               5                   10                  15


Ser Thr Lys Ser Pro Ile Phe Gly Pro Glu Glu Val Asn Ser Val Glu
            20                  25                  30


Gly Asn Ser Val Ser Ile Thr Cys Tyr Tyr Pro Pro Thr Ser Val Asn
            35                  40                  45


Arg His Thr Arg Lys Tyr Trp Cys Arg Gln Gly Ala Arg Gly Gly Cys
        50                  55                  60


Ile Thr Leu Ile Ser Ser Glu Gly Tyr Val Ser Ser Lys Tyr Ala Gly
65                  70                  75                  80


Arg Ala Asn Leu Thr Asn Phe Pro Glu Asn Gly Thr Phe Val Val Asn
                85                  90                  95


Ile Ala Gln Leu Ser Gln Asp Asp Ser Gly Arg Tyr Lys Cys Gly Leu
            100                 105                 110


Gly Ile Asn Ser Arg Gly Leu Ser Phe Asp Val Ser Leu Glu Val Ser
            115                 120                 125


Gln Gly Pro Gly Leu Leu Asn Asp Thr Lys Val Tyr Thr Val Asp Leu
        130                 135                 140


Gly Arg Thr Val Thr Ile Asn Cys Pro Phe Lys Thr Glu Asn Ala Gln
145                 150                 155                 160


Lys Arg Lys Ser Leu Tyr Lys Gln Ile Gly Leu Tyr Pro Val Leu Val
                165                 170                 175


Ile Asp Ser Ser Gly Tyr Val Asn Pro Asn Tyr Thr Gly Arg Ile Arg
```

```
                   180                    185                    190

    Leu Asp Ile Gln Gly Thr Gly Gln Leu Leu Phe Ser Val Val Ile Asn
            195                    200                    205


    Gln Leu Arg Leu Ser Asp Ala Gly Gln Tyr Leu Cys Gln Ala Gly Asp
            210                    215                    220


    Asp Ser Asn Ser Asn Lys Lys Asn Ala Asp Leu Gln Val Leu Lys Pro
    225                    230                    235                    240


    Glu Pro Glu Leu Val Tyr Glu Asp Leu Arg Gly Ser Val Thr Phe His
                           245                    250                    255


    Cys Ala Leu Gly Pro Glu Val Ala Asn Val Ala Lys Phe Leu Cys Arg
                   260                    265                    270


    Gln Ser Ser Gly Glu Asn Cys Asp Val Val Val Asn Thr Leu Gly Lys
            275                    280                    285


    Arg Ala Pro Ala Phe Glu Gly Arg Ile Leu Leu Asn Pro Gln Asp Lys
            290                    295                    300


    Asp Gly Ser Phe Ser Val Val Ile Thr Gly Leu Arg Lys Glu Asp Ala
    305                    310                    315                    320


    Gly Arg Tyr Leu Cys Gly Ala His Ser Asp Gly Gln Leu Gln Glu Gly
                   325                    330                    335


    Ser Pro Ile Gln Ala Trp Gln Leu Phe Val Asn Glu Glu Ser Thr Ile
            340                    345                    350


    Pro Arg Ser Pro Thr Val Val Lys Gly Val Ala Gly Gly Ser Val Ala
            355                    360                    365


    Val Leu Cys Pro Tyr Asn Arg Lys Glu Ser Lys Ser Ile Lys Tyr Trp
            370                    375                    380


    Cys Leu Trp Glu Gly Ala Gln Asn Gly Arg Cys Pro Leu Leu Val Asp
    385                    390                    395                    400


    Ser Glu Gly Trp Val Lys Ala Gln Tyr Glu Gly Arg Leu Ser Leu Leu
                   405                    410                    415


    Glu Glu Pro Gly Asn Gly Thr Phe Thr Val Ile Leu Asn Gln Leu Thr
```

                420                     425                     430

Ser Arg Asp Ala Gly Phe Tyr Trp Cys Leu Thr Asn Gly Asp Thr Leu
    435                     440                     445

Trp Arg Thr Thr Val Glu Ile Lys Ile Ile Glu Gly Glu Pro Asn Leu
    450                     455                     460

Lys Val Pro Gly Asn Val Thr Ala Val Leu Gly Glu Thr Leu Lys Val
465                     470                     475                     480

Pro Cys His Phe Pro Cys Lys Phe Ser Ser Tyr Glu Lys Tyr Trp Cys
                485                     490                     495

Lys Trp Asn Asn Thr Gly Cys Gln Ala Leu Pro Ser Gln Asp Glu Gly
                500                     505                     510

Pro Ser Lys Ala Phe Val Asn Cys Asp Glu Asn Ser Arg Leu Val Ser
                515                     520                     525

Leu Thr Leu Asn Leu Val Thr Arg Ala Asp Glu Gly Trp Tyr Trp Cys
    530                     535                     540

Gly Val Lys Gln Gly His Phe Tyr Gly Glu Thr Ala Ala Val Tyr Val
545                     550                     555                     560

Ala Val Glu Glu Arg Lys Ala Ala Gly Ser Arg Asp Val Ser Leu Ala
                565                     570                     575

Lys Ala Asp Ala Ala Pro Asp Glu Lys Val Leu Asp Ser Gly Phe Arg
                580                     585                     590

Glu Ile Glu Asn Lys Ala Ile Gln Asp Pro Arg Leu Phe Ala Glu Glu
    595                     600                     605

Lys Ala Val Ala Asp Thr Arg Asp Gln Ala Asp Gly Ser Arg Ala Ser
    610                     615                     620

Val Asp Ser Gly Ser Ser Glu Glu Gln Gly Gly Ser Ser Arg Ala Leu
625                     630                     635                     640

Val Ser Thr Leu Val Pro Leu Gly Leu Val Leu Ala Val Gly Ala Val
                645                     650                     655

Ala Val Gly Val Ala Arg Ala Arg His Arg Lys Asn Val Asp Arg Val

```
              660                665                670

Ser Ile Arg Ser Tyr Arg Thr Asp Ile Ser Met Ser Asp Phe Glu Asn
        675                680                685


Ser Arg Glu Phe Gly Ala Asn Asp Asn Met Gly Ala Ser Ser Ile Thr
    690                695                700


Gln Glu Thr Ser Leu Gly Gly Lys Glu Glu Phe Val Ala Thr Thr Glu
705                710                715                720


Ser Thr Thr Glu Thr Lys Glu Pro Lys Lys Ala Lys Arg Ser Ser Lys
                725                730                735


Glu Glu Ala Glu Met Ala Tyr Lys Asp Phe Leu Leu Gln Ser Ser Thr
                740                745                750


Val Ala Ala Glu Ala Gln Asp Gly Pro Gln Glu Ala
        755                760
```

```
<210>  16
<211>  318
<212>  PRT
<213>  Homo Sapiens


<220>
<221>  MISC_FEATURE
<222>  (1)..(318)
<223>  Accession No: Q92820

<400>  16

Met Ala Ser Pro Gly Cys Leu Leu Cys Val Leu Gly Leu Leu Leu Cys
1              5                10                15


Gly Ala Ala Ser Leu Glu Leu Ser Arg Pro His Gly Asp Thr Ala Lys
                20                25                30


Lys Pro Ile Ile Gly Ile Leu Met Gln Lys Cys Arg Asn Lys Val Met
            35                40                45


Lys Asn Tyr Gly Arg Tyr Tyr Ile Ala Ala Ser Tyr Val Lys Tyr Leu
        50                55                60


Glu Ser Ala Gly Ala Arg Val Val Pro Val Arg Leu Asp Leu Thr Glu
65                70                75                80


Lys Asp Tyr Glu Ile Leu Phe Lys Ser Ile Asn Gly Ile Leu Phe Pro
```

                        85                    90                    95

Gly Gly Ser Val Asp Leu Arg Arg Ser Asp Tyr Ala Lys Val Ala Lys
            100                 105                 110

Ile Phe Tyr Asn Leu Ser Ile Gln Ser Phe Asp Asp Gly Asp Tyr Phe
            115                 120                 125

Pro Val Trp Gly Thr Cys Leu Gly Phe Glu Glu Leu Ser Leu Leu Ile
        130                 135                 140

Ser Gly Glu Cys Leu Leu Thr Ala Thr Asp Thr Val Asp Val Ala Met
145                 150                 155                 160

Pro Leu Asn Phe Thr Gly Gly Gln Leu His Ser Arg Met Phe Gln Asn
                165                 170                 175

Phe Pro Thr Glu Leu Leu Leu Ser Leu Ala Val Glu Pro Leu Thr Ala
            180                 185                 190

Asn Phe His Lys Trp Ser Leu Ser Val Lys Asn Phe Thr Met Asn Glu
            195                 200                 205

Lys Leu Lys Lys Phe Phe Asn Val Leu Thr Thr Asn Thr Asp Gly Lys
            210                 215                 220

Ile Glu Phe Ile Ser Thr Met Glu Gly Tyr Lys Tyr Pro Val Tyr Gly
225                 230                 235                 240

Val Gln Trp His Pro Glu Lys Ala Pro Tyr Glu Trp Lys Asn Leu Asp
                245                 250                 255

Gly Ile Ser His Ala Pro Asn Ala Val Lys Thr Ala Phe Tyr Leu Ala
            260                 265                 270

Glu Phe Phe Val Asn Glu Ala Arg Lys Asn Asn His His Phe Lys Ser
            275                 280                 285

Glu Ser Glu Glu Glu Lys Ala Leu Ile Tyr Gln Phe Ser Pro Ile Tyr
        290                 295                 300

Thr Gly Asn Ile Ser Ser Phe Gln Gln Cys Tyr Ile Phe Asp
305                 310                 315

<210> 17

```
<211>   315
<212>   PRT
<213>   Homo Sapiens


<220>
<221>   MISC_FEATURE
<222>   (1)..(315)
<223>   Accession No: P27216

<400>   17

Gly Asn Arg His Ala Lys Ala Ser Ser Pro Gln Gly Phe Asp Val Asp
1               5                   10                  15


Arg Asp Ala Lys Lys Leu Asn Lys Ala Cys Lys Gly Met Gly Thr Asn
            20                  25                  30


Glu Ala Ala Ile Ile Glu Ile Leu Ser Gly Arg Thr Ser Asp Glu Arg
            35                  40                  45


Gln Gln Ile Lys Gln Lys Tyr Lys Ala Thr Tyr Gly Lys Glu Leu Glu
        50                  55                  60


Glu Val Leu Lys Ser Glu Leu Ser Gly Asn Phe Glu Lys Thr Ala Leu
65                  70                  75                  80


Ala Leu Leu Asp Arg Pro Ser Glu Tyr Ala Ala Arg Gln Leu Gln Lys
                85                  90                  95


Ala Met Lys Gly Leu Gly Thr Asp Glu Ser Val Leu Ile Glu Phe Leu
                100                 105                 110


Cys Thr Arg Thr Asn Lys Glu Ile Ile Ala Ile Lys Glu Ala Tyr Gln
            115                 120                 125


Arg Leu Phe Asp Arg Ser Leu Glu Ser Asp Val Lys Gly Asp Thr Ser
        130                 135                 140


Gly Asn Leu Lys Lys Ile Leu Val Ser Leu Leu Gln Ala Asn Arg Asn
145                 150                 155                 160


Glu Gly Asp Asp Val Asp Lys Asp Leu Ala Gly Gln Asp Ala Lys Asp
                165                 170                 175


Leu Tyr Asp Ala Gly Glu Gly Arg Trp Gly Thr Asp Glu Leu Ala Phe
            180                 185                 190


Asn Glu Val Leu Ala Lys Arg Ser Tyr Lys Gln Leu Arg Ala Thr Phe
```

```
               195                 200                 205


Gln Ala Tyr Gln Ile Leu Ile Gly Lys Asp Ile Glu Glu Ala Ile Glu
    210             215             220


Glu Glu Thr Ser Gly Asp Leu Gln Lys Ala Tyr Leu Thr Leu Val Arg
225             230             235             240


Cys Ala Gln Asp Cys Glu Asp Tyr Phe Ala Glu Arg Leu Tyr Lys Ser
                245             250             255


Met Lys Gly Ala Gly Thr Asp Glu Glu Thr Leu Ile Arg Ile Val Val
                260             265             270


Thr Arg Ala Glu Val Asp Leu Gln Gly Ile Lys Ala Lys Phe Gln Glu
        275             280             285


Lys Tyr Gln Lys Ser Leu Ser Asp Met Val Arg Ser Asp Thr Ser Gly
    290             295             300


Asp Phe Arg Lys Leu Leu Val Ala Leu Leu His
305             310             315


<210>  18
<211>  265
<212>  PRT
<213>  Homo Sapiens


<220>
<221>  MISC_FEATURE
<222>  (1)..(265)
<223>  Accession No: Q14002

<400>  18

Met Gly Ser Pro Ser Ala Cys Pro Tyr Arg Val Cys Ile Pro Trp Gln
1               5               10              15


Gly Leu Leu Leu Thr Ala Ser Leu Leu Thr Phe Trp Asn Leu Pro Asn
            20              25              30


Ser Ala Gln Thr Asn Ile Asp Val Val Pro Phe Asn Val Ala Glu Gly
        35              40              45


Lys Glu Val Leu Leu Val Val His Asn Glu Ser Gln Asn Leu Tyr Gly
    50              55              60


Tyr Asn Trp Tyr Lys Gly Glu Arg Val His Ala Asn Tyr Arg Ile Ile
```

```
            65                    70                    75                    80


Gly Tyr Val Lys Asn Ile Ser Gln Glu Asn Ala Pro Gly Pro Ala His
                85                    90                    95


Asn Gly Arg Glu Thr Ile Tyr Pro Asn Gly Thr Leu Leu Ile Gln Asn
                100                   105                   110


Val Thr His Asn Asp Ala Gly Phe Tyr Thr Leu His Val Ile Lys Glu
        115                   120                   125


Asn Leu Val Asn Glu Glu Val Thr Arg Gln Phe Tyr Val Phe Ser Glu
        130                   135                   140


Pro Pro Lys Pro Ser Ile Thr Ser Asn Asn Phe Asn Pro Val Glu Asn
145                   150                   155                   160


Lys Asp Ile Val Val Leu Thr Cys Gln Pro Glu Thr Gln Asn Thr Thr
                165                   170                   175


Tyr Leu Trp Trp Val Asn Asn Gln Ser Leu Leu Val Ser Pro Arg Leu
                180                   185                   190


Leu Leu Ser Thr Asp Asn Arg Thr Leu Val Leu Leu Ser Ala Thr Lys
        195                   200                   205


Asn Asp Ile Gly Pro Tyr Glu Cys Glu Ile Gln Asn Pro Val Gly Ala
        210                   215                   220


Ser Arg Ser Asp Pro Val Thr Leu Asn Val Arg Tyr Glu Ser Val Gln
225                   230                   235                   240


Ala Ser Ser Pro Asp Leu Ser Ala Gly Thr Ala Val Ser Ile Met Ile
                245                   250                   255


Gly Val Leu Ala Gly Met Ala Leu Ile
                260                   265


<210>   19
<211>   765
<212>   PRT
<213>   Homo Sapiens


<220>
<221>   DOMAIN
<222>   (1)..(765)
```

<223> Extracellular domain of Q12864

<400> 19

Gln Glu Gly Lys Phe Ser Gly Pro Leu Lys Pro Met Thr Phe Ser Ile
1               5                   10                  15

Tyr Glu Gly Gln Glu Pro Ser Gln Ile Ile Phe Gln Phe Lys Ala Asn
            20                  25                  30

Pro Pro Ala Val Thr Phe Glu Leu Thr Gly Glu Thr Asp Asn Ile Phe
            35                  40                  45

Val Ile Glu Arg Glu Gly Leu Leu Tyr Tyr Asn Arg Ala Leu Asp Arg
            50                  55                  60

Glu Thr Arg Ser Thr His Asn Leu Gln Val Ala Ala Leu Asp Ala Asn
65                  70                  75                  80

Gly Ile Ile Val Glu Gly Pro Val Pro Ile Thr Ile Lys Val Lys Asp
                    85                  90                  95

Ile Asn Asp Asn Arg Pro Thr Phe Leu Gln Ser Lys Tyr Glu Gly Ser
            100                 105                 110

Val Arg Gln Asn Ser Arg Pro Gly Lys Pro Phe Leu Tyr Val Asn Ala
            115                 120                 125

Thr Asp Leu Asp Asp Pro Ala Thr Pro Asn Gly Gln Leu Tyr Tyr Gln
            130                 135                 140

Ile Val Ile Gln Leu Pro Met Ile Asn Asn Val Met Tyr Phe Gln Ile
145                 150                 155                 160

Asn Asn Lys Thr Gly Ala Ile Ser Leu Thr Arg Glu Gly Ser Gln Glu
                    165                 170                 175

Leu Asn Pro Ala Lys Asn Pro Ser Tyr Asn Leu Val Ile Ser Val Lys
            180                 185                 190

Asp Met Gly Gly Gln Ser Glu Asn Ser Phe Ser Asp Thr Thr Ser Val
            195                 200                 205

Asp Ile Ile Val Thr Glu Asn Ile Trp Lys Ala Pro Lys Pro Val Glu
            210                 215                 220

Met Val Glu Asn Ser Thr Asp Pro His Pro Ile Lys Ile Thr Gln Val

                    225                 230                 235                 240

Arg Trp Asn Asp Pro Gly Ala Gln Tyr Ser Leu Val Asp Lys Glu Lys
                245                 250                 255

Leu Pro Arg Phe Pro Phe Ser Ile Asp Gln Glu Gly Asp Ile Tyr Val
                260                 265                 270

Thr Gln Pro Leu Asp Arg Glu Glu Lys Asp Ala Tyr Val Phe Tyr Ala
                275                 280                 285

Val Ala Lys Asp Glu Tyr Gly Lys Pro Leu Ser Tyr Pro Leu Glu Ile
    290                 295                 300

His Val Lys Val Lys Asp Ile Asn Asp Asn Pro Pro Thr Cys Pro Ser
305                 310                 315                 320

Pro Val Thr Val Phe Glu Val Gln Glu Asn Glu Arg Leu Gly Asn Ser
                325                 330                 335

Ile Gly Thr Leu Thr Ala His Asp Arg Asp Glu Glu Asn Thr Ala Asn
                340                 345                 350

Ser Phe Leu Asn Tyr Arg Ile Val Glu Gln Thr Pro Lys Leu Pro Met
                355                 360                 365

Asp Gly Leu Phe Leu Ile Gln Thr Tyr Ala Gly Met Leu Gln Leu Ala
                370                 375                 380

Lys Gln Ser Leu Lys Lys Gln Asp Thr Pro Gln Tyr Asn Leu Thr Ile
385                 390                 395                 400

Glu Val Ser Asp Lys Asp Phe Lys Thr Leu Cys Phe Val Gln Ile Asn
                405                 410                 415

Val Ile Asp Ile Asn Asp Gln Ile Pro Ile Phe Glu Lys Ser Asp Tyr
                420                 425                 430

Gly Asn Leu Thr Leu Ala Glu Asp Thr Asn Ile Gly Ser Thr Ile Leu
                435                 440                 445

Thr Ile Gln Ala Thr Asp Ala Asp Glu Pro Phe Thr Gly Ser Ser Lys
                450                 455                 460

Ile Leu Tyr His Ile Ile Lys Gly Asp Ser Glu Gly Arg Leu Gly Val

465                     470                     475                     480

Asp Thr Asp Pro His Thr Asn Thr Gly Tyr Val Ile Ile Lys Lys Pro
            485                 490                 495

Leu Asp Phe Glu Thr Ala Ala Val Ser Asn Ile Val Phe Lys Ala Glu
            500                 505                 510

Asn Pro Glu Pro Leu Val Phe Gly Val Lys Tyr Asn Ala Ser Ser Phe
            515                 520                 525

Ala Lys Phe Thr Leu Ile Val Thr Asp Val Asn Glu Ala Pro Gln Phe
            530                 535                 540

Ser Gln His Val Phe Gln Ala Lys Val Ser Glu Asp Val Ala Ile Gly
545                 550                 555                 560

Thr Lys Val Gly Asn Val Thr Ala Lys Asp Pro Glu Gly Leu Asp Ile
            565                 570                 575

Ser Tyr Ser Leu Arg Gly Asp Thr Arg Gly Trp Leu Lys Ile Asp His
            580                 585                 590

Val Thr Gly Glu Ile Phe Ser Val Ala Pro Leu Asp Arg Glu Ala Gly
            595                 600                 605

Ser Pro Tyr Arg Val Gln Val Val Ala Thr Glu Val Gly Gly Ser Ser
            610                 615                 620

Leu Ser Ser Val Ser Glu Phe His Leu Ile Leu Met Asp Val Asn Asp
625                 630                 635                 640

Asn Pro Pro Arg Leu Ala Lys Asp Tyr Thr Gly Leu Phe Phe Cys His
            645                 650                 655

Pro Leu Ser Ala Pro Gly Ser Leu Ile Phe Glu Ala Thr Asp Asp Asp
            660                 665                 670

Gln His Leu Phe Arg Gly Pro His Phe Thr Phe Ser Leu Gly Ser Gly
            675                 680                 685

Ser Leu Gln Asn Asp Trp Glu Val Ser Lys Ile Asn Gly Thr His Ala
            690                 695                 700

Arg Leu Ser Thr Arg His Thr Glu Phe Glu Glu Arg Glu Tyr Val Val

```
                705                 710                 715                 720



Leu Ile Arg Ile Asn Asp Gly Gly Arg Pro Pro Leu Glu Gly Ile Val
                    725                 730                 735



Ser Leu Pro Val Thr Phe Cys Ser Cys Val Glu Gly Ser Cys Phe Arg
                    740                 745                 750



Pro Ala Gly His Gln Thr Gly Ile Pro Thr Val Gly Met
        755                 760                 765



<210>  20
<211>  108
<212>  PRT
<213>  Homo Sapiens


<220>
<221>  MISC_FEATURE
<222>  (1)..(108)
<223>  Commercially available recombinant protein of Q12864

<400>  20

Glu Gly Lys Phe Ser Gly Pro Leu Lys Pro Met Thr Phe Ser Ile Tyr
1               5                   10                  15



Glu Gly Gln Glu Pro Ser Gln Ile Ile Phe Gln Phe Lys Ala Asn Pro
                20                  25                  30



Pro Ala Val Thr Phe Glu Leu Thr Gly Glu Thr Asp Asn Ile Phe Val
        35                  40                  45



Ile Glu Arg Glu Gly Leu Leu Tyr Tyr Asn Arg Ala Leu Asp Arg Glu
    50                  55                  60



Thr Arg Ser Thr His Asn Leu Gln Val Ala Ala Leu Asp Ala Asn Gly
65                  70                  75                  80



Ile Ile Val Glu Gly Pro Val Pro Ile Thr Ile Glu Val Lys Asp Ile
                85                  90                  95



Asn Asp Asn Arg Pro Thr Phe Leu Gln Ser Lys Tyr
                100                 105


<210>  21
<211>  214
<212>  PRT
<213>  Homo Sapiens
```

```
<220>
<221>   DOMAIN
<222>   (1)..(214)
<223>   Extracellualr domain of Q99795

<400>   21

Ile Ser Val Glu Thr Pro Gln Asp Val Leu Arg Ala Ser Gln Gly Lys
1               5                   10                  15


Ser Val Thr Leu Pro Cys Thr Tyr His Thr Ser Thr Ser Ser Arg Glu
            20                  25                  30


Gly Leu Ile Gln Trp Asp Lys Leu Leu Leu Thr His Thr Glu Arg Val
        35                  40                  45


Val Ile Trp Pro Phe Ser Asn Lys Asn Tyr Ile His Gly Glu Leu Tyr
        50                  55                  60


Lys Asn Arg Val Ser Ile Ser Asn Asn Ala Glu Gln Ser Asp Ala Ser
65                  70                  75                  80


Ile Thr Ile Asp Gln Leu Thr Met Ala Asp Asn Gly Thr Tyr Glu Cys
                85                  90                  95


Ser Val Ser Leu Met Ser Asp Leu Glu Gly Asn Thr Lys Ser Arg Val
            100                 105                 110


Arg Leu Leu Val Leu Val Pro Pro Ser Lys Pro Glu Cys Gly Ile Glu
        115                 120                 125


Gly Glu Thr Ile Ile Gly Asn Asn Ile Gln Leu Thr Cys Gln Ser Lys
        130                 135                 140


Glu Gly Ser Pro Thr Pro Gln Tyr Ser Trp Lys Arg Tyr Asn Ile Leu
145                 150                 155                 160


Asn Gln Glu Gln Pro Leu Ala Gln Pro Ala Ser Gly Gln Pro Val Ser
                165                 170                 175


Leu Lys Asn Ile Ser Thr Asp Thr Ser Gly Tyr Tyr Ile Cys Thr Ser
            180                 185                 190


Ser Asn Glu Glu Gly Thr Gln Phe Cys Asn Ile Thr Val Ala Val Arg
            195                 200                 205


Ser Pro Ser Met Asn Val
```

210

<210> 22
<211> 525
<212> PRT
<213> Homo Sapiens

<220>
<221> DOMAIN
<222> (1)..(525)
<223> Extracellular domain of P29323

<400> 22

Val Glu Glu Thr Leu Met Asp Ser Thr Thr Ala Thr Ala Glu Leu Gly
1               5                   10                  15

Trp Met Val His Pro Pro Ser Gly Trp Glu Glu Val Ser Gly Tyr Asp
                20                  25                  30

Glu Asn Met Asn Thr Ile Arg Thr Tyr Gln Val Cys Asn Val Phe Glu
            35                  40                  45

Ser Ser Gln Asn Asn Trp Leu Arg Thr Lys Phe Ile Arg Arg Arg Gly
        50                  55                  60

Ala His Arg Ile His Val Glu Met Lys Phe Ser Val Arg Asp Cys Ser
65                  70                  75                  80

Ser Ile Pro Ser Val Pro Gly Ser Cys Lys Glu Thr Phe Asn Leu Tyr
                85                  90                  95

Tyr Tyr Glu Ala Asp Phe Asp Ser Ala Thr Lys Thr Phe Pro Asn Trp
                100                 105                 110

Met Glu Asn Pro Trp Val Lys Val Asp Thr Ile Ala Ala Asp Glu Ser
            115                 120                 125

Phe Ser Gln Val Asp Leu Gly Gly Arg Val Met Lys Ile Asn Thr Glu
        130                 135                 140

Val Arg Ser Phe Gly Pro Val Ser Arg Ser Gly Phe Tyr Leu Ala Phe
145                 150                 155                 160

Gln Asp Tyr Gly Gly Cys Met Ser Leu Ile Ala Val Arg Val Phe Tyr
                165                 170                 175

Arg Lys Cys Pro Arg Ile Ile Gln Asn Gly Ala Ile Phe Gln Glu Thr

**109**

```
                    180                      185                      190

        Leu Ser Gly Ala Glu Ser Thr Ser Leu Val Ala Ala Arg Gly Ser Cys
                195                  200                  205

        Ile Ala Asn Ala Glu Glu Val Asp Val Pro Ile Lys Leu Tyr Cys Asn
                210                  215                  220

        Gly Asp Gly Glu Trp Leu Val Pro Ile Gly Arg Cys Met Cys Lys Ala
        225                  230                  235                  240

        Gly Phe Glu Ala Val Glu Asn Gly Thr Val Cys Arg Gly Cys Pro Ser
                        245                  250                  255

        Gly Thr Phe Lys Ala Asn Gln Gly Asp Glu Ala Cys Thr His Cys Pro
                    260                  265                  270

        Ile Asn Ser Arg Thr Thr Ser Glu Gly Ala Thr Asn Cys Val Cys Arg
                275                  280                  285

        Asn Gly Tyr Tyr Arg Ala Asp Leu Asp Pro Leu Asp Met Pro Cys Thr
                290                  295                  300

        Thr Ile Pro Ser Ala Pro Gln Ala Val Ile Ser Ser Val Asn Glu Thr
        305                  310                  315                  320

        Ser Leu Met Leu Glu Trp Thr Pro Pro Arg Asp Ser Gly Gly Arg Glu
                        325                  330                  335

        Asp Leu Val Tyr Asn Ile Ile Cys Lys Ser Cys Gly Ser Gly Arg Gly
                    340                  345                  350

        Ala Cys Thr Arg Cys Gly Asp Asn Val Gln Tyr Ala Pro Arg Gln Leu
                    355                  360                  365

        Gly Leu Thr Glu Pro Arg Ile Tyr Ile Ser Asp Leu Leu Ala His Thr
                    370                  375                  380

        Gln Tyr Thr Phe Glu Ile Gln Ala Val Asn Gly Val Thr Asp Gln Ser
        385                  390                  395                  400

        Pro Phe Ser Pro Gln Phe Ala Ser Val Asn Ile Thr Thr Asn Gln Ala
                        405                  410                  415

        Ala Pro Ser Ala Val Ser Ile Met His Gln Val Ser Arg Thr Val Asp
```

```
                420                     425                     430

Ser Ile Thr Leu Ser Trp Ser Gln Pro Asp Gln Pro Asn Gly Val Ile
        435                 440                 445

Leu Asp Tyr Glu Leu Gln Tyr Tyr Glu Lys Glu Leu Ser Glu Tyr Asn
        450                 455                 460

Ala Thr Ala Ile Lys Ser Pro Thr Asn Thr Val Thr Val Gln Gly Leu
465                 470                 475                 480

Lys Ala Gly Ala Ile Tyr Val Phe Gln Val Arg Ala Arg Thr Val Ala
                485                 490                 495

Gly Tyr Gly Arg Tyr Ser Gly Lys Met Tyr Phe Gln Thr Met Thr Glu
                500                 505                 510

Ala Glu Tyr Gln Thr Ser Ile Gln Glu Lys Leu Pro Leu
            515                 520                 525


<210>   23
<211>   100
<212>   PRT
<213>   Homo Sapiens


<220>
<221>   MISC_FEATURE
<222>   (1)..(100)
<223>   Commercially available recombinant protein of P29323

<400>   23

Cys Ile Ala Asn Ala Glu Glu Val Asp Val Pro Ile Lys Leu Tyr Cys
1               5                   10                  15

Asn Gly Asp Gly Glu Trp Leu Val Pro Ile Gly Arg Cys Met Cys Lys
            20                  25                  30

Ala Gly Phe Glu Ala Val Glu Asn Gly Thr Val Cys Arg Gly Cys Pro
            35                  40                  45

Ser Gly Thr Phe Lys Ala Asn Gln Gly Asp Glu Ala Cys Thr His Cys
            50                  55                  60

Pro Ile Asn Ser Arg Thr Thr Ser Glu Gly Ala Thr Asn Cys Val Cys
65                  70                  75                  80

Arg Asn Gly Tyr Tyr Arg Ala Asp Leu Asp Pro Leu Asp Met Pro Cys
```

```
                    85                  90                  95


Thr Thr Ile Pro
          100


<210>  24
<211>  487
<212>  PRT
<213>  Homo Sapiens


<220>
<221>  MISC_FEATURE
<222>  (1)..(487)
<223>  Commercially available recombinant protein of P29323


<400>  24

Gly Phe Glu Arg Ala Asp Ser Glu Tyr Thr Asp Lys Leu Gln His Tyr
1               5                   10                  15


Thr Ser Gly His Met Thr Pro Gly Met Lys Ile Tyr Ile Asp Pro Phe
          20                  25                  30


Thr Tyr Glu Asp Pro Asn Glu Ala Val Arg Glu Phe Ala Lys Glu Ile
          35                  40                  45


Asp Ile Ser Cys Val Lys Ile Glu Gln Val Ile Gly Ala Gly Glu Phe
          50                  55                  60


Gly Glu Val Cys Ser Gly His Leu Lys Leu Pro Gly Lys Arg Glu Ile
65                  70                  75                  80


Phe Val Ala Ile Lys Thr Leu Lys Ser Gly Tyr Thr Glu Lys Gln Arg
                  85                  90                  95


Arg Asp Phe Leu Ser Glu Ala Ser Ile Met Gly Gln Phe Asp His Pro
          100                 105                 110


Asn Val Ile His Leu Glu Gly Val Val Thr Lys Ser Thr Pro Val Met
          115                 120                 125


Ile Ile Thr Glu Phe Met Glu Asn Gly Ser Leu Asp Ser Phe Leu Arg
          130                 135                 140


Gln Asn Asp Gly Gln Phe Thr Val Ile Gln Leu Val Gly Met Leu Arg
145                 150                 155                 160


Gly Ile Ala Ala Gly Met Lys Tyr Leu Ala Asp Met Asn Tyr Val His
```

```
                    165                    170                       175

Arg Asp Leu Ala Ala Arg Asn Ile Leu Val Asn Ser Asn Leu Val Cys
            180                 185                 190

Lys Val Ser Asp Phe Gly Leu Ser Arg Phe Leu Glu Asp Asp Thr Ser
            195                 200                 205

Asp Pro Thr Tyr Thr Ser Ala Leu Gly Gly Lys Ile Pro Ile Arg Trp
            210                 215                 220

Thr Ala Pro Glu Ala Ile Gln Tyr Arg Lys Phe Thr Ser Ala Ser Asp
225                 230                 235                 240

Val Trp Ser Tyr Gly Ile Val Met Trp Glu Val Met Ser Tyr Gly Glu
                245                 250                 255

Arg Pro Tyr Trp Asp Met Thr Asn Gln Asp Val Ile Asn Ala Ile Glu
                260                 265                 270

Gln Asp Tyr Arg Leu Pro Pro Pro Met Asp Cys Pro Ser Ala Leu His
                275                 280                 285

Gln Leu Met Leu Asp Cys Trp Gln Lys Asp Arg Asn His Arg Pro Lys
            290                 295                 300

Phe Gly Gln Ile Val Asn Thr Leu Asp Lys Met Ile Arg Asn Pro Asn
305                 310                 315                 320

Ser Leu Lys Ala Met Ala Pro Leu Ser Ser Gly Ile Asn Leu Pro Leu
                325                 330                 335

Leu Asp Arg Thr Ile Pro Asp Tyr Thr Ser Phe Asn Thr Val Asp Glu
            340                 345                 350

Trp Leu Glu Ala Ile Lys Met Gly Gln Tyr Lys Glu Ser Phe Ala Asn
            355                 360                 365

Ala Gly Phe Thr Ser Phe Asp Val Val Ser Gln Met Met Met Glu Asp
            370                 375                 380

Ile Leu Arg Val Gly Val Thr Leu Ala Gly His Gln Lys Lys Ile Leu
385                 390                 395                 400

Asn Ser Ile Gln Val Met Arg Ala Gln Met Asn Gln Ile Gln Ser Val
```

```
                          405                    410                       415

Glu Gly Gln Pro Leu Ala Arg Arg Pro Arg Ala Thr Gly Arg Thr Lys
            420                 425                 430

Arg Cys Gln Pro Arg Asp Val Thr Lys Lys Thr Cys Asn Ser Asn Asp
        435                 440                 445

Gly Lys Lys Lys Gly Met Gly Lys Lys Lys Thr Asp Pro Gly Arg Gly
    450                 455                 460

Arg Glu Ile Gln Gly Ile Phe Phe Lys Glu Asp Ser His Lys Glu Ser
465                 470                 475                 480

Asn Asp Cys Ser Cys Gly Gly
                485


<210>  25
<211>  779
<212>  PRT
<213>  Homo Sapiens


<220>
<221>  DOMAIN
<222>  (1)..(779)
<223>  Extracellular domain of Q9Y5Y6

<400>  25

Trp His Leu Gln Tyr Arg Asp Val Arg Val Gln Lys Val Phe Asn Gly
1               5                   10                  15

Tyr Met Arg Ile Thr Asn Glu Asn Phe Val Asp Ala Tyr Glu Asn Ser
            20                  25                  30

Asn Ser Thr Glu Phe Val Ser Leu Ala Ser Lys Val Lys Asp Ala Leu
        35                  40                  45

Lys Leu Leu Tyr Ser Gly Val Pro Phe Leu Gly Pro Tyr His Lys Glu
    50                  55                  60

Ser Ala Val Thr Ala Phe Ser Glu Gly Ser Val Ile Ala Tyr Tyr Trp
65                  70                  75                  80

Ser Glu Phe Ser Ile Pro Gln His Leu Val Glu Glu Ala Glu Arg Val
            85                  90                  95

Met Ala Glu Glu Arg Val Val Met Leu Pro Pro Arg Ala Arg Ser Leu
```

```
                    100                    105                    110

Lys Ser Phe Val Val Thr Ser Val Val Ala Phe Pro Thr Asp Ser Lys
        115                    120                    125

Thr Val Gln Arg Thr Gln Asp Asn Ser Cys Ser Phe Gly Leu His Ala
        130                    135                    140

Arg Gly Val Glu Leu Met Arg Phe Thr Thr Pro Gly Phe Pro Asp Ser
145                    150                    155                    160

Pro Tyr Pro Ala His Ala Arg Cys Gln Trp Ala Leu Arg Gly Asp Ala
                    165                    170                    175

Asp Ser Val Leu Ser Leu Thr Phe Arg Ser Phe Asp Leu Ala Ser Cys
                    180                    185                    190

Asp Glu Arg Gly Ser Asp Leu Val Thr Val Tyr Asn Thr Leu Ser Pro
                    195                    200                    205

Met Glu Pro His Ala Leu Val Gln Leu Cys Gly Thr Tyr Pro Pro Ser
        210                    215                    220

Tyr Asn Leu Thr Phe His Ser Ser Gln Asn Val Leu Leu Ile Thr Leu
225                    230                    235                    240

Ile Thr Asn Thr Glu Arg Arg His Pro Gly Phe Glu Ala Thr Phe Phe
                    245                    250                    255

Gln Leu Pro Arg Met Ser Ser Cys Gly Gly Arg Leu Arg Lys Ala Gln
                    260                    265                    270

Gly Thr Phe Asn Ser Pro Tyr Tyr Pro Gly His Tyr Pro Pro Asn Ile
        275                    280                    285

Asp Cys Thr Trp Asn Ile Glu Val Pro Asn Asn Gln His Val Lys Val
        290                    295                    300

Arg Phe Lys Phe Phe Tyr Leu Leu Glu Pro Gly Val Pro Ala Gly Thr
305                    310                    315                    320

Cys Pro Lys Asp Tyr Val Glu Ile Asn Gly Glu Lys Tyr Cys Gly Glu
                    325                    330                    335

Arg Ser Gln Phe Val Val Thr Ser Asn Ser Asn Lys Ile Thr Val Arg
```

```
                    340                    345                    350

Phe His Ser Asp Gln Ser Tyr Thr Asp Thr Gly Phe Leu Ala Glu Tyr
        355                 360                 365

Leu Ser Tyr Asp Ser Ser Asp Pro Cys Pro Gly Gln Phe Thr Cys Arg
        370                 375                 380

Thr Gly Arg Cys Ile Arg Lys Glu Leu Arg Cys Asp Gly Trp Ala Asp
385                 390                 395                 400

Cys Thr Asp His Ser Asp Glu Leu Asn Cys Ser Cys Asp Ala Gly His
        405                 410                 415

Gln Phe Thr Cys Lys Asn Lys Phe Cys Lys Pro Leu Phe Trp Val Cys
        420                 425                 430

Asp Ser Val Asn Asp Cys Gly Asp Asn Ser Asp Glu Gln Gly Cys Ser
        435                 440                 445

Cys Pro Ala Gln Thr Phe Arg Cys Ser Asn Gly Lys Cys Leu Ser Lys
        450                 455                 460

Ser Gln Gln Cys Asn Gly Lys Asp Asp Cys Gly Asp Gly Ser Asp Glu
465                 470                 475                 480

Ala Ser Cys Pro Lys Val Asn Val Val Thr Cys Thr Lys His Thr Tyr
        485                 490                 495

Arg Cys Leu Asn Gly Leu Cys Leu Ser Lys Gly Asn Pro Glu Cys Asp
        500                 505                 510

Gly Lys Glu Asp Cys Ser Asp Gly Ser Asp Glu Lys Asp Cys Asp Cys
        515                 520                 525

Gly Leu Arg Ser Phe Thr Arg Gln Ala Arg Val Val Gly Gly Thr Asp
        530                 535                 540

Ala Asp Glu Gly Glu Trp Pro Trp Gln Val Ser Leu His Ala Leu Gly
545                 550                 555                 560

Gln Gly His Ile Cys Gly Ala Ser Leu Ile Ser Pro Asn Trp Leu Val
                565                 570                 575

Ser Ala Ala His Cys Tyr Ile Asp Asp Arg Gly Phe Arg Tyr Ser Asp
```

```
                580                    585                       590

Pro Thr Gln Trp Thr Ala Phe Leu Gly Leu His Asp Gln Ser Gln Arg
        595                 600                 605

Ser Ala Pro Gly Val Gln Glu Arg Arg Leu Lys Arg Ile Ile Ser His
        610                 615                 620

Pro Phe Phe Asn Asp Phe Thr Phe Asp Tyr Asp Ile Ala Leu Leu Glu
625                 630                 635                 640

Leu Glu Lys Pro Ala Glu Tyr Ser Ser Met Val Arg Pro Ile Cys Leu
              645                 650                 655

Pro Asp Ala Ser His Val Phe Pro Ala Gly Lys Ala Ile Trp Val Thr
              660                 665                 670

Gly Trp Gly His Thr Gln Tyr Gly Gly Thr Gly Ala Leu Ile Leu Gln
              675                 680                 685

Lys Gly Glu Ile Arg Val Ile Asn Gln Thr Thr Cys Glu Asn Leu Leu
        690                 695                 700

Pro Gln Gln Ile Thr Pro Arg Met Met Cys Val Gly Phe Leu Ser Gly
705                 710                 715                 720

Gly Val Asp Ser Cys Gln Gly Asp Ser Gly Gly Pro Leu Ser Ser Val
              725                 730                 735

Glu Ala Asp Gly Arg Ile Phe Gln Ala Gly Val Val Ser Trp Gly Asp
              740                 745                 750

Gly Cys Ala Gln Arg Asn Lys Pro Gly Val Tyr Thr Arg Leu Pro Leu
              755                 760                 765

Phe Arg Asp Trp Ile Lys Glu Asn Thr Gly Val
        770                 775


<210>   26
<211>   103
<212>   PRT
<213>   Homo Sapiens


<220>
<221>   MISC_FEATURE
<222>   (1)..(103)
```

<223> Commercially available recombinant protein of Q9Y5Y6

<400> 26

Pro Pro Ser Tyr Asn Leu Thr Phe His Ser Ser Gln Asn Val Leu Leu
1               5                  10                 15

Ile Thr Leu Ile Thr Asn Thr Glu Arg Arg His Pro Gly Phe Glu Ala
            20                 25                 30

Thr Phe Phe Gln Leu Pro Arg Met Ser Ser Cys Gly Gly Arg Leu Arg
        35                 40                 45

Lys Ala Gln Gly Thr Phe Asn Ser Pro Tyr Tyr Pro Gly His Tyr Pro
        50                 55                 60

Pro Asn Ile Asp Cys Thr Trp Asn Ile Glu Val Pro Asn Asn Gln His
65                 70                 75                 80

Val Lys Val Arg Phe Lys Phe Phe Tyr Leu Leu Glu Pro Gly Val Pro
                85                 90                 95

Ala Gly Thr Cys Pro Lys Asp
            100

<210> 27
<211> 123
<212> PRT
<213> Homo Sapiens


<220>
<221> DOMAIN
<222> (1)..(123)
<223> Extracellular domain of P18433

<400> 27

Asn Asn Ala Thr Thr Val Ala Pro Ser Val Gly Ile Thr Arg Leu Ile
1               5                  10                 15

Asn Ser Ser Thr Ala Glu Pro Val Lys Glu Glu Ala Lys Thr Ser Asn
            20                 25                 30

Pro Thr Ser Ser Leu Thr Ser Leu Ser Val Ala Pro Thr Phe Ser Pro
        35                 40                 45

Asn Ile Thr Leu Gly Pro Thr Tyr Leu Thr Thr Val Asn Ser Ser Asp
        50                 55                 60

Ser Asp Asn Gly Thr Thr Arg Thr Ala Ser Thr Asn Ser Ile Gly Ile

```
                    65                  70                  75                  80

Thr Ile Ser Pro Asn Gly Thr Trp Leu Pro Asp Asn Gln Phe Thr Asp
                    85                  90                  95

Ala Arg Thr Glu Pro Trp Glu Gly Asn Ser Ser Thr Ala Ala Thr Thr
                    100                 105                 110

Pro Glu Thr Phe Pro Pro Ser Asp Glu Thr Pro
                    115                 120


<210>   28
<211>   99
<212>   PRT
<213>   Homo Sapiens


<220>
<221>   MISC_FEATURE
<222>   (1)..(99)
<223>   Commercially available recombinant protein of Q6PIM3

<400>   28

Ser Leu Lys Val Lys Gly Gly Ala Ser Glu Leu Gln Glu Asp Glu Ser
1               5                   10                  15

Phe Thr Leu Arg Gly Pro Pro Gly Ala Ala Pro Ser Ala Thr Gln Ile
                20                  25                  30

Thr Val Val Leu Pro His Ser Ser Cys Glu Leu Leu Tyr Leu Gly Thr
                35                  40                  45

Glu Ser Gly Asn Val Phe Val Val Gln Leu Pro Ala Phe Arg Ala Leu
            50                  55                  60

Glu Asp Arg Thr Ile Ser Ser Asp Ala Val Leu Gln Arg Leu Pro Glu
65                  70                  75                  80

Glu Ala Arg His Arg Arg Val Phe Glu Met Val Glu Ala Leu Gln Glu
                85                  90                  95

His Pro Arg


<210>   29
<211>   663
<212>   PRT
<213>   Homo Sapiens
```

```
<220>
<221>  DOMAIN
<222>  (1)..(663)
<223>  Extracellular domain of Q9UN66

<400>  29

Ala Glu Pro Arg Ser Tyr Ser Val Val Glu Glu Thr Glu Gly Ser Ser
1               5                   10                  15


Phe Val Thr Asn Leu Ala Lys Asp Leu Gly Leu Glu Gln Arg Glu Phe
                20                  25                  30


Ser Arg Arg Gly Val Arg Val Val Ser Arg Gly Asn Lys Leu His Leu
                35                  40                  45


Gln Leu Asn Gln Glu Thr Ala Asp Leu Leu Leu Asn Glu Lys Leu Asp
            50                  55                  60


Arg Glu Asp Leu Cys Gly His Thr Glu Pro Cys Val Leu Arg Phe Gln
65                  70                  75                  80


Val Leu Leu Glu Ser Pro Phe Glu Phe Phe Gln Ala Glu Leu Gln Val
                85                  90                  95


Ile Asp Ile Asn Asp His Ser Pro Val Phe Leu Asp Lys Gln Met Leu
                100                 105                 110


Val Lys Val Ser Glu Ser Ser Pro Pro Gly Thr Ala Phe Pro Leu Lys
                115                 120                 125


Asn Ala Glu Asp Leu Asp Ile Gly Gln Asn Asn Ile Glu Asn Tyr Ile
            130                 135                 140


Ile Ser Pro Asn Ser Tyr Phe Arg Val Leu Thr Arg Lys Arg Ser Asp
145                 150                 155                 160


Gly Arg Lys Tyr Pro Glu Leu Val Leu Asp Asn Ala Leu Asp Arg Glu
                165                 170                 175


Glu Glu Ala Glu Leu Arg Leu Thr Leu Thr Ala Leu Asp Gly Gly Ser
            180                 185                 190


Pro Pro Arg Ser Gly Thr Ala Gln Val Tyr Ile Glu Val Val Asp Val
            195                 200                 205


Asn Asp Asn Ala Pro Glu Phe Gln Gln Pro Phe Tyr Arg Val Gln Ile
```

```
                210                 215                 220


Ser Glu Asp Ser Pro Ile Ser Phe Leu Val Val Lys Val Ser Ala Thr
225                 230                 235                 240


Asp Val Asp Thr Gly Val Asn Gly Glu Ile Ser Tyr Ser Leu Phe Gln
                245                 250                 255


Ala Ser Asp Glu Ile Ser Lys Thr Phe Lys Val Asp Phe Leu Thr Gly
                260                 265                 270


Glu Ile Arg Leu Lys Lys Gln Leu Asp Phe Glu Lys Phe Gln Ser Tyr
                275                 280                 285


Glu Val Asn Ile Glu Ala Arg Asp Ala Gly Gly Phe Ser Gly Lys Cys
                290                 295                 300


Thr Val Leu Ile Gln Val Ile Asp Val Asn Asp His Ala Pro Glu Val
305                 310                 315                 320


Thr Met Ser Ala Phe Thr Ser Pro Ile Pro Glu Asn Ala Pro Glu Thr
                325                 330                 335


Val Val Ala Leu Phe Ser Val Ser Asp Leu Asp Ser Gly Glu Asn Gly
                340                 345                 350


Lys Ile Ser Cys Ser Ile Gln Glu Asp Leu Pro Phe Leu Leu Lys Ser
                355                 360                 365


Ser Val Gly Asn Phe Tyr Thr Leu Leu Thr Glu Thr Pro Leu Asp Arg
                370                 375                 380


Glu Ser Arg Ala Glu Tyr Asn Val Thr Ile Thr Val Thr Asp Leu Gly
385                 390                 395                 400


Thr Pro Arg Leu Thr Thr His Leu Asn Met Thr Val Leu Val Ser Asp
                405                 410                 415


Val Asn Asp Asn Ala Pro Ala Phe Thr Gln Thr Ser Tyr Thr Leu Phe
                420                 425                 430


Val Arg Glu Asn Asn Ser Pro Ala Leu His Ile Gly Ser Val Ser Ala
                435                 440                 445


Thr Asp Arg Asp Ser Gly Thr Asn Ala Gln Val Thr Tyr Ser Leu Leu
```

                    450                   455                   460

Pro Pro Gln Asp Pro His Leu Pro Leu Ala Ser Leu Val Ser Ile Asn
465             470             475                     480

Thr Asp Asn Gly His Leu Phe Ala Leu Arg Ser Leu Asp Tyr Glu Ala
                485             490                 495

Leu Gln Ala Phe Glu Phe Arg Val Gly Ala Ser Asp Arg Gly Ser Pro
            500             505                 510

Ala Leu Ser Ser Glu Ala Leu Val Arg Val Leu Val Leu Asp Ala Asn
            515             520                 525

Asp Asn Ser Pro Phe Val Leu Tyr Pro Leu Gln Asn Gly Ser Ala Pro
            530             535                 540

Cys Thr Glu Leu Val Pro Arg Ala Ala Glu Pro Gly Tyr Leu Val Thr
545             550             555                     560

Lys Val Val Ala Val Asp Gly Asp Ser Gly Gln Asn Ala Trp Leu Ser
                565             570                 575

Tyr Gln Leu Leu Lys Ala Thr Glu Pro Gly Leu Phe Gly Val Trp Ala
            580             585                 590

His Asn Gly Glu Val Arg Thr Ala Arg Leu Leu Ser Glu Arg Asp Ala
            595             600                 605

Ala Lys Gln Arg Leu Val Val Leu Val Lys Asp Asn Gly Glu Pro Pro
            610             615                 620

Cys Ser Ala Thr Ala Thr Leu His Leu Leu Leu Val Asp Gly Phe Ser
625             630             635                     640

Gln Pro Tyr Leu Pro Leu Pro Glu Ala Ala Pro Ala Gln Gly Gln Ala
            645             650                 655

Asp Ser Leu Thr Val Tyr Leu
            660

<210> 30
<211> 242
<212> PRT
<213> Homo Sapiens


<220>

```
<221>   DOMAIN
<222>   (1)..(242)
<223>   Extracellular domain of P16422

<400>   30

Gln Glu Glu Cys Val Cys Glu Asn Tyr Lys Leu Ala Val Asn Cys Phe
1               5                   10                  15


Val Asn Asn Asn Arg Gln Cys Gln Cys Thr Ser Val Gly Ala Gln Asn
            20                  25                  30


Thr Val Ile Cys Ser Lys Leu Ala Ala Lys Cys Leu Val Met Lys Ala
        35                  40                  45


Glu Met Asn Gly Ser Lys Leu Gly Arg Arg Ala Lys Pro Glu Gly Ala
        50                  55                  60


Leu Gln Asn Asn Asp Gly Leu Tyr Asp Pro Asp Cys Asp Glu Ser Gly
65                  70                  75                  80


Leu Phe Lys Ala Lys Gln Cys Asn Gly Thr Ser Met Cys Trp Cys Val
                85                  90                  95


Asn Thr Ala Gly Val Arg Arg Thr Asp Lys Asp Thr Glu Ile Thr Cys
                100                 105                 110


Ser Glu Arg Val Arg Thr Tyr Trp Ile Ile Ile Glu Leu Lys His Lys
            115                 120                 125


Ala Arg Glu Lys Pro Tyr Asp Ser Lys Ser Leu Arg Thr Ala Leu Gln
        130                 135                 140


Lys Glu Ile Thr Thr Arg Tyr Gln Leu Asp Pro Lys Phe Ile Thr Ser
145                 150                 155                 160


Ile Leu Tyr Glu Asn Asn Val Ile Thr Ile Asp Leu Val Gln Asn Ser
                165                 170                 175


Ser Gln Lys Thr Gln Asn Asp Val Asp Ile Ala Asp Val Ala Tyr Tyr
            180                 185                 190


Phe Glu Lys Asp Val Lys Gly Glu Ser Leu Phe His Ser Lys Lys Met
            195                 200                 205


Asp Leu Thr Val Asn Gly Glu Gln Leu Asp Leu Asp Pro Gly Gln Thr
            210                 215                 220
```

```
Leu Ile Tyr Tyr Val Asp Glu Lys Ala Pro Glu Phe Ser Met Gln Gly
225                 230                 235                 240


Leu Lys



<210>  31
<211>  90
<212>  PRT
<213>  Homo Sapiens


<220>
<221>  MISC_FEATURE
<222>  (1)..(90)
<223>  Commercially available recombinant protein of O95994

<400>  31

Arg Asp Thr Thr Val Lys Pro Gly Ala Lys Lys Asp Thr Lys Asp Ser
1               5                   10                  15


Arg Pro Lys Leu Pro Gln Thr Leu Ser Arg Gly Trp Gly Asp Gln Leu
            20                  25                  30


Ile Trp Thr Gln Thr Tyr Glu Glu Ala Leu Tyr Lys Ser Lys Thr Ser
            35                  40                  45


Asn Lys Pro Leu Met Ile Ile His His Leu Asp Glu Cys Pro His Ser
        50                  55                  60


Gln Ala Leu Lys Lys Val Phe Ala Glu Asn Lys Glu Ile Gln Lys Leu
65                  70                  75                  80


Ala Glu Gln Phe Val Leu Leu Asn Leu Val
                85                  90


<210>  32
<211>  620
<212>  PRT
<213>  Homo Sapiens


<220>
<221>  DOMAIN
<222>  (1)..(620)
<223>  Extracellular domain of P01833

<400>  32

Lys Ser Pro Ile Phe Gly Pro Glu Glu Val Asn Ser Val Glu Gly Asn
```

```
                1               5                       10                      15

Ser Val Ser Ile Thr Cys Tyr Tyr Pro Pro Thr Ser Val Asn Arg His
            20                  25                  30

Thr Arg Lys Tyr Trp Cys Arg Gln Gly Ala Arg Gly Gly Cys Ile Thr
            35                  40                  45

Leu Ile Ser Ser Glu Gly Tyr Val Ser Ser Lys Tyr Ala Gly Arg Ala
            50                  55                  60

Asn Leu Thr Asn Phe Pro Glu Asn Gly Thr Phe Val Val Asn Ile Ala
65                  70                  75                  80

Gln Leu Ser Gln Asp Asp Ser Gly Arg Tyr Lys Cys Gly Leu Gly Ile
                85                  90                  95

Asn Ser Arg Gly Leu Ser Phe Asp Val Ser Leu Glu Val Ser Gln Gly
            100                 105                 110

Pro Gly Leu Leu Asn Asp Thr Lys Val Tyr Thr Val Asp Leu Gly Arg
            115                 120                 125

Thr Val Thr Ile Asn Cys Pro Phe Lys Thr Glu Asn Ala Gln Lys Arg
            130                 135                 140

Lys Ser Leu Tyr Lys Gln Ile Gly Leu Tyr Pro Val Leu Val Ile Asp
145                 150                 155                 160

Ser Ser Gly Tyr Val Asn Pro Asn Tyr Thr Gly Arg Ile Arg Leu Asp
                165                 170                 175

Ile Gln Gly Thr Gly Gln Leu Leu Phe Ser Val Val Ile Asn Gln Leu
            180                 185                 190

Arg Leu Ser Asp Ala Gly Gln Tyr Leu Cys Gln Ala Gly Asp Asp Ser
            195                 200                 205

Asn Ser Asn Lys Lys Asn Ala Asp Leu Gln Val Leu Lys Pro Glu Pro
            210                 215                 220

Glu Leu Val Tyr Glu Asp Leu Arg Gly Ser Val Thr Phe His Cys Ala
225                 230                 235                 240

Leu Gly Pro Glu Val Ala Asn Val Ala Lys Phe Leu Cys Arg Gln Ser
```

                        245                    250                         255

Ser Gly Glu Asn Cys Asp Val Val Val Asn Thr Leu Gly Lys Arg Ala
            260                     265                 270

Pro Ala Phe Glu Gly Arg Ile Leu Leu Asn Pro Gln Asp Lys Asp Gly
            275                     280                 285

Ser Phe Ser Val Val Ile Thr Gly Leu Arg Lys Glu Asp Ala Gly Arg
            290                     295                 300

Tyr Leu Cys Gly Ala His Ser Asp Gly Gln Leu Gln Glu Gly Ser Pro
305                     310                     315                 320

Ile Gln Ala Trp Gln Leu Phe Val Asn Glu Glu Ser Thr Ile Pro Arg
                325                     330                 335

Ser Pro Thr Val Val Lys Gly Val Ala Gly Gly Ser Val Ala Val Leu
            340                     345                 350

Cys Pro Tyr Asn Arg Lys Glu Ser Lys Ser Ile Lys Tyr Trp Cys Leu
            355                     360                 365

Trp Glu Gly Ala Gln Asn Gly Arg Cys Pro Leu Leu Val Asp Ser Glu
            370                     375                 380

Gly Trp Val Lys Ala Gln Tyr Glu Gly Arg Leu Ser Leu Leu Glu Glu
385                     390                     395                 400

Pro Gly Asn Gly Thr Phe Thr Val Ile Leu Asn Gln Leu Thr Ser Arg
                405                     410                 415

Asp Ala Gly Phe Tyr Trp Cys Leu Thr Asn Gly Asp Thr Leu Trp Arg
                420                     425                 430

Thr Thr Val Glu Ile Lys Ile Ile Glu Gly Glu Pro Asn Leu Lys Val
            435                     440                 445

Pro Gly Asn Val Thr Ala Val Leu Gly Glu Thr Leu Lys Val Pro Cys
            450                     455                 460

His Phe Pro Cys Lys Phe Ser Ser Tyr Glu Lys Tyr Trp Cys Lys Trp
465                     470                     475                 480

Asn Asn Thr Gly Cys Gln Ala Leu Pro Ser Gln Asp Glu Gly Pro Ser

                        485                    490                        495

Lys Ala Phe Val Asn Cys Asp Glu Asn Ser Arg Leu Val Ser Leu Thr
            500                    505                    510

Leu Asn Leu Val Thr Arg Ala Asp Glu Gly Trp Tyr Trp Cys Gly Val
            515                    520                    525

Lys Gln Gly His Phe Tyr Gly Glu Thr Ala Ala Val Tyr Val Ala Val
            530                    535                    540

Glu Glu Arg Lys Ala Ala Gly Ser Arg Asp Val Ser Leu Ala Lys Ala
545                    550                    555                    560

Asp Ala Ala Pro Asp Glu Lys Val Leu Asp Ser Gly Phe Arg Glu Ile
                565                    570                    575

Glu Asn Lys Ala Ile Gln Asp Pro Arg Leu Phe Ala Glu Glu Lys Ala
            580                    585                    590

Val Ala Asp Thr Arg Asp Gln Ala Asp Gly Ser Arg Ala Ser Val Asp
            595                    600                    605

Ser Gly Ser Ser Glu Glu Gln Gly Gly Ser Ser Arg
        610                    615                    620


<210>   33
<211>   110
<212>   PRT
<213>   Homo Sapiens


<220>
<221>   MISC_FEATURE
<222>   (1)..(110)
<223>   Commercially available recombinant protein of P01833

<400>   33

Pro Ile Phe Gly Pro Glu Glu Val Asn Ser Val Glu Gly Asn Ser Val
1                   5                   10                      15

Ser Ile Thr Cys Tyr Tyr Pro Pro Thr Ser Val Asn Arg His Thr Arg
            20                      25                      30

Lys Tyr Trp Cys Arg Gln Gly Ala Arg Gly Gly Cys Ile Thr Leu Ile
            35                      40                      45

Ser Ser Glu Gly Tyr Val Ser Ser Lys Tyr Ala Gly Arg Ala Asn Leu

```
                50                    55                    60


        Thr Asn Phe Pro Glu Asn Gly Thr Phe Val Val Asn Ile Ala Gln Leu
        65                  70                  75                  80


        Ser Gln Asp Asp Ser Gly Arg Tyr Lys Cys Gly Leu Gly Ile Asn Ser
                        85                  90                  95


        Arg Gly Leu Ser Phe Asp Val Ser Leu Glu Val Ser Gln Gly
                    100                 105                 110



        <210>  34
        <211>  11
        <212>  PRT
        <213>  Homo Sapiens

        <400>  34

        Ala Ala Gly Ser Arg Asp Val Ser Leu Ala Lys
        1               5                   10



        <210>  35
        <211>  8
        <212>  PRT
        <213>  Homo Sapiens

        <400>  35

        Ala Asp Ala Ala Pro Asp Glu Lys
        1               5



        <210>  36
        <211>  11
        <212>  PRT
        <213>  Homo Sapiens

        <400>  36

        Ala Asp Glu Gly Trp Tyr Trp Cys Gly Val Lys
        1               5                   10



        <210>  37
        <211>  12
        <212>  PRT
        <213>  Homo Sapiens

        <400>  37

        Ala Glu Asn Pro Glu Pro Leu Val Phe Gly Val Lys
        1               5                   10



        <210>  38
```

```
<211>  9
<212>  PRT
<213>  Homo Sapiens

<400>  38

Ala Glu Val Asp Leu Gln Gly Ile Lys
1               5


<210>  39
<211>  16
<212>  PRT
<213>  Homo Sapiens

<400>  39

Ala Glu Tyr Asn Val Thr Ile Thr Val Thr Asp Leu Gly Thr Pro Arg
1               5                   10                  15


<210>  40
<211>  10
<212>  PRT
<213>  Homo Sapiens

<400>  40

Ala Phe Val Asn Cys Asp Glu Asn Ser Arg
1               5                   10


<210>  41
<211>  7
<212>  PRT
<213>  Homo Sapiens

<400>  41

Ala Leu Leu Ser Asp Glu Arg
1               5


<210>  42
<211>  26
<212>  PRT
<213>  Homo Sapiens

<400>  42

Ala Asn Leu Thr Asn Phe Pro Glu Asn Gly Thr Phe Val Val Asn Ile
1               5                   10                  15


Ala Gln Leu Ser Gln Asp Asp Ser Gly Arg
            20                  25


<210>  43
<211>  7
```

```
<212>  PRT
<213>  Homo Sapiens

<400>  43

Ala Pro Ala Phe Glu Gly Arg
1               5


<210>  44
<211>  10
<212>  PRT
<213>  Homo Sapiens

<400>  44

Ala Pro Glu Phe Ser Met Gln Gly Leu Lys
1               5                   10


<210>  45
<211>  6
<212>  PRT
<213>  Homo Sapiens

<400>  45

Ala Pro Tyr Glu Trp Lys
1               5


<210>  46
<211>  6
<212>  PRT
<213>  Homo Sapiens

<400>  46

Ala Gln Ile His Met Arg
1               5


<210>  47
<211>  6
<212>  PRT
<213>  Homo Sapiens

<400>  47

Ala Gln Tyr Glu Gly Arg
1               5


<210>  48
<211>  11
<212>  PRT
<213>  Homo Sapiens

<400>  48

Ala Ser Ser Pro Gln Gly Phe Asp Val Asp Arg
```

```
                1                5                          10


<210>  49
<211>  15
<212>  PRT
<213>  Homo Sapiens


<400>  49


Ala Ser Ser Pro Gln Gly Phe Asp Val Asp Arg Asp Ala Lys Lys
1                5                          10                          15


<210>  50
<211>  16
<212>  PRT
<213>  Homo Sapiens


<400>  50


Ala Ser Val Asp Ser Gly Ser Ser Glu Glu Gln Gly Gly Ser Ser Arg
1                5                          10                          15


<210>  51
<211>  14
<212>  PRT
<213>  Homo Sapiens


<400>  51


Ala Thr Phe Ala Phe Ser Pro Glu Glu Gln Gln Ala Gln Arg
1                5                          10


<210>  52
<211>  12
<212>  PRT
<213>  Homo Sapiens


<400>  52


Ala Thr Phe Gln Ala Tyr Gln Ile Leu Ile Gly Lys
1                5                          10


<210>  53
<211>  9
<212>  PRT
<213>  Homo Sapiens


<400>  53


Ala Val Ile Asn Ser Ala Gly Tyr Lys
1                5


<210>  54
<211>  7
```

```
<212>  PRT
<213>  Homo Sapiens

<400>  54

Ala Tyr Leu Thr Leu Val Arg
1               5


<210>  55
<211>  7
<212>  PRT
<213>  Homo Sapiens

<400>  55

Ala Tyr Pro Gln Tyr Tyr Arg
1               5


<210>  56
<211>  12
<212>  PRT
<213>  Homo Sapiens

<400>  56

Cys Ala Gln Asp Cys Glu Asp Tyr Phe Ala Glu Arg
1               5                   10


<210>  57
<211>  8
<212>  PRT
<213>  Homo Sapiens

<400>  57

Cys Gly Leu Gly Ile Asn Ser Arg
1               5


<210>  58
<211>  12
<212>  PRT
<213>  Homo Sapiens

<400>  58

Cys Pro Leu Leu Val Asp Ser Glu Gly Trp Val Lys
1               5                   10


<210>  59
<211>  15
<212>  PRT
<213>  Homo Sapiens

<400>  59

Cys Pro Thr Gln Phe Pro Leu Ile Leu Trp His Pro Tyr Ala Arg
```

```
                1              5                    10                      15


<210>  60
<211>  16
<212>  PRT
<213>  Homo Sapiens


<400>  60

Cys Ser Val Pro Glu Gly Pro Phe Pro Gly His Leu Val Asp Val Arg
1               5                    10                      15


<210>  61
<211>  16
<212>  PRT
<213>  Homo Sapiens


<400>  61

Asp Ala Gly Phe Tyr Trp Cys Leu Thr Asn Gly Asp Thr Leu Trp Arg
1               5                    10                      15


<210>  62
<211>  10
<212>  PRT
<213>  Homo Sapiens


<400>  62

Asp Ala Tyr Val Phe Tyr Ala Val Ala Lys
1               5                    10


<210>  63
<211>  11
<212>  PRT
<213>  Homo Sapiens


<400>  63

Asp Glu Asp Glu Asp Ile Gln Ser Ile Leu Arg
1               5                    10


<210>  64
<211>  13
<212>  PRT
<213>  Homo Sapiens


<400>  64

Asp Glu Glu Asn Thr Ala Asn Ser Phe Leu Asn Tyr Arg
1               5                    10


<210>  65
<211>  16
```

```
<212>  PRT
<213>  Homo Sapiens

<400>  65

Asp Glu Tyr Gly Lys Pro Leu Ser Tyr Pro Leu Glu Ile His Val Lys
1               5                   10                  15


<210>  66
<211>  12
<212>  PRT
<213>  Homo Sapiens

<400>  66

Asp Gly Ser Phe Ser Val Val Ile Thr Gly Leu Arg
1               5                   10


<210>  67
<211>  16
<212>  PRT
<213>  Homo Sapiens

<400>  67

Asp Ile Glu Glu Ala Ile Glu Glu Glu Thr Ser Gly Asp Leu Gln Lys
1               5                   10                  15


<210>  68
<211>  13
<212>  PRT
<213>  Homo Sapiens

<400>  68

Asp Ile Asn Asp Asn Arg Pro Thr Phe Leu Gln Ser Lys
1               5                   10


<210>  69
<211>  9
<212>  PRT
<213>  Homo Sapiens

<400>  69

Asp Leu Tyr Asp Ala Gly Glu Gly Arg
1               5


<210>  70
<211>  15
<212>  PRT
<213>  Homo Sapiens

<400>  70

Asp Asn Val Glu Ser Ala Gln Ala Ser Glu Val Lys Pro Leu Arg
```

```
        1                   5                       10                          15


<210>  71
<211>  7
<212>  PRT
<213>  Homo Sapiens


<400>  71


Asp Gln Ala Asp Gly Ser Arg
1               5



<210>  72
<211>  7
<212>  PRT
<213>  Homo Sapiens


<400>  72


Asp Arg Asn His Arg Pro Lys
1               5



<210>  73
<211>  9
<212>  PRT
<213>  Homo Sapiens


<400>  73


Asp Thr Glu Ile Thr Cys Ser Glu Arg
1               5



<210>  74
<211>  14
<212>  PRT
<213>  Homo Sapiens


<400>  74


Asp Val Ser Leu Ala Lys Ala Asp Ala Ala Pro Asp Glu Lys
1               5                   10



<210>  75
<211>  7
<212>  PRT
<213>  Homo Sapiens


<400>  75


Asp Tyr Glu Ile Leu Phe Lys
1               5



<210>  76
<211>  11
```

```
<212>  PRT
<213>  Homo Sapiens

<400>  76

Glu Ala Tyr Glu Glu Pro Pro Glu Gln Leu Arg
1               5                   10


<210>  77
<211>  14
<212>  PRT
<213>  Homo Sapiens

<400>  77

Glu Glu Phe Val Ala Thr Thr Glu Ser Thr Thr Glu Thr Lys
1               5                   10


<210>  78
<211>  9
<212>  PRT
<213>  Homo Sapiens

<400>  78

Glu Glu Leu Cys Lys Ser Ile Gln Arg
1               5


<210>  79
<211>  9
<212>  PRT
<213>  Homo Sapiens

<400>  79

Glu Gly His Gln Ser Glu Gly Leu Arg
1               5


<210>  80
<211>  8
<212>  PRT
<213>  Homo Sapiens

<400>  80

Glu Gly Leu Ile Gln Trp Asp Lys
1               5


<210>  81
<211>  8
<212>  PRT
<213>  Homo Sapiens

<400>  81

Glu Gly Leu Leu Tyr Tyr Asn Arg
```

```
                          1                  5


<210>  82
<211>  11
<212>  PRT
<213>  Homo Sapiens

<400>  82

Glu Gly Ser Pro Thr Pro Gln Tyr Ser Trp Lys
1                  5                   10


<210>  83
<211>  7
<212>  PRT
<213>  Homo Sapiens

<400>  83

Glu Lys Pro Tyr Asp Ser Lys
1                  5


<210>  84
<211>  7
<212>  PRT
<213>  Homo Sapiens

<400>  84

Glu Leu Glu Ile Pro Pro Arg
1                  5


<210>  85
<211>  7
<212>  PRT
<213>  Homo Sapiens

<400>  85

Glu Met Gly Glu Met His Arg
1                  5


<210>  86
<211>  9
<212>  PRT
<213>  Homo Sapiens

<400>  86

Glu Arg Glu Glu Glu Asp Asp Tyr Arg
1                  5


<210>  87
<211>  14
```

```
<212>  PRT
<213>  Homo Sapiens

<400>  87

Glu Arg Glu Glu Glu Asp Asp Tyr Arg Gln Glu Glu Gln Arg
1               5                   10


<210>  88
<211>  17
<212>  PRT
<213>  Homo Sapiens

<400>  88

Glu Val Thr Asp Met Asn Leu Asn Val Ile Asn Glu Gly Gly Ile Asp
1               5                   10                  15


Lys



<210>  89
<211>  12
<212>  PRT
<213>  Homo Sapiens

<400>  89

Phe Asp Thr His Glu Tyr Arg Asn Glu Ser Arg Arg
1               5                   10


<210>  90
<211>  7
<212>  PRT
<213>  Homo Sapiens

<400>  90

Phe Glu Glu Val Leu Ser Lys
1               5


<210>  91
<211>  12
<212>  PRT
<213>  Homo Sapiens

<400>  91

Phe Phe Asn Val Leu Thr Thr Asn Thr Asp Gly Lys
1               5                   10


<210>  92
<211>  10
<212>  PRT
```

```
<213>  Homo Sapiens

<400>  92

Phe Gly Gln Ile Val Asn Thr Leu Asp Lys
1               5               10


<210>  93
<211>  16
<212>  PRT
<213>  Homo Sapiens

<400>  93

Phe Ile Gly Val Glu Ala Gly Gly Thr Leu Glu Leu His Gly Ala Arg
1               5               10              15


<210>  94
<211>  13
<212>  PRT
<213>  Homo Sapiens

<400>  94

Phe Ile Met Leu Asn Leu Met His Glu Thr Thr Asp Lys
1               5               10


<210>  95
<211>  10
<212>  PRT
<213>  Homo Sapiens

<400>  95

Phe Leu Arg Pro Gly His Asp Pro Val Arg
1               5               10


<210>  96
<211>  7
<212>  PRT
<213>  Homo Sapiens

<400>  96

Phe Gln Glu Lys Tyr Gln Lys
1               5


<210>  97
<211>  14
<212>  PRT
<213>  Homo Sapiens

<400>  97

Phe Gln Glu Lys Tyr Gln Lys Ser Leu Ser Asp Met Val Arg
```

```
                    1              5                    10


<210>  98
<211>  11
<212>  PRT
<213>  Homo Sapiens

<400>  98

Phe Gln Glu Leu Ile Phe Glu Asp Phe Ala Arg
1              5                    10


<210>  99
<211>  13
<212>  PRT
<213>  Homo Sapiens

<400>  99

Phe Arg Pro His Gln Asp Ala Asn Pro Glu Lys Pro Arg
1              5                    10


<210>  100
<211>  6
<212>  PRT
<213>  Homo Sapiens

<400>  100

Phe Ser Ser Tyr Glu Lys
1              5


<210>  101
<211>  16
<212>  PRT
<213>  Homo Sapiens

<400>  101

Phe Thr Thr Pro Gly Phe Pro Asp Ser Pro Tyr Pro Ala His Ala Arg
1              5                    10                   15


<210>  102
<211>  11
<212>  PRT
<213>  Homo Sapiens

<400>  102

Gly Ala Gly Thr Asp Glu Glu Thr Leu Ile Arg
1              5                    10


<210>  103
<211>  12
```

```
<212>   PRT
<213>   Homo Sapiens

<400>   103

Gly Asp Ala Asp Ser Val Leu Ser Leu Thr Phe Arg
1               5                   10


<210>   104
<211>   8
<212>   PRT
<213>   Homo Sapiens

<400>   104

Gly Asp Thr Arg Gly Trp Leu Lys
1               5


<210>   105
<211>   9
<212>   PRT
<213>   Homo Sapiens

<400>   105

Gly Asp Thr Ser Gly Asn Leu Lys Lys
1               5


<210>   106
<211>   8
<212>   PRT
<213>   Homo Sapiens

<400>   106

Gly Glu Ser Leu Phe His Ser Lys
1               5


<210>   107
<211>   15
<212>   PRT
<213>   Homo Sapiens

<400>   107

Gly Gly Ala Ser Glu Leu Gln Glu Asp Glu Ser Phe Thr Leu Arg
1               5                   10                  15


<210>   108
<211>   16
<212>   PRT
<213>   Homo Sapiens

<400>   108

Gly Gly Cys Ile Thr Leu Ile Ser Ser Glu Gly Tyr Val Ser Ser Lys
```

1                5                          10                          15

```
<210>  109
<211>  15
<212>  PRT
<213>  Homo Sapiens

<400>  109

Gly His Ser Pro Ala Phe Leu Gln Pro Gln Asn Gly Asn Ser Arg
1                5                          10                          15


<210>  110
<211>  16
<212>  PRT
<213>  Homo Sapiens

<400>  110

Gly Met Gly Thr Asn Glu Ala Ala Ile Ile Glu Ile Leu Ser Gly Arg
1                5                          10                          15


<210>  111
<211>  18
<212>  PRT
<213>  Homo Sapiens

<400>  111

Gly Ser Val Thr Phe His Cys Ala Leu Gly Pro Glu Val Ala Asn Val
1                5                          10                          15


Ala Lys


<210>  112
<211>  18
<212>  PRT
<213>  Homo Sapiens

<400>  112

Gly Trp Gly Asp Gln Leu Ile Trp Thr Gln Thr Tyr Glu Glu Ala Leu
1                5                          10                          15


Tyr Lys


<210>  113
<211>  12
<212>  PRT
<213>  Homo Sapiens

<400>  113
```

```
Gly Tyr Thr Ile His Trp Asn Gly Pro Ala Pro Arg
1               5                   10


<210>  114
<211>  16
<212>  PRT
<213>  Homo Sapiens

<400>  114

His Glu Ile Glu Gly Thr Gly Leu Pro Gln Ala Gln Leu Leu Trp Arg
1               5                   10                  15


<210>  115
<211>  11
<212>  PRT
<213>  Homo Sapiens

<400>  115

His Leu Ser Pro Asp Gly Gln Tyr Val Pro Arg
1               5                   10


<210>  116
<211>  5
<212>  PRT
<213>  Homo Sapiens

<400>  116

His Asn Leu Tyr Arg
1               5


<210>  117
<211>  13
<212>  PRT
<213>  Homo Sapiens

<400>  117

His Pro Gly Phe Glu Ala Thr Phe Phe Gln Leu Pro Arg
1               5                   10


<210>  118
<211>  7
<212>  PRT
<213>  Homo Sapiens

<400>  118

His Thr Glu Phe Glu Glu Arg
1               5


<210>  119
```

```
<211>  16
<212>  PRT
<213>  Homo Sapiens

<400>  119

Ile Asp His Val Thr Gly Glu Ile Phe Ser Val Ala Pro Leu Asp Arg
1               5                   10                  15


<210>  120
<211>  15
<212>  PRT
<213>  Homo Sapiens

<400>  120

Ile Glu Glu Tyr Glu Pro Val His Ser Leu Glu Glu Leu Gln Arg
1               5                   10                  15


<210>  121
<211>  11
<212>  PRT
<213>  Homo Sapiens

<400>  121

Ile Glu Phe Ile Ser Thr Met Glu Gly Tyr Lys
1               5                   10


<210>  122
<211>  16
<212>  PRT
<213>  Homo Sapiens

<400>  122

Ile Phe Gln Ala Gly Val Val Ser Trp Gly Asp Gly Cys Ala Gln Arg
1               5                   10                  15


<210>  123
<211>  9
<212>  PRT
<213>  Homo Sapiens

<400>  123

Ile Ile Glu Gly Glu Pro Asn Leu Lys
1               5


<210>  124
<211>  8
<212>  PRT
<213>  Homo Sapiens

<400>  124
```

```
Ile Leu Leu Asn Pro Gln Asp Lys
1               5

<210>  125
<211>  10
<212>  PRT
<213>  Homo Sapiens

<400>  125

Ile Leu Val Ser Leu Leu Gln Ala Asn Arg
1               5                   10

<210>  126
<211>  11
<212>  PRT
<213>  Homo Sapiens

<400>  126

Ile Met Phe Val Asp Pro Ser Leu Thr Val Arg
1               5                   10

<210>  127
<211>  14
<212>  PRT
<213>  Homo Sapiens

<400>  127

Ile Pro Ile Arg Trp Thr Ala Pro Glu Ala Ile Gln Tyr Arg
1               5                   10

<210>  128
<211>  15
<212>  PRT
<213>  Homo Sapiens

<400>  128

Ile Val Phe Ser Gly Asn Leu Phe Gln His Gln Glu Asp Ser Lys
1               5                   10                  15

<210>  129
<211>  8
<212>  PRT
<213>  Homo Sapiens

<400>  129

Lys Glu Leu Glu Ile Pro Pro Arg
1               5

<210>  130
```

```
<211>   13
<212>   PRT
<213>   Homo Sapiens

<400>   130

Lys Phe Cys Ile Gln Gln Val Gly Asp Met Thr Asn Arg
1               5                   10


<210>   131
<211>   13
<212>   PRT
<213>   Homo Sapiens

<400>   131

Lys Phe Phe Asn Val Leu Thr Thr Asn Thr Asp Gly Lys
1               5                   10


<210>   132
<211>   6
<212>   PRT
<213>   Homo Sapiens

<400>   132

Lys His Asn Leu Tyr Arg
1               5


<210>   133
<211>   6
<212>   PRT
<213>   Homo Sapiens

<400>   133

Lys Lys Arg Met Ala Lys
1               5


<210>   134
<211>   20
<212>   PRT
<213>   Homo Sapiens

<400>   134

Lys Asn Ala Asp Leu Gln Val Leu Lys Pro Glu Pro Glu Leu Val Tyr
1               5                   10                  15


Glu Asp Leu Arg
            20


<210>   135
<211>   7
```

```
<212>  PRT
<213>  Homo Sapiens

<400>  135

Lys Asn Asn His His Phe Lys
1               5


<210>  136
<211>  7
<212>  PRT
<213>  Homo Sapiens

<400>  136

Lys Val Phe Ala Glu Asn Lys
1               5


<210>  137
<211>  10
<212>  PRT
<213>  Homo Sapiens

<400>  137

Lys Tyr Asp Tyr Asp Ser Ser Ser Val Arg
1               5               10


<210>  138
<211>  11
<212>  PRT
<213>  Homo Sapiens

<400>  138

Lys Tyr Asp Tyr Asp Ser Ser Ser Val Arg Lys
1               5               10


<210>  139
<211>  12
<212>  PRT
<213>  Homo Sapiens

<400>  139

Lys Tyr Asp Tyr Asp Ser Ser Ser Val Arg Lys Arg
1               5               10


<210>  140
<211>  5
<212>  PRT
<213>  Homo Sapiens

<400>  140

Lys Tyr Trp Cys Arg
```

1               5

<210>  141
<211>  9
<212>  PRT
<213>  Homo Sapiens

<400>  141

Leu Ala Ala Lys Cys Leu Val Met Lys
1               5

<210>  142
<211>  19
<212>  PRT
<213>  Homo Sapiens

<400>  142

Leu Asp Ile Gln Gly Thr Gly Gln Leu Leu Phe Ser Val Val Ile Asn
1               5                       10                      15

Gln Leu Arg

<210>  143
<211>  6
<212>  PRT
<213>  Homo Sapiens

<400>  143

Leu Phe Ala Glu Glu Lys
1               5

<210>  144
<211>  11
<212>  PRT
<213>  Homo Sapiens

<400>  144

Leu Phe Asp Arg Ser Leu Glu Ser Asp Val Lys
1               5                       10

<210>  145
<211>  7
<212>  PRT
<213>  Homo Sapiens

<400>  145

Leu Gly Glu Tyr Met Glu Lys
1               5

```
<210>  146
<211>  8
<212>  PRT
<213>  Homo Sapiens

<400>  146

Leu Leu Leu Thr His Thr Glu Arg
1               5


<210>  147
<211>  7
<212>  PRT
<213>  Homo Sapiens

<400>  147

Leu Asn Leu Trp Ile Ser Arg
1               5


<210>  148
<211>  7
<212>  PRT
<213>  Homo Sapiens

<400>  148

Leu Pro Asp Asn Thr Val Lys
1               5


<210>  149
<211>  7
<212>  PRT
<213>  Homo Sapiens

<400>  149

Leu Pro Gln Thr Leu Ser Arg
1               5


<210>  150
<211>  18
<212>  PRT
<213>  Homo Sapiens

<400>  150

Leu Pro Ser Val Glu Glu Ala Glu Val Pro Lys Pro Leu Pro Pro Ala
1               5                   10                  15


Ser Lys


<210>  151
```

```
<211>  12
<212>  PRT
<213>  Homo Sapiens

<400>  151

Leu Gln Glu Asp Gly Leu Ser Val Trp Phe Gln Arg
1               5                   10


<210>  152
<211>  22
<212>  PRT
<213>  Homo Sapiens

<400>  152

Leu Ser Leu Leu Glu Glu Pro Gly Asn Gly Thr Phe Thr Val Ile Leu
1               5                   10                  15

Asn Gln Leu Thr Ser Arg
                20


<210>  153
<211>  17
<212>  PRT
<213>  Homo Sapiens

<400>  153

Leu Tyr Ala Tyr Glu Pro Ala Asp Thr Ala Leu Leu Leu Asp Asn Met
1               5                   10                  15

Lys


<210>  154
<211>  7
<212>  PRT
<213>  Homo Sapiens

<400>  154

Leu Tyr Thr Tyr Glu Pro Arg
1               5


<210>  155
<211>  7
<212>  PRT
<213>  Homo Sapiens

<400>  155

Met Asp Asn Tyr Leu Leu Arg
1               5
```

```
<210>  156
<211>  12
<212>  PRT
<213>  Homo Sapiens

<400>  156

Met Glu Ser Leu Arg Leu Asp Gly Leu Gln Gln Arg
1               5                   10


<210>  157
<211>  7
<212>  PRT
<213>  Homo Sapiens

<400>  157

Met Gly Trp Met Gly Glu Lys
1               5


<210>  158
<211>  9
<212>  PRT
<213>  Homo Sapiens

<400>  158

Met Ile Arg Asn Pro Asn Ser Leu Lys
1               5


<210>  159
<211>  15
<212>  PRT
<213>  Homo Sapiens

<400>  159

Met Pro Ala Met Leu Thr Gly Leu Cys Gln Gly Cys Gly Thr Arg
1               5                   10                  15


<210>  160
<211>  13
<212>  PRT
<213>  Homo Sapiens

<400>  160

Asn Leu Asp Gly Ile Ser His Ala Pro Asn Ala Val Lys
1               5                   10


<210>  161
<211>  11
<212>  PRT
```

<213>  Homo Sapiens

<400>  161

Asn Leu Ser Pro Asp Gly Gln Tyr Val Pro Arg
1                 5                 10

<210>  162
<211>  12
<212>  PRT
<213>  Homo Sapiens

<400>  162

Asn Leu Ser Ser Thr Thr Asp Asp Glu Ala Pro Arg
1                 5                 10

<210>  163
<211>  6
<212>  PRT
<213>  Homo Sapiens

<400>  163

Asn Asn His His Phe Lys
1                 5

<210>  164
<211>  8
<212>  PRT
<213>  Homo Sapiens

<400>  164

Asn Ser Trp Gln Leu Thr Pro Arg
1                 5

<210>  165
<211>  9
<212>  PRT
<213>  Homo Sapiens

<400>  165

Asn Tyr Ile His Gly Glu Leu Tyr Lys
1                 5

<210>  166
<211>  10
<212>  PRT
<213>  Homo Sapiens

<400>  166

Gln Ala Gly Ser His Ser Asn Ser Phe Arg

```
1               5                      10
```

<210> 167
<211> 14
<212> PRT
<213> Homo Sapiens

<400> 167

```
Gln Glu Thr Asp Tyr Val Leu Asn Asn Gly Phe Asn Pro Arg
1               5                      10
```

<210> 168
<211> 18
<212> PRT
<213> Homo Sapiens

<400> 168

```
Gln Gly His Phe Tyr Gly Glu Thr Ala Ala Val Tyr Val Ala Val Glu
1               5                      10                      15
```

```
Glu Arg
```

<210> 169
<211> 19
<212> PRT
<213> Homo Sapiens

<400> 169

```
Gln Gly His Phe Tyr Gly Glu Thr Ala Ala Val Tyr Val Ala Val Glu
1               5                      10                      15
```

```
Glu Arg Lys
```

<210> 170
<211> 8
<212> PRT
<213> Homo Sapiens

<400> 170

```
Gln Leu Gly Leu Thr Glu Pro Arg
1               5
```

<210> 171
<211> 15
<212> PRT
<213> Homo Sapiens

<400> 171

EP 2 375 255 A1

```
Gln Pro Gly Leu Val Met Glu Arg Ala Leu Leu Ser Asp Glu Arg
1               5               10              15


<210>  172
<211>  16
<212>  PRT
<213>  Homo Sapiens

<400>  172

Gln Ser Ser Gly Glu Asn Cys Asp Val Val Val Asn Thr Leu Gly Lys
1               5               10              15


<210>  173
<211>  17
<212>  PRT
<213>  Homo Sapiens

<400>  173

Gln Ser Ser Gly Glu Asn Cys Asp Val Val Val Asn Thr Leu Gly Lys
1               5               10              15


Arg



<210>  174
<211>  13
<212>  PRT
<213>  Homo Sapiens

<400>  174

Gln Val Val Glu Ala Ala Gln Ala Pro Ile Gln Glu Arg
1               5               10


<210>  175
<211>  8
<212>  PRT
<213>  Homo Sapiens

<400>  175

Arg Ala Pro Ala Phe Glu Gly Arg
1               5


<210>  176
<211>  24
<212>  PRT
<213>  Homo Sapiens

<400>  176

Arg Ala Thr Ala Ser Glu Gln Pro Leu Ala Gln Glu Pro Pro Ala Ser
```

154

EP 2 375 255 A1

```
                1               5                       10                          15

Gly Gly Ser Pro Ala Thr Thr Lys
                  20


<210>  177
<211>  14
<212>  PRT
<213>  Homo Sapiens

<400>  177

Arg Glu Asp Val Ser Gly Ile Ala Ser Cys Val Phe Thr Lys
1                   5                       10


<210>  178
<211>  16
<212>  PRT
<213>  Homo Sapiens

<400>  178

Arg Lys Asn Leu Asp Leu Ala Ala Pro Thr Ala Glu Glu Ala Gln Arg
1                   5                       10                      15


<210>  179
<211>  16
<212>  PRT
<213>  Homo Sapiens

<400>  179

Arg Pro Glu Leu Glu Glu Ile Phe His Gln Tyr Ser Gly Glu Asp Arg
1                   5                       10                      15


<210>  180
<211>  8
<212>  PRT
<213>  Homo Sapiens

<400>  180

Arg Pro Pro Gln Thr Leu Ser Arg
1                   5


<210>  181
<211>  6
<212>  PRT
<213>  Homo Sapiens

<400>  181

Arg Ser Asp Tyr Ala Lys
1                   5
```

```
<210>  182
<211>  8
<212>  PRT
<213>  Homo Sapiens

<400>  182

Arg Ser Glu Ser Val Lys Ser Arg
1               5


<210>  183
<211>  11
<212>  PRT
<213>  Homo Sapiens

<400>  183

Ser Ala Glu Asp Ser Phe Thr Gly Phe Val Arg
1               5                   10


<210>  184
<211>  13
<212>  PRT
<213>  Homo Sapiens

<400>  184

Ser Asp Glu Gly Glu Ser Met Pro Thr Phe Gly Lys Lys
1               5                   10


<210>  185
<211>  9
<212>  PRT
<213>  Homo Sapiens

<400>  185

Ser Asp Pro Val Thr Leu Asn Val Arg
1               5


<210>  186
<211>  8
<212>  PRT
<213>  Homo Sapiens

<400>  186

Ser Asp Thr Ser Gly Asp Phe Arg
1               5


<210>  187
<211>  9
<212>  PRT
```

```
<213>  Homo Sapiens

<400>  187

Ser Glu Leu Ser Gly Asn Phe Glu Lys
1               5


<210>  188
<211>  7
<212>  PRT
<213>  Homo Sapiens

<400>  188

Ser Glu Ser Glu Glu Glu Lys
1               5


<210>  189
<211>  15
<212>  PRT
<213>  Homo Sapiens

<400>  189

Ser Phe Val Val Thr Ser Val Val Ala Phe Pro Thr Asp Ser Lys
1               5                   10                  15


<210>  190
<211>  15
<212>  PRT
<213>  Homo Sapiens

<400>  190

Ser Ile Asn Gly Ile Leu Phe Pro Gly Gly Ser Val Asp Leu Arg
1               5                   10                  15


<210>  191
<211>  16
<212>  PRT
<213>  Homo Sapiens

<400>  191

Ser Ile Asn Gly Ile Leu Phe Pro Gly Gly Ser Val Asp Leu Arg Arg
1               5                   10                  15


<210>  192
<211>  12
<212>  PRT
<213>  Homo Sapiens

<400>  192

Ser Leu Ala Pro Gly Met Ala Leu Gly Ser Gly Arg
```

```
                     1                5                     10


    <210>  193
    <211>  7
    <212>  PRT
    <213>  Homo Sapiens

    <400>  193

    Ser Leu Glu Ser Asp Val Lys
    1                5


    <210>  194
    <211>  7
    <212>  PRT
    <213>  Homo Sapiens

    <400>  194

    Ser Leu Ser Asp Met Val Arg
    1                5


    <210>  195
    <211>  15
    <212>  PRT
    <213>  Homo Sapiens

    <400>  195

    Ser Val Thr Leu Pro Cys Thr Tyr His Thr Ser Thr Ser Ser Arg
    1                5                     10                    15


    <210>  196
    <211>  14
    <212>  PRT
    <213>  Homo Sapiens

    <400>  196

    Thr Ala Phe Tyr Leu Ala Glu Phe Phe Val Asn Glu Ala Arg
    1                5                     10


    <210>  197
    <211>  15
    <212>  PRT
    <213>  Homo Sapiens

    <400>  197

    Thr Ala Leu Ala Leu Leu Asp Arg Pro Ser Glu Tyr Ala Ala Arg
    1                5                     10                    15


    <210>  198
    <211>  12
```

```
<212>  PRT
<213>  Homo Sapiens

<400>  198

Thr Asp Ile Ser Met Ser Asp Phe Glu Asn Ser Arg
1               5                   10


<210>  199
<211>  10
<212>  PRT
<213>  Homo Sapiens

<400>  199

Thr Asp Met Asp Gln Ile Ile Thr Ser Lys
1               5                   10


<210>  200
<211>  14
<212>  PRT
<213>  Homo Sapiens

<400>  200

Thr Glu Asp Val Glu Pro Gln Ser Val Pro Leu Leu Ala Arg
1               5                   10


<210>  201
<211>  7
<212>  PRT
<213>  Homo Sapiens

<400>  201

Thr Glu Asn Ala Gln Lys Arg
1               5


<210>  202
<211>  8
<212>  PRT
<213>  Homo Sapiens

<400>  202

Thr Gly Ala Ile Ser Leu Thr Arg
1               5


<210>  203
<211>  10
<212>  PRT
<213>  Homo Sapiens

<400>  203

Thr Leu Glu Asp Glu Glu Glu Gln Glu Arg
```

```
                    1               5                       10
```

<210> 204
<211> 16
<212> PRT
<213> Homo Sapiens

<400> 204

```
Thr Leu Asn Ser Ser Gly Leu Pro Phe Gly Ser Tyr Thr Phe Glu Lys
1               5                       10                      15
```

<210> 205
<211> 9
<212> PRT
<213> Homo Sapiens

<400> 205

```
Thr Leu Val Leu Leu Ser Ala Thr Lys
1               5
```

<210> 206
<211> 10
<212> PRT
<213> Homo Sapiens

<400> 206

```
Thr Leu Tyr Phe Ala Asp Thr Tyr Leu Lys
1               5                       10
```

<210> 207
<211> 16
<212> PRT
<213> Homo Sapiens

<400> 207

```
Thr Gln Asn Asp Val Asp Ile Ala Asp Val Ala Tyr Tyr Phe Glu Lys
1               5                       10                      15
```

<210> 208
<211> 19
<212> PRT
<213> Homo Sapiens

<400> 208

```
Thr Gln Asn Asp Val Asp Ile Ala Asp Val Ala Tyr Tyr Phe Glu Lys
1               5                       10                      15
```

```
Asp Val Lys
```

```
<211>  11
<212>  PRT
<213>  Homo Sapiens

<400>  209

Thr Val Ala Gly Tyr Gly Arg Tyr Ser Gly Lys
1               5                   10


<210>  210
<211>  9
<212>  PRT
<213>  Homo Sapiens

<400>  210

Thr Val Thr Ile Asn Cys Pro Phe Lys
1               5


<210>  211
<211>  11
<212>  PRT
<213>  Homo Sapiens

<400>  211

Val Glu Gly Pro Ala Phe Thr Asp Ala Ile Arg
1               5                   10


<210>  212
<211>  15
<212>  PRT
<213>  Homo Sapiens

<400>  212

Val Phe Ala Gln Asn Glu Glu Ile Gln Glu Met Ala Gln Asn Lys
1               5                   10                  15


<210>  213
<211>  13
<212>  PRT
<213>  Homo Sapiens

<400>  213

Val Phe Glu Met Val Glu Ala Leu Gln Glu His Pro Arg
1               5                   10


<210>  214
<211>  7
<212>  PRT
<213>  Homo Sapiens

<400>  214
```

```
Val Leu Asp Ser Gly Phe Arg
1               5


<210>  215
<211>  12
<212>  PRT
<213>  Homo Sapiens

<400>  215

Val Leu Asp Ser Gly Phe Arg Glu Ile Glu Asn Lys
1               5                   10


<210>  216
<211>  8
<212>  PRT
<213>  Homo Sapiens

<400>  216

Val Pro Cys His Phe Pro Cys Lys
1               5


<210>  217
<211>  7
<212>  PRT
<213>  Homo Sapiens

<400>  217

Val Pro Pro Ala Glu Arg Arg
1               5


<210>  218
<211>  13
<212>  PRT
<213>  Homo Sapiens

<400>  218

Val Gln Gln Val Gln Pro Ala Met Gln Ala Val Ile Arg
1               5                   10


<210>  219
<211>  8
<212>  PRT
<213>  Homo Sapiens

<400>  219

Val Ser Asp Phe Gly Leu Ser Arg
1               5


<210>  220
```

```
<211>  10
<212>  PRT
<213>  Homo Sapiens

<400>  220

Val Ser Glu Asp Val Ala Leu Gly Thr Lys
1               5                   10


<210>  221
<211>  9
<212>  PRT
<213>  Homo Sapiens

<400>  221

Val Ser Val Ala His Phe Gly Ser Arg
1               5


<210>  222
<211>  16
<212>  PRT
<213>  Homo Sapiens

<400>  222

Val Thr Val Asp Ala Ile Ser Val Glu Thr Pro Gln Asp Val Leu Arg
1               5                   10                  15


<210>  223
<211>  7
<212>  PRT
<213>  Homo Sapiens

<400>  223

Val Val Met Leu Pro Pro Arg
1               5


<210>  224
<211>  8
<212>  PRT
<213>  Homo Sapiens

<400>  224

Val Tyr Thr Val Asp Leu Gly Arg
1               5


<210>  225
<211>  14
<212>  PRT
<213>  Homo Sapiens

<400>  225
```

```
Trp Gly Thr Asp Glu Leu Ala Phe Asn Glu Val Leu Ala Lys
1               5                   10
```

```
<210>   226
<211>   15
<212>   PRT
<213>   Homo Sapiens

<400>   226
```

```
Trp Gly Thr Asp Glu Leu Ala Phe Asn Glu Val Leu Ala Lys Arg
1               5                   10                  15
```

```
<210>   227
<211>   13
<212>   PRT
<213>   Homo Sapiens

<400>   227
```

```
Trp Asn Asp Pro Gly Ala Gln Tyr Ser Leu Val Asp Lys
1               5                   10
```

```
<210>   228
<211>   6
<212>   PRT
<213>   Homo Sapiens

<400>   228
```

```
Trp Ser Leu Ser Val Lys
1               5
```

```
<210>   229
<211>   10
<212>   PRT
<213>   Homo Sapiens

<400>   229
```

```
Trp Thr Ala Pro Glu Ala Ile Gln Tyr Arg
1               5                   10
```

```
<210>   230
<211>   9
<212>   PRT
<213>   Homo Sapiens

<400>   230
```

```
Tyr Asp Tyr Asp Ser Ser Ser Val Arg
1               5
```

```
<210>   231
```

```
<211>  10
<212>  PRT
<213>  Homo Sapiens

<400>  231

Tyr Asp Tyr Asp Ser Ser Ser Val Arg Lys
1                5                    10


<210>  232
<211>  11
<212>  PRT
<213>  Homo Sapiens

<400>  232

Tyr Asp Tyr Asp Ser Ser Ser Val Arg Lys Arg
1                5                    10


<210>  233
<211>  7
<212>  PRT
<213>  Homo Sapiens

<400>  233

Tyr Glu Lys Ala Glu Ile Lys
1                5


<210>  234
<211>  15
<212>  PRT
<213>  Homo Sapiens

<400>  234

Tyr Gly Gln Gly Phe Tyr Leu Ile Ser Pro Ser Glu Phe Glu Arg
1                5                    10                   15


<210>  235
<211>  15
<212>  PRT
<213>  Homo Sapiens

<400>  235

Tyr Gly Gln Gly Phe Tyr Leu Leu Ser Pro Ser Glu Phe Glu Arg
1                5                    10                   15


<210>  236
<211>  10
<212>  PRT
<213>  Homo Sapiens

<400>  236
```

```
Tyr Lys Cys Gly Leu Gly Ile Asn Ser Arg
1               5               10


<210>   237
<211>   10
<212>   PRT
<213>   Homo Sapiens

<400>   237

Tyr Leu Ala Asp Met Asn Tyr Val His Arg
1               5               10


<210>   238
<211>   32
<212>   PRT
<213>   Homo Sapiens

<400>   238

Tyr Leu Cys Gly Ala His Ser Asp Gly Gln Leu Gln Glu Gly Ser Pro
1               5               10              15


Ile Gln Ala Trp Gln Leu Phe Val Asn Glu Glu Ser Thr Ile Pro Arg
            20              25              30


<210>   239
<211>   8
<212>   PRT
<213>   Homo Sapiens

<400>   239

Tyr Leu Glu Ser Ala Gly Ala Arg
1               5


<210>   240
<211>   22
<212>   PRT
<213>   Homo Sapiens

<400>   240

Tyr Asn Ile Leu Asn Gln Glu Gln Pro Leu Ala Gln Pro Ala Ser Gly
1               5               10              15


Gln Pro Val Ser Leu Lys
            20


<210>   241
<211>   8
<212>   PRT
```

```
<213>  Homo Sapiens

<400>  241

Tyr Pro Pro Leu Pro Val Asp Lys
1                   5


<210>  242
<211>  12
<212>  PRT
<213>  Homo Sapiens

<400>  242

Tyr Pro Val Tyr Gly Val Gln Trp His Pro Glu Lys
1                   5                   10


<210>  243
<211>  18
<212>  PRT
<213>  Homo Sapiens

<400>  243

Tyr Pro Val Tyr Gly Val Gln Trp His Pro Glu Lys Ala Pro Tyr Glu
1                   5                   10                  15


Trp Lys



<210>  244
<211>  12
<212>  PRT
<213>  Homo Sapiens

<400>  244

Tyr Trp Cys Leu Trp Glu Gly Ala Gln Asn Gly Arg
1                   5                   10


<210>  245
<211>  9
<212>  PRT
<213>  Homo Sapiens

<400>  245

Tyr Tyr Ile Ala Ala Ser Tyr Val Lys
1                   5
```

**EP 2 375 255 A1**

**Claims**

1. A method of diagnosing colorectal cancer in a subject, differentiating causes of colorectal cancer in a subject, guiding therapy in a subject suffering from colorectal cancer, assessing the risk of relapse in a subject suffering from colorectal cancer, or assigning a prognostic risk of one or more future clinical outcomes to a subject suffering from colorectal cancer, the method comprising:

   (a) performing assays configured to detect a soluble polypeptide derived from a protein defined by SEQ ID No 4 as a marker in one or more samples obtained from said subject; and
   (b) correlating the results of said assay(s) to the presence or absence of colorectal cancer in the subject, to a therapeutic regimen to be used in the subject, to a risk of relapse in the subject, or to the prognostic risk of one or more clinical outcomes for the subject suffering from colorectal cancer.

2. A method according to claim 1 wherein the marker comprises an amino acid sequence selected from SEQ ID Nos 101, 103, 117, 122, 189 and 223.

3. A method according to claim 1 or 2 wherein the marker sequence overlaps with or is preferably within a sequence corresponding to an extracellular portion of a protein defined by SEQ ID No 4 (i.e. overlaps with or is preferably within a sequence corresponding to SEQ ID No 25).

4. A method according to any one of claims 1 to 3, wherein the method comprises performing assays configured to detect an additional one, two, three, four or more said markers, wherein said markers are soluble polypeptides derived from at least one, two, three or four different proteins selected from the proteins defined by SEQ ID Nos 1-3 and 5-18.

5. The method according to claim 4 wherein the additional one, two, three, four or more said markers comprise an amino acid sequence selected from any one of SEQ ID Nos 34-35, 37, 38, 40-42, 44, 47-56, 59-60, 62, 64-83, 85-87, 89-92, 95-100, 102, 104-116, 118-121, 123-127, 132, 133, 137-141, 144-147, 149, 151-153, 155-161, 164, 165, 167-175, 177-179, 182-187, 190, 193-195, 197-200, 202, 205-209, 211, 213-222, 224-227, 229-241 and 243 or an amino acid sequence recited in column 4 of Table 2, namely any one of SEQ ID Nos 36, 39-40, 42, 43, 45-47, 57-58, 61, 63, 66, 75, 84, 88, 91, 93, 94, 98, 100, 108, 111, 115, 121, 123, 124, 126, 128-131, 134-136, 140, 142, 143, 147-150, 152-154, 160-163, 166, 168, 172, 174-176, 180, 181, 188, 190-192, 196, 200, 201, 203, 204, 212, 214, 216, 218, 224, 228, 238-239, 242, 244, and 245 and/or wherein the marker is derived from a protein in an isoform **characterized by** a pI and MW as listed in columns 2 and 3 of Table 2.

6. A method according to claim 4 or 5 wherein the additional one, two, three, four or more said marker sequences overlap with or are preferably within sequences corresponding to the extracellular portions of the proteins having a sequence selected from any one of SEQ ID Nos 1-3, 5, 8, 9 and 15 (i.e. overlap with or are preferably within sequences corresponding to the sequences selected from SEQ ID Nos 19, 21, 22, 27, 29, 30 and 32).

7. An antibody or other affinity reagent such as an Affibody, Nanobody or Unibody capable of specific binding to a soluble polypeptide derived from a protein defined by SEQ ID No 4.

8. An affinity reagent according to claim 7 which contains or is conjugated to a diagnostic or a therapeutic moiety.

9. A kit comprising one or more affinity reagents as defined in claim 7 or claim 8, wherein said affinity reagent is suitable for use in diagnosis.

10. Use of an affinity reagent according to claim 7 or claim 8 or a kit according to claim 9 for diagnosing colorectal cancer in a subject, differentiating causes of colorectal cancer in a subject, guiding therapy in a subject suffering from colorectal cancer, assessing the risk of relapse in a subject suffering from colorectal cancer, or assigning a prognostic risk of one or more future clinical outcomes to a subject suffering from colorectal cancer.

11. A method for identifying the presence or absence of colorectal cancer cells in a biological sample obtained from a human subject, which comprises the step of identifying the presence or absence of one or more soluble polypeptides derived from a protein defined by SEQ ID No 4, wherein the presence of one or more said soluble polypeptides indicates the presence of colorectal cancer in the patient.

**12.** A method of detecting or diagnosing colorectal cancer in a subject, differentiating causes of colorectal cancer in a subject, guiding therapy in a subject suffering from colorectal cancer, assessing the risk of relapse in a subject suffering from colorectal cancer, or assigning a prognostic risk of one or more future clinical outcomes to a subject suffering from colorectal cancer, the method comprising:

(a) bringing into contact with a sample to be tested from said subject one or more affinity reagents as defined in claim 7 or claim 8; and
(b) thereby detecting the presence of one or more soluble polypeptides derived from a protein defined by SEQ ID No 4 in the sample, wherein the presence of one or more said soluble polypeptides indicates the presence of colorectal cancer in the patient.

**13.** A method according to any one of the preceding claims wherein the soluble polypeptide derived from a protein defined by SEQ ID No 4, is detected by a method which involves use of an imaging technology, wherein the imaging technology optionally involves use of labelled antibodies or Affibodies.

**14.** A method for identifying the presence of colorectal cancer in a subject which comprises the step of carrying out immunohistochemistry to determine the localisation of colorectal cancer cells, particularly metastatic colorectal cancer cells, in tissue sections, by the use of labeled affinity reagents according to claim 7 or claim 8, or derivatives and analogs thereof, in order to determine presence or amount of one or more soluble polypeptides derived from a protein defined by SEQ ID No 4, wherein the presence or amount of one or more of said soluble polypeptides indicates the presence of colorectal cancer in the subject.

**15.** A method of treating or preventing colorectal cancer comprising administering to a subject in need thereof a therapeutically effective amount of an affinity reagent according to claim 7 or claim 8 or a fragment thereof.

## Figure 1: Box Plot data for CRCMP#19

Oneway Analysis of AGP2 By Diagnosis

Excluded Rows       16

**Quantiles**

| Level | Minimum | 10% | 25% | Median | 75% | 90% | Maximum |
|---|---|---|---|---|---|---|---|
| Colon | 0.227 | 0.227 | 0.26275 | 0.6275 | 1.09125 | 2.099 | 2.099 |
| Normal | 0.071 | 0.071 | 0.0925 | 0.146 | 0.19525 | 0.303 | 0.303 |

**Oneway Anova**

Means for Oneway Anova

| Level | Number | Mean | Std Error | Lower 95% | Upper 95% |
|---|---|---|---|---|---|
| Colon | 8 | 0.773375 | 0.15810 | 0.4343 | 1.1125 |
| Normal | 8 | 0.154875 | 0.15810 | -0.1842 | 0.4940 |

Std Error uses a pooled estimate of error variance

**Means Comparisons**

Comparisons for each pair using Student's t

| Level | - Level | Difference | Lower CL | Upper CL | p-Value | |
|---|---|---|---|---|---|---|
| Colon | Normal | 0.6185000 | 0.1389403 | 1.098060 | 0.0152* | |

## Figure 2: Box Plot data for CRCMP#6

**Oneway Analysis of Matriptase, ng/ml By Diagnosis**

Excluded Rows    21

**Quantiles**

| Level | Minimum | 10% | 25% | Median | 75% | 90% | Maximum |
|---|---|---|---|---|---|---|---|
| Colon | 0.554945 | 0.554945 | 0.658045 | 1.094872 | 1.785822 | 2.345228 | 2.345228 |
| Normal | 0.315512 | 0.324223 | 0.409648 | 0.480572 | 0.721868 | 0.915032 | 0.927906 |

**Oneway Anova**

**Means for Oneway Anova**

| Level | Number | Mean | Std Error | Lower 95% | Upper 95% |
|---|---|---|---|---|---|
| Colon | 9 | 1.22124 | 0.15431 | 0.89568 | 1.5468 |
| Normal | 10 | 0.56250 | 0.14639 | 0.25364 | 0.8714 |

Std Error uses a pooled estimate of error variance

**Means Comparisons**

**Comparisons for each pair using Student's t**

| Level | - Level | Difference | Lower CL | Upper CL | p-Value | |
|---|---|---|---|---|---|---|
| Colon | Normal | 0.6587467 | 0.2099853 | 1.107508 | 0.0065* | |

171

## Figure 3: Box Plot data for CRCMP#22

Oneway Analysis of Assay 1 (01181_01801) By Diagnosis (Cancer)

Excluded Rows    20

### Quantiles

| Level | Minimum | 10% | 25% | Median | 75% | 90% | Maximum |
|---|---|---|---|---|---|---|---|
| Colon | 0.7 | 0.7 | 1.045 | 4.28 | 13.095 | 19.287 | 19.81 |
| normal | 0.31 | 0.325 | 0.565 | 0.905 | 1.555 | 2.059 | 2.1 |

### Oneway Anova

#### Means for Oneway Anova

| Level | Number | Mean | Std Error | Lower 95% | Upper 95% |
|---|---|---|---|---|---|
| Colon | 10 | 6.74400 | 1.5219 | 3.547 | 9.9415 |
| normal | 10 | 1.01900 | 1.5219 | -2.178 | 4.2165 |

Std Error uses a pooled estimate of error variance

### Means Comparisons

#### Comparisons for each pair using Student's t

| Level | - Level | Difference | Lower CL | Upper CL | p-Value |
|---|---|---|---|---|---|
| Colon | normal | 5.725000 | 1.203073 | 10.24693 | 0.0160* |

## Figure 4: Box Plot data for CRCMP#10

Oneway Analysis of Protocadherin Beta 8, ng/ml (assay 2) By Diagnosis

Excluded Rows    20

**Quantiles**

| Level | Minimum | 10% | 25% | Median | 75% | 90% | Maximum |
|---|---|---|---|---|---|---|---|
| colon | 0 | 0 | 0 | 0.020257 | 0.482163 | 0.644041 | 0.653451 |
| Normal | 0 | 0 | 0 | 0 | 0.023616 | 0.113222 | 0.115575 |

**Oneway Anova**

**Means for Oneway Anova**

| Level | Number | Mean | Std Error | Lower 95% | Upper 95% |
|---|---|---|---|---|---|
| colon | 10 | 0.214823 | 0.06228 | 0.0840 | 0.34566 |
| Normal | 10 | 0.020843 | 0.06228 | -0.1100 | 0.15168 |

Std Error uses a pooled estimate of error variance

**Means Comparisons**

**Comparisons for each pair using Student's t**

| Level | - Level | Difference | Lower CL | Upper CL | p-Value | |
|---|---|---|---|---|---|---|
| colon | Normal | 0.1939800 | 0.0089469 | 0.3790130 | 0.0409* | |

## Figure 5: Box Plot data for CRCMP#9

Oneway Analysis of AGP3 By Diagnosis

Excluded Rows    16

**Quantiles**

| Level | Minimum | 10% | 25% | Median | 75% | 90% | Maximum |
|---|---|---|---|---|---|---|---|
| Colon | 0.23 | 0.23 | 0.7 | 1.945 | 3.58 | 4.37 | 4.37 |
| Normal | 1.7 | 1.7 | 2.8525 | 4.205 | 9.435 | 19.37 | 19.37 |

**Oneway Anova**

**Means for Oneway Anova**

| Level | Number | Mean | Std Error | Lower 95% | Upper 95% |
|---|---|---|---|---|---|
| Colon | 8 | 2.16375 | 1.5110 | -1.077 | 5.4044 |
| Normal | 8 | 6.64125 | 1.5110 | 3.401 | 9.8819 |

Std Error uses a pooled estimate of error variance

**Means Comparisons**

**Comparisons for each pair using Student's t**

| Level | - Level | Difference | Lower CL | Upper CL | p-Value | |
|---|---|---|---|---|---|---|
| Normal | Colon | 4.477500 | -0.105522 | 9.060522 | 0.0548 | |

## Figure 6: ROC curve data for CRCMP#19

| Curve | Area | SE | p | 95% CI of Area | | Colon vs Normals = colon cancer |
|-------|------|-----|---|----------------|---|------------------------------|
| AGP2 ng/ml | 0.621 | 0.0501 | 0.0079 | 0.523 | to 0.719 | have higher values |

## Figure 7: Box Plot data for CRCMP#19

| AGP2 ng/ml by Diagnosis | n | Mean | SD | SE | 95% CI of Mean | | Median | IQR | 95% CI of Median | |
|---|---|---|---|---|---|---|---|---|---|---|
| colon cancer | 65 | 0.762 | 2.1409 | 0.2656 | 0.231 | to 1.293 | 0.110 | 0.221 | 0.081 | to 0.171 |
| normal | 61 | 0.140 | 0.3044 | 0.0390 | 0.062 | to 0.218 | 0.082 | 0.072 | 0.063 | to 0.098 |

## Figure 8: ROC curve data for CRCMP#9

| Curve | Area | SE | p | 95% CI of Area | | Colon vs Normals = colon cancer |
|---|---|---|---|---|---|---|
| AGP3ng/ml | 0.863 | 0.0334 | <0.0001 | 0.797 | to 0.928 | have lower values |

EP 2 375 255 A1

## Figure 9: Box Plot data for CRCMP#9

| AGP3ng/ml by Diagnosis | n | Mean | SD | SE | 95% CI of Mean | | Median | IQR | 95% CI of Median | |
|---|---|---|---|---|---|---|---|---|---|---|
| colon cancer | 65 | 2.460 | 2.9292 | 0.3633 | 1.734 | to 3.186 | 1.656 | 1.944 | 1.203 | to 2.187 |
| normal | 61 | 7.248 | 7.1884 | 0.9204 | 5.407 | to 9.089 | 4.849 | 6.050 | 3.985 | to 7.451 |

177

## Figure 10(a):

**CRCMP #1**

**Peptide Source:** 1D-GE CRC

**SP: Q12864 (SEQ ID No: 1)**

MILQAHLHSLCLLMLYLATGYGQ*EGKFSGPLKPMTFSIYEGQEPSQIIFQFKANPPAVTFELTGETDNIFVIER***E
GLLYYNR***ALDRETRSTHNLQVAALDANGIIVEGPVPITIKVK**DINDNRPTFLQSK**YEGSVRQNSRPGKPFLYVNA
TDLDDPATPNGQLYYQIVIQLPMINNVMYFQINNK**TGAISLTR**EGSQELNPAKNPSYNLVISVKDMGGQSENSFS
DTTSVDIIVTENIWKAPKPVEMVENSTDPHPIKITQVR**WNDPGAQYSLVDK**EKLPRFPFSIDQEGDIYVTQPLDR
EEK*DAYVFYAVAK***DEYGKPLSYPLEIHVK**VKDINDNPPTCPSPVTVFEVQENERLGNSIGTLTAHDR**DEENTANS
FLNYR**IVEQTPKLPMDGLFLIQTYAGMLQLAKQSLKKQDTPQYNLTIEVSDKDFKTLCFVQINVIDINDQIPIFE
KSDYGNLTLAEDTNIGSTILTIQATDADEPFTGSSKILYHIIKGDSEGRLGVDTDPHTNTGYVIIKKPLDFETAA
VSNIVFK**AENPEPLVFGVK**YNASSFAKFTLIVTDVNEAPQFSQHVFQAK*VSEDVAIGTK*VGNVTAKDPEGLDISY
SLR**GDTRGWLKIDHVTGEIFSVAPLDR**EAGSPYRVQVVATEVGGSSLSSVSEFHLILMDVNDNPPRLAKDYTGLF
FCHPLSAPGSLIFEATDDDQHLFRGPHFTFSLGSGSLQNDWEVSKINGTHARLSTR**HTEFEER**EYVVLIRINDGG
RPPLEGIVSLPVTFCSCVEGSCFRPAGHQTGIPTVGMAVGILLTTLLVIGIILAVVFIRIKKDKGK**DNVESAQAS
EVKPLR**S

### Mass Match Peptides (bold):

    AENPEPLVFGVK [37]
    DEENTANSFLNYR [64]
    DEYGKPLSYPLEIHVK [65]
    DINDNRPTFLQSK [68]
    DNVESAQASEVKPLR [70]
    EGLLYYNR [81]
    GDTRGWLK [104]
    HTEFEER [118]
    IDHVTGEIFSVAPLDR [119]
    TGAISLTR [202]
    WNDPGAQYSLVDK [227]

### Tandem Peptides (double underline):

    AENPEPLVFGVK [37]
    DAYVFYAVAK [62]
    DEENTANSFLNYR [64]
    DNVESAQASEVKPLR [70]
    VSEDVALGTK [220]
    WNDPGAQYSLVDK [227]

### Extracellular Domain (underline): (SEQ ID No: 19)

    QEGKFSGPLKPMTFSIYEGQEPSQIIFQFKANPPAVTFELTGETDNIFVIEREGLLYYNRALDRET
RSTHNLQVAALDANGIIVEGPVPITIKVKDINDNRPTFLQSKYEGSVRQNSRPGKPFLYVNATDLDDPATPNGQ
LYYQIVIQLPMINNVMYFQINNKTGAISLTREGSQELNPAKNPSYNLVISVKDMGGQSENSFSDTTSVDIIVTE
NIWKAPKPVEMVENSTDPHPIKITQVRWNDPGAQYSLVDKEKLPRFPFSIDQEGDIYVTQPLDREEKDAYVFYA
VAKDEYGKPLSYPLEIHVKVKDINDNPPTCPSPVTVFEVQENERLGNSIGTLTAHDRDEENTANSFLNYRIVEQ
TPKLPMDGLFLIQTYAGMLQLAKQSLKKQDTPQYNLTIEVSDKDFKTLCFVQINVIDINDQIPIFEKSDYGNLT
LAEDTNIGSTILTIQATDADEPFTGSSKILYHIIKGDSEGRLGVDTDPHTNTGYVIIKKPLDFETAAVSNIVFK
AENPEPLVFGVKYNASSFAKFTLIVTDVNEAPQFSQHVFQAKVSEDVAIGTKVGNVTAKDPEGLDISYSLRGDT
RGWLKIDHVTGEIFSVAPLDREAGSPYRVQVVATEVGGSSLSSVSEFHLILMDVNDNPPRLAKDYTGLFFCHPL
SAPGSLIFEATDDDQHLFRGPHFTFSLGSGSLQNDWEVSKINGTHARLSTRHTEFEEREYVVLIRINDGGRPPL
EGIVSLPVTFCSCVEGSCFRPAGHQTGIPTVGM

### Recombinant Protein (italics): (SEQ ID No: 20)

    EGKFSGPLKPMTFSIYEGQEPSQIIFQFKANPPAVTFELTGETDNIFVIEREGLLYYNRALDRETR
STHNLQVAALDANGIIVEGPVPITIEVKDINDNRPTFLQSKY

**Figure 10(b):**

**CRCMP #2**

**Peptide Source:** 1D-GE CRC

**SP: Q99795 (SEQ ID No: 2)**

MVGKMWPVLWTLCAVR<u>VTVDAISVETPQDVLRASQGK</u>**SVTLPCTYHTSTSSREGLIQWDKLLLTHTER**VVIWPFS
NK**NYIHGELYK**NRVSISNNAEQSDASITIDQLTMADNGTYECSVSLMSDLEGNTKSRVRLLVLVPPSKPECGIEG
ETIIGNNIQLTCQSK**EGSPTPQYSWK**R**YNILNQEQPLAQPASGQPVSLK**NISTDTSGYYICTSSNEEGTQFCNIT
VAVRSPSMNVALYVGIAVGVVAALIIIGIIIYCCCCRGKDDNTEDKEDARPNR**EAYEEPPEQLR**ELSR**EREEEDD**
**YRQEEQR**STGRESPDHLDQ

**Mass Match Peptides (bold):**
    EAYEEPPEQLR [76]
    EGLIQWDK [80]
    EGSPTPQYSWK [82]
    EREEEDDYR [86]
    EREEEDDYRQEEQR [87]
    LLLTHTER [146]
    NYIHGELYK [165]
    SVTLPCTYHTSTSSR [195]
    YNILNQEQPLAQPASGQPVSLK [240]

**Tandem Peptides (double underline):**
    EAYEEPPEQLR [76]
    LLLTHTER [146]
    VTVDAISVETPQDVLR [222]

**Extracellular Domain (underline): (SEQ ID No: 21)**
        ISVETPQDVLRASQGKSVTLPCTYHTSTSSREGLIQWDKLLLTHTERVVIWPFSNKNYIHGELYKN
RVSISNNAEQSDASITIDQLTMADNGTYECSVSLMSDLEGNTKSRVRLLVLVPPSKPECGIEGETIIGNNIQLT
CQSKEGSPTPQYSWKRYNILNQEQPLAQPASGQPVSLKNISTDTSGYYICTSSNEEGTQFCNITVAVRSPSMNV

## Figure 10(c):

**CRCMP #5**

**Peptide Source:** 1D-GE CRC

**SP: P29323 (SEQ ID No: 3)**

MALRRLGAALLLLPLLAAVEETLMDSTTATAELGWMVHPPSGWEEVSGYDENMNTIRTYQVCNVFESSQNNWLRT
KFIRRRGAHRIHVEMKFSVRDCSSIPSVPGSCKETFNLYYYEADFDSATKTFPNWMENPWVKVDTIAADESFSQV
DLGGRVMKINTEVRSFGPVSRSGFYLAFQDYGGCMSLIAVRVFYRKCPRIIQNGAIFQETLSGAESTSLVAARGS
*CIANAEEVDVPIKLYCNGDGEWLVPIGRCMCKAGFEAVENGTVCRGCPSGTFKANQGDEACTHCPINSRTTSEGA*
*TNCVCRNGYYRADLDPLDMPCTTIP*SAPQAVISSVNETSLMLEWTPPRDSGGREDLVYNIICKSCGSGRGACTRC
GDNVQYAPR**QLGLTEPR**IYISDLLAHTQYTFEIQAVNGVTDQSPFSPQFASVNITTNQAAPSAVSIMHQVSRTVD
SITLSWSQPDQPNGVILDYELQYYEKELSEYNATAIKSPTNTVTVQGLKAGAIYVFQVRAR**TVAGYGRYSGK**MYF
QTMTEAEYQTSIQEKLPLIIGSSAAGLVFLIAVVVIAIVCNR*RGFERADSEYTDKLQHYTSGHMTPGMKIYIDPF*
*TYEDPNEAVREFAKEIDISCVKIEQVIGAGEFGEVCSGHLKLPGKREIFVAIKTLKSGYTEKQRRDFLSEASIMG*
*QFDHPNVIHLEGVVTKSTPVMIITEFMENGSLDSFLRQNDGQFTVIQLVGMLRGIAAGMK****YLADMNYVHR****DLAAR*
*NILVNSNLVCK****VSDFGLSR****FLEDDTSDPTYTSALGGK****IPIRWTAPEAIQYR****KFTSASDVWSYGIVMWEVMSYGER*
*PYWDMTNQDVINAIEQDYRLPPPMDCPSALHQLMLDCWQK***DRNHRPK**FGQIVNTLDK***MIRNPNSLK****AMAPLSSGI*
*NLPLLDRTIPDYTSFNTVDEWLEAIKMGQYKESFANAGFTSFDVVSQMMMEDILRVGVTLAGHQKKILNSIQVMR*
*AQMNQIQSVEGQPLARRPRATGRTKRCQPRDVTKKTCNSNDGKKKGMGKKKTDPGRGREIQGIFFKEDSHKESND*
*CSCGG*

### Mass Match Peptides (bold):
DRNHRPK [72]
IPIRWTAPEAIQYR [127]
MIRNPNSLK [158]
QLGLTEPR [170]
TVAGYGRYSGK [209]
VSDFGLSR [219]
WTAPEAIQYR [229]
YLADMNYVHR [237]

### Tandem Peptides (double underline):
FGQIVNTLDK [92]
WTAPEAIQYR [229]

### Extracellular Domain (underline): (SEQ ID No: 22)
VEETLMDSTTATAELGWMVHPPSGWEEVSGYDENMNTIRTYQVCNVFESSQNNWLRTKFIRRRGAH
RIHVEMKFSVRDCSSIPSVPGSCKETFNLYYYEADFDSATKTFPNWMENPWVKVDTIAADESFSQVDLGGRVMK
INTEVRSFGPVSRSGFYLAFQDYGGCMSLIAVRVFYRKCPRIIQNGAIFQETLSGAESTSLVAARGSCIANAEE
VDVPIKLYCNGDGEWLVPIGRCMCKAGFEAVENGTVCRGCPSGTFKANQGDEACTHCPINSRTTSEGATNCVCR
NGYYRADLDPLDMPCTTIPSAPQAVISSVNETSLMLEWTPPRDSGGREDLVYNIICKSCGSGRGACTRCGDNVQ
YAPRQLGLTEPRIYISDLLAHTQYTFEIQAVNGVTDQSPFSPQFASVNITTNQAAPSAVSIMHQVSRTVDSITL
SWSQPDQPNGVILDYELQYYEKELSEYNATAIKSPTNTVTVQGLKAGAIYVFQVRARTVAGYGRYSGKMYFQTM
TEAEYQTSIQEKLPL

### Recombinant Protein (italics): (SEQ ID Nos: 23, 24)
CIANAEEVDVPIKLYCNGDGEWLVPIGRCMCKAGFEAVENGTVCRGCPSGTFKANQGDEACTHCPI
NSRTTSEGATNCVCRNGYYRADLDPLDMPCTTIP
GFERADSEYTDKLQHYTSGHMTPGMKIYIDPFTYEDPNEAVREFAKEIDISCVKIEQVIGAGEFGE
VCSGHLKLPGKREIFVAIKTLKSGYTEKQRRDFLSEASIMGQFDHPNVIHLEGVVTKSTPVMIITEFMENGSLD
SFLRQNDGQFTVIQLVGMLRGIAAGMKYLADMNYVHRDLAARNILVNSNLVCKVSDFGLSRFLEDDTSDPTYTS
ALGGKIPIRWTAPEAIQYRKFTSASDVWSYGIVMWEVMSYGERPYWDMTNQDVINAIEQDYRLPPPMDCPSALH
QLMLDCWQKDRNHRPKFGQIVNTLDKMIRNPNSLKAMAPLSSGINLPLLDRTIPDYTSFNTVDEWLEAIKMGQY
KESFANAGFTSFDVVSQMMMEDILRVGVTLAGHQKKILNSIQVMRAQMNQIQSVEGQPLARRPRATGRTKRCQP
RDVTKKTCNSNDGKKKGMGKKKTDPGRGREIQGIFFKEDSHKESNDCSCGG

## Figure 10(d):

## CRCMP #6

**Peptide Source:** 1D-GE CRC

## SP: Q9Y5Y6 (SEQ ID No: 4)

MGSDRARKGGGGPKDFGAGLKYNSRHEKVNGLEEGVEFLPVNNVKKVEKHGPGRWVVLAAVLIGLLLVLLGIGFL
VWHLQYRDVRVQKVFNGYMRITNENFVDAYENSNSTEFVSLASKVKDALKLLYSGVPFLGPYHKESAVTAFSEGS
VIAYYWSEFSIPQHLVEEAERVMAEER**VVMLPPR**ARSLK**SFVVTSVVAFPTDSK**TVQRTQDNSCSFGLHARGVEL
MR**FTTPGFPDSPYPAHAR**CQWALR*GDADSVLSLTFR*SFDLASCDERGSDLVTVYNTLSPMEPHALVQLCGTY*PPS
YNLTFHSSQNVLLITLITNTERR**HPGFEATFFQLPR***MSSCGGRLRKAQGTFNSPYYPGHYPPNIDCTWNIEVPNN
QHVKVRFKFFYLLEPGVPAGTCPKD*YVEINGEKYCGERSQFVVTSNSNKITVRFHSDQSYTDTGFLAEYLSYDSS
DPCPGQFTCRTGRCIRKELRCDGWADCTDHSDELNCSCDAGHQFTCKNKFCKPLFWVCDSVNDCGDNSDEQGCSC
PAQTFRCSNGKCLSKSQQCNGKDDCGDGSDEASCPKVNVVTCTKHTYRCLNGLCLSKGNPECDGKEDCSDGSDEK
DCDCGLRSFTRQARVVGGTDADEGEWPWQVSLHALGQGHICGASLISPNWLVSAAHCYIDDRGFRYSDPTQWTAF
LGLHDQSQRSAPGVQERRLKRIISHPFFNDFTFDYDIALLELEKPAEYSSMVRPICLPDASHVFPAGKAIWVTGW
GHTQYGGTGALILQKGEIRVINQTTCENLLPQQITPRMMCVGFLSGGVDSCQGDSGGPLSSVEADGR**IFQAGVVS
WGDGCAQR**NKPGVYTRLPLFRDWIKENTGV

## Mass Match Peptides (bold):

        FTTPGFPDSPYPAHAR [101]
        HPGFEATFFQLPR [117]
        IFQAGVVSWGDGCAQR [122]
        SFVVTSVVAFPTDSK [189]
        VVMLPPR [223]

## Tandem Peptides (double underline):

        FTTPGFPDSPYPAHAR [101]
        GDADSVLSLTFR [103]
        SFVVTSVVAFPTDSK [189]

## Extracellular Domain (underline): (SEQ ID No: 25)

        WHLQYRDVRVQKVFNGYMRITNENFVDAYENSNSTEFVSLASKVKDALKLLYSGVPFLGPYHKESA
VTAFSEGSVIAYYWSEFSIPQHLVEEAERVMAEERVVMLPPRARSLKSFVVTSVVAFPTDSKTVQRTQDNSCSF
GLHARGVELMRFTTPGFPDSPYPAHARCQWALRGDADSVLSLTFRSFDLASCDERGSDLVTVYNTLSPMEPHAL
VQLCGTYPPSYNLTFHSSQNVLLITLITNTERRHPGFEATFFQLPRMSSCGGRLRKAQGTFNSPYYPGHYPPNI
DCTWNIEVPNNQHVKVRFKFFYLLEPGVPAGTCPKDYVEINGEKYCGERSQFVVTSNSNKITVRFHSDQSYTDT
GFLAEYLSYDSSDPCPGQFTCRTGRCIRKELRCDGWADCTDHSDELNCSCDAGHQFTCKNKFCKPLFWVCDSVN
DCGDNSDEQGCSCPAQTFRCSNGKCLSKSQQCNGKDDCGDGSDEASCPKVNVVTCTKHTYRCLNGLCLSKGNPE
CDGKEDCSDGSDEKDCDCGLRSFTRQARVVGGTDADEGEWPWQVSLHALGQGHICGASLISPNWLVSAAHCYID
DRGFRYSDPTQWTAFLGLHDQSQRSAPGVQERRLKRIISHPFFNDFTFDYDIALLELEKPAEYSSMVRPICLPD
ASHVFPAGKAIWVTGWGHTQYGGTGALILQKGEIRVINQTTCENLLPQQITPRMMCVGFLSGGVDSCQGDSGGP
LSSVEADGRIFQAGVVSWGDGCAQRNKPGVYTRLPLFRDWIKENTGV

## Recombinant Protein (italics): (SEQ ID No: 26)

        PPSYNLTFHSSQNVLLITLITNTERRHPGFEATFFQLPRMSSCGGRLRKAQGTFNSPYYPGHYPPN
IDCTWNIEVPNNQHVKVRFKFFYLLEPGVPAGTCPKD

## Figure 10(e):

**CRCMP #7**

**Peptide Source:** 1D-GE CRC

**SP: P18433 (SEQ ID No: 5)**

MDSWFILVLLGSGLICVSANNATTVAPSVGITRLINSSTAEPVKEEAKTSNPTSSLTSLSVAPTFSPNITLGPTY
LTTVNSSDSDNGTTRTASTNSIGITISPNGTWLPDNQFTDARTEPWEGNSSTAATTPETFPPSDETPIIAVMVAL
SSLLVIVFIIIVLYMLRFKKYKQAGSHSNSKQAGSHSNSFRLSNGR<u>**TEDVEPQSVPLLAR**</u>SPSTNRK**YPPLPVDK**
LEEEINRRMADDNKLFREEFNALPACPIQATCEAASKEENKEKNRYVNILPYDHSRVHLTPVEGVPDSDYINASF
INGYQEKNKFIAAQGPKEETVNDFWRMIWEQNTATIVMVTNLKERKECKCAQYWPDQGCWTYGNIRVSVEDVTVL
VDYTVRKFCIQQVGDMTNRKPQRLITQFHFTSWPDFGVPFTPIGMLKFLKKVKACNPQYAGAIVVHCSAGVGRTG
TFVVIDAMLDMMHTERKVDVYGFVSRIRAQRCQMVQTDMQYVFIYQALLEHYLYGDTELEVTSLETHLQKIYNKI
PGTSNNGLEEEFKKLTSIKIQNDKMRTGNLPANMKKNRVLQIIPYEFNRVIIPVKRGEENTDYVNASFIDGYRQK
DSYIASQGPLLHTIEDFWRMIWEWKSCSIVMLTELEERGQEKCAQYWPSDGLVSYGDITVELKKEEECESYTVRD
LLVTNTRENKSRQIRQFHFHGWPEVGIPSDGKGMISIIAAVQKQQQQSGNHPITVHCSAGAGRTGTFCALSTVLE
RVKAEGILDVFQTVKSLRLQRPHMVQTLEQYEFCYKVVQEYIDAFSDYANFK

**Mass Match Peptides (bold):**
    TEDVEPQSVPLLAR [200]
    YPPLPVDK [241]

**Tandem Peptides (double underline):**
    TEDVEPQSVPLLAR [200]

**Extracellular Domain (underline): (SEQ ID No: 27)**
    NNATTVAPSVGITRLINSSTAEPVKEEAKTSNPTSSLTSLSVAPTFSPNITLGPTYLTTVNSSDSD
NGTTRTASTNSIGITISPNGTWLPDNQFTDARTEPWEGNSSTAATTPETFPPSDETP

**Peptide Source:** 2D-GE

**SP: P18433 (SEQ ID No: 5)**

MDSWFILVLLGSGLICVS<u>ANNATTVAPSVGITRLINSSTAEPVKEEAKTSNPTSSLTSLSVAPTFSPNITLGPTY
LTTVNSSDSDNGTTRTASTNSIGITISPNGTWLPDNQFTDARTEPWEGNSSTAATTPETFPPSDETP</u>IIAVMVAL
SSLLVIVFIIIVLYMLRFKKYKQAGSHSNSK**QAGSHSNSFR**LSNGR<u>TEDVEPQSVPLLAR</u>SPSTNRKYPPLPVDK
LEEEINRRMADDNKLFREEFNALPACPIQATCEAASKEENKEKNRYVNILPYDHSRVHLTPVEGVPDSDYINASF
INGYQEKNKFIAAQGPKEETVNDFWRMIWEQNTATIVMVTNLKERKECKCAQYWPDQGCWTYGNIRVSVEDVTVL
VDYTVR<u>KFCIQQVGDMTNR</u>KPQRLITQFHFTSWPDFGVPFTPIGMLKFLKKVKACNPQYAGAIVVHCSAGVGRTG
TFVVIDAMLDMMHTERKVDVYGFVSRIRAQRCQMVQTDMQYVFIYQALLEHYLYGDTELEVTSLETHLQKIYNKI
PGTSNNGLEEEFKKLTSIKIQNDKMRTGNLPANMKKNRVLQIIPYEFNRVIIPVKRGEENTDYVNASFIDGYRQK
DSYIASQGPLLHTIEDFWRMIWEWKSCSIVMLTELEERGQEKCAQYWPSDGLVSYGDITVELKKEEECESYTVRD
LLVTNTRENKSRQIRQFHFHGWPEVGIPSDGKGMISIIAAVQKQQQQSGNHPITVHCSAGAGRTGTFCALSTVLE
RVKAEGILDVFQTVKSLRLQRPHMVQTLEQYEFCYKVVQEYIDAFSDYANFK

**Mass Match Peptides (bold):**
    QAGSHSNSFR [166]

**Tandem Peptides (double underline):**
    KFCIQQVGDMTNR [130]
    TEDVEPQSVPLLAR [200]
    QAGSHSNSFR [166]

**Extracellular Domain (underline): (SEQ ID No: 27)**
    NNATTVAPSVGITRLINSSTAEPVKEEAKTSNPTSSLTSLSVAPTFSPNITLGPTYLTTVNSSDSD
NGTTRTASTNSIGITISPNGTWLPDNQFTDARTEPWEGNSSTAATTPETFPPSDETP

## Figure 10(f):

**CRCMP #8**

**Peptide Source:** 1D-GE CRC

**SP: Q6P1M3 (SEQ ID No: 6)**

MRR**FLRPGHDPVR**ERLKRDLFQFNKTVEHGFPHQPSALGYSPSLRILAIGTRSGAIKLYGAPGVEFMGLHQENNA
VTQIHLLPGQCQLVTLLDDNSLHLW*SLKVK***GGASELQEDESFTLR***GPPGAAPSATQITVVLPHSSCELLYLGTES
GNVFVVQLPAFRALEDRTISSDAVLQRLPEEARHRR***VFEMVEALQEHPR***DPNQILIGYSRGLVVIWDLQGSRVLY
HFLSSQQLENIWWQRDGRLLVSCHSDGSYCQWPVSSEAQQPEPLRSLVPYGPFPCKAITRILWLTTRQGLPFTIF
QGGMPRASYGDRHCISVIHDGQQTAFDFTSRVIGFTVLTEADPAATFDDPYALVVLAEEELVVIDLQTAGWPPVQ
LPYLASLHCSAITCSHHVSNIPLKLWERIIAAGSRQNAHFSTMEWPIDGGTSLTPAPPQRDLLLTGHEDGTVRFW
DASGVCLRLLYKLSTVRVFLTDTDPNENFSAQGEDEWPPLRKVGSFDPYSDDPRLGIQKIFLCKYSGYLAVAGTA
GQVLVLELNDEAAEQAVEQVEADLLQDQEGYRWKGHERLAARSGPVRFEPGFQPFVLVQCQPPAVVTSLALHSEW
RLVAFGTSHGFGLFDHQQRRQVFVKCTLHPSDQLALEGPLSRVKSLKKSLRQSFRRMRRSRVSSRKRHPAGPPGE
AQEGSAKAERPGLQNMELAPVQRKIEAR**SAEDSFTGFVR**<u>TLYFADTYLK</u>DSSRHCPSLWAGTNGGTIYAFSLR**VP**
**PAERR**MDEPVRAEQAKEIQLMHRAPVVGILVLDGHSVPLPEPLEVAHDLSKSPDMQGSHQLLVVSEEQFKVFTLP
KVSAKLKLKLTALEGSRVRR**VSVAHFGSR**RAEDYGEHHLAVLTNLGDIQVVSLPLLKPQVRYSCIR**REDVSGIAS**
**CVFTK**<u>YGQGFYLISPSEFER</u>FSLSTKWLVEPRCLVDSAETKNHRPGNGAGPKKAPSRARNSGTQSDGEEK**QPGLV**
**MERALLSDER**AATGVHIEPPWGAASAMAEQSEWLSVQAAR

**Mass Match Peptides (bold):**

ALLSDER [41]
FLRPGHDPVR [95]
GGASELQEDESFTLR [107]
QPGLVMERALLSDER [171]
REDVSGIASCVFTK [177]
SAEDSFTGFVR [183]
VFEMVEALQEHPR [213]
VPPAERR [217]
VSVAHFGSR [221]
YGQGFYLISPSEFER [234]

**Tandem Peptides (double underline):**

GGASELQEDESFTLR [107]
TLYFADTYLK [206]
SAEDSFTGFVR [183]
YGQGFYLLSPSEFER [235]

**Recombinant Protein (italics): (SEQ ID No: 28)**

SLKVKGGASELQEDESFTLRGPPGAAPSATQITVVLPHSSCELLYLGTESGNVFVVQLPAFRALED
RTISSDAVLQRLPEEARHRRVFEMVEALQEHPR

## Figure 10(g):

**CRCMP #9**

**Peptide Source**: 1D-GE CRC

**SP: Q8TD06 (SEQ ID No: 7)**

```
MMLHSALGLCLLLVTVSSNLAIAIKKEKRPPQTLSRGWGDDITWVQTYEEGLFYAQKSKKPLMVIHHLEDCQYSQ
ALKKVFAQNEEIQEMAQNKFIMLNLMHETTDKNLSPDGQYVPRIMFVDPSLTVRADIAGRYSNRLYTYEPRDLPL
LIENMKKALRLIQSEL
```

**Mass Match Peptides (bold):**
          IMFVDPSLTVR [126]
          NLSPDGQYVPR [161]

**Tandem Peptides (double underline):**
          IMFVDPSLTVR [126]

**Peptide Source**: 2D-GE

**SP: Q8TD06 (SEQ ID No: 7)**

```
MMLHSALGLCLLLVTVSSNLAIAIKKEKRPPQTLSRGWGDDITWVQTYEEGLFYAQKSKKPLMVIHHLEDCQYSQ
ALKKVFAQNEEIQEMAQNKFIMLNLMHETTDKNLSPDGQYVPRIMFVDPSLTVRADIAGRYSNRLYTYEPRDLPL
LIENMKKALRLIQSEL
```

**Mass Match Peptides (bold):**
          FIMLNLMHETTDK [94]
          IMFVDPSLTVR [126]
          LYTYEPR [154]
          NLSPDGQYVPR [161]
          RPPQTLSR [180]
          VFAQNEEIQEMAQNK [212]

**Tandem Peptides (double underline):**
          IMFVDPSLTVR [126]
          NLSPDGQYVPR [161]

## Figure 10(h):

**CRCMP #10**

**Peptide Source:** 1D-GE CRC

**SP: Q9UN66 (SEQ ID No: 8)**

```
MEASGKLICRQRQVLFSFLLLGLSLAGAAEPRSYSVVEETEGSSFVTNLAKDLGLEQREFSRRGVRVVSRGNKLH
LQLNQETADLLLNEKLDREDLCGHTEPCVLRFQVLLESPFEFFQAELQVIDINDHSPVFLDKQMLVKVSESSPPG
TAFPLKNAEDLDIGQNNIENYIISPNSYFRVLTRKRSDGRKYPELVLDNALDREEEAELRLTLTALDGGSPPRSG
TAQVYIEVVDVNDNAPEFQQPFYRVQISEDSPISFLVVKVSATDVDTGVNGEISYSLFQASDEISKTFKVDFLTG
EIRLKKQLDFEKFQSYEVNIEARDAGGFSGKCTVLIQVIDVNDHAPEVTMSAFTSPIPENAPETVVALFSVSDLD
SGENGKISCSIQEDLPFLLKSSVGNFYTLLTETPLDRESRAEYNVTITVTDLGTPRLTTHLNMTVLVSDVNDNAP
AFTQTSYTLFVRENNSPALHIGSVSATDRDSGTNAQVTYSLLPPQDPHLPLASLVSINTDNGHLFALRSLDYEAL
QAFEFRVGASDRGSPALSSEALVRVLVLDANDNSPFVLYPLQNGSAPCTELVPRAAEPGYLVTKVVAVDGDSGQN
AWLSYQLLKATEPGLFGVWAHNGEVRTARLLSERDAAKQRLVVLVKDNGEPPCSATATLHLLLVDGFSQPYLPLP
EAAPAQGQADSLTVYLVVALASVSSLFLFSVLLFVAVLLCRRSRAASVGRCSVPEGPFPGHLVDVRGTGSLSQNY
QYEVCLAGGSGTNEFQFLKPVLPNIQGHSFGPEMEQNSNFRNGFGFSLQLK
```

## Mass Match Peptides (bold):


## Tandem Peptides (double underline):
```
    CSVPEGPFPGHLVDVR [60]
```
## Extracellular Domain (underline): (SEQ ID No: 29)
```
        AEPRSYSVVEETEGSSFVTNLAKDLGLEQREFSRRGVRVVSRGNKLHLQLNQETADLLLNEKLDRE
DLCGHTEPCVLRFQVLLESPFEFFQAELQVIDINDHSPVFLDKQMLVKVSESSPPGTAFPLKNAEDLDIGQNNI
ENYIISPNSYFRVLTRKRSDGRKYPELVLDNALDREEEAELRLTLTALDGGSPPRSGTAQVYIEVVDVNDNAPE
FQQPFYRVQISEDSPISFLVVKVSATDVDTGVNGEISYSLFQASDEISKTFKVDFLTGEIRLKKQLDFEKFQSY
EVNIEARDAGGFSGKCTVLIQVIDVNDHAPEVTMSAFTSPIPENAPETVVALFSVSDLDSGENGKISCSIQEDL
PFLLKSSVGNFYTLLTETPLDRESRAEYNVTITVTDLGTPRLTTHLNMTVLVSDVNDNAPAFTQTSYTLFVREN
NSPALHIGSVSATDRDSGTNAQVTYSLLPPQDPHLPLASLVSINTDNGHLFALRSLDYEALQAFEFRVGASDRG
SPALSSEALVRVLVLDANDNSPFVLYPLQNGSAPCTELVPRAAEPGYLVTKVVAVDGDSGQNAWLSYQLLKATE
PGLFGVWAHNGEVRTARLLSERDAAKQRLVVLVKDNGEPPCSATATLHLLLVDGFSQPYLPLPEAAPAQGQADS
LTVYL
```

**Figure 10(h) continued…**

**Peptide Source:** 2D-GE

**SP: Q9UN66 (SEQ ID No: 8)**

MEASGKLICRQRQVLFSFLLLGLSLAGAAEPRSYSVVEETEGSSFVTNLAKDLGLEQREFSRRGVRVVSRGNKLH
LQLNQETADLLLNEKLDREDLCGHTEPCVLRFQVLLESPFEFFQAELQVIDINDHSPVFLDKQMLVKVSESSPPG
TAFPLKNAEDLDIGQNNIENYIISPNSYFRVLTRKRSDGRKYPELVLDNALDREEEAELRLTLTALDGGSPPRSG
TAQVYIEVVDVNDNAPEFQQPFYRVQISEDSPISFLVVKVSATDVDTGVNGEISYSLFQASDEISKTFKVDFLTG
EIRLKKQLDFEKFQSYEVNIEARDAGGFSGKCTVLIQVIDVNDHAPEVTMSAFTSPIPENAPETVVALFSVSDLD
SGENGKISCSIQEDLPFLLKSSVGNFYTLLTETPLDRESR<u><u>AEYNVTITVTDLGTPR</u></u>LTTHLNMTVLVSDVNDNAP
AFTQTSYTLFVRENNSPALHIGSVSATDRDSGTNAQVTYSLLPPQDPHLPLASLVSINTDNGHLFALRSLDYEAL
QAFEFRVGASDRGSPALSSEALVRVLVLDANDNSPFVLYPLQNGSAPCTELVPRAAEPGYLVTKVVAVDGDSGQN
AWLSYQLLKATEPGLFGVWAHNGEVRTARLLSERDAAKQRLVVLVKDNGEPPCSATATLHLLLVDGFSQPYLPLP
EAAPAQGQADSLTVYLVVALASVSSLFLFSVLLFVAVLLCRRSRAASVGRCSVPEGPFPGHLVDVRGTGSLSQNY
QYEVCLAGGSGTNEFQFLKPVLPNIQGHSFGPEMEQNSNFRNGFGFSLQLK

**Mass Match Peptides (bold):**
**Tandem Peptides (double underline):**
  AEYNVTITVTDLGTPR [39]
**Extracellular Domain (underline): (SEQ ID No: 29)**
   AEPRSYSVVEETEGSSFVTNLAKDLGLEQREFSRRGVRVVSRGNKLHLQLNQETADLLLNEKLDRE
DLCGHTEPCVLRFQVLLESPFEFFQAELQVIDINDHSPVFLDKQMLVKVSESSPPGTAFPLKNAEDLDIGQNNI
ENYIISPNSYFRVLTRKRSDGRKYPELVLDNALDREEEAELRLTLTALDGGSPPRSGTAQVYIEVVDVNDNAPE
FQQPFYRVQISEDSPISFLVVKVSATDVDTGVNGEISYSLFQASDEISKTFKVDFLTGEIRLKKQLDFEKFQSY
EVNIEARDAGGFSGKCTVLIQVIDVNDHAPEVTMSAFTSPIPENAPETVVALFSVSDLDSGENGKISCSIQEDL
PFLLKSSVGNFYTLLTETPLDRESRAEYNVTITVTDLGTPRLTTHLNMTVLVSDVNDNAPAFTQTSYTLFVREN
NSPALHIGSVSATDRDSGTNAQVTYSLLPPQDPHLPLASLVSINTDNGHLFALRSLDYEALQAFEFRVGASDRG
SPALSSEALVRVLVLDANDNSPFVLYPLQNGSAPCTELVPRAAEPGYLVTKVVAVDGDSGQNAWLSYQLLKATE
PGLFGVWAHNGEVRTARLLSERDAAKQRLVVLVKDNGEPPCSATATLHLLLVDGFSQPYLPLPEAAPAQGQADS
LTVYL

## Figure 10(i):

**CRCMP #12**

**Peptide Source:** 1D-GE CRC

**SP: P16422 (SEQ ID No: 9)**

MAPPQVLAFGLLLAAATATFAAAQEECVCENYKLAVNCFVNNNRQCQCTSVGAQNTVICSK**LAAKCLVMK**AEMNG
SKLGRRAKPEGALQNNDGLYDPDCDESGLFKAKQCNGTSMCWCVNTAGVRRTDK**DTEITCSER**VRTYWIIIELKH
KAR**EKPYDSK**SLRTALQKEITTRYQLDPKFITSILYENNVITIDLVQNSSQK**TQNDVDIADVAYYFEKDVKGESL
FHSKK**MDLTVNGEQLDLDPGQTLIYYVDEK**APEFSMQGLK**AGVIAVIVVVIAVVAGIVVLVISR**KKRMAKYEKA
EIKEMGEMHR**ELNA

**Mass Match Peptides (bold):**
     APEFSMQGLK [44]
     DTEITCSER [73]
     EKPYDSK [83]
     EMGEMHR [85]
     GESLFHSK [106]
     KKRMAK [133]
     LAAKCLVMK [141]
     TQNDVDIADVAYYFEK [207]
     TQNDVDIADVAYYFEKDVK [208]
     YEKAEIK [233]

**Tandem Peptides (double underline):**
     APEFSMQGLK [44]
     TQNDVDIADVAYYFEK [207]

**Extracellular Domain (underline): (SEQ ID No: 30)**
     QEECVCENYKLAVNCFVNNNRQCQCTSVGAQNTVICSKLAAKCLVMKAEMNGSKLGRRAKPEGALQ
NNDGLYDPDCDESGLFKAKQCNGTSMCWCVNTAGVRRTDKDTEITCSERVRTYWIIIELKHKAREKPYDSKSLR
TALQKEITTRYQLDPKFITSILYENNVITIDLVQNSSQKTQNDVDIADVAYYFEKDVKGESLFHSKKMDLTVNG
EQLDLDPGQTLIYYVDEKAPEFSMQGLK

## Figure 10(j):

**CRCMP #14**

**Peptide Source:** 1D-GE CRC

**ENST00000322765 (SEQ ID No: 10)**

MLCGRWRRCRRPPEEPPVAAQVAAQVAAPVALPSPPTPSDGGTKRPGLRALKKMGLTEDEDVRAMLRGSRLRKIR
SRTWHKERLYR<u>LQEDGLSVWFQR</u>RIPRAPSQHIFFVQHIEAVR**EGHQSEGLR**RFGGAFAPARCLTIAFKGR**RKNL**
**DLAAPTAEEAQR**WVRASYLRAGGSLACCCYFLSTHTWIHSYLHRADSNQDSKMSFKEIKSLLRMVNVDMNDMYAY
LLFKECDHSNNDRLEGAEIEEFLRRLLK**RPELEEIFHQYSGEDR**VLSAPELLEFLEDQGEEGATLARAQQLIQTY
ELNETAKQHELMTLDGFMMYLLSPEGAALDNTHTCVFQDMNQPLAHYFISSSHNTYLTDSQIGGPSSTEAYVRAF
AQGCRCVELDCWEGPGGEPVIYHGHTLTSKILFRDVVQAVRDHAFTLSPYPVILSLENHCGLEQQAAMARHLCTI
LGDMLVTQALDSPNPEELPSPEQLKGRVLVKGKKLPAARSEDGRALSDREEEEEDDEEEEEEVEAAAQRRLLHPA
PNAPQPCQVSSLSERKAKKLIREAGNSFVRHNARQLTRVYPLGLRMNSANYSPQEMWNSGCQLVALNFQTPGYEM
DLNAGRFLVNGQCGYVLKPACLRQPDSTFDPEYPGPPRTTLSIQVLTAQQLPKLNAEKPHSIVDPLVRIEIHGVP
ADCAR**QETDYVLNNGFNPR**WGQTLQFQLRAPELALVRFVVEDYDATSPNDFVGQFTLPLSSLKQGYRHIHLLSKD
GASLSPATLFIQIRIQRS

**Mass Match Peptides (bold):**
        EGHQSEGLR [79]
        QETDYVLNNGFNPR [167]
        RKNLDLAAPTAEEAQR [178]
        RPELEEIFHQYSGEDR [179]
**Tandem Peptides (double underline):**
        LQEDGLSVWFQR [151]
        QETDYVLNNGFNPR [167]

## Figure 10(k):

**CRCMP #17**

**Peptide Source:** 1D-GE CRC

**SP: O00515 (SEQ ID No: 11)**

```
MAVSRKDWSALSSLARQRTLEDEEEQERERRRRHRNLSSTTDDEAPRLSQNGDRQASASERLPSVEEAEVPKPLP
PASKDEDEDIQSILRTRQERRQRRQVVEAAQAPIQERLEAEEGRNSLSPVQATQKPLVSKKELEIPPRRRLSREQ
RGPWPLEEESLVGREPEERKKGVPEKSPVLEKSSMPKKTAPEKSLVSDKTSISEKVLASEKTSLSEKIAVSEKRN
SSEKKSVLEKTSVSEKSLAPGMALGSGRRLVSEKASIFEKALASEKSPTADAKPAPKRATASEQPLAQEPPASGG
SPATTKEQRGRALPGKNLPSLAKQGASDPPTVASRLPPVTLQVKIPSKEEEADMSSPTQRTYSSSLKRSSPRTIS
FRMKPKKENSETTLTRSASMKLPDNTVKLGEKLERYHTAIRRSESVKSRGLPCTELFVAPVGVASKRHLFEKELA
GQSRAEPASSRKENLRLSGVVTSRLNLWISRTQESGDQDPQEAQKASSATERTQWGQKSDSSLDAEV
```

### Mass Match Peptides (bold):

```
        LNLWISR [147]
        QVVEAAQAPIQER [174]
        RSESVKSR [182]
```

### Tandem Peptides (double underline):

```
        QVVEAAQAPIQER [174]
```

**Peptide Source:** 2D-GE

**SP: O00515 (SEQ ID No: 11)**

```
MAVSRKDWSALSSLARQRTLEDEEEQERERRRRHRNLSSTTDDEAPRLSQNGDRQASASERLPSVEEAEVPKPLP
PASKDEDEDIQSILRTRQERRQRRQVVEAAQAPIQERLEAEEGRNSLSPVQATQKPLVSKKELEIPPRRRLSREQ
RGPWPLEEESLVGREPEERKKGVPEKSPVLEKSSMPKKTAPEKSLVSDKTSISEKVLASEKTSLSEKIAVSEKRN
SSEKKSVLEKTSVSEKSLAPGMALGSGRRLVSEKASIFEKALASEKSPTADAKPAPKRATASEQPLAQEPPASGG
SPATTKEQRGRALPGKNLPSLAKQGASDPPTVASRLPPVTLQVKIPSKEEEADMSSPTQRTYSSSLKRSSPRTIS
FRMKPKKENSETTLTRSASMKLPDNTVKLGEKLERYHTAIRRSESVKSRGLPCTELFVAPVGVASKRHLFEKELA
GQSRAEPASSRKENLRLSGVVTSRLNLWISRTQESGDQDPQEAQKASSATERTQWGQKSDSSLDAEV
```

### Mass Match Peptides (bold):

```
        DEDEDIQSILR [63]
        ELEIPPR [84]
        KELEIPPR [129]
        LNLWISR [147]
        LPDNTVK [148]
        LPSVEEAEVPKPLPPASK [150]
        NLSSTTDDEAPR [162]
        QVVEAAQAPIQER [174]
        RATASEQPLAQEPPASGGSPATTK [176]
        SLAPGMALGSGR [192]
        TLEDEEEQER [203]
```

### Tandem Peptides (double underline):

```
        DEDEDIQSILR [63]
        NLSSTTDDEAPR [162]
        QVVEAAQAPIQER [174]
```

## Figure 10(I):

**CRCMP #18**

**Peptide Source:** 1D-GE CRC

**SP: Q96TA1 (SEQ ID No: 12)**

MGWMGEKTGKILTEFLQFYEDQYGVALFNSMRHEIEGTGLPQAQLLWRKVPLDERIVFSGNLFQHQEDSKKWRNR
FSLVPHNYGLVLYENKAAYERQVPPRAVINSAGYKILTSVDQYLELIGNSLPGTTAKSGSAPILKCPTQFPLILW
HPYARHYYFCMMTEAEQDKWQAVLQDCIRHCNNGIPEDSKVEGPAFTDAIRMYRQSKELYGTWEMLCGNEVQILS
NLVMEELGPELKAELGPRLKGKPQERQRQWIQISDAVYHMVYEQAKARFEEVLSKVQQVQPAMQAVIRTDMDQII
TSKEHLASKIRAFILPKAEVCVRNHVQPYIPSILEALMVPTSQGFTEVRDVFFKEVTDMNLNVINEGGIDKLGEY
MEKLSRLAYHPLKMQSCYEKMESLRLDGLQQRFDVSSTSVFKQRAQIHMREQMDNAVYTFETLLHQELGKGPTKE
ELCKSIQRVLERVLKKYDYDSSSVRKRFFREALLQISIPFLLKKLAPTCKSELPRFQELIFEDFARFILVENTYE
EVVLQTVMKDILQAVKEAAVQRKHNLYRDSMVMHNSDPNLHLLAEGAPIDWGEEYSNSGGGGSPSPSTPESATLS
EKRRRAKQVVSVVQDEEVGLPFEASPESPPPASPDGVTEIRGLLAQGLRPESPPPAGPLLNGAPAGESPQPKAAP
EASSPPASPLQHLLPGKAVDLGPPKPSDQETGEQVSSPSSHPALHTTTEDSAGVQTEF

**Mass Match Peptides (bold):**

        AVINSAGYK [53]
        CPTQFPLILWHPYAR [59]
        EELCKSIQR [78]
        FQELIFEDFAR [98]
        HEIEGTGLPQAQLLWR [114]
        HNLYR [116]
        KHNLYR [132]
        KYDYDSSSVR [137]
        KYDYDSSSVRK [138]
        KYDYDSSSVRKR [139]
        MESLRLDGLQQR [156]
        MGWMGEK [157]
        TDMDQIITSK [199]
        VQQVQPAMQAVIR [218]
        YDYDSSSVRK [231]
        YDYDSSSVRKR [232]

**Tandem Peptides (double underline):**

        FEEVLSK [90]
        FQELIFEDFAR [98]
        LGEYMEK [145]
        TDMDQIITSK [199]
        VEGPAFTDAIR [211]
        YDYDSSSVR [230]

Figure 10(I) continued…

**Peptide Source:** 2D-GE

**SP: Q96TA1 (SEQ ID No: 12)**

MGWMGEKTGKILTEFLQFYEDQYGVALFNSMRHEIEGTGLPQAQLLWRKVPLDER**IVFSGNLFQHQEDSK**KWRNR
FSLVPHNYGLVLYENKAAYERQVPPRAVINSAGYKILTSVDQYLELIGNSLPGTTAKSGSAPILKCPTQFPLILW
HPYARHYYFCMMTEAEQDKWQAVLQDCIRHCNNGIPEDSKVEGPAFTDAIRMYRQSKELYGTWEMLCGNEVQILS
NLVMEELGPELKAELGPRLKGKPQERQRQWIQISDAVYHMVYEQAKARFEEVLSK**VQQVQPAMQAVIR**TDMDQII
TSKEHLASKIRAFILPKAEVCVRNHVQPYIPSILEALMVPTSQGFTEVRDVFFK**EVTDMNLNVINEGGIDK**LGEY
MEKLSRLAYHPLKMQSCYEKMESLRLDGLQQRFDVSSTSVFKQR**AQIHMR**EQMDNAVYTFETLLHQELGKGPTKE
ELCKSIQRVLERVLKKYDYDSSSVRKRFFREALLQISIPFLLKKLAPTCKSELPR**FQELIFEDFAR**FILVENTYE
EVVLQTVMKDILQAVKEAAVQRKHNLYRDSMVMHNSDPNLHLLAEGAPIDWGEEYSNSGGGGSPSPSTPESATLS
EKRRRAKQVVSVVQDEEVGLPFEASPESPPPASPDGVTEIRGLLAQGLRPESPPPAGPLLNGAPAGESPQPKAAP
EASSPPASPLQHLLPGKAVDLGPPKPSDQETGEQVSSPSSHPALHTTTEDSAGVQTEF

**Mass Match Peptides (bold):**
      AQIHMR [46]
      EVTDMNLNVINEGGIDK [88]
      FQELIFEDFAR [98]
      IVFSGNLFQHQEDSK [128]
      VQQVQPAMQAVIR [218]

**Tandem Peptides (double underline):**
      FQELIFEDFAR [98]

**Figure 10(m):**

**CRCMP #19**

**Peptide Source:** 1D-GE CRC

**SP: O95994 (SEQ ID No: 13)**

MEKIPVSAFLLLVALSYTLAR*DTTVKPGAKKDTKDSRPK***LPQTLSRGWGDQLIWTQTYEEALYK***SKTSNKPLMII*
*HHLDECPHSQALKKVFAENKEIQKLAEQFVLLNL*VYETTDK<u>**HLSPDGQYVPRIMFVDPSLTVR**</u>ADITGRYSNR<u>**LY**</u>
<u>**AYEPADTALLLDNMK**</u>KALKLLKTEL

**Mass Match Peptides (bold):**
      GWGDQLIWTQTYEEALYK [112]
      HLSPDGQYVPR [115]
      IMFVDPSLTVR [126]
      LPQTLSR [149]
      LYAYEPADTALLLDNMK [153]

**Tandem Peptides (double underline):**
      HLSPDGQYVPR [115]
      IMFVDPSLTVR [126]

**Recombinant Protein (italics): (SEQ ID No: 31)**
      RDTTVKPGAKKDTKDSRPKLPQTLSRGWGDQLIWTQTYEEALYKSKTSNKPLMIIHHLDECPHSQA
LKKVFAENKEIQKLAEQFVLLNLV

**Peptide Source:** 2D-GE

**SP: O95994 (SEQ ID No: 13)**

MEKIPVSAFLLLVALSYTLAR*DTTVKPGAKKDTKDSRPK***LPQTLSR***GWGDQLIWTQTYEEALYKSKTSNKPLMII*
*HHLDECPHSQALK***KVFAENK***EIQKLAEQFVLLNL*VYETTDK<u>**HLSPDGQYVPRIMFVDPSLTVR**</u>ADITGRYSNR<u>**LY**</u>
<u>**AYEPADTALLLDNMK**</u>KALKLLKTEL

**Mass Match Peptides (bold):**
      HLSPDGQYVPR [115]
      IMFVDPSLTVR [126]
      KVFAENK [136]
      LPQTLSR [149]
      LYAYEPADTALLLDNMK [153]

**Tandem Peptides (double underline):**
      HLSPDGQYVPR [115]
      IMFVDPSLTVR [126]
      LYAYEPADTALLLDNMK [153]

**Recombinant Protein (italics): (SEQ ID No: 31)**
      RDTTVKPGAKKDTKDSRPKLPQTLSRGWGDQLIWTQTYEEALYKSKTSNKPLMIIHHLDECPHSQA
LKKVFAENKEIQKLAEQFVLLNLV

**Figure 10(n):**

**CRCMP #20**

**Peptide Source:** 1D-GE CRC

**SP: Q9UHN6 (SEQ ID No: 14)**

MYATDSR**GHSPAFLQPQNGNSR**HPSGYVPGKVVPLRPPPPPKSQASAKFTSIRREDR**ATFAFSPEEQQAQR**ESQK
QKRHKNTFICFAITSFSFFIALAIILGISSKYAPDENCPDQNPRLRNWDPGQDSAKQVVIKEGDMLRLTSDATVH
SIVIQDGGLLVFGDNKDGSRNITLRTHYILIQDGGALHIGAEKCRYKSKATITLYGK**SDEGESMPTFGKK**FIGVE
AGGTLELHGARKASWTLLARTLNSSGLPFGSYTFEKDFSRGLNVRVIDQDTAKILESER**FDTHEYRNESRR**LQEF
LRFQDPGRIVAIAVGDSAAKSLLQGTIQMIQERLGSELIQGLGYRQAWALVGVIDGGSTSCNESVRNYENHSSGG
KALAQREFYTVDGQKFSVTAYSEWIEGVSLSGFRVEVVDGVKLNLLDDVSSWKPGDQIVVASTDYSMYQAEEFTL
LPCSECSHFQVKVKETPQFLHMGEIIDGVDMRAEVGILTRNIVIQGEVEDSCYAENQCQFFDYDTFGGHIMIMKN
FTSVHLSYVELKHMGQQQMGRYPVHFHLCGDVDYKGGYRHATFVDGLSIHHSFSRCITVHGTNGLLIKDTIGFDT
LGHCFFLEDGIEQRNTLFHNLGLLTKPGTLLPTDRNNSMCTTMRDKVFGNYIPVPATDCMAVSTFWIAHPNNNLI
NNAAAGSQDAGIWYLFHKEPTGESSGLQLLAKPELTPLGIFYNNRVHSNFKAGLFIDKGVKTTNSSAADPREYLC
LDNSAR**FRPHQDANPEKPR**VAALIDRLIAFKNNDNGAWVRGGDIIVQNSAFADNGIGLTFASDGSFPSDEGSSQE
VSESLFVGESRNYGFQGGQNKYVGTGGIDQKPRTLPRNRTFPIRGFQIYDGPIHLTRSTFKKYVPTPDRYSSAIG
FLMK**NSWQITPR**NNISLVKFGPHVSLNVFFGKPGPWFEDCEMDGDKNSIFHDIDGSVTGYKDAYVGR**MDNYLIR**H
PSCVNVSKWNAVICSGTYAQVYVQTWSTQNLSMTITRDEYPSNPMVLRGINQKAAFPQYQPVVMLEK**GYTIHWNG**
**PAPR**TTFLYLVNFNKNDWIRVGLCYPSNTSFQVTFGYLQRQNGSLSK**IEEYEPVHSLEELQR**KQSERKFYFDSST
GLLFLYLKAKSHRHGHSYCSSQGCERVKIQAATDSKDISNCMAK**AYPQYYR**KPSVVKR**MPAMLTGLCQGCGTR**QV
VFTSDPHKSYLPVQFQSPDKAETQRGDPSVISVNGTDFTFRSAGVLLLVVDPCSVPFRLTEKTVFPLADVSRIEE
YLKTGIPPRSIVLLSTRGEIKQLNISHLLVPLGLAKPAHLYDKGSTIFLGFSGNFKPSWTKLFTSPAGQGLGVLE
QFIPLQLDEYGCPRATTVRRRDLELLKQASKAH

### Mass Match Peptides (bold):
    ATFAFSPEEQQAQR [51]
    AYPQYYR [55]
    FDTHEYRNESRR [89]
    FRPHQDANPEKPR [99]
    GHSPAFLQPQNGNSR [109]
    GYTIHWNGPAPR [113]
    IEEYEPVHSLEELQR [120]
    MDNYLLR [155]
    MPAMLTGLCQGCGTR [159]
    NSWQLTPR [164]
    SDEGESMPTFGKK [184]

### Tandem Peptides (double underline):
    ATFAFSPEEQQAQR [51]
    GHSPAFLQPQNGNSR [109]

**Figure 10(n) continued...**

**Peptide Source:** 2D-GE

**SP: Q9UHN6 (SEQ ID No: 14)**

```
MYATDSRGHSPAFLQPQNGNSRHPSGYVPGKVVPLRPPPPPKSQASAKFTSIRREDRATFAFSPEEQQAQRESQK
QKRHKNTFICFAITSFSFFIALAIILGISSKYAPDENCPDQNPRLRNWDPGQDSAKQVVIKEGDMLRLTSDATVH
SIVIQDGGLLVFGDNKDGSRNITLRTHYILIQDGGALHIGAEKCRYKSKATITLYGKSDEGESMPTFGKKFIGVE
AGGTLELHGARKASWTLLARTLNSSGLPFGSYTFEKDFSRGLNVRVIDQDTAKILESERFDTHEYRNESRRLQEF
LRFQDPGRIVAIAVGDSAAKSLLQGTIQMIQERLGSELIQGLGYRQAWALVGVIDGGSTSCNESVRNYENHSSGG
KALAQREFYTVDGQKFSVTAYSEWIEGVSLSGFRVEVVDGVKLNLLDDVSSWKPGDQIVVASTDYSMYQAEEFTL
LPCSECSHFQVKVKETPQFLHMGEIIDGVDMRAEVGILTRNIVIQGEVEDSCYAENQCQFFDYDTFGGHIMIMKN
FTSVHLSYVELKHMGQQQMGRYPVHFHLCGDVDYKGGYRHATFVDGLSIHHSFSRCITVHGTNGLLIKDTIGFDT
LGHCFFLEDGIEQRNTLFHNLGLLTKPGTLLPTDRNNSMCTTMRDKVFGNYIPVPATDCMAVSTFWIAHPNNNLI
NNAAAGSQDAGIWYLFHKEPTGESSGLQLLAKPELTPLGIFYNNRVHSNFKAGLFIDKGVKTTNSSAADPREYLC
LDNSARFRPHQDANPEKPRVAALIDRLIAFKNNDNGAWVRGGDIIVQNSAFADNGIGLTFASDGSFPSDEGSSQE
VSESLFVGESRNYGFQGGQNKYVGTGGIDQKPRTLPRNRTFPIRGFQIYDGPIHLTRSTFKKYVPTPDRYSSAIG
FLMKNSWQITPRNNISLVKFGPHVSLNVFFGKPGPWFEDCEMDGDKNSIFHDIDGSVTGYKDAYVGRMDNYLIRH
PSCVNVSKWNAVICSGTYAQVYVQTWSTQNLSMTITRDEYPSNPMVLRGINQKAAFPQYQPVVMLEKGYTIHWNG
PAPRTTFLYLVNFNKNDWIRVGLCYPSNTSFQVTFGYLQRQNGSLSKIEEYEPVHSLEELQRKQSERKFYFDSST
GLLFLYLKAKSHRHGHSYCSSQGCERVKIQAATDSKDISNCMAKAYPQYYRKPSVVKRMPAMLTGLCQGCGTRQV
VFTSDPHKSYLPVQFQSPDKAETQRGDPSVISVNGTDFTFRSAGVLLLVVDPCSVPFRLTEKTVFPLADVSRIEE
YLKTGIPPRSIVLLSTRGEIKQLNISHLLVPLGLAKPAHLYDKGSTIFLGFSGNFKPSWTKLFTSPAGQGLGVLE
QFIPLQLDEYGCPRATTVRRRDLELLKQASKAH
```

**Mass Match Peptides (bold):**

      TLNSSGLPFGSYTFEK [204]

**Tandem Peptides (double underline):**

      FIGVEAGGTLELHGAR [93]

## Figure 10(o):

**CRCMP #22**

**Peptide Source:** 1D-GE CRC

**SP: P01833 (SEQ ID No: 15)**

MLLFVLTCLLAVFPAISTKS*PIFGPEEVNSVEGNSVSITCYYPPTSVNRHTR***KYWCR**QGAR***GGCITLISSEGYVS***
***SK****YAGR***ANLTNFPENGTFVVNIAQLSQDDSGRYKCGLGINSR***GLSFDVSLEVSQGPGLLNDTK**VYTVDLGR**TVTI
NCPFKTENAQKRKSLYKQIGLYPVLVIDSSGYVNPNYTGRIRLDIQGTGQLLFSVVINQLRLSDAGQYLCQAGDD
SNSNKKNADLQVLKPEPELVYEDLR**GSVTFHCALGPEVANVAK**FLCRQ**SSGENCDVVVNTLGKRAPAFEGRILLN**
**PQDKDGSFSVVITGLR**KEDAGRYLCGAHSDGQLQEGSPIQAWQLFVNEESTIPRSPTVVKGVAGGSVAVLCPYNR
KESKSIKYWCLWEGAQNGRCPLLVDSEGWVK**AQYEGR**LSLLEEPGNGTFTVILNQLTSRDAGFYWCLTNGDTLWR
TTVEIK**IIEGEPNLK**VPGNVTAVLGETLK**VPCHFPCKFSSYEK**YWCKWNNTGCQALPSQDEGPSK**AFVNCDENSR**
LVSLTLNLVTRADEGWYWCGVK**QGHFYGETAAVYVAVEERKAAGSRDVSLAKADAAPDEKVLDSGFREIENK**AIQ
DPRLFAEEKAVADTR**DQADGSRASVDSGSSEEQGGSSR**ALVSTLVPLGLVLAVGAVAVGVARARHRKNVDRVSIR
SYR**TDISMSDFENSR**EFGANDNMGASSITQETSLGGK**EEFVATTESTTETK**EPKKAKRSSKEEAEMAYKDFLLQS
STVAAEAQDGPQEA

## Mass Match Peptides (bold):

AAGSRDVSLAK [34]
ADAAPDEK [35]
AFVNCDENSR [40]
ANLTNFPENGTFVVNIAQLSQDDSGR [42]
AQYEGR [47]
ASVDSGSSEEQGGSSR [50]
DGSFSVVITGLR [66]
DQADGSR [71]
DVSLAKADAAPDEK [74]
EEFVATTESTTETK [77]
FSSYEK [100]
GGCITLISSEGYVSSK [108]
GSVTFHCALGPEVANVAK [111]
IIEGEPNLK [123]
ILLNPQDK [124]
KYWCR [140]
QGHFYGETAAVYVAVEER [168]
QGHFYGETAAVYVAVEERK [169]
QSSGENCDVVVNTLGK [172]
QSSGENCDVVVNTLGKR [173]
RAPAFEGR [175]
TDISMSDFENSR [198]
VLDSGFR [214]
VLDSGFREIENK [215]
VPCHFPCK [216]
VYTVDLGR [224]
YKCGLGINSR [236]

## Tandem Peptides (double underline):

ASVDSGSSEEQGGSSR [50]
DGSFSVVITGLR [66]
EEFVATTESTTETK [77]
ILLNPQDK [124]
LSLLEEPGNGTFTVILNQLTSR [152]
RAPAFEGR [175]
TDISMSDFENSR [198]
VYTVDLGR [224]
YLCGAHSDGQLQEGSPIQAWQLFVNEESTIPR [238]

## Figure 10(o) continued…

### Extracellular Domain (underline): (SEQ ID No: 32)

KSPIFGPEEVNSVEGNSVSITCYYPPTSVNRHTRKYWCRQGARGGCITLISSEGYVSSKYAGRANL
TNFPENGTFVVNIAQLSQDDSGRYKCGLGINSRGLSFDVSLEVSQGPGLLNDTKVYTVDLGRTVTINCPFKTEN
AQKRKSLYKQIGLYPVLVIDSSGYVNPNYTGRIRLDIQGTGQLLFSVVINQLRLSDAGQYLCQAGDDSNSNKKN
ADLQVLKPEPELVYEDLRGSVTFHCALGPEVANVAKFLCRQSSGENCDVVVNTLGKRAPAFEGRILLNPQDKDG
SFSVVITGLRKEDAGRYLCGAHSDGQLQEGSPIQAWQLFVNEESTIPRSPTVVKGVAGGSVAVLCPYNRKESKS
IKYWCLWEGAQNGRCPLLVDSEGWVKAQYEGRLSLLEEPGNGTFTVILNQLTSRDAGFYWCLTNGDTLWRTTVE
IKIIEGEPNLKVPGNVTAVLGETLKVPCHFPCKFSSYEKYWCKWNNTGCQALPSQDEGPSKAFVNCDENSRLVS
LTLNLVTRADEGWYWCGVKQGHFYGETAAVYVAVEERKAAGSRDVSLAKADAAPDEKVLDSGFREIENKAIQDP
RLFAEEKAVADTRDQADGSRASVDSGSSEEQGGSSR

### Recombinant Protein (italics): (SEQ ID No: 33)

*PIFGPEEVNSVEGNSVSITCYYPPTSVNRHTRKYWCRQGARGGCITLISSEGYVSSKYAGRANLTN
FPENGTFVVNIAQLSQDDSGRYKCGLGINSRGLSFDVSLEVSQG*

**Peptide Source:** 2D-GE

**SP: P01833 (SEQ ID No: 15)**

MLLFVLTCLLAVFPAISTKS*PIFGPEEVNSVEGNSVSITCYYPPTSVNRHTR***KYWCR**QGAR**GGCITLISSEGYVS
SK**YAGR**ANLTNFPENGTFVVNIAQLSQDDSGR**YK**CGLGINSR**GLSFDVSLEVSQGPGLLNDTK**VYTVDLGRTVTI
NCPFKTENAQKR**KSLYKQIGLYPVLVIDSSGYVNPNYTGRIRLDIQGTGQLLFSVVINQLRLSDAGQYLCQAGDD
SNSNK**KNADLQVLKPEPELVYEDLRGSVTFHCALGPEVANVAK**FLCR**QSSGENCDVVVNTLGKRAPAFEGRILLN
PQDKDGSFSVVITGLR**KEDAGRYLCGAHSDGQLQEGSPIQAWQLFVNEESTIPRSPTVVKGVAGGSVAVLCPYNR
KESKSIK**YWCLWEGAQNGRCPLLVDSEGWVKAQYEGR**LSLLEEPGNGTFTVILNQLTSRDAGFYWCLTNGDTLWR
TTVEIK**IIEGEPNLK**VPGNVTAVLGETLK**VPCHFPCKFSSYEK**YWCKWNNTGCQALPSQDEGPSK**AFVNCDENSR
LVSLTLNLVTR**ADEGWYWCGVKQGHFYGETAAVYVAVEER**KAAGSRDVSLAKADAAPDEK**VLDSGFR**EIENKAIQ
DPR**LFAEEK**AVADTRDQADGSRASVDSGSSEEQGGSSRALVSTLVPLGLVLAVGAVAVGVARARHRKNVDRVSIR
SYRTDISMSDFENSREFGANDNMGASSITQETSLGGKEEFVATTESTTETKEPKKAKRSSKEEAEMAYKDFLLQS
STVAAEAQDGPQEA

### Mass Match Peptides (bold):

ADEGWYWCGVK [36]
AFVNCDENSR [40]
ANLTNFPENGTFVVNIAQLSQDDSGR [42]
APAFEGR [43]
AQYEGR [47]
CGLGINSR [57]
CPLLVDSEGWVK [58]
DGSFSVVITGLR [66]
FSSYEK [100]
GGCITLISSEGYVSSK [108]
GSVTFHCALGPEVANVAK [111]
IIEGEPNLK [123]
ILLNPQDK [124]
KNADLQVLKPEPELVYEDLR [134]
KYWCR [140]
LFAEEK [143]
QGHFYGETAAVYVAVEER [168]
QSSGENCDVVVNTLGK [172]
RAPAFEGR [175]
TENAQKR [201]
TVTINCPFK [210]
VLDSGFR [214]
VPCHFPCK [216]
VYTVDLGR [224]
YWCLWEGAQNGR [244]

**Figure 10(o) continued…**

**Tandem Peptides (double underline):**

```
AFVNCDENSR [40]
DAGFYWCLTNGDTLWR [61]
DGSFSVVITGLR [66]
IIEGEPNLK [123]
ILLNPQDK [124]
LDIQGTGQLLFSVVINQLR [142]
LSLLEEPGNGTFTVILNQLTSR [152]
QSSGENCDVVVNTLGK [172]
VYTVDLGR [224]
YLCGAHSDGQLQEGSPIQAWQLFVNEESTIPR [238]
```

**Extracellular Domain (underline): (SEQ ID No 32)**

```
        KSPIFGPEEVNSVEGNSVSITCYYPPTSVNRHTRKYWCRQGARGGCITLISSEGYVSSKYAGRANLT
NFPENGTFVVNIAQLSQDDSGRYKCGLGINSRGLSFDVSLEVSQGPGLLNDTKVYTVDLGRTVTINCPFKTENAQ
KRKSLYKQIGLYPVLVIDSSGYVNPNYTGRIRLDIQGTGQLLFSVVINQLRLSDAGQYLCQAGDDSNSNKKNADL
QVLKPEPELVYEDLRGSVTFHCALGPEVANVAKFLCRQSSGENCDVVVNTLGKRAPAFEGRILLNPQDKDGSFSV
VITGLRKEDAGRYLCGAHSDGQLQEGSPIQAWQLFVNEESTIPRSPTVVKGVAGGSVAVLCPYNRKESKSIKYWC
LWEGAQNGRCPLLVDSEGWVKAQYEGRLSLLEEPGNGTFTVILNQLTSRDAGFYWCLTNGDTLWRTTVEIKIIEG
EPNLKVPGNVTAVLGETLKVPCHFPCKFSSYEKYWCKWNNTGCQALPSQDEGPSKAFVNCDENSRLVSLTLNLVT
RADEGWYWCGVKQGHFYGETAAVYVAVEERKAAGSRDVSLAKADAAPDEKVLDSGFREIENKAIQDPRLFAEEKA
VADTRDQADGSRASVDSGSSEEQGGSSR
```

**Recombinant Protein (italics): (SEQ ID No 33)**

```
        PIFGPEEVNSVEGNSVSITCYYPPTSVNRHTRKYWCRQGARGGCITLISSEGYVSSKYAGRANLTNF
PENGTFVVNIAQLSQDDSGRYKCGLGINSRGLSFDVSLEVSQG
```

**Figure 10(p):**

**CRCMP #23**

**Peptide Source:** 1D-GE CRC

**SP: Q92820 (SEQ ID No: 16)**

MASPGCLLCVLGLLLCGAASLELSRPHGDTAKKPIIGILMQKCRNKVMKNYGRYYIAASYVK<u>**YLESAGAR**</u>VVPVR
LDLTEK<u>DYEILFK</u>**SINGILFPGGSVDLR**RSDYAKVAKIFYNLSIQSFDDGDYFPVWGTCLGFEELSLLISGECLL
TATDTVDVAMPLNFTGGQLHSRMFQNFPTELLLSLAVEPLTANFHKWSLSVKNFTMNEKLKK<u>FFNVLTTNTDGKI</u>
<u>EFISTMEGYK</u>**YPVYGVQWHPEKAPYEWKNLDGISHAPNAVK**TAFYLAEFFVNEARKNNHHFKSESEEEKALIYQF
SPIYTGNISSFQQCYIFD

**Mass Match Peptides (bold):**
NLDGISHAPNAVK [160]
SINGILFPGGSVDLR [190]
YLESAGAR [239]
YPVYGVQWHPEKAPYEWK [243]

**Tandem Peptides (double underline):**
DYEILFK [75]
FFNVLTTNTDGK [91]
IEFISTMEGYK [121]
SINGILFPGGSVDLR [190]
YLESAGAR [239]

**Peptide Source:** 2D-GE

**SP: Q92820 (SEQ ID No: 16)**

MASPGCLLCVLGLLLCGAASLELSRPHGDTAKKPIIGILMQKCRNKVMKNYGR**YYIAASYVKYLESAGAR**VVPVR
LDLTEK**DYEILFKSINGILFPGGSVDLRRSDYAK**VAKIFYNLSIQSFDDGDYFPVWGTCLGFEELSLLISGECLL
TATDTVDVAMPLNFTGGQLHSRMFQNFPTELLLSLAVEPLTANFHK**WSLSVK**NFTMNEKLK**FFNVLTTNTDGKI**
**EFISTMEGYKYPVYGVQWHPEKAPYEWKNLDGISHAPNAVKTAFYLAEFFVNEARKNNHHFKSESEEEK**ALIYQF
SPIYTGNISSFQQCYIFD

## Figure 10(p) continued…

**Mass Match Peptides (bold):**
```
APYEWK [45]
DYEILFK [75]
FFNVLTTNTDGK [91]
IEFISTMEGYK [121]
KFFNVLTTNTDGK [131]
KNNHHFK [135]
NLDGISHAPNAVK [160]
NNHHFK [163]
RSDYAK [181]
SESEEEK [188]
SINGILFPGGSVDLR [190]
SINGILFPGGSVDLRR [191]
TAFYLAEFFVNEAR [196]
WSLSVK [228]
YLESAGAR [239]
YPVYGVQWHPEK [242]
YYIAASYVK [245]
```

**Tandem Peptides (double underline):**
```
FFNVLTTNTDGK [91]
IEFISTMEGYK [121]
NLDGISHAPNAVK [160]
SINGILFPGGSVDLR [190]
TAFYLAEFFVNEAR [196]
YLESAGAR [239]
YPVYGVQWHPEK [242]
```

**Figure 10(q):**

**CRCMP #25**

**Peptide Source:** 1D-GE CRC

**SP: P27216 (SEQ ID No: 17)**

GNRHAK**ASSPQGFDVDRDAKK**LNKACK**GMGTNEAAIIEILSGR**TSDERQQIKQKYKATYGKELEEVLKSELSGNF
EK**TALALLDRPSEYAAR**QLQKAMKGLGTDESVLIEFLCTRTNKEIIAIKEAYQR**LFDRSLESDVKGDTSGNLKKI
LVSLLQANR**NEGDDVDKDLAGQDAK**DLYDAGEGRWGTDELAFNEVLAKR**SYKQLRATFQAYQILIGKDIEEAIEE
ETSGDLQK**AYLTLVR**CAQDCEDYFAERLYKSMK**GAGTDEETLIR**IVVTR**AEVDLQGIK**AK**FQEKYQKSLSDMVRS
DTSGDFR**KLLVALLH

**Mass Match Peptides (bold):**
    AEVDLQGIK [38]
    ASSPQGFDVDR [48]
    ASSPQGFDVDRDAKK [49]
    AYLTLVR [54]
    DLYDAGEGR [69]
    FQEKYQK [96]
    FQEKYQKSLSDMVR [97]
    GAGTDEETLIR [102]
    GDTSGNLKK [105]
    GMGTNEAAIIEILSGR [110]
    LFDRSLESDVK [144]
    ILVSLLQANR [125]
    SDTSGDFR [186]
    SLESDVK [193]
    SLSDMVR [194]
    TALALLDRPSEYAAR [197]
    WGTDELAFNEVLAK [225]
    WGTDELAFNEVLAKR [226]

**Tandem Peptides (double underline):**
    AEVDLQGIK [38]
    ASSPQGFDVDR [48]
    ATFQAYQILIGK [52]
    CAQDCEDYFAER [56]
    DIEEAIEEETSGDLQK [67]
    DLYDAGEGR [69]
    GAGTDEETLIR [102]
    GMGTNEAAIIEILSGR [110]
    ILVSLLQANR [125]
    SDTSGDFR [186]
    SELSGNFEK [187]
    TALALLDRPSEYAAR [197]
    WGTDELAFNEVLAK [225]

## Figure 10(r):

**CRCMP #26**

**Peptide Source:** 1D-GE CRC

**SP: Q14002 (SEQ ID No: 18)**

```
MGSPSACPYRVCIPWQGLLLTASLLTFWNLPNSAQTNIDVVPFNVAEGKEVLLVVHNESQNLYGYNWYKGERVHA
NYRIIGYVKNISQENAPGPAHNGRETIYPNGTLLIQNVTHNDAGFYTLHVIKENLVNEEVTRQFYVFSEPPKPSI
TSNNFNPVENKDIVVLTCQPETQNTTYLWWVNNQSLLVSPRLLLSTDNRTLVLLSATKNDIGPYECEIQNPVGAS
RSDPVTLNVRYESVQASSPDLSAGTAVSIMIGVLAGMALI
```

**Mass Match Peptides (bold):**
        SDPVTLNVR [185]
**Tandem Peptides (double underline):**
        TLVLLSATK [205]

Europäisches
Patentamt

European
Patent Office

Office européen
des brevets

**EUROPEAN SEARCH REPORT**

Application Number

EP 11 17 2448

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/134794 A1 (MADISON EDWIN L [US] ET AL) 17 July 2003 (2003-07-17) * claims 1-93; sequence 2 * | 1-15 | INV.<br>G01N33/574<br>G01N33/68 |
| X | WO 01/29056 A1 (UNIV ARKANSAS [US]) 26 April 2001 (2001-04-26) * claims 1-52; sequence 2 * * figures 5, 8; examples 5, 7, 11 * * table 1 * | 1-15 | |
| X | WO 03/087831 A2 (OXFORD GLYCOSCIENCES UK LTD [GB]; HUDSON LYNDSEY J [GB]; STAMPS ALASDA) 23 October 2003 (2003-10-23) * page 69; sequence 257 * * claims 1, 8, 9 * | 7-9 | |
| Y | REYMOND M A ET AL: "Expression and functional proteomics studies in colorectal cancer", PATHOLOGY RESEARCH AND PRACTICE, GUSTAV FISCHER, STUTTGART, DE, vol. 200, no. 2, 30 April 2004 (2004-04-30), pages 119-127, XP004959031, ISSN: 0344-0338 * the whole document * | 1-15 | TECHNICAL FIELDS SEARCHED (IPC)<br><br>G01N |
| Y | WO 98/42736 A (PROTEOME SCIENCES PLC [GB]; HOCHSTRASSER DENIS FRANCOIS [CH]; REYMOND) 1 October 1998 (1998-10-01) * the whole document * | 1-15 | |

The present search report has been drawn up for all claims

| Place of search | Date of completion of the search | Examiner |
|---|---|---|
| Munich | 6 September 2011 | Bayer, Martin |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 11 17 2448

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2011

| Patent document cited in search report | | | Publication date | Patent family member(s) | | | Publication date |
|---|---|---|---|---|---|---|---|
| US 2003134794 | A1 | | 17-07-2003 | NONE | | | |
| WO 0129056 | A1 | | 26-04-2001 | AU | 774106 | B2 | 17-06-2004 |
| | | | | AU | 1221501 | A | 30-04-2001 |
| | | | | CA | 2388450 | A1 | 26-04-2001 |
| | | | | EP | 1226150 | A1 | 31-07-2002 |
| | | | | JP | 2003512036 | A | 02-04-2003 |
| | | | | MX | PA02004046 | A | 11-10-2002 |
| WO 03087831 | A2 | | 23-10-2003 | AU | 2003222604 | A1 | 27-10-2003 |
| WO 9842736 | A | | 01-10-1998 | AT | 259504 | T | 15-02-2004 |
| | | | | AU | 740707 | B2 | 15-11-2001 |
| | | | | AU | 6739698 | A | 20-10-1998 |
| | | | | AU | 726738 | B2 | 16-11-2000 |
| | | | | AU | 6739998 | A | 20-10-1998 |
| | | | | CA | 2283640 | A1 | 01-10-1998 |
| | | | | CA | 2284272 | A1 | 01-10-1998 |
| | | | | DE | 69821604 | D1 | 18-03-2004 |
| | | | | DE | 69821604 | T2 | 05-01-2005 |
| | | | | DK | 970377 | T3 | 14-06-2004 |
| | | | | EP | 0971951 | A1 | 19-01-2000 |
| | | | | EP | 0970377 | A1 | 12-01-2000 |
| | | | | ES | 2217541 | T3 | 01-11-2004 |
| | | | | WO | 9843091 | A1 | 01-10-1998 |
| | | | | JP | 2001526644 | A | 18-12-2001 |
| | | | | JP | 2001521631 | A | 06-11-2001 |
| | | | | PT | 970377 | E | 30-06-2004 |
| | | | | US | 2002150569 | A1 | 17-10-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 41216899 A
- EP 320308 A
- US 20050148024 A
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 6316409 B
- US 9002545 W
- US 4816567 A, Cabilly
- US 4816397 A, Boss
- US 5585089 A, Queen
- WO 8702671 A
- EP 184187 A
- EP 171496 A
- EP 173494 A
- WO 8601533 A
- EP 125023 A
- US 5225539 A
- US 5625126 A
- US 5633425 A
- US 5569825 A
- US 5661016 A
- US 5545806 A
- US 5571698 A, Ladner
- US 6057098 A
- GB 9101134 W
- WO 9002809 A
- WO 9110737 A
- WO 9201047 A
- WO 9218619 A
- WO 9311236 A
- WO 9515982 A
- WO 9520401 A
- US 5698426 A
- US 5223409 A
- US 5403484 A
- US 5580717 A
- US 5427908 A
- US 5750753 A
- US 5821047 A
- US 5516637 A
- US 5780225 A
- US 5658727 A
- US 5733743 A
- US 5969108 A
- WO 9222324 A
- US 4946778 A
- US 5258498 A
- WO 9308829 A
- WO 9404690 A
- US 5831012 A
- US 6291158 B
- US 6582915 B
- US 6593081 B
- US 6172197 B
- US 6696245 B
- US 20040110941 A
- EP 1433846 A
- EP 0368684 A
- EP 0616640 A
- WO 05035572 A
- WO 04101790 A
- WO 04081026 A
- WO 04058821 A
- WO 04003019 A
- WO 03002609 A
- WO 04041867 A
- US 6765087 B
- US 6838254 B
- WO 06079372 A
- WO 8605807 A
- WO 8901036 A
- US 5122464 A
- US 4741900 A
- US 4676980 A, Segal
- US 43639202 P
- US 0341426 W
- US 33112702 A
- US 0341453 W
- WO 9636882 A
- US 6064754 A
- WO 9853323 A
- WO 09094996 A
- GB 9901742 W
- US 09412168 B
- US 6064654 A

- WO 9823950 A

**Non-patent literature cited in the description**

- Lectins as Indicators of Disease-Associated Glyco-forms. **SUMAR et al.** Lectins and Glycobiology. 1993, 158-174
- **INNIS et al.** PCR Protocols. Academic Press, Inc, 1990
- **OHARA et al.** *DNA Research,* 1997, vol. 4, 53-59
- Current Protocols in Molecular Biology. 1987
- **DAVIS et al.** *Basic Methods in Molecular Biology,* 1986
- **SAMBROOK et al.** Molecular Cloning: A Laboratory Manual. Cold Spring Harbour laboratory Press, 1989
- *Proc. Natl. Acad. Sci. U.S.A.,* 1968, vol. 60, 160
- *Nucleic Acids Research,* 1981, vol. 9, 309
- *Journal of Molecular Biology,* 1978, vol. 120, 517
- *Journal of Molecular Biology,* 1969, vol. 41, 459
- *Gene,* 1983, vol. 24, 255
- *Journal of Biochemistry,* 1984, vol. 95, 87
- *Proc. Natl. Acad. Sci. U.S.A.,* 1972, vol. 69, 2110
- *Gene,* 1982, vol. 17, 107
- *Molecular & General Genetics,* 1979, vol. 168, 111
- *Methods in Enzymology,* 1991, vol. 194, 182-187
- *Proc. Natl. Acad. Sci. U.S.A.,* 1978, vol. 75, 1929
- New Cell Technology, Experimental Protocol. *Cell Technology,* 1995, vol. 8, 263-267
- *Virology,* 1973, vol. 52, 456
- **NG ; ILAG.** *J. Cell Mol. Med.,* 2002, vol. 6, 329-340
- Near Patient Tests: Triage® Cardiac System. The Immunoassay Handbook. Nature Publishing Group, 2001
- Tietz Textbook of Clinical Chemistry. W.B. Saunders and Company, 496
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **GRISWOLD.** *Comput. Methods Programs Biomed.,* 1988, vol. 27, 65-8
- Antibodies, A Laboratory Manual. Cold Spring Harbor Laboratory, 1988
- Antibody Engineering: A Practical Approach. Oxford University Press, 1995
- *J. Immunol.,* 1992, vol. 149, 3914-3920
- Guide to Proteins Purification. Meth. Enzymol. 1990, vol. 182
- Solid Phase Peptide Synthesis. Meth. Enzymol. 1997, vol. 289
- **KISO et al.** *Chem. Pharm. Bull. (Tokyo,* 1990, vol. 38, 1192-99
- **MOSTAFAVI et al.** *Biomed. Pept. Proteins Nucleic Acids,* 1995, vol. 1, 255-60
- **FUJIWARA et al.** *Chem. Pharm. Bull. (Tokyo,* 1996, vol. 44, 1326-31
- **KOHLER ; MILSTEIN.** *Nature,* 1975, vol. 256, 495-497
- **KOZBOR et al.** *Immunology Today,* 1983, vol. 4, 72
- **COLE et al.** Monoclonal Antibodies and Cancer Therapy. Alan R. Liss, Inc, 1985, 77-96
- **BETTER et al.** *Science,* 1988, vol. 240, 1041-1043
- **LIU et al.** *Proc. Natl. Acad. Sci. USA,* 1987, vol. 84, 3439-3443
- **LIU et al.** *J. Immunol.,* 1987, vol. 139, 3521-3526
- **SUN et al.** *Proc. Natl. Acad. Sci. USA,* 1987, vol. 84, 214-218
- **NISHIMURA et al.** *Canc. Res.,* 1987, vol. 47, 999-1005
- **WOOD et al.** *Nature,* 1985, vol. 314, 446-449
- **SHAW et al.** *J. Natl. Cancer Inst.,* 1988, vol. 80, 1553-1559
- **MORRISON.** *Science,* 1985, vol. 229, 1202-1207
- **OI et al.** *Bio/Techniques,* 1986, vol. 4, 214
- **JONES et al.** *Nature,* 1986, vol. 321, 552-525
- **VERHOEYAN et al.** *Science,* 1988, vol. 239, 1534
- **BEIDLER et al.** *J. Immunol.,* 1988, vol. 141, 4053-4060
- **LONBERG ; HUSZAR.** *Int. Rev. Immunol.,* 1995, vol. 13, 65-93
- **JESPERS et al.** *Bio/technology,* 1994, vol. 12, 899-903
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **BRINKMAN et al.** *J. Immunol. Methods,* 1995, vol. 182, 41-50

- **AMES et al.** *J. Immunol. Methods,* 1995, vol. 184, 177-186
- **KETTLEBOROUGH et al.** *Eur. J. Immunol.,* 1994, vol. 24, 952-958
- **PERSIC et al.** *Gene,* 1997, vol. 187, 9-18
- **BURTON et al.** *Advances in Immunology,* 1994, vol. 57, 191-280
- **MULLINAX et al.** *BioTechniques,* 1992, vol. 12 (6), 864-869
- **SAWAI et al.** *AJRI,* 1995, vol. 34, 26-34
- **HUSTON et al.** *Methods in Enzymology,* 1991, vol. 203, 46-88
- **SHU et al.** *PNAS,* 1993, vol. 90, 7995-7999
- **SKERRA et al.** *Science,* 1988, vol. 240, 1038-1040
- **MILSTEIN et al.** *Nature,* 1983, vol. 305, 537-539
- **TRAUNECKER et al.** *EMBO J.,* 1991, vol. 10, 3655-3659
- **SURESH et al.** *Methods in Enzymology,* 1986, vol. 121, 210
- **BIRD.** *Science,* 1988, vol. 242, 423-42
- **HUSTON et al.** *Proc. Natl. Acad. Sci. USA,* 1988, vol. 85, 5879-5883
- **WARD et al.** *Nature,* 1989, vol. 334, 544-54
- **SKERRA et al.** *Science,* 1988, vol. 242, 1038-1041
- **NORD K ; GUNNERIUSSON E ; RINGDAHL J ; STAHL S ; UHLEN M ; NYGREN PA.** Binding proteins selected from combinatorial libraries of an α-helical bacterial receptor domain. *Nat Biotechnol,* 1997, vol. 15, 772-7
- **RONMARK J ; GRONLUND H ; UHLEN M ; NYGREN PA.** Human immunoglobulin A (IgA)-specific ligands from combinatorial engineering of protein A. *Eur J Biochem,* 2002, vol. 269, 2647-55
- **RONMARK J ; HANSSON M ; NGUYEN T et al.** Construction and characterization of affibody-Fc chimeras produced in Escherichia coli. *J Immunol Methods,* 2002, vol. 261, 199-211
- **SANDSTORM K ; XU Z ; FORSBERG G ; NYGREN PA.** Inhibition of the CD28-CD80 co-stimulation signal by a CD28-binding Affibody ligand developed by combinatorial protein engineering. *Protein Eng,* 2003, vol. 16, 691-7
- **KUTMEIER et al.** *BioTechniques,* 1994, vol. 17, 242
- **HUSE et al.** *Science,* 1989, vol. 246, 1275-1281
- **CLACKSON et al.** *Nature,* 1991, vol. 352, 624
- **HANE et al.** *Proc. Natl. Acad. Sci. USA,* 1997, vol. 94, 4937
- **HUTCHINSON et al.** *J. Biol. Chem.,* 1978, vol. 253, 6551
- **MORRISON et al.** *Proc. Natl. Acad. Sci.,* 1984, vol. 81, 851-855
- **NEUBERGER et al.** *Nature,* 1984, vol. 312, 604-608
- **TAKEDA et al.** *Nature,* 1985, vol. 314, 452-454
- **SAMBROOK et al.** Molecular Cloning, A Laboratory Manual. Cold Spring Harbor Laboratory, 1990
- Current Protocols in Molecular Biology. John Wiley & Sons, 1998
- **FOECKING et al.** *Gene,* 1986, vol. 45, 101
- **COCKETT et al.** *Bio/Technology,* 1990, vol. 8, 2
- **RUTHER et al.** *EMBO J.,* 1983, vol. 2, 1791
- **INOUYE ; INOUYE.** *Nucleic Acids Res.,* 1985, vol. 13, 3101-3109
- **VAN HEEKE ; SCHUSTER.** *J. Biol. Chem.,* 1989, vol. 24, 5503-5509
- **BEBBINGTON ; HENTSCHEL.** The use of vectors based on gene amplification for the expression of cloned genes in mammalian cells. *DNA cloning,* 1987, vol. 3
- **CROUSE et al.** *Mol. Cell. Biol.,* 1983, vol. 3, 257
- **PROUDFOOT.** *Nature,* 1986, vol. 322, 52
- **KOHLER.** *Proc. Natl. Acad. Sci. USA,* 1980, vol. 77, 2197
- **JANKNECHT et al.** *Proc. Natl. Acad. Sci. USA,* 1991, vol. 88, 8972-897
- **RONNMARK J ; GRONLUND H ; UHLE' N, M. ; NYGREN P.A°.** Human immunoglobulin A (IgA)-specific ligands from combinatorial engineering of protein A. *Eur. J. Biochem.,* 2002, vol. 269, 2647-2655
- **NORD, K. ; NILSSON, J. ; NILSSON, B. ; UHLE' N, M. ; NYGREN, P.A°.** A combinatorial library of an a-helical bacterial receptor domain. *Protein Eng.,* 1995, vol. 8, 601-608
- **NORD, K. ; GUNNERIUSSON, E. ; RINGDAHL, J. ; STA°HL, S. ; UHLE' N, M. ; NYGREN, P.A°.** Binding proteins selected from combinatorial libraries of an a-helical bacterial receptor domain. *Nat. Biotechnol.,* 1997, vol. 15, 772-777
- Monoclonal Antibodies For Immunotargeting Of Drugs In Cancer Therapy. **ARNON et al.** Monoclonal Antibodies And Cancer Therapy. Alan R. Liss, Inc, 1985, 243-56
- Antibodies For Drug Delivery. **HELLSTROM et al.** Controlled Drug Delivery. Marcel Dekker, Inc, 1987, 623-53
- Antibody Carriers Of Cytotoxic Agents In Cancer Therapy: A Review. **THORPE et al.** Monoclonal Antibodies '84: Biological And Clinical Applications. 1985, 475-506
- Analysis, Results, And Future Prospective Of The Therapeutic Use Of Radiolabeled Antibody In Cancer Therapy. Monoclonal Antibodies For Cancer Detection And Therapy. Academic Press, 1985, 303-16
- **THORPE et al.** The Preparation And Cytotoxic Properties Of Antibody-Toxin Conjugates. *Immunol. Rev.,* 1982, vol. 62, 119-58

- **HANLEY et al.** *Radiology,* 1982, vol. 143, 29-36
- **FISCHER et al.** *Intensive Care Med.,* 2003, vol. 29, 1043-51
- Merck Manual of Diagnosis and Therapy. Merck Research Laboratories, 1999
- Vaccine design - the subunit and adjuvant approach. Plenum Press, 1995
- *NeuroRx - The Journal of the American Society for Experimental NeuroTherapeutics,* 2005, vol. 2 (2), 348-360
- **ORLOVA A ; MAGNUSSON M ; ERIKSSON TL ; NILSSON M ; LARSSON B ; HOIDEN-GUTHEN-BERG I ; WIDSTROM C ; CARLSSON J ; TOL-MACHEV V ; STAHL S.** Tumor imaging using a picomolar affinity HER2 binding affibody molecule. *Cancer Res.,* 15 April 2006, vol. 66 (8), 4339-48
- **BODEY B.** The significance of immunohistochemistry in the diagnosis and therapy of neoplasms. *Expert Opin Biol Ther.,* April 2002, vol. 2 (4), 371-93
- Current Protocols in Molecular Biology. Green Publishing Associates, Inc., and John Wiley & Sons, Inc, 1989, vol. II
- **DAVID A. BASIJI.** Development of a High-throughput Fluorescence Scanner Employing Internal Reflection Optics and Phase-sensitive Detection (Total Internal Reflection, Electrophoresis. *Dissertation Abstracts International,* 1997, vol. 58/12-B, 6686
- **ENG et al.** *J. Am. Soc. Mass Spectrom.,* 1994, vol. 5, 976-989
- **GASKELL et al.** *Rapid Commun. Mass Spectrom.,* 1992, vol. 6, 658-662
- **VENTER, J.C. et al.** The sequence of the human genome. *Science,* 2001, vol. 16, 1304-51
- Initial sequencing and analysis of the human genome Nature. International Human Genome Sequencing Consortium, 2001, vol. 409, 860-921
- **H PEARSON.** What is a gene?. *Nature,* 2006, vol. 24, 399-401
- **HOCHSTRASSER et al.** *Analytical Biochemistry,* 1988, vol. 173, 412-423
- **AMESS et al.** *Electrophoresis,* 1995, vol. 16, 1255-1267